# EUROPEAN PATENT APPLICATION

(11) **EP 0 885 869 A1**
(43) Date of publication of application: **23.12.1998**
(21) Application number: 97902712.5
(22) Date of filing: 17.02.1997
(51) Int. Cl.: C07C 49/04, C07C 49/29, C07C 49/385, C07C 49/563, C07C 49/76, C07C 59/185, C07C 59/76, C07C 69/716, C07C 69/738, C07C 233/30, C07D 207/267

(54) **THERAPEUTIC AGENT FOR DIABETES**

(30) Priority: 19.02.1996 JP 56883/96
(71) Applicant: Japan Tobacco Inc., Minato-Ku Tokyo 105 (JP)
(72) Inventor: SHINKAI, Hisashi-Cent. Pharm. Res. Inst. of Japan, Osaka 569-11 (JP); OZEKI, Hidekazu-Cent. Pharm. Res. Insti. of Japan, Osaka 569-11 (JP); FURUKAWA, Noboru-Cent. Pharm. Res. Inst. of Japan, Osaka 569-11 (JP)
(74) Representative: von Kreisler, Alek, Dipl.-Chem.
(86) International application number: JP9700422
(87) International publication number: WO9730017

(57) **Abstract**

A therapeutic agent for diabetes, which comprises a compound of the formula [I] 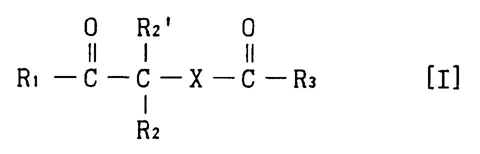 wherein
- X: is a group of the formula 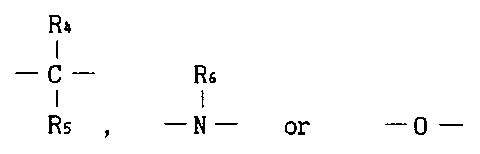
wherein R₄ and R₅ are the same or different and each is a hydrogen atom, an optionally substituted alkyl having 1 to 5 carbon atoms and the like, and R₆ is a hydrogen atom or an amino-protecting group; R₁ is an optionally substituted alkyl having 1 to 5 carbon atoms, an optionally substituted alkenyl having 2 to 6 carbon atoms and the like, R₂ is a hydrogen atom, an optionally substituted alkyl having 1 to 5 carbon atoms and the like, R₂' is a hydrogen atom, and R₃ is an optionally substituted alkyl having 1 to 5 carbon atoms and the like, a prodrug thereof, a pharmaceutically acceptable salt thereof, a hydrate thereof and a solvate thereof.

The compound of the present invention shows superior blood sugar decreasing action on the state of hyperglycemia, but does not affect the blood sugar when it is in the normal range or in the hypoglycemic state, which means that it is free of serious side effects such as hypoglycemia. Therefore, the compound of the present invention is useful as a therapeutic drug for diabetes and also useful as a preventive of the chronic complications of diabetes.

## Description

### Technical Field

The present invention relates to a novel therapeutic drug for diabetes. More particularly, the present invention relates to a novel therapeutic drug for diabetes containing a compound having a dicarbonyl structure, a prodrug compound thereof, a pharmaceutically acceptable salt thereof, a hydrate thereof or a solvate thereof, which drug being free of serious side effects such as hypoglycemia and having superior pharmacological activity to improve only hyperglycemia.

### Background Art

Diadetes is a chronic disease caused by insulin deficiency and characterized by abnormal metabolism of glucose, lipid and amino acid. When untreated, it shows persistent hyperglycemia and incidence of glycosuria. Diabetes is classified into an insulin-dependent type and a non-insulin-dependent type, and about 90% of the diabetic patients belong to the latter.

The insulin-dependent diabetes mellitus tends to develop ketonemia and acidosis due to the disappearance of insulin secretory function, and if left untreated, it ultimately induces diabetic coma. No therapeutic effect can be expected by diet therapy or administration of oral hypoglycemic agent, since this disease is only treatable with insulin.

In the non-insulin-dependent diabetes mellitus, in contrast, although the action of insulin is not sufficient, insulin is not necessarily needed for the treatment, because the tendency toward ketonemia and acidosis is less.

Some of the causes of hyperglycemia found in patients with non-insulin-dependent diabetes mellitus are considered to be abnormal glucose stimulated insulin secretion and increased insulin resistance of target cells. The abnormal secretion of insulin is considered to be due to the abnormal functions of detecting blood glucose concentration in pancreatic β cells and secreting insulin depending on the detected concentration, though the details of the mechanism have not been elucidated yet. The abnormal insulin secretion includes not only deficiency of insulin secretion but also disappearance of secretion in early phase and delayed secretion, as a result of which hyperglycemia is induced. The insulin resistance means a decrease in insulin action or the uptake of glucose into the cells by insulin. In this sense, abnormality of insulin itself, abnormality of insulin receptor in the target cells, and abnormality of signal-transduction system in the cells appear to be the causes. However, what causes the resistance of insulin waits for further elucidation (*Chiryogaku*, 29 (4), pp. 378-381 (1995)).

At present, there exist insulin preparations, sulfonylurea agents, biguanide agents, therapeutic agents for diabetes which improve insulin resistance, α-glucosidase inhibitors and the like for treating hyperglycemia. The insulin preparations are used to treat insulin-dependent diabetes mellitus, and certainly decrease blood glucose levels. Yet, they can only be administered by injection, and are associated with the possibility of inducing hypoglycemia. The sulfonylurea agents stimulate pancreatic β cells and promote endogeneous insulin secretion, wherein the timing and amount of insulin secreted depend on the timing of drug administration and doses, irrespective of blood glucose levels. Thus, hypoglycemia caused by long action of drugs is observed as a side effect. In addition, a digestive symptom such as anorexia appears. The sulfonylurea agents should be avoided for the patients with grave ketosis or liver or renal function disorders. The biguanide drugs are free of pancreatic β cell-stimulating action, and do not cause hypoglycemia by a single administration thereof to both healthy human and diabetic patients. Its action mechanism is considered to be based on an increased use of glucose by anaerobic glycolysis, suppression of glyconeogenesis, suppression of intestinal absorption of glucose and the like. Comparatively severe lactic acidosis tends to occur as a side effect. The pharmaceutical agents to improve insulin resistance include thiazolidine derivatives. The thiazolidine derivatives do not promote insulin secretion, but enhance insulin activity, activate insulin receptor kinase, promote glucose uptake into peripheral tissues, and suppress excess sugar production in liver. They also cause disorders of digestive organs, edema and the like as side effects. In addition, they decrease erythrocyte count, hematocrit value and hemoglobin concentration, and increase LDH (*Atarashii Tonyobyo Chiryogaku*, pp. 90-99 (1994), Pharmaceutical Journal Corp.).

On the other hand, other therapeutic drugs of diabetes exist which inhibit α-glucosidase. The α-glucosidase inhibitors delay digestion and absorption of glucose in the digestive tract and suppress postcibal increase in blood glucose level. At the same time, however, they cause side effects such as sense of fullness, rumbling sound, diarrhea and the like (JOSLIN'S DIABETES MELLITUS 13Th Edition 521-522). Also, Japanese Patent Unexamined Publication No. 54321/1988 discloses an oral hypoglycemic agent which shows acute and transient insulin secretion and which is of fast and short acting type. Inasmuch as it does not secrete insulin according to the blood glucose level and its effect depends on doses and timing of the drug administration, the blood glucose cannot be easily controlled by this drug and misadministration of the drug may result in hypoglycemia. In addition, Japanese Patent Unexamined Publication No. 128266/1992 teaches that an aldose reductase inhibitor also has a glucose concentration-dependent insulin secretion-stimulating action. This inhibitor needs to be administered in greater amounts, and blood glucose cannot be controlled satisfactorily. Therefore, there has been a demand for a pharmaceutical agent which does not merely lower the blood glucose level but is capable of controlling the blood glucose level to be in the normal range.

Certain compounds used as the active ingredients in the inventive pharmaceutical agent of diabetes are known. However, no known publications have disclosed heretofore that such compounds are effective as superior therapeutic drugs of diabetes, let alone the data suggestive of such effects. Hereunder follow structural formulas of such known compounds, the names of the publications [Registry Number (R.N.), patent publication Nos. or name of the publications] and the main use disclosed therein which are summarized in Tables 1 to 8.

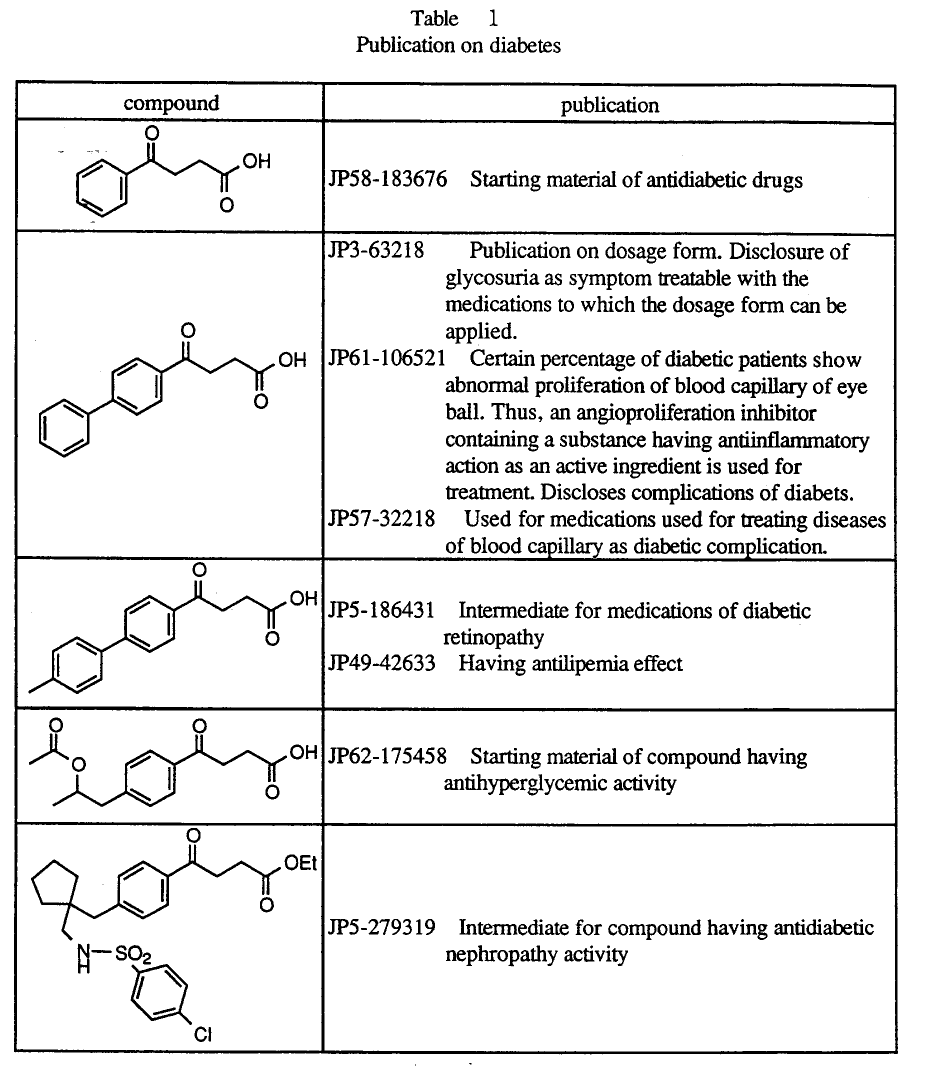

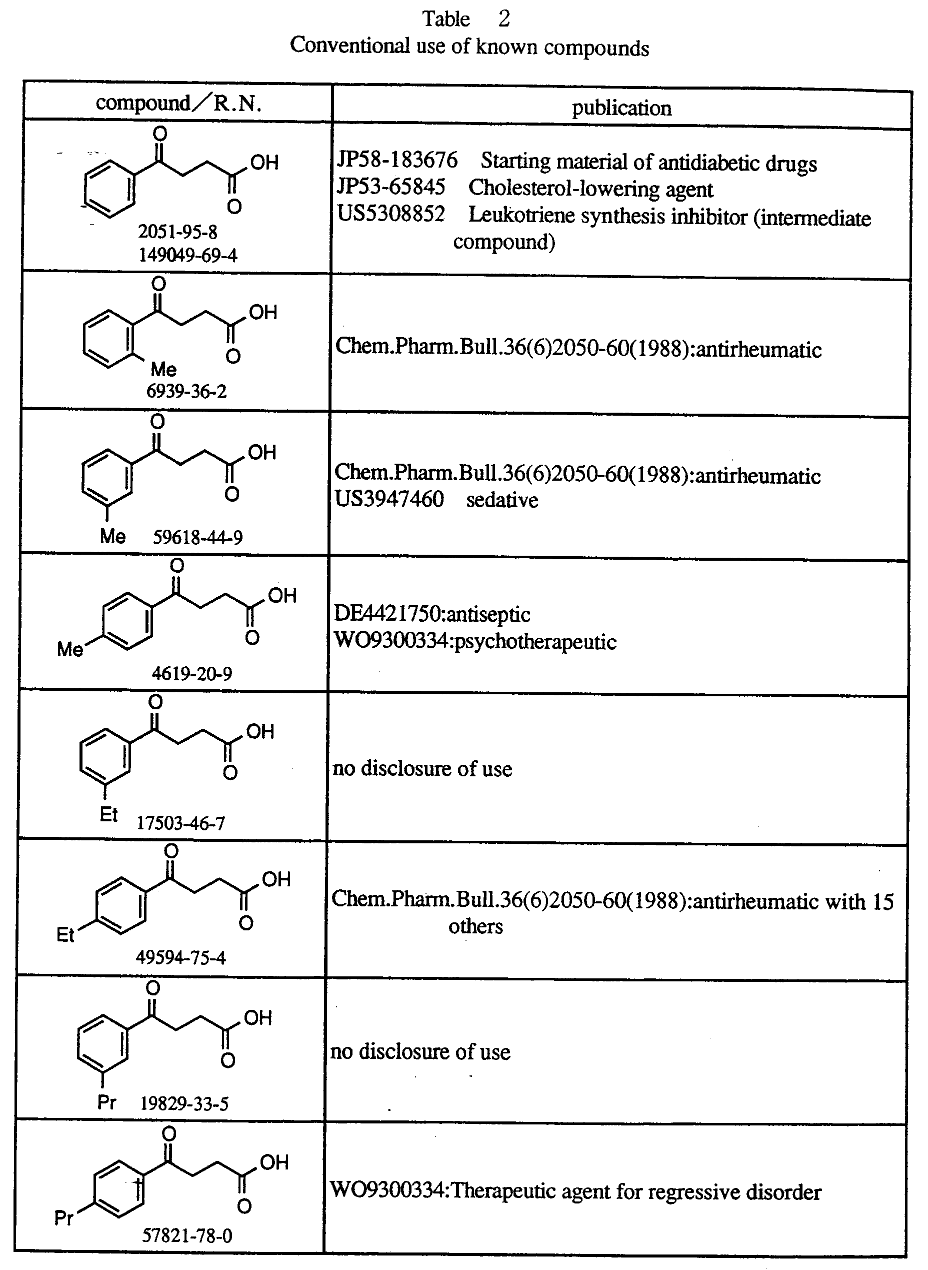

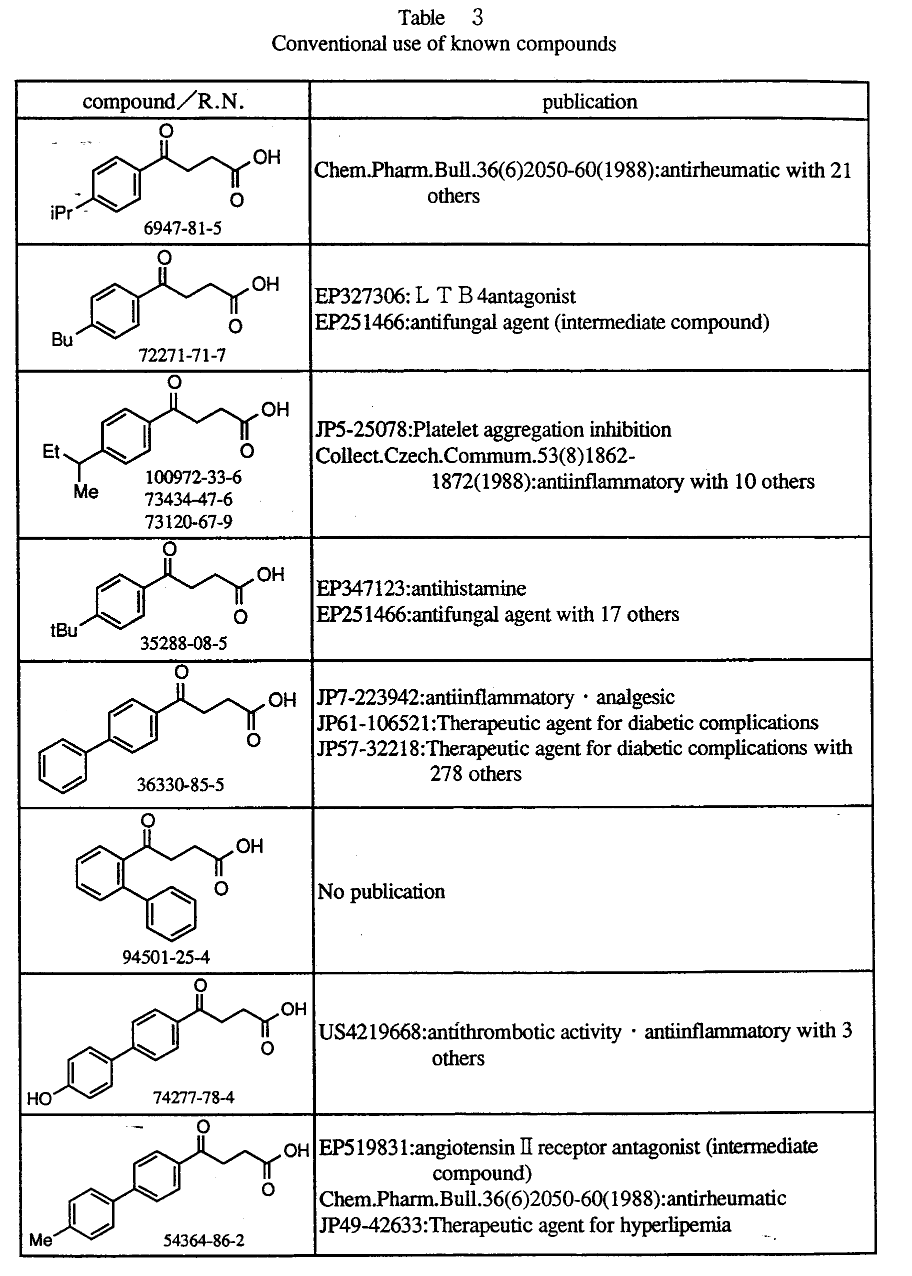

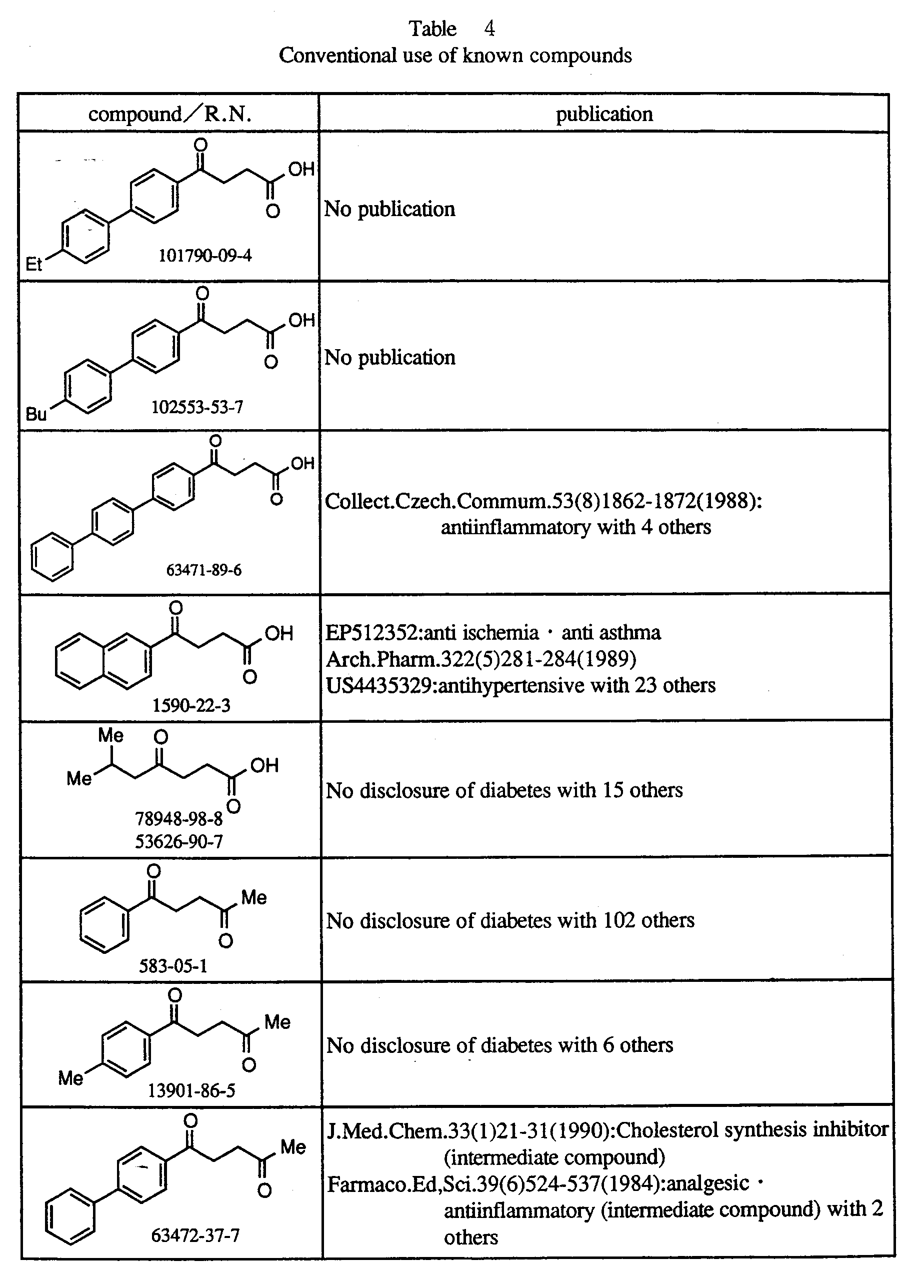

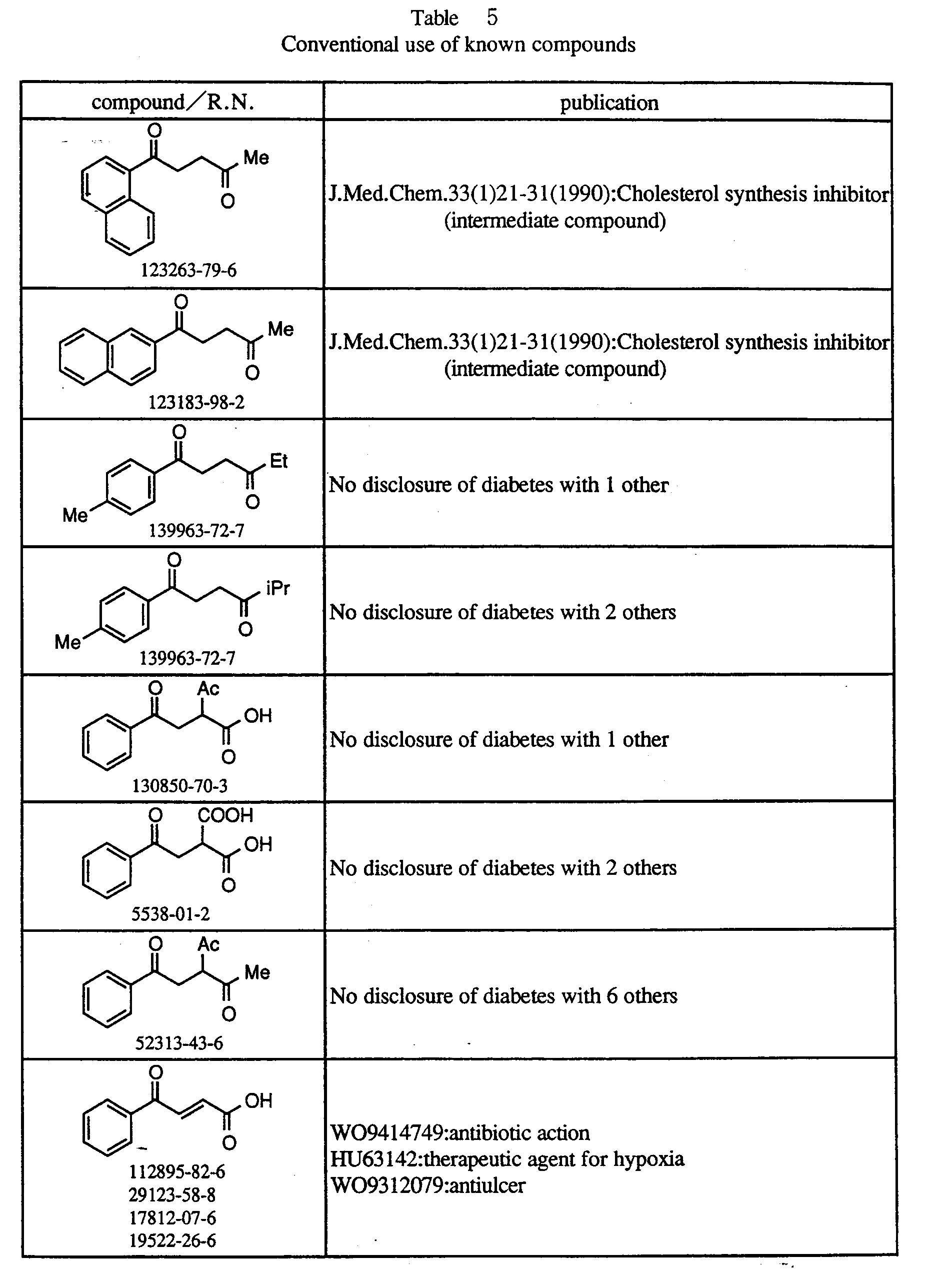

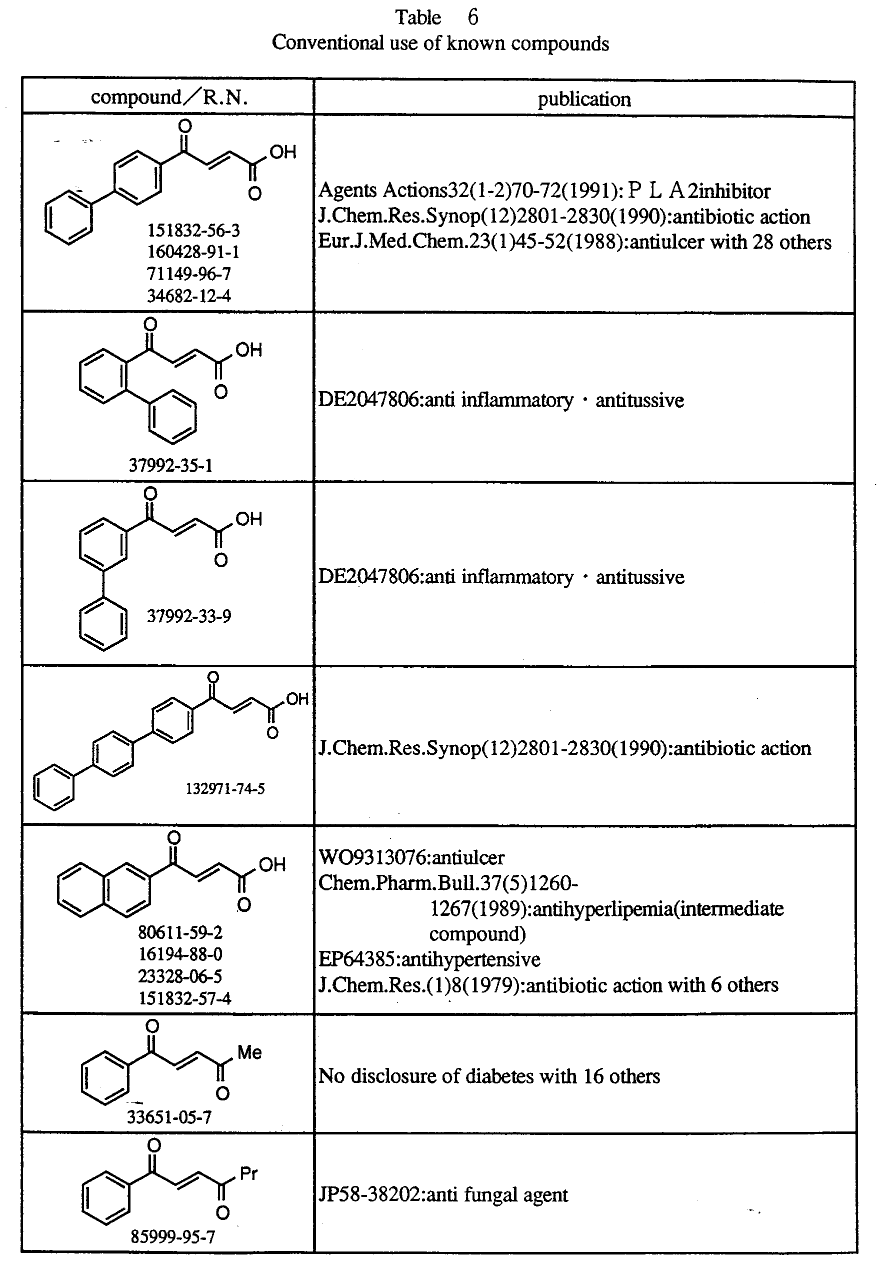

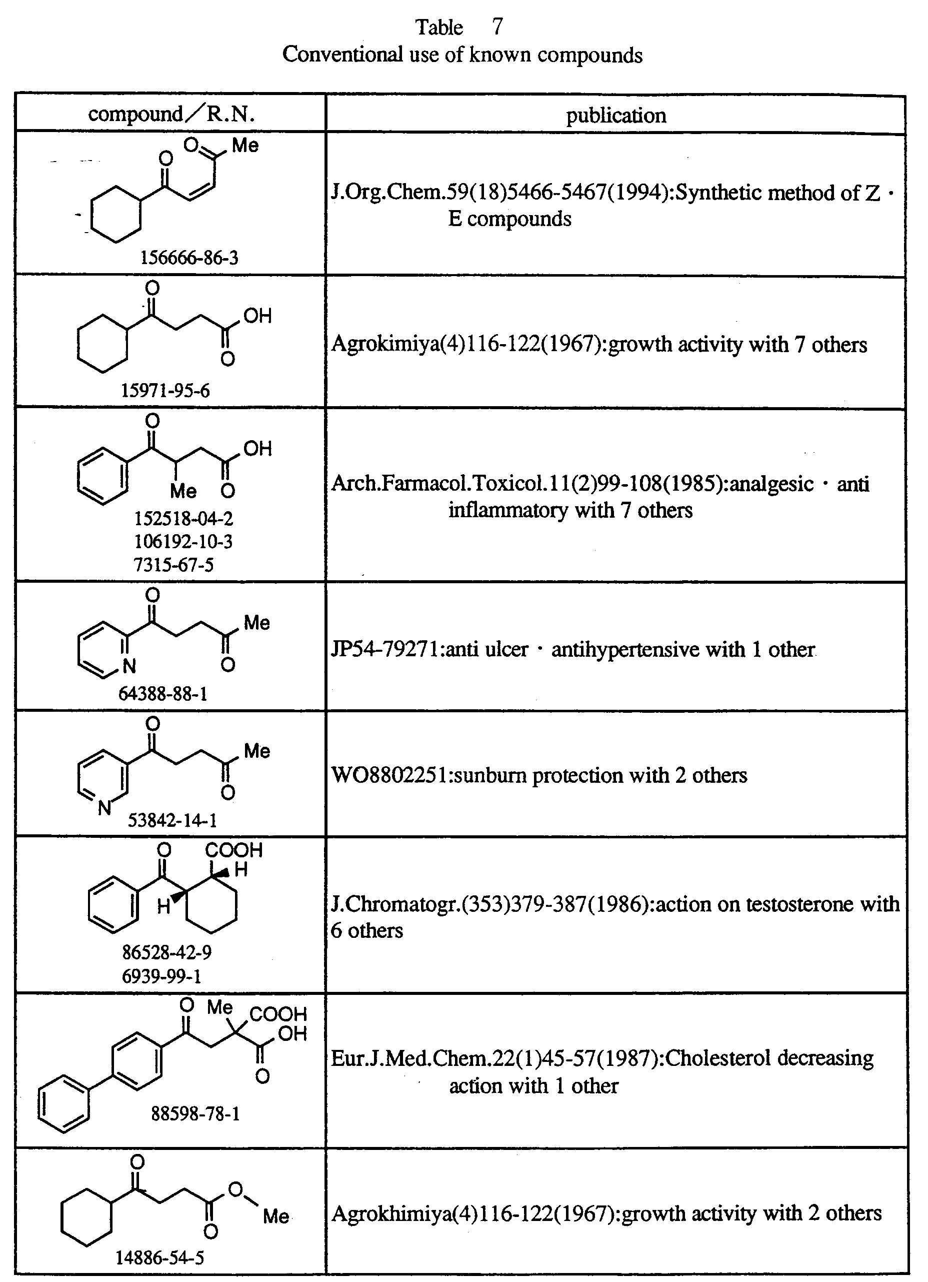

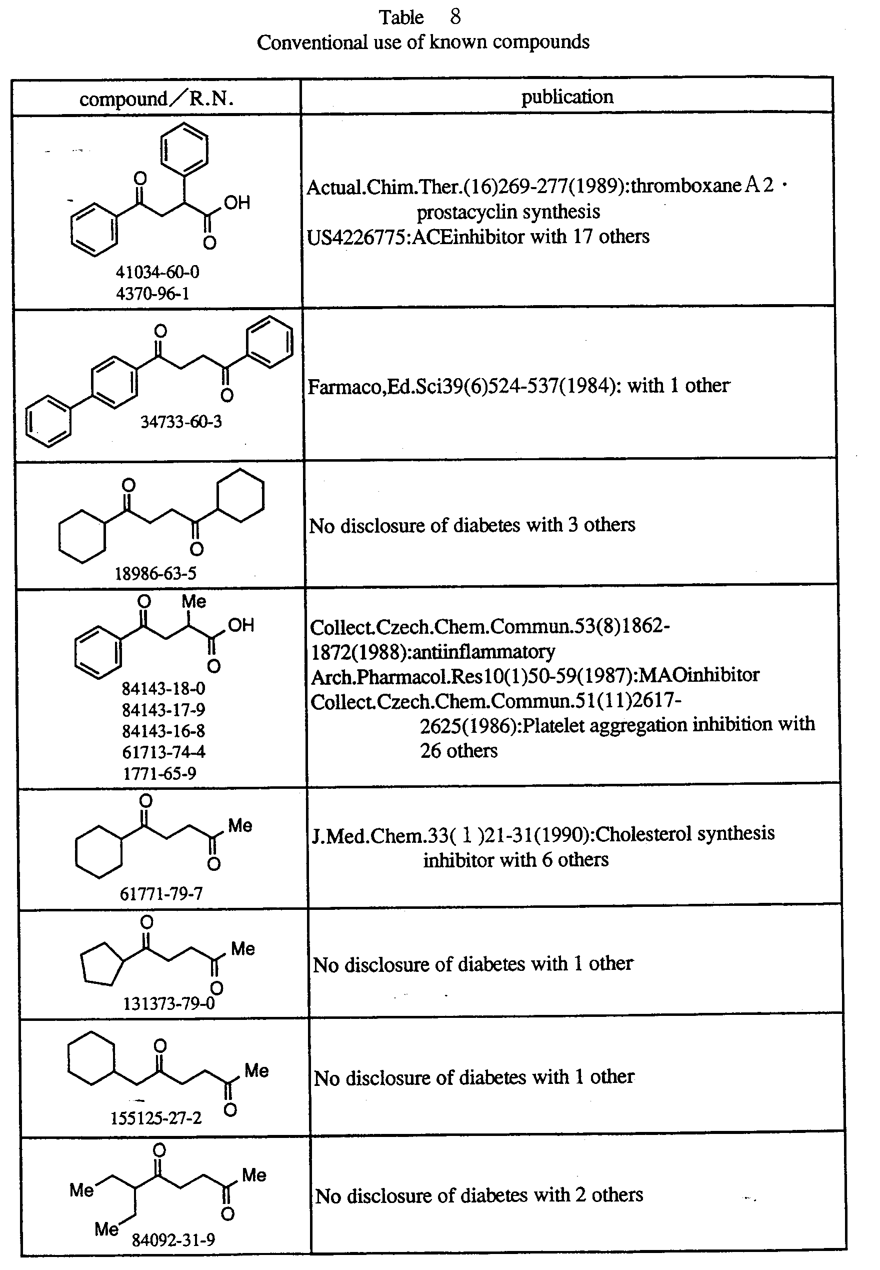

### Disclosure of the Invention

The present invention aims at providing a pharmaceutical agent which has a blood glucose lowering action on the condition of hyperglycemia and which does not cause side effects such as hypoglycemia and the like. The present inventors have conducted intensive studies and found a pharmaceutical agent which does not cause severe side effects after administration such as hypoglycemia, exerts effects only on hyperglycemia, and is useful as a treatment drug of diabetes and a preventive drug of chronic complications of diabetes, which resulted in the completion of the invention.

That is, the present invention relates to therapeutic agents for diabetes containing the compounds shown in the following (1) to (11), pharmaceutically acceptable salts thereof, hydrates thereof or solvates thereof, the novel compounds shown in the following (12) to (28), prodrugs thereof, pharmaceutically acceptable salts thereof, hydrates thereof and solvates thereof, encompassing their active metabolites. It also relates to the pharmaceutical agents and compositions of (29) to (32), and therapeutic agents for diabetes of (33).
(1) A therapeutic agent for diabetes, which comprises a compound of the formula [I] 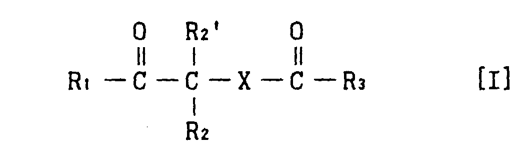 wherein
   X is a group of the formula 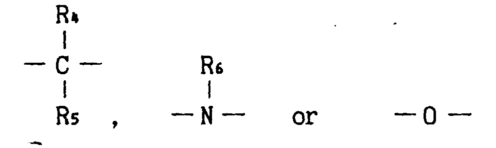 wherein
   R₄ and R₅ are the same or different and each is a hydrogen atom, an optionally substituted alkyl having 1 to 5 carbon atoms, an optionally substituted alkenyl having 2 to 6 carbon atoms, an optionally substituted aryl, an optionally substituted acyl having 2 to 5 carbon atoms, a carboxy or an optionally substituted alkoxycarbonyl having 2 to 5 carbon atoms, and R₆ is a hydrogen atom or an amino-protecting group;
   R₁ is an optionally substituted alkyl having 1 to 5 carbon atoms, an optionally substituted alkenyl having 2 to 6 carbon atoms, an optionally substituted aryl, an optionally substituted heterocyclic group having at least one nitrogen atom, oxygen atom or sulfur atom, an optionally substituted cycloalkyl having 3 to 7 carbon atoms, a cycloalkenyl having 5 to 7 carbon atoms having at least one double bond in the ring, an optionally substituted adamantyl, an optionally substituted indanyl, an optionally substituted fluorenyl, or a group of the formula 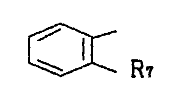
   R₂ is a hydrogen atom, an optionally substituted alkyl having 1 to 5 carbon atoms, an optionally substituted alkenyl having 2 to 6 carbon atoms, an optionally substituted aryl, an optionally substituted acyl having 2 to 5 carbon atoms, a carboxy or an optionally substituted alkoxycarbonyl having 2 to 5 carbon atoms;
   R₂ 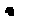 is a hydrogen atom;
   R₃ is an optionally substituted alkyl having 1 to 5 carbon atoms, an optionally substituted alkoxy having 1 to 4 carbon atoms, a hydroxy, an optionally substituted aryl, an optionally substituted cycloalkyl having 3 to 7 carbon atoms or an amino;
   R₂ and R₇ combinedly form a group of the formula

   ―CH₂― or ―(CH₂)₂― ;

   R₂ and R₅ optionally combinedly form a bond, or a group of the formula

   ―CH₂― , ―(CH₂)₂―, ―(CH₂)₃― or ―(CH₂)₄― ;

   R₂, R₂', R₄ and R₅ optionally combinedly form a group of the formula

   =CH―CH=CH―CH=;

   R₃ and R₅ optionally combinedly form a group of the formula 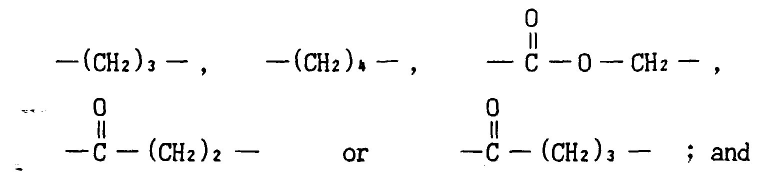 R₂' and R₃ optionally combinedly form a group of the formula 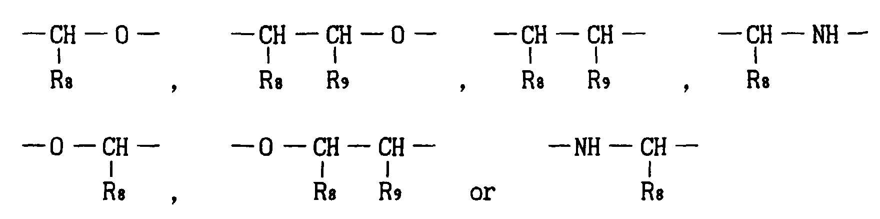 wherein
   R₈ and R₉ are the same or different and each is a hydrogen atom, an optionally substituted alkyl having 1 to 5 carbon atoms, an optionally substituted alkoxy having 1 to 4 carbon atoms, an optionally substituted alkoxycarbonyl having 2 to 5 carbon atoms or an optionally substituted acyloxy having 2 to 5 carbon atoms; a prodrug thereof, a pharmaceutically acceptable salt thereof, a hydrate thereof or a solvate thereof.
(2) The therapeutic agent for diabetes of the above (1), wherein, in the formula [I],
   - X: is a group of the formula 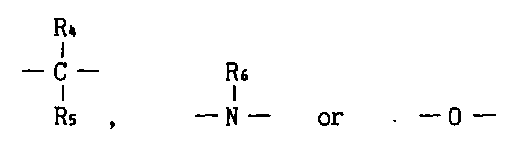 wherein
   R₄ is a hydrogen atom, an alkyl having 1 to 5 carbon atoms, an aryl, an acyl having 2 to 5 carbon atoms which is optionally substituted by alkoxy having 1 to 4 carbon atoms, a carboxy or an alkoxycarbonyl having 2 to 5 carbon atoms, R₅ is a hydrogen atom or an alkyl having 1 to 5 carbon atoms, and R₆ is a hydrogen atom or an acyl having 2 to 5 carbon atoms;
   - R₁: is an alkyl having 1 to 5 carbon atoms which is optionally substituted by cycloalkyl having 3 to 7 carbon atoms or aryl, an alkenyl having 2 to 6 carbon atoms which is optionally substituted by cycloalkyl having 3 to 7 carbon atoms or aryl, an aryl selected from the group consisting of phenyl, biphenyl, naphthyl and terphenyl (these aryl groups being optionally substituted by one or more substituents selected from the group consisting of alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, alkoxycarbonyl having 2 to 5 carbon atoms, hydroxy, cycloalkyl having 3 to 7 carbon atoms, alkoxy having 1 to 4 carbon atoms, amino, alkyl-substituted amino having 1 to 5 carbon atoms, C₁-C₅ alkyl-disubstituted amino, acyl-substituted amino having 2 to 5 carbon atoms, C₂-C₅ acyl-disubstituted amino, heterocyclic group and halogen atom), a heterocyclic group selected from the group consisting of furyl, thienyl, pyridyl, benzothienyl and benzofuryl (these heterocyclic groups being optionally substituted by alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, alkoxycarbonyl having 2 to 5 carbon atoms, hydroxy, cycloalkyl having 3 to 7 carbon atoms, alkoxy having 1 to 4 carbon atoms, amino, alkyl-substituted amino having 1 to 5 carbon atoms, C₁-C₅ alkyl-disubstituted amino, acyl-substituted amino having 2 to 5 carbon atoms, C₂-C₅ acyl-disubstituted amino and halogen atom), a cycloalkyl having 3 to 7 carbon atoms which is optionally substituted by alkyl having 1 to 5 carbon atoms or aryl, a cycloalkenyl having 5 to 7 carbon atoms having at least one double bond in the ring, an adamantyl, an indanyl optionally substituted by alkyl having 1 to 5 carbon atoms or aryl, a fluorenyl, or a group of the formula 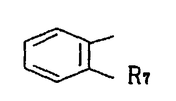
   - R₂: is a hydrogen atom or an alkyl having 1 to 5 carbon atoms which is optionally substituted by aryl or carboxy;
   - R₂': is a hydrogen atom;
   - R₃: is an alkyl having 1 to 5 carbon atoms which is optionally substituted by alkoxy having 1 to 4 carbon atoms or aryl, an alkoxy having 1 to 4 carbon atoms, a hydroxy, an aryl selected from the group consisting of phenyl, biphenyl, naphthyl and terphenyl, a cycloalkyl having 3 to 7 carbon atoms which is optionally substituted by alkyl having 1 to 5 carbon atoms or an amino;
   - R₂ and R₇: combinedly form a group of the formula

   ―CH₂― or ―(CH₂)₂― ;
   - R₂ and R₅: combinedly form a bond or a group of the formula

   ―CH₂― , ―(CH₂)₂― , ―(CH₂)₃― or ―(CH₂)₄― ;
   - R₂, R₂', R₄ and R₅: combinedly form a group of the formula

   =CH―CH=CH―CH= ;
   - R₃ and R₅: combinedly form a group of the formula 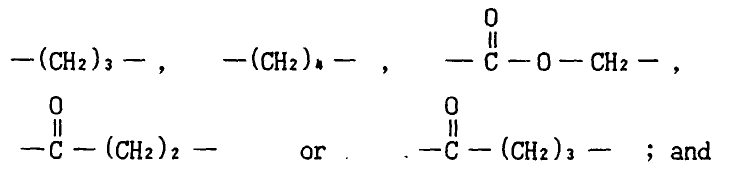
   - R₂' and R₃: combinedly form a group of the formula 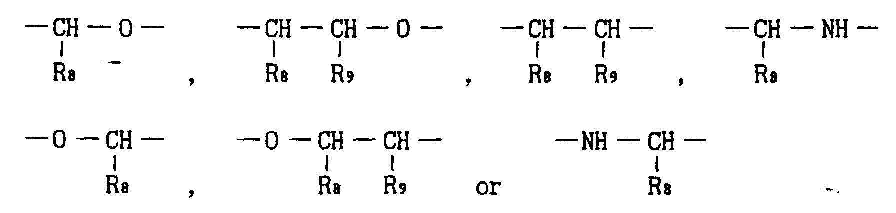 wherein
   R₈ and R₉ are the same or different and each is a hydrogen atom, an alkyl having 1 to 5 carbon atoms, an alkoxy having 1 to 4 carbon atoms, an alkoxycarbonyl having 2 to 5 carbon atoms or an acyloxy having 2 to 5 carbon atoms.
(3) The therapeutic agent for diabetes of the above (1), wherein, in the formula [I],
   - X: is a group of the formula 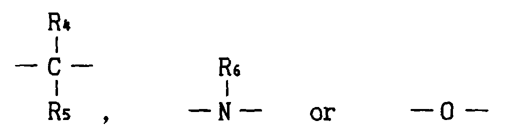 wherein R₄ is a hydrogen atom, an alkyl having 1 to 5 carbon atoms, a phenyl, an acyl having 2 to 5 carbon atoms, a carboxy or an alkoxycarbonyl having 2 to 5 carbon atoms, R₅ is a hydrogen atom or an alkyl having 1 to 5 carbon atoms, and R₆ is a hydrogen atom or an acyl having 2 to 5 carbon atoms;
   - R₁: is an alkyl having 1 to 5 carbon atoms which is optionally substituted by cycloalkyl having 3 to 7 carbon atoms or aryl, an aryl selected from the group consisting of phenyl, biphenyl, naphthyl and terphenyl (these aryl groups being optionally substituted by one or more substituents selected from the group consisting of alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group and hydroxy), a heterocyclic group selected from the group consisting of furyl, thienyl, benzothienyl and pyridyl (these heterocyclic groups being optionally substituted by alkyl having 1 to 5 carbon atoms, alkyl having 1 to 5 carbon atoms which is substituted by hydroxy, alkenyl having 2 to 6 carbon atoms, acyl having 1 to 5 carbon atoms, carboxy, cycloalkyl having 3 to 7 carbon atoms or hydroxy), a cycloalkyl having 3 to 7 carbon atoms which is optionally substituted by alkyl having 1 to 5 carbon atoms or aryl, an adamantyl, an indanyl, a fluorenyl or a group of the formula 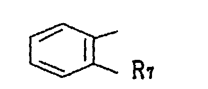
   - R₂: is a hydrogen atom or an alkyl having 1 to 5 carbon atoms;
   - R₂': is a hydrogen atom;
   - R₃: is an alkyl having 1 to 5 carbon atoms which is optionally substituted by alkoxy having 1 to 4 carbon atoms or aryl, alkoxy having 1 to 4 carbon atoms, a hydroxy, an aryl selected from the group consisting of phenyl, biphenyl, naphthyl and terphenyl or a cycloalkyl having 3 to 7 carbon atoms which is optionally substituted by alkyl having 1 to 5 carbon atoms;
   - R₂ and R₇: combinedly form a group of the formula

   ―CH₂― or ―(CH₂)₂― ;
   - R₂ and R₅: optionally combinedly form a bond or a group of the formula

   ―CH₂― , ―(CH₂)₂― , ―(CH₂)₃― or ―(CH₂)₄― ;
   - R₂, R₂', R₄ and R₅: optionally combinedly form a group of the formula

   =CH―CH=CH―CH= ;
   - R₃ and R₅: optionally combinedly form a group of the formula 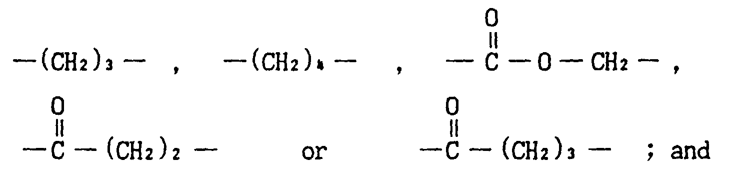
   - R₂' and R₃: optionally combinedly form a group of the formula 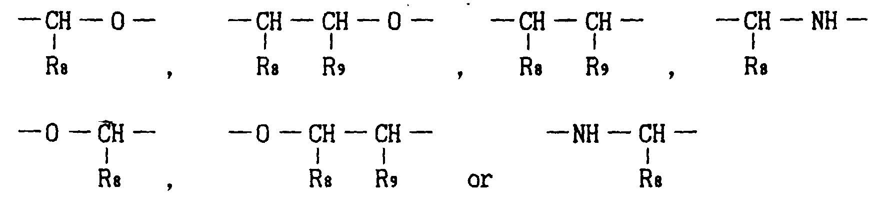 wherein
   R₈ and R₉ are the same or different and each is a hydrogen atom, an alkyl having 1 to 5 carbon atoms, an alkoxy having 1 to 4 carbon atoms, an alkoxycarbonyl having 2 to 5 carbon atoms or an acyloxy having 2 to 5 carbon atoms.
(4) The therapeutic agent for diabetes of the above (1), wherein, in the formula [I],
   - X: is a group of the formula 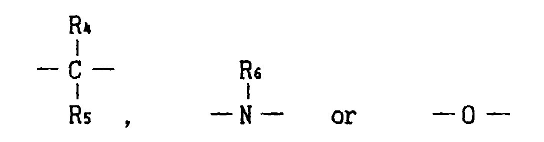 wherein
   R₄ is a hydrogen atom, an alkyl having 1 to 5 carbon atoms, an acyl having 2 to 5 carbon atoms, a carboxy or an alkoxycarbonyl having 2 to 5 carbon atoms, R₅ is a hydrogen atom or an alkyl having 1 to 5 carbon atoms, and R₆ is a hydrogen atom;
   - R₁: is an alkyl having 1 to 5 carbon atoms which is optionally substituted by cycloalkyl having 3 to 7 carbon atoms or aryl, an aryl selected from the group consisting of phenyl, biphenyl, naphthyl and terphenyl (these aryl groups being optionally substituted by one or more substituents selected from the group consisting of alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group and hydroxy), a heterocyclic group selected from furyl, thienyl, benzothienyl and pyridyl, a cycloalkyl having 3 to 7 carbon atoms which is optionally substituted by alkyl having 1 to 5 carbon atoms or aryl, an adamantyl, an indanyl, a fluorenyl, or a group of the formula 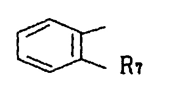
   - R₂: is a hydrogen atom or an alkyl having 1 to 5 carbon atoms;
   - R₂': is a hydrogen atom;
   - R₃: is an alkyl having 1 to 5 carbon atoms which is optionally substituted by alkoxy having 1 to 4 carbon atoms or aryl, an alkoxy having 1 to 4 carbon atoms, a hydroxy or an aryl selected from the group consisting of phenyl, biphenyl, naphthyl and terphenyl;
   - R₂ and R₇: combinedly form a group of the formula

   ―(CH₂)₂― ;
   - R₂ and R₅: optionally combinedly form a bond or a group of the formula

   ―CH₂― , ―(CH₂)₂― , ―(CH₂)₃― or ―(CH₂)₄― ;
   - R₂, R₂', R₄ and R₅: optionally combinedly form a group of the formula

   =CH―CH=CH―CH= ; and
   - R₂' and R₃: optionally combinedly form a group of the formula 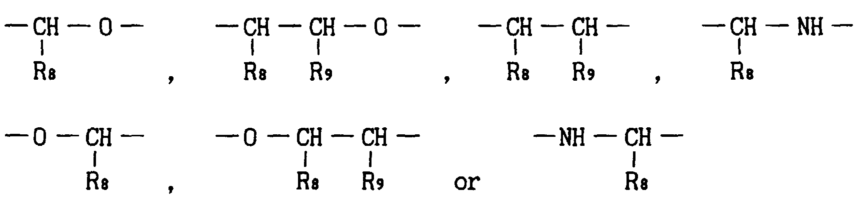
   wherein R₈ and R₉ are each a hydrogen atom.
(5) The therapeutic agent for diabetes of the above (1), wherein, in the formula [I],
   - X: is a group of the formula 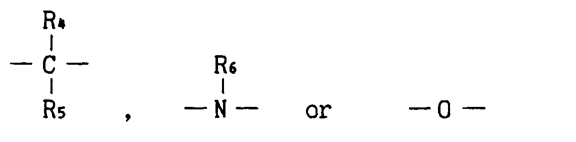 wherein
   R₄ is a hydrogen atom, an alkyl having 1 to 5 carbon atoms, an acyl having 2 to 5 carbon atoms, carboxy or an alkoxycarbonyl having 2 to 5 carbon atoms, R₅ is a hydrogen atom or an alkyl having 1 to 5 carbon atoms, and R₆ is a hydrogen atom;
   - R₁: is an alkyl having 1 to 5 carbon atoms optionally substituted by cycloalkyl having 3 to 7 carbon atoms or aryl, an aryl selected from the group consisting of phenyl, biphenyl, naphthyl and terphenyl (these aryl groups being optionally substituted by alkyl having 1 to 5 carbon atoms, alkyl having 1 to 5 carbon atoms which is substituted by hydroxy, alkyl having 1 to 5 carbon atoms substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group or hydroxy), a heterocyclic group selected from the group consisting of furyl, thienyl, benzothienyl and pyridyl, a cycloalkyl having 3 to 7 carbon atoms which is optionally substituted by alkyl having 1 to 5 carbon atoms or aryl, an adamantyl, an indanyl or a fluorenyl;
   - R₂: is a hydrogen atom, or an alkyl having 1 to 5 carbon atoms;
   - R₂': is a hydrogen atom;
   - R₃: is an alkyl having 1 to 5 carbon atoms, an alkoxy having 1 to 4 carbon atoms, a hydroxy or an aryl selected from the group consisting of phenyl, biphenyl, naphthyl and terphenyl;
   - R₂ and R₅: optionally together form a bond or a group of the formula

   ―(CH₂)₃― or ―(CH₂)₄― ; and
   - R₂' and R₃: optionally combinedly form a group of the formula 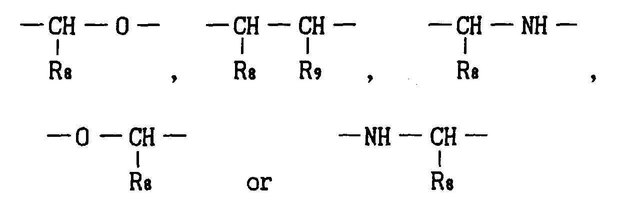 wherein R₈ and R₉ are each hydrogen atom.
(6) The therapeutic agent for diabetes of the above (1), wherein, in the formula [I],
   X is a group of the formula 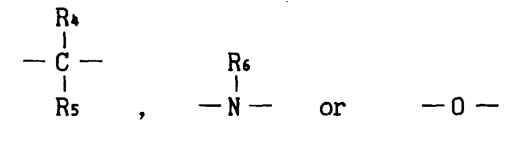 wherein
   R₄ is a hydrogen atom, an alkyl having 1 to 5 carbon atoms, an acyl having 2 to 5 carbon atoms, a carboxy or an alkoxycarbonyl having 2 to 5 carbon atoms, R₅ is a hydrogen atom or an alkyl having 1 to 5 carbon atoms and R₆ is a hydrogen atom;
   R₁ is an alkyl having 1 to 5 carbon atoms optionally substituted by cycloalkyl having 3 to 7 carbon atoms or aryl, an aryl selected from phenyl, biphenyl, naphthyl and terphenyl (these aryl groups being optionally substituted by alkyl having 1 to 5 carbon atoms, alkyl having 1 to 5 carbon atoms substituted by hydroxy, alkyl having 1 to 5 carbon atoms substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group or hydroxy), a cycloalkyl having 3 to 7 carbon atoms which is optionally substituted by alkyl having 1 to 5 carbon atoms or aryl, an adamantyl, an indanyl or a fluorenyl;
   R₂ is a hydrogen atom;
   R₂ 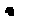 is a hydrogen atom;
   R₃ is an alkyl having 1 to 5 carbon atoms, alkoxy having 1 to 4 carbon atoms or hydroxy;
   R₂ and R₅ optionally combinedly show a bond; and
   R₂ 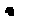 and R₃ optionally combinedly form a group of the formula 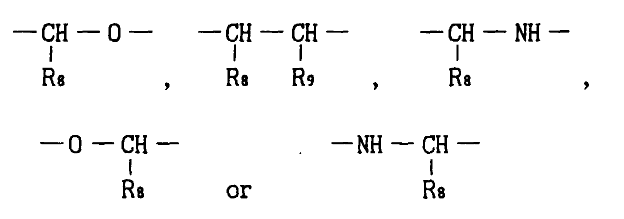 wherein R₈ and R₉ are each hydrogen atom.
(7) The therapeutic agent for diabetes of the above (1), wherein, in the formula [I],
   X is a group of the formula 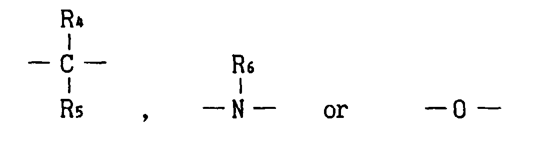 wherein
   R₄ is a hydrogen atom, an alkyl having 1 to 5 carbon atoms, an acyl having 2 to 5 carbon atoms, a carboxy or an alkoxycarbonyl having 2 to 5 carbon atoms, R₅ is a hydrogen atom or an alkyl having 1 to 5 carbon atoms, and R₆ is a hydrogen atom;
   R₁ is an alkyl having 1 to 5 carbon atoms optionally substituted by cycloalkyl having 3 to 7 carbon atoms or aryl, an aryl selected from phenyl, biphenyl, naphthyl and terphenyl (these aryl groups being optionally substituted by alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group or hydroxy), or cycloalkyl having 3 to 7 carbon atoms which is optionally substituted by alkyl having 1 to 5 carbon atoms or aryl;
   R₂ is a hydrogen atom;
   R₂ 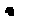 is a hydrogen atom;
   R₃ is an alkyl having 1 to 5 carbon atoms, an alkoxy having 1 to 4 carbon atoms or a hydroxy; and
   R₂ 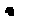 and R₃ combinedly form a group of the formula 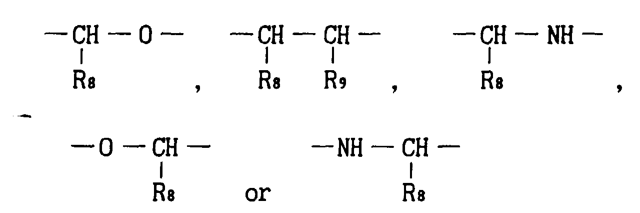 wherein R₈ and R₉ are each a hydrogen atom.
(8) The therapeutic agent for diabetes of the above (1), wherein, in the formula [I],
   X is a group of the formula 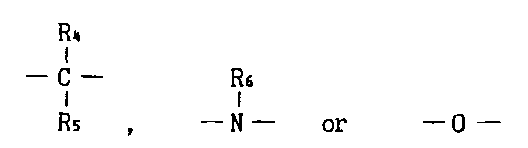 wherein
   R₄ is a hydrogen atom, an alkyl having 1 to 5 carbon atoms, an acyl having 2 to 5 carbon atoms, a carboxy or an alkoxycarbonyl having 2 to 5 carbon atoms, R₅ is a hydrogen atom or an alkyl having 1 to 5 carbon atoms, and R₆ is a hydrogen atom;
   R₁ is an aryl selected from the group consisting of phenyl, biphenyl, naphthyl and terphenyl (these aryl groups being optionally substituted by alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group or hydroxy), or a cycloalkyl having 3 to 7 carbon atoms which is optionally substituted by alkyl having 1 to 5 carbon atoms or aryl;
   R₂ is a hydrogen atom;
   R₂ 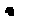 is a hydrogen atom; and
   R₃ is an alkyl having 1 to 5 carbon atoms, an alkoxy having 1 to 4 carbon atoms or a hydroxy.
(9) The therapeutic agent for diabetes of the above (1), wherein, in the formula [I],
   X is a group of the formula 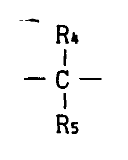 wherein
   R₄ is a hydrogen atom, an alkyl having 1 to 5 carbon atoms, an acyl having 2 to 5 carbon atoms, a carboxy or an alkoxycarbonyl having 2 to 5 carbon atoms, and R₅ is a hydrogen atom or an alkyl having 1 to 5 carbon atoms;
   R₁ is an aryl selected from phenyl, biphenyl, naphthyl and terphenyl (these aryl groups being optionally substituted by alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group or hydroxy), or a cycloalkyl having 3 to 7 carbon atoms which is optionally substituted by alkyl having 1 to 5 carbon atoms or aryl;
   R₂ is a hydrogen atom;
   R₂ 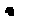 is a hydrogen atom; and
   R₃ is an alkyl having 1 to 5 carbon atoms, an alkoxy having 1 to 4 carbon atoms or a hydroxy.
(10) The therapeutic agent for diabetes of the above (1), wherein, in the formula [I],
   X is a group of the formula 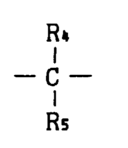 wherein
   R₄ and R₅ are each a hydrogen atom;
   R₁ is an aryl selected from the group consisting of phenyl, biphenyl, naphthyl and terphenyl (these aryl groups being optionally substituted by alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group or hydroxy), or cycloalkyl having 3 to 7 carbon atoms which is optionally substituted by alkyl having 1 to 5 carbon atoms or aryl;
   R₂ is a hydrogen atom;
   R₂ 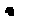 is a hydrogen atom; and
   R₃ is an alkyl having 1 to 5 carbon atoms, an alkoxy having 1 to 4 carbon atoms or a hydroxy.
(11) The therapeutic agent for diabetes of the above (1), wherein, in the formula [I],
   X is a group of the formula 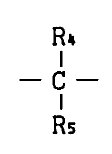 wherein
   R₄ and R₅ are each a hydrogen atom;
   R₁ is an aryl selected from the group consisting of phenyl, biphenyl, naphthyl and terphenyl (these aryl groups being optionally substituted by alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group or hydroxy), or a cycloalkyl having 3 to 7 carbon atoms which is optionally substituted by alkyl having 1 to 5 carbon atoms or aryl;
   R₂ is a hydrogen atom;
   R₂ 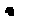 is a hydrogen atom; and
   R₃ is a hydroxy.
(12) A compound of the formula 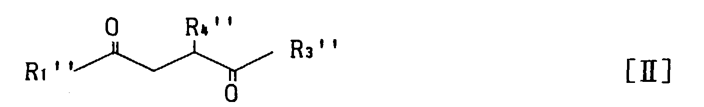 wherein,
   when R₃'' is a hydroxy,
   - R₄'': is a hydrogen atom, an acyl having 2 to 5 carbon atoms, a carboxy or an alkoxycarbonyl having 2 to 5 carbon atoms, and
   - R₁'': is a cycloalkyl having 3 to 7 carbon atoms which is mono-substituted by a substituent selected from the group consisting of alkyl having 1 to 4 carbon atoms, hydroxy, alkoxy having 1 to 4 carbon atoms, aryl, acyl having 2 to 5 carbon atoms, amino, carboxy and alkoxycarbonyl having 2 to 5 carbon atoms; and
   when R₃'' is an alkyl having 1 to 4 carbon atoms or an alkoxy having 1 to 4 carbon atoms,
   - R₄'': is an acyl having 2 to 5 carbon atoms, a carboxy or an alkoxycarbonyl having 2 to 5 carbon atoms, and
   - R₁'': is a cycloalkyl having 3 to 7 carbon atoms which is mono-substituted by a substituent selected from the group consisting of alkyl having 1 to 4 carbon atoms, hydroxy, alkoxy having 1 to 4 carbon atoms, aryl, acyl having 2 to 5 carbon atoms, amino, carboxy and alkoxycarbonyl having 2 to 5 carbon atoms;
   a prodrug thereof, a pharmaceutically acceptable salt thereof, a hydrate thereof and a solvate thereof.
(13) The compound of the above (12), wherein R₃'' is a hydroxy; a prodrug thereof, a pharmaceutically acceptable salt thereof, a hydrate thereof and a solvate thereof.
(14) The compound of the above (12) or (13), wherein R₄'' is a hydrogen atom; a prodrug thereof, a pharmaceutically acceptable salt thereof, a hydrate thereof and a solvate thereof.
(15) The compound of any one of the above (12) to (14), wherein R₁'' is a cycloalkyl having 3 to 7 carbon atoms which is mono-substituted by alkyl having 1 to 4 carbon atoms; a prodrug thereof, a pharmaceutically acceptable salt thereof, a hydrate thereof and a solvate thereof.
(16) The compound of any one of the above (12) to (15), wherein R₁'' is a cycloalkyl having 3 to 7 carbon atoms which is mono-substituted by methyl; a prodrug thereof, a pharmaceutically acceptable salt thereof, a hydrate thereof and a solvate thereof.
(17) The compound of any one of the above (12) to (16), which is selected from the group consisting of
   1) 4- (1-methylcyclohexyl)-4-oxobutanoic acid,
   2) trans-4-(4-methylcyclohexyl)-4-oxobutanoic acid,
   3) trans-4-(4-ethylcyclohexyl)-4-oxobutanoic acid,
   4) trans-4-(4-isopropylcyclohexyl)-4-oxobutanoic acid,
   5) trans-4-(4-tert-butylcyclohexyl)-4-oxobutanoic acid,
   6) trans-4-(4-phenylcyclohexyl)-4-oxobutanoic acid,
   7) cis-4-(4-methylcyclohexyl)-4-oxobutanoic acid,
   8) 4-(3-methylcyclohexyl)-4-oxobutanoic acid,
   9) dimethyl 2-[2-(1-methylcyclohexyl)-2-oxoethyl]propenedioate and
   10) methyl 2-acetyl-4-(1-methylcyclohexyl)-4-oxobutanoate; a prodrug thereof, a pharmaceutically acceptable salt thereof, a hydrate thereof and a solvate thereof.
(18) The compound of any one of the above (12) to (17) which is selected from the group consisting of
   1) 4-(1-methylcyclohexyl)-4-oxobutanoic acid,
   2) trans-4-(4-methylcyclohexyl)-4-oxobutanoic acid,
   3) trans-4-(4-ethylcyclohexyl)-4-oxobutanoic acid,
   4) trans-4-(4-isopropylcyclohexyl)-4-oxobutanoic acid,
   5) trans-4-(4-tert-butylcyclohexyl)-4-oxobutanoic acid,
   6) trans-4-(4-phenylcyclohexyl)-4-oxobutanoic acid,
   7) cis-4-(4-methylcyclohexyl)-4-oxobutanoic acid and
   8) 4-(3-methylcyclohexyl)-4-oxobutanoic acid; a prodrug thereof, a pharmaceutically acceptable salt thereof, a hydrate thereof and a solvate thereof.
(19) A compound of the formula 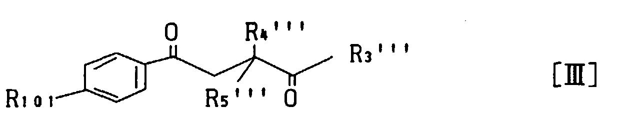 wherein,
   when R₃''' is a hydroxy,
   - R₄''': is a hydrogen atom, an alkyl having 1 to 4 carbon atoms, an acyl having 2 to 5 carbon atoms, a carboxy or an alkoxycarbonyl having 2 to 5 carbon atoms,
   - R₅''': is a hydrogen atom, an alkyl having 1 to 4 carbon atoms, an acyl having 2 to 5 carbon atoms, a carboxy or an alkoxycarbonyl having 2 to 5 carbon atoms, and
   - R₁₀₁: is an alkyl having 1 to 4 carbon atoms which is substituted by hydroxy;
   when R₃''' is an alkyl having 1 to 4 carbon atoms,
   - R₄''': is a hydrogen atom, an alkyl having 1 to 4 carbon atoms, an acyl having 2 to 5 carbon atoms, a carboxy or an alkoxycarbonyl having 2 to 5 carbon atoms;
   - R₅''': is a hydrogen atom, an alkyl having 1 to 4 carbon atoms, an acyl having 2 to 5 carbon atoms, a carboxy or an alkoxycarbonyl having 2 to 5 carbon atoms, and
   - R₁₀₁: is an alkyl having 1 to 4 carbon atoms which is substituted by hydroxy or an aryl substituted by a substituent selected from the group consisting of carboxy, alkoxycarbonyl having 2 to 5 carbon atoms, hydroxy, alkyl having 1 to 4 carbon atoms, alkoxy having 1 to 4 carbon atoms, aryl, acyl having 2 to 5 carbon atoms and amino; and
   when R₃''' is an alkoxy having 1 to 4 carbon atoms,
   - R₄''': is a hydrogen atom, an alkyl having 1 to 4 carbon atoms, an acyl having 2 to 5 carbon atoms, a carboxy or an alkoxycarbonyl having 2 to 5 carbon atoms,
   - R₅''': is a hydrogen atom, an alkyl having 1 to 4 carbon atoms, an acyl having 2 to 5 carbon atoms, a carboxy or an alkoxycarbonyl having 2 to 5 carbon atoms, and
   - R₁₀₁: is an alkyl having 1 to 4 carbon atoms which is substituted by hydroxy or an aryl substituted by a substituent selected from the group consisting of carboxy, alkoxycarbonyl having 2 to 5 carbon atoms, hydroxy, aryl, acyl having 2 to 5 carbon atoms and amino;
   a prodrug thereof, a pharmaceutically acceptable salt thereof, a hydrate thereof and a solvate thereof.
(20) The compound of the above (19), wherein R₃''' is a hydroxy; a prodrug thereof, a pharmaceutically acceptable salt thereof, a hydrate thereof and a solvate thereof.
(21) The compound of the above (19) or (20), wherein R₄''' or R₅''' is a hydrogen atom; a prodrug thereof, a pharmaceutically acceptable salt thereof, a hydrate thereof and a solvate thereof.
(22) The compound of any one of the above (19) to (21), wherein R₄''' and R₅''' are each a hydrogen atom; a prodrug thereof, a pharmaceutically acceptable salt thereof, a hydrate thereof and a solvate thereof.
(23) The compound of any one of the above (19) to (22), wherein R₁₀₁ is an alkyl having 1 to 4 carbon atoms which is substituted by hydroxy; a prodrug thereof, a pharmaceutically acceptable salt thereof, a hydrate thereof and a solvate thereof.
(24) The compound of any one of the above (19) to (23), which is selected from the group consisting of
   1) 4-[4-(hydroxymethyl)phenyl]-4-oxobutanoic acid,
   2) 1-(4-hydroxymethylphenyl)-1,4-pentanedione,
   3) 1-[4-(1-hydroxyethyl)phenyl]-1,4-pentanedione and
   4) 1-[4-(2-hydroxyethyl)phenyl]-1,4-pentanedione; a prodrug thereof, a pharmaceutically acceptable salt thereof, a hydrate thereof and a solvate thereof.
(25) A compound of the formula 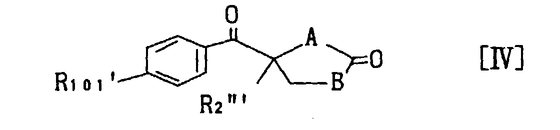 wherein A and B are the same or different and each is C, NH or O; and
   when R₂''' is a hydrogen atom,
   - R₁₀₁': is an alkyl having 1 to 4 carbon atoms which is substituted by hydroxy or an optionally substituted aryl; and
   when R₂''' is an alkyl having 1 to 4 carbon atoms,
   - R₁₀₁': is a hydrogen atom, an alkyl having 1 to 4 carbon atoms which is substituted by hydroxy or an optionally substituted aryl;
   a prodrug thereof, a pharmaceutically acceptable salt thereof, a hydrate thereof and a solvate thereof.
(26) The compound of the above (25), wherein R₂''' is an alkyl having 1 to 4 carbon atoms; a prodrug thereof, a pharmaceutically acceptable salt thereof, a hydrate thereof and a solvate thereof.
(27) The compound of the above (25) or (26), wherein R₂''' is a methyl; a prodrug thereof, a pharmaceutically acceptable salt thereof, a hydrate thereof and a solvate thereof.
(28) The compound of any one of the above (25) to (27), which is selected from the group consisting of
   1) (±)-3-benzoyl-3-methyl-1-cyclopentanone
   2) (±)-dihydro-4-(4'-phenylbenzoyl)-2(3H)-furanone
   3) (±)-dihydro-4-methyl-4-(4'-phenylbenzoyl)-2(3H)-furanone
   4) (±)-dihydro-4-(4'-hydroxymethylbenzoyl)-2(3H)-furanone
   5) (±)-dihydro-5-(4'-phenylbenzoyl)-2(3H)-furanone
   6) (±)-dihydro-5-(4'-hydroxymethylbenzoyl)-2(3H)-furanone
   7) (±)-dihydro-5-(4'-hydroxymethylbenzoyl)-5-methyl-2(3H)-furanone
   8) (-)-dihydro-5-methyl-5-(4'-phenylbenzoyl)-2(3H)-furanone
   9) (+)-dihydro-5-methyl-5-(4'-phenylbenzoyl)-2(3H)-furanone
   10) (S)-(-)-5-(4'-phenylbenzoyl)-2-pyrrolidinone and
   11) (±)-4-(4'-hydroxymethylbenzoyl)-2-pyrrolidinone;
   a prodrug thereof, a pharmaceutically acceptable salt thereof, a hydrate thereof and a solvate thereof.
(29) A pharmaceutical agent comprising the compound of any one of the above (12)-(16), (19)-(23) and (25)-(27), a prodrug thereof, a pharmaceutically acceptable salt thereof, a hydrate thereof or a solvate thereof.
(30) A pharmaceutical composition comprising the compound of any one of the above (12)-(16), (19)-(23) and (25)-(27), a prodrug thereof, a pharmaceutically acceptable salt thereof, a hydrate thereof or a solvate thereof.
(31) A pharmaceutical agent comprising the compound of any one of the above (17), (18), (24) and (28), a prodrug thereof, a pharmaceutically acceptable salt thereof, a hydrate thereof or a solvate thereof.
(32) A pharmaceutical composition comprising the compound of any one of the above (17), (18), (24) and (28), a prodrug thereof, a pharmaceutically acceptable salt thereof, a hydrate thereof or a solvate thereof.
(33) An agent for treating diabetes, comprising the compound of any one of the above (17), (18), (24) and (28), a prodrug thereof, a pharmaceutically acceptable salt thereof, a hydrate thereof or a solvate thereof.

### Brief Description of the Drawings

Fig. 1 is a graph showing changes in blood sugar (ordinates) and time course (abscissa) when the compound of the present invention (Example 93) and carboxymethylcellulose (control) were orally administered after glucose loading.

Fig. 2 is a graph showing changes in blood sugar (ordinates) and time course (abscissa) when the compound of the present invention (Example 65) in various amounts or carboxymethylcellulose (control) was orally administered after glucose loading.

Fig. 3 is a graph showing changes in blood sugar (ordinates) and time course (abscissa) when tolbutamide or carboxymethylcellulose (control) was orally administered after glucose loading.

Fig. 4 is a graph showing changes in fasting blood sugar (ordinates) and time course (abscissa) when the compound of the present invention (Example 93), tolbutamide or carboxymethylcellulose (control) was orally administered after glucose loading.

### Detailed Description of the Invention

In the present invention, the alkyl having 1 to 5 carbon atoms in the optionally substituted alkyl having 1 to 5 carbon atoms is a linear or branched alkyl having 1 to 5 carbon atoms, which is exemplified by methyl, ethyl, propyl, isopropyl, butyl, isobutyl, sec-butyl, tert-butyl, pentyl, isopentyl, neopentyl, 3-pentyl, tert-pentyl and the like. Preferable alkyl having 1 to 5 carbon atoms at R₁ is, for example, isopropyl, tert-butyl, 3-pentyl, and preferable alkyl having 1 to 5 carbon atoms at R₂, R₃, R₄, R₅, R₈ and R₉ is, for example, methyl and ethyl. The substituent is exemplified by aryl such as phenyl, naphthyl, biphenyl, terphenyl and the like; cycloalkyl such as cyclopropyl, cyclobutyl, cyclopentyl, cyclohexyl, cycloheptyl and the like; hydroxy; alkoxy such as methoxy, ethoxy, propoxy, butoxy and the like; acyl such as formyl, acetyl, propionyl, butyryl, valeryl and the like; carboxy; alkoxycarbonyl such as methoxycarbonyl, ethoxycarbonyl, propoxycarbonyl, butoxycarbonyl and the like; alkenyl such as ethenyl, propenyl, butenyl, pentenyl and the like; heterocyclic group such as thienyl, furyl, pyrrolyl, imidazolyl, pyrazolyl, thiazolyl, isothiazolyl, oxazolyl, isoxazolyl, morpholino, piperazinyl, piperidinyl, pyrranyl, pyridyl, thiopyrranyl, benzothienyl, benzofuryl and the like; amino; alkylamino such as methylamino, ethylamino, propylamino, butylamino and the like; dialkylamino such as dimethylamino, diethylamino, dipropylamino, dibutylamino and the like; acylamino such as formylamino, acetylamino, propionylamino, butyrylamino, valerylamino and the like; halogen atom such as fluorine atom, chlorine atom, bromine atom, iodine atom and the like; and the like. These substituents may be substituted by other substituents besides those mentioned above. Preferable substituents of the alkyl having 1 to 5 carbon atoms at R₁ include aryl, cycloalkyl, hydroxy, alkoxy, amino and halogen atom, with particular preference given to aryl and cycloalkyl. Preferable substituents of the alkyl having 1 to 5 carbon atoms at R₂, R₄, R₅, R₈ and R₉ include acyl, hydroxy, alkoxy and carboxy, with particular preference given to carboxy. Preferable substituents of the alkyl having 1 to 5 carbon atoms at R₃ include aryl, cycloalkyl and alkoxy.

The alkyl having 1 to 5 carbon atoms in the present invention is a linear or branched alkyl having 1 to 5 carbon atoms which is exemplified by methyl, ethyl, propyl, isopropyl, butyl, isobutyl, sec-butyl, tert-butyl, pentyl, isopentyl, neopentyl, 3-pentyl, tert-pentyl and the like.

The alkyl having 1 to 4 carbon atoms means a linear or branched alkyl having 1 to 4 carbon atoms which is exemplified by methyl, ethyl, propyl, isopropyl, butyl, isobutyl, sec-butyl, tert-butyl and the like. The alkyl having 1 to 4 carbon atoms at R₁'' is preferably methyl and ethyl, and particularly preferably methyl. Preferable alkyl having 1 to 4 carbon atoms at R₃'' is methyl and ethyl, particularly methyl. The alkyl having 1 to 4 carbon atoms at R₄''' is preferably methyl and ethyl, particularly preferably methyl. The alkyl having 1 to 4 carbon atoms at R₅''' is preferably methyl and ethyl, particularly preferably methyl. The alkyl having 1 to 4 carbon atoms at R₁₀₁ is preferably methyl and ethyl, particularly preferably methyl. The alkyl having 1 to 4 carbon atoms at R₃''' is preferably methyl and ethyl, particularly preferably methyl. The alkyl having 1 to 4 carbon atoms at R₁₀₁' is preferably methyl and ethyl, particularly preferably methyl. The alkyl having 1 to 4 carbon atoms at R₂''' is preferably methyl and ethyl, particularly preferably methyl.

The alkenyl having 2 to 6 carbon atoms in the optionally substituted alkenyl having 2 to 6 carbon atoms is a linear or branched alkenyl having 2 to 6 carbon atoms, which is exemplified by ethenyl, propenyl, butenyl, pentenyl, hexenyl and the like. Alkenyl having 2 to 6 carbon atoms at R₁, R₂, R₄ or R₅ is preferably ethenyl or propenyl. The substituent is exemplified by aryl such as phenyl, naphthyl, biphenyl, terphenyl and the like; cycloalkyl such as cyclopropyl, cyclobutyl, cyclopentyl, cyclohexyl, cycloheptyl and the like; hydroxy; alkoxy such as methoxy, ethoxy, propoxy, butoxy and the like; acyl such as formyl, acetyl, propionyl, butyryl, valeryl and the like; carboxy; alkoxycarbonyl such as methoxycarbonyl, ethoxycarbonyl, propoxycarbonyl, butoxycarbonyl and the like; heterocyclic group such as thienyl, furyl, pyrrolyl, imidazolyl, pyrazolyl, thiazolyl, isothiazolyl, oxazolyl, isoxazolyl, morpholino, piperazinyl, piperidinyl, pyrranyl, pyridyl, thiopyrranyl, benzothienyl, benzofuryl and the like; amino; alkylamino such as methylamino, ethylamino, propylamino, butylamino and the like; dialkylamino such as dimethylamino, diethylamino, dipropylamino, dibutylamino and the like; acylamino such as formylamino, acetylamino, propionylamino, butyrylamino, valerylamino and the like; halogen atom such as fluorine atom, chlorine atom, bromine atom, iodine atom and the like; and the like. These substituents may be substituted by other substituents besides those mentioned above. The substituent of alkenyl having 2 to 6 carbon atoms at R₁ is preferably aryl, cycloalkyl and alkoxy, particularly preferably aryl and cycloalkyl. The substituent of alkenyl having 2 to 6 carbon atoms at R₂, R₄ and R₅ is preferalby aryl, cycloalkyl and alkoxy, particularly preferably aryl and cycloalkyl.

The alkenyl having 2 to 6 carbon atoms in the present invention is a linear or branched alkenyl having 2 to 6 carbon atoms, which is exemplified by ethenyl, propenyl, butenyl, pentenyl, hexenyl and the like.

The aryl in the optionally substituted aryl is specifically phenyl, biphenyl, naphthyl, terphenyl and the like. The preferable aryl at R₁, R₂, R₃, R₄ and R₅ is phenyl, biphenyl and naphthyl, particularly preferably phenyl and biphenyl. The substituent is exemplified by alkyl such as methyl, ethyl, propyl, isopropyl, butyl, isobutyl, sec-butyl, tert-butyl, pentyl, isopentyl, neopentyl, 3-pentyl, tert-pentyl and the like; hydroxyalkyl such as hydroxymethyl, hydroxyethyl, hydroxypropyl, hydroxybutyl, hydroxypentyl and the like; alkenyl such as ethenyl, propenyl, butenyl, pentenyl, hexenyl and the like; cycloalkyl such as cyclopropyl, cyclobutyl, cyclopentyl, cyclohexyl, cycloheptyl and the like; hydroxy; alkoxy such as methoxy, ethoxy, propoxy, butoxy and the like; acyl such as formyl, acetyl, propionyl, butyryl, valeryl and the like; carboxy; alkoxycarbonyl such as methoxycarbonyl, ethoxycarbonyl, propoxycarbonyl, butoxycarbonyl and the like; heterocyclic group such as thienyl, furyl, pyrrolyl, imidazolyl, pyrazolyl, thiazolyl, isothiazolyl, oxazolyl, isoxazolyl, morpholino, piperazinyl, piperidinyl, pyrranyl, pyridyl, thiopyrranyl, benzothienyl, benzofuryl and the like; amino; alkylamino such as methylamino, ethylamino, propylamino, butylamino and the like; dialkylamino such as dimethylamino, diethylamino, dipropylamino, dibutylamino and the like; acylamino such as formylamino, acetylamino, propionylamino, butyrylamino, valerylamino and the like; halogen atom such as fluorine atom, chlorine atom, bromine atom, iodine atom and the like; and the like. These substituents may be substituted by other substituents besides those mentioned above. The substituent of aryl at R₁ is preferably alkyl, hydroxyalkyl, alkenyl, cycloalkyl, hydroxy, alkoxy, acyl, amino, acylamino and halogen atom, particularly preferably alkyl, hydroxyalkyl and hydroxy and most preferably hydroxymethyl. The substituent of aryl at R₂, R₃, R₄, R₅ and R₆ is preferably alkyl and hydroxyalkyl.

The aryl in the present invention is exemplified by phenyl, biphenyl, naphthyl, terphenyl and the like, and aryl at R₁'' is preferably phenyl and biphenyl, particularly preferably phenyl. The aryl at R₁₀₁ is preferably phenyl and biphenyl, particularly preferably phenyl. The aryl at R₁₀₁' is preferably phenyl and biphenyl, particularly preferably phenyl.

The heterocyclic group at the optionally substituted heterocyclic group is an aromatic heterocyclic ring or saturated heterocyclic ring containing, besides carbon atom, 1 to 3 hetero atoms selected from nitrogen atom, oxygen atom and sulfur atom as the atom constituting the ring. Specific examples thereof include thienyl, furyl, pyrrolyl, imidazolyl, pyrazolyl, thiazolyl, isothiazolyl, oxazolyl, isoxazolyl, morpholino, piperazinyl, piperidinyl, pyrranyl, pyridyl, thiopyrranyl, benzothienyl, benzofuryl and the like. The heterocyclic group at R₁ is preferably piperazinyl, pyridyl, benzothienyl and benzofuryl, particularly preferably pyridyl, benzothienyl and benzofuryl. As the substituent, exemplified are alkyl such as methyl, ethyl, propyl, isopropyl, butyl, isobutyl, sec-butyl, tert-butyl, pentyl, isopentyl, neopentyl, 3-pentyl, tert-pentyl and the like; hydroxyalkyl such as hydroxymethyl, hydroxyethyl, hydroxypropyl, hydroxybutyl, hydroxypentyl and the like; alkenyl such as ethenyl, propenyl, butenyl, pentenyl, hexenyl and the like; cycloalkyl such as cyclopropyl, cyclobutyl, cyclopentyl, cyclohexyl, cycloheptyl and the like; hydroxy; alkoxy such as methoxy, ethoxy, propoxy, butoxy and the like; acyl such as formyl, acetyl, propionyl, butyryl, valeryl and the like; carboxy; alkoxycarbonyl such as methoxycarbonyl, ethoxycarbonyl, propoxycarbonyl, butoxycarbonyl and the like; amino; alkylamino such as methylamino, ethylamino, propylamino, butylamino and the like; dialkylamino such as dimethylamino, diethylamino, dipropylamino, dibutylamino and the like; acylamino such as formylamino, acetylamino, propionylamino, butyrylamino, valerylamino and the like; halogen atom such as fluorine atom, chlorine atom, bromine atom, iodine atom and the like; and the like. These substituents may be substituted by other substituents besides those mentioned above.

Preferable substituents of the heterocyclic group at R₁ are, for example, alkyl, hydroxyalkyl, alkenyl, cycloalkyl, hydroxy, alkoxy, acyl, amino, acylamino and halogen atom, particularly preferably alkyl, hydroxyalkyl and hydroxy.

The cycloalkyl having 3 to 7 carbon atoms of the optionally substituted cycloalkyl having 3 to 7 carbon atoms is specifically cyclopropyl, cyclobutyl, cyclopentyl, cyclohexyl and cycloheptyl, and the cycloalkyl having 3 to 7 carbon atoms at R₁ and R₃ is preferably cyclopentyl and cyclohexyl. As the substituent, exemplified are alkyl such as methyl, ethyl, propyl, isopropyl, butyl, isobutyl, sec-butyl, tert-butyl, pentyl, isopentyl, neopentyl, 3-pentyl, tert-pentyl and the like; hydroxyalkyl such as hydroxymethyl, hydroxyethyl, hydroxypropyl, hydroxybutyl, hydroxypentyl and the like; alkenyl such as ethenyl, propenyl, butenyl, pentenyl, hexenyl and the like; cycloalkyl such as cyclopropyl, cyclobutyl, cyclopentyl, cyclohexyl, cycloheptyl and the like; hydroxy; alkoxy such as methoxy, ethoxy, propoxy, butoxy and the like; acyl such as formyl, acetyl, propionyl, butyryl, valeryl and the like; aryl such as phenyl, naphthyl, biphenyl, terphenyl and the like; heterocyclic group such as thienyl, furyl, pyrrolyl, imidazolyl, pyrazolyl, thiazolyl, isothiazolyl, oxazolyl, isoxazolyl, morpholino, piperazinyl, piperidinyl, pyrranyl, pyridyl, thiopyrranyl, benzothienyl, benzofuryl and the like; carboxy; alkoxycarbonyl such as methoxycarbonyl, ethoxycarbonyl, propoxycarbonyl, butoxycarbonyl and the like; amino; alkylamino such as methylamino, ethylamino, propylamino, butylamino and the like; dialkylamino such as dimethylamino, diethylamino, dipropylamino, dibutylamino and the like; acylamino such as formylamino, acetylamino, propionylamino, butyrylamino, valerylamino and the like; halogen atom such as fluorine atom, chlorine atom, bromine atom, iodine atom and the like; and the like. These substituents may be substituted by other substituents besides those mentioned above. The substituent of cycloalkyl having 3 to 7 carbon atoms at R₁ is preferably alkyl, hydroxyalkyl and aryl, particularly preferably alkyl and aryl and most preferably alkyl. The substituent of cycloalkyl having 3 to 7 carbon atoms at R₃ is preferably alkyl and aryl, particularly preferably alkyl.

The cycloalkyl having 3 to 7 carbon atoms in the present invention is exemplified by cyclopropyl, cyclobutyl, cyclopentyl, cyclohexyl, cycloheptyl and the like. The cycloalkyl having 3 to 7 carbon atoms at R₁, R₃ and R₁'' is preferably cyclopentyl or cyclohexyl, particularly preferably cyclohexyl.

The cycloalkenyl having 5 to 7 carbon atoms of the optionally substituted cycloalkenyl having 5 to 7 carbon atoms is specifically cyclopentenyl, cyclohexenyl and cycloheptenyl, and the substituent is exemplified by alkyl such as methyl, ethyl, propyl, isopropyl, butyl, isobutyl, sec-butyl, tert-butyl, pentyl, isopentyl, neopentyl, 3-pentyl, tert-pentyl and the like; hydroxyalkyl such as hydroxymethyl, hydroxyethyl, hydroxypropyl, hydroxybutyl, hydroxypentyl and the like; alkenyl such as ethenyl, propenyl, butenyl, pentenyl, hexenyl and the like; cycloalkyl such as cyclopropyl, cyclobutyl, cyclopentyl, cyclohexyl, cycloheptyl and the like; hydroxy; alkoxy such as methoxy, ethoxy, propoxy, butoxy and the like; acyl such as formyl, acetyl, propionyl, butyryl, valeryl and the like; aryl such as phenyl, naphthyl, biphenyl, terphenyl and the like; heterocyclic group such as thienyl, furyl, pyrrolyl, imidazolyl, pyrazolyl, thiazolyl, isothiazolyl, oxazolyl, isoxazolyl, morpholino, piperazinyl, piperidinyl, pyrranyl, pyridyl, thiopyrranyl, benzothienyl, benzofuryl and the like; carboxy; alkoxycarbonyl such as methoxycarbonyl, ethoxycarbonyl, propoxycarbonyl, butoxycarbonyl and the like; amino; alkylamino such as methylamino, ethylamino, propylamino, butylamino and the like; dialkylamino such as dimethylamino, diethylamino, dipropylamino, dibutylamino and the like; acylamino such as formylamino, acetylamino, propionylamino, butyrylamino, valerylamino and the like; halogen atom such as fluorine atom, chlorine atom, bromine atom, iodine atom and the like; and the like. These substituents may be substituted by other substituents besides those mentioned above. The substituent of cycloalkenyl having 5 to 7 carbon atoms at R₁ is preferably alkyl, hydroxyalkyl, aryl and halogen atom, particularly preferably alkyl and aryl.

The alkoxy having 1 to 4 carbon atoms of the optionally substituted alkoxy having 1 to 4 carbon atoms is specifically methoxy, ethoxy, propoxy, isopropoxy, butoxy, isobutoxy, sec-butoxy and tert-butoxy, and preferable alkoxy having 1 to 4 carbon atoms at R₃, R₈ and R₉ is methoxy or ethoxy, particularly preferably methoxy. The substituent is exemplified by cycloalkyl such as cyclopropyl, cyclobutyl, cyclopentyl, cyclohexyl, cycloheptyl and the like; hydroxy; alkoxy such as methoxy, ethoxy, propoxy, butoxy and the like; acyl such as formyl, acetyl, propionyl, butyryl, valeryl and the like; aryl such as phenyl, naphthyl, biphenyl, terphenyl and the like; heterocyclic group such as thienyl, furyl, pyrrolyl, imidazolyl, pyrazolyl, thiazolyl, isothiazolyl, oxazolyl, isoxazolyl, morpholino, piperazinyl, piperidinyl, pyrranyl, pyridyl, thiopyrranyl, benzothienyl, benzofuryl and the like; carboxy; alkoxycarbonyl such as methoxycarbonyl, ethoxycarbonyl, propoxycarbonyl, butoxycarbonyl and the like; amino; alkylamino such as methylamino, ethylamino, propylamino, butylamino and the like; dialkylamino such as dimethylamino, diethylamino, dipropylamino, dibutylamino and the like; acylamino such as formylamino, acetylamino, propionylamino, butyrylamino, valerylamino and the like; halogen atom such as fluorine atom, chlorine atom, bromine atom, iodine atom and the like; and the like. These substituents may be substituted by other substituents besides those mentioned above. The substituent of alkoxy having 1 to 4 carbon atoms at R₃ is preferably cycloalkyl or aryl, and the substituent of alkoxy having 1 to 4 carbon atoms at R₈ and R₉ is preferably hydroxy, alkoxy or amino.

As the alkoxy having 1 to 4 carbon atoms of the present invention, exemplified are methoxy, ethoxy, propoxy, isopropoxy, butoxy, isobutoxy, sec-butoxy, tert-butoxy and the like. The alkoxy having 1 to 4 carbon atoms at R₁'' is preferably methoxy or ethoxy, particularly preferably methoxy. The alkoxy having 1 to 4 carbon atoms at R₃'' is preferably methoxy or ethoxy, particularly preferably methoxy. The alkoxy having 1 to 4 carbon atoms at R₁₀₁ is preferably methoxy or ethoxy, particularly preferably methoxy.

The alkoxycarbonyl having 2 to 5 carbon atoms of the optionally substituted alkoxycarbonyl having 2 to 5 carbon atoms is exemplified by methoxycarbonyl, ethoxycarbonyl, propoxycarbonyl, isopropoxycarbonyl, butoxycarbonyl, isobutoxycarbonyl, sec-butoxycarbonyl and tert-butoxycarbonyl, and the alkoxycarbonyl having 2 to 5 carbon atoms at R₂, R₄, R₅, R₈ and R₉ is preferably methoxycarbonyl or ethoxycarbonyl, particularly preferably methoxycarbonyl. The substituent is exemplified by cycloalkyl such as cyclopropyl, cyclobutyl, cyclopentyl, cyclohexyl, cycloheptyl and the like; hydroxy; alkoxy such as methoxy, ethoxy, propoxy, butoxy and the like; acyl such as formyl, acetyl, propionyl, butyryl, valeryl and the like; aryl such as phenyl, naphthyl, biphenyl, terphenyl and the like; heterocyclic group such as thienyl, furyl, pyrrolyl, imidazolyl, pyrazolyl, thiazolyl, isothiazolyl, oxazolyl, isoxazolyl, morpholino, piperazinyl, piperidinyl, pyrranyl, pyridyl, thiopyrranyl, benzothienyl, benzofuryl and the like; carboxy; alkoxycarbonyl such as methoxycarbonyl, ethoxycarbonyl, propoxycarbonyl, butoxycarbonyl and the like; amino; alkylamino such as methylamino, ethylamino, propylamino, butylamino and the like; dialkylamino such as dimethylamino, diethylamino, dipropylamino, dibutylamino and the like; acylamino such as formylamino, acetylamino, propionylamino, butyrylamino, valerylamino and the like; halogen atom such as fluorine atom, chlorine atom, bromine atom, iodine atom and the like; and the like. These substituents may be substituted by other substituents besides those mentioned above. The substituent of alkoxycarbonyl having 2 to 5 carbon atoms at R₂, R₄, R₅, R₈ and R₉ is preferably hydroxy, alkoxy, halogen atom, acyl, aryl or amino.

The alkoxycarbonyl having 2 to 5 carbon atoms in the present invention is exemplified by methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl and the like, and the alkoxycarbonyl having 2 to 5 carbon atoms at R₄'' is preferably methoxycarbonyl and ethoxycarbonyl, particularly preferably methoxycarbonyl. The alkoxycarbonyl having 2 to 5 carbon atoms at R₁'' is preferably methoxycarbonyl and ethoxycarbonyl, particularly preferably methoxycarbonyl. The alkoxycarbonyl having 2 to 5 carbon atoms at R₄''' is preferably methoxycarbonyl and ethoxycarbonyl, particularly preferably methoxycarbonyl. The alkoxycarbonyl having 2 to 5 carbon atoms at R₅''' is preferably methoxycarbonyl and ethoxycarbonyl, particularly preferably methoxycarbonyl. The alkoxycarbonyl having 2 to 5 carbon atoms at R₁₀₁ is preferably methoxycarbonyl and ethoxycarbonyl, particularly preferably methoxycarbonyl.

The acyloxy having 2 to 5 carbon atoms of the optionally substituted acyloxy having 2 to 5 carbon atoms is exemplified by acetyloxy, propionyloxy and butyryloxy, and the acyloxy having 2 to 5 carbon atoms at R₈ and R₉ is preferably acetyloxy and propionyloxy. The substituent is exemplified by cycloalkyl such as cyclopropyl, cyclobutyl, cyclopentyl, cyclohexyl, cycloheptyl and the like; hydroxy; alkoxy such as methoxy, ethoxy, propoxy, butoxy and the like; acyl such as formyl, acetyl, propionyl, butyryl, valeryl and the like; aryl such as phenyl, naphthyl, biphenyl, terphenyl and the like; heterocyclic group such as thienyl, furyl, pyrrolyl, imidazolyl, pyrazolyl, thiazolyl, isothiazolyl, oxazolyl, isoxazolyl, morpholino, piperazinyl, piperidinyl, pyrranyl, pyridyl, thiopyrranyl, benzothienyl, benzofuryl and the like; carboxy; alkoxycarbonyl such as methoxycarbonyl, ethoxycarbonyl, propoxycarbonyl, butoxycarbonyl and the like; amino; alkylamino such as methylamino, ethylamino, propylamino, butylamino and the like; dialkylamino such as dimethylamino, diethylamino, dipropylamino, dibutylamino and the like; acylamino such as formylamino, acetylamino, propionylamino, butyrylamino, valerylamino and the like; halogen atom such as fluorine atom, chlorine atom, bromine atom, iodine atom and the like; and the like. These substituents may be further substituted by the substituents other than those mentioned above. The substituent of acyloxy having 2 to 5 carbon atoms at R₈ and R₉ is preferably hydroxy, aryl or amino.

Examples of the substituent of the optionally substituted adamantyl include alkyl such as methyl, ethyl, propyl, isopropyl, butyl, isobutyl, sec-butyl, tert-butyl, pentyl, isopentyl, neopentyl, 3-pentyl, tert-pentyl and the like; hydroxyalkyl such as hydroxymethyl, hydroxyethyl, hydroxypropyl, hydroxybutyl, hydroxypentyl and the like; alkenyl such as ethenyl, propenyl, butenyl, pentenyl, hexenyl and the like; cycloalkyl such as cyclopropyl, cyclobutyl, cyclopentyl, cyclohexyl, cycloheptyl and the like; alkoxy such as methoxy, ethoxy, propoxy, butoxy and the like; acyl such as formyl, acetyl, propionyl, butyryl, valeryl and the like; aryl such as phenyl, naphthyl, biphenyl, terphenyl and the like; heterocyclic group such as thienyl, furyl, pyrrolyl, imidazolyl, pyrazolyl, thiazolyl, isothiazolyl, oxazolyl, isoxazolyl, morpholino, piperazinyl, piperidinyl, pyrranyl, pyridyl, thiopyrranyl, benzothienyl, benzofuryl and the like; carboxy; alkoxycarbonyl such as methoxycarbonyl, ethoxycarbonyl, propoxycarbonyl, butoxycarbonyl and the like; amino; alkylamino such as methylamino, ethylamino, propylamino, butylamino and the like; dialkylamino such as dimethylamino, diethylamino, dipropylamino, dibutylamino and the like; acylamino such as formylamino, acetylamino, propionylamino, butyrylamino, valerylamino and the like; halogen atom such as fluorine atom, chlorine atom, bromine atom, iodine atom and the like; and the like. These substituents may be further substituted by a substituent other than those mentioned above. The substituent of admantyl at R₁ is preferably alkyl, hydroxyalkyl or halogen atom, particularly preferably alkyl.

Examples of the substituent of optionally substituted indanyl include alkyl such as methyl, ethyl, propyl, isopropyl, butyl, isobutyl, sec-butyl, tert-butyl, pentyl, isopentyl, neopentyl, 3-pentyl, tert-pentyl and the like; hydroxyalkyl such as hydroxymethyl, hydroxyethyl, hydroxypropyl, hydroxybutyl, hydroxypentyl and the like; alkenyl such as ethenyl, propenyl, butenyl, pentenyl, hexenyl and the like; cycloalkyl such as cyclopropyl, cyclobutyl, cyclopentyl, cyclohexyl, cycloheptyl and the like; alkoxy such as methoxy, ethoxy, propoxy, butoxy and the like; acyl such as formyl, acetyl, propionyl, butyryl, valeryl and the like; aryl such as phenyl, naphthyl, biphenyl, terphenyl and the like; heterocyclic group such as thienyl, furyl, pyrrolyl, imidazolyl, pyrazolyl, thiazolyl, isothiazolyl, oxazolyl, isoxazolyl, morpholino, piperazinyl, piperidinyl, pyrranyl, pyridyl, thiopyrranyl, benzothienyl, benzofuryl and the like; carboxy; alkoxycarbonyl such as methoxycarbonyl, ethoxycarbonyl, propoxycarbonyl, butoxycarbonyl and the like; amino; alkylamino such as methylamino, ethylamino, propylamino, butylamino and the like; dialkylamino such as dimethylamino, diethylamino, dipropylamino, dibutylamino and the like; acylamino such as formylamino, acetylamino, propionylamino, butyrylamino, valerylamino and the like; halogen atom such as fluorine atom, chlorine atom, bromine atom, iodine atom and the like; and the like. These substituents may be further substituted by a substituent other than those mentioned above. The substituent of indanyl at R₁ is preferably alkyl, hydroxyalkyl or halogen atom, particularly preferably alkyl.

Examples of the substituent of optionally substituted fluorenyl include alkyl such as methyl, ethyl, propyl, isopropyl, butyl, isobutyl, sec-butyl, tert-butyl, pentyl, isopentyl, neopentyl, 3-pentyl, tert-pentyl and the like; hydroxyalkyl such as hydroxymethyl, hydroxyethyl, hydroxypropyl, hydroxybutyl, hydroxypentyl and the like; alkenyl such as ethenyl, propenyl, butenyl, pentenyl, hexenyl and the like; cycloalkyl such as cyclopropyl, cyclobutyl, cyclopentyl, cyclohexyl, cycloheptyl and the like; hydroxy; alkoxy such as methoxy, ethoxy, propoxy, butoxy and the like; acyl such as formyl, acetyl, propionyl, butyryl, valeryl and the like; aryl such as phenyl, naphthyl, biphenyl, terphenyl and the like; heterocyclic group such as thienyl, furyl, pyrrolyl, imidazolyl, pyrazolyl, thiazolyl, isothiazolyl, oxazolyl, isoxazolyl, morpholino, piperazinyl, piperidinyl, pyrranyl, pyridyl, thiopyrranyl, benzothienyl, benzofuryl and the like; carboxy; alkoxycarbonyl such as methoxycarbonyl, ethoxycarbonyl, propoxycarbonyl, butoxycarbonyl and the like; amino; alkylamino such as methylamino, ethylamino, propylamino, butylamino and the like; dialkylamino such as dimethylamino, diethylamino, dipropylamino, dibutylamino and the like; acylamino such as formylamino, acetylamino, propionylamino, butyrylamino, valerylamino and the like; halogen atom such as fluorine atom, chlorine atom, bromine atom, iodine atom and the like; and the like. These substituents may be further substituted by a substituent other than those mentioned above. The substituent of fluorenyl at R₁ is preferably alkyl, hydroxyalkyl or halogen atom, particularly preferably alkyl.

The acyl having 2 to 5 carbon atoms of the optionally substituted acyl having 2 to 5 carbon atoms is exemplified by acetyl, propionyl, butyryl, valeryl and the like and the acyl having 2 to 5 carbon atoms at R₂, R₄ and R₅ is preferably acetyl or propionyl, particularly preferably acetyl. Examples of the substituent include cycloalkyl such as cyclopropyl, cyclobutyl, cyclopentyl, cyclohexyl, cycloheptyl and the like; hydroxy; alkoxy such as methoxy, ethoxy, propoxy, butoxy and the like; acyl such as formyl, acetyl, propionyl, butyryl, valeryl and the like; aryl such as phenyl, naphthyl, biphenyl, terphenyl and the like; heterocyclic group such as thienyl, furyl, pyrrolyl, imidazolyl, pyrazolyl, thiazolyl, isothiazolyl, oxazolyl, isoxazolyl, morpholino, piperazinyl, piperidinyl, pyrranyl, pyridyl, thiopyrranyl, benzothienyl, benzofuryl and the like; carboxy; alkoxycarbonyl such as methoxycarbonyl, ethoxycarbonyl, propoxycarbonyl, butoxycarbonyl and the like; amino; alkylamino such as methylamino, ethylamino, propylamino, butylamino and the like; dialkylamino such as dimethylamino, diethylamino, dipropylamino, dibutylamino and the like; acylamino such as formylamino, acetylamino, propionylamino, butyrylamino, valerylamino and the like; halogen atom such as fluorine atom, chlorine atom, bromine atom, iodine atom and the like; and the like. These substituents may be further substituted by a substituent other than those mentioned above. The substituent of acyl having 2 to 5 carbon atoms at R₂, R₄ and R₅ is preferably hydroxy, alkoxy or halogen atom, particularly preferably alkoxy.

The acyl having 2 to 5 carbon atoms in the present invention is specifically exemplified by acetyl, propionyl, butyryl, valeryl and the like, and the acyl having 2 to 5 carbon atoms at R₆ is preferably acetyl or propionyl, particularly preferably acetyl. The acyl having 2 to 5 carbon atoms at R₄'' is preferably acetyl and propionyl, particularly preferably acetyl. The acyl having 2 to 5 carbon atoms at R₁'' is preferably acetyl or propionyl, particularly preferably acetyl. The acyl having 2 to 5 carbon atoms at R₄''' is preferably acetyl or propionyl, particularly preferably acetyl. The acyl having 2 to 5 carbon atoms at R₅''' is preferably acetyl or propionyl, particularly preferably acetyl. The acyl having 2 to 5 carbon atoms at R₁₀₁ is preferably acetyl or propionyl, particularly preferably acetyl.

The acyl having 1 to 5 carbon atoms in the present invention is exemplified by formyl, acetyl, propionyl, butyryl, valeryl and the like. The substituent of aryl is preferably formyl or acetyl.

The amino-protecting group is one conventionally used for organic synthesis, such as benzyl, 3,4-dimethoxybenzyl, 1-phenylethyl, biphenylmethyl, bis(4-methoxyphenyl)methyl, 2-nitrobenzyl, triphenylmethyl, phenacyl, acetyl, trifluoroacetyl, benzyloxycarbonyl, tert-butoxycarbonyl and the like.

The prodrug is a derivative obtained by chemically modifying a drug molecule, and it does not show physiological activity by itself. After administration, it restores to the original drug molecule in the body and exhibits pharmaceutical effects.

The active metabolite is a substance which enhances or induces the action by a drug metabolic enzyme.

The pharmaceutically acceptable salt includes, but not limited to, various alkali metal salts such as sodium salt and potassium salt; various alkaline earth metal salts such as magnesium salt and calcium salt; aluminum salt; ammonium salt; and the like. The compound of the present invention may comprise asymmetric carbon, in which case optically pure enantiomer, racemate thereof, and mixtures having optional combinations and ratios exist. The present invention encompasses therapeutic drugs for diabetes comprising any of such isomers. In the case of racemates, moreover, optical resolution gives either of the optically active compounds, if necessary. An asymmetric synthesis directly gives only one optically active compound. In addition, the compound of the present invention may be in the form of a hydrate or solvate as occasion demands.

The pharmaceutical preparation containing the compound of the present invention can be formulated by admixing the compound with pharmacologically acceptable carriers, excipients, diluents, extenders, disintegrators, stabilizers, preservatives, buffers, emulsifying agents, aromatics, colorings, sweeteners, thickeners, flavors, solubilizers and other additives known *per se,* such as water, vegetable oil, alcohols such as ethanol, benzyl alcohol and hydroxy propyl alcohol, polyethylene glycol, glycerol triacetate, gelatin, carbohydrates such as lactose and starch, magnesium stearate, talc, lanolin, vaselin, sucrose, glucose, mannit, sorbit, crystalline cellulose, acacia, dextrin, hydroxypropylmethylcellulose, polyvinylpyrrolidone, macrogol, carnauba wax, polyoxyethylene, polyoxypropylene, glycol, cacao butter, lauric acid, lecithin, glycerin, sodium parahydoroxybenzoate, sodium benzoate, salicylic acid, potassium sorbate and the like to give tablets, pills, powders, granules, suppositories, injections, liqids, capsules, troches and the like which can be administered orally or parenterally. While the dose varies depending on the stages of the disease, compound to be administered, administration route, and age, sex, body weight and the like of patients, 0.001-1,000 mg, particularly 0.1-100 mg of the compound of the present invention is generally administered to an adult per day.

The present invention is described in more detail by referring to the production method of the dicarbonyl compounds thereof, to which the method of producing the compounds of the present invention is not limited.

Hereunder follow synthesis methods, by way of which the present invention is explained in more detail.

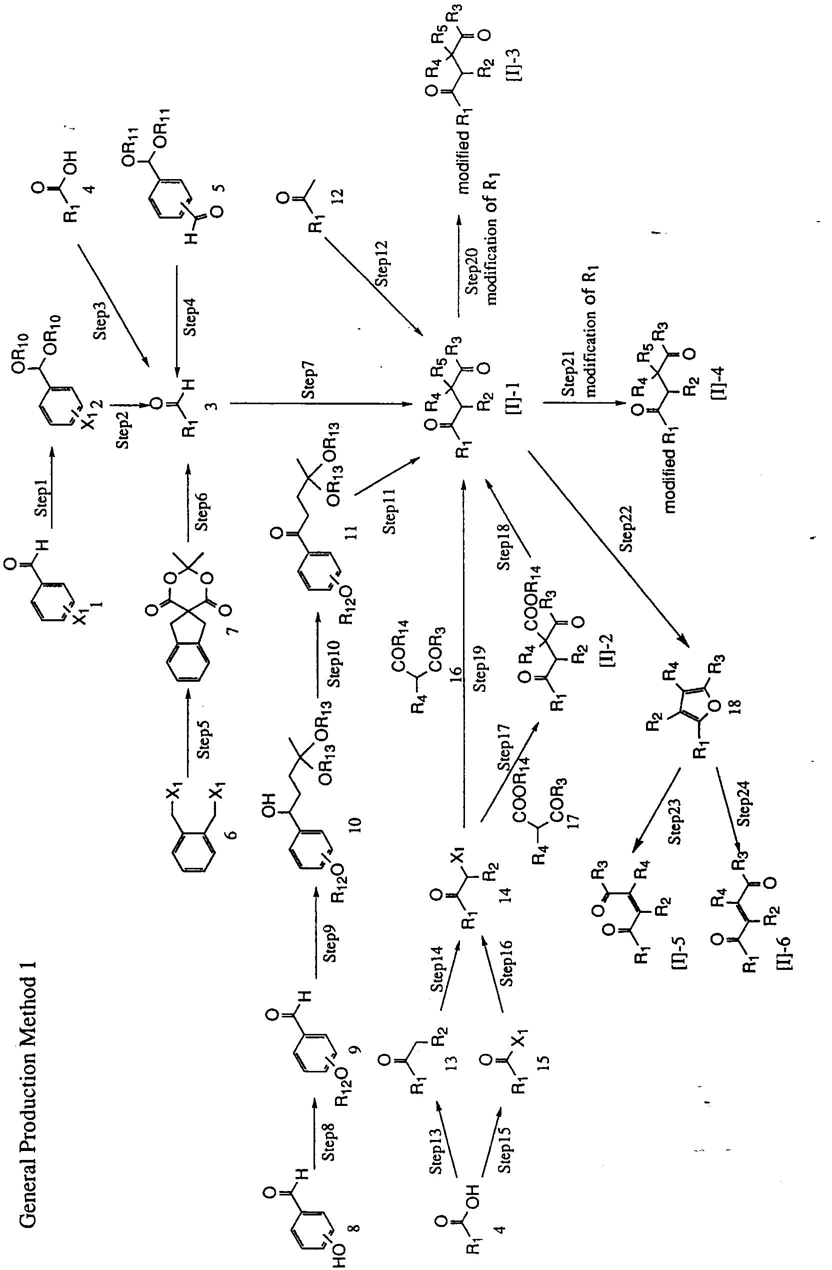

The reaction flow on the previous page schematically show the General Production Method 1, wherein R₁, R₂, R₃, R₄ and R₅ are used within the general sense of the symbols as used in the compound of the present invention. The same symbol generally shows the same meaning throughout all formulas, though there may occur cases where the range shown by each symbol differs depending on respective reactions from the corresponding symbol.

The General Production Method 1 is described in detail following each step. 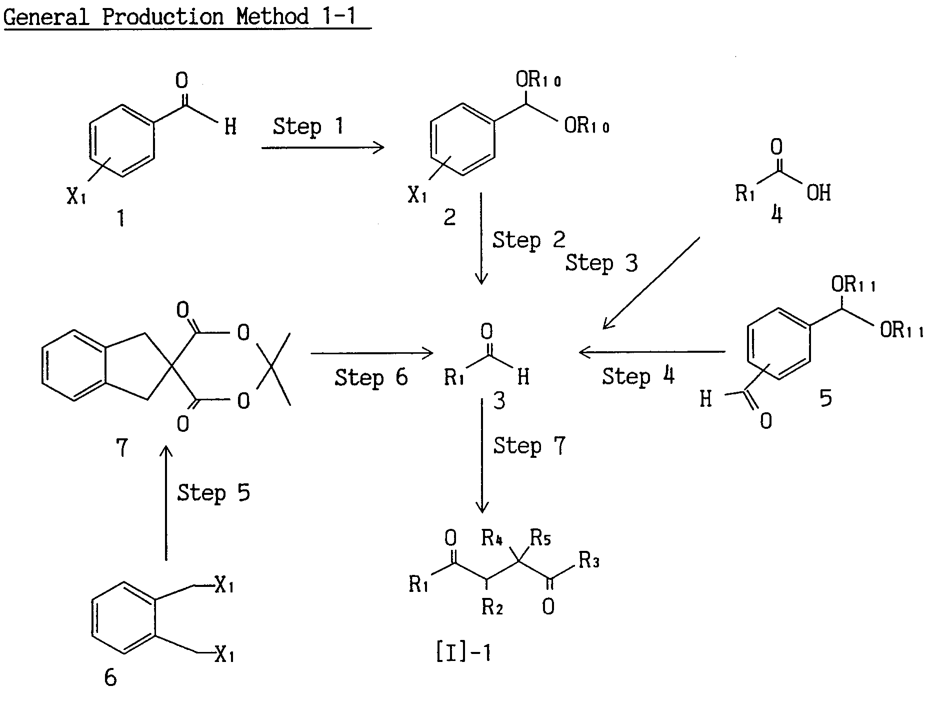 wherein R₁, R₂, R₃, R₄ and R₅ are as defined above, X₁ is halogen atom such as chlorine atom, bromine atom, iodine atom and the like, R₁₀ is lower alkyl such as methyl, ethyl, propyl, butyl and the like or two R₁₀ in combination show lower alkylene such as ethylene, trimethylene and the like, R₁₁ is lower alkyl such as methyl, ethyl, propyl, butyl and the like, or two R₁₁ combinedly show lower alkylene such as ethylene, trimethylene and the like.

### Step 1

Compound 1 is reacted with an alcohol such as methanol, ethanol, propanol, butanol, ethylene glycol, trimethylene glycol and the like in a solvent such as benzene, toluene, xylene, methylene chloride, chloroform, 1,2-dichloroethane and the like or a mixed solvent thereof or without solvent, in the presence of an inorganic acid such as hydrochloric acid, sulfuric acid, hydrobromic acid or an organic acid such as p-toluenesulfonic acid, benzenesulfonic acid, methanesulfonic acid, trifluoromethanesulfonic acid and the like under heating, preferably under reflux with heating to give compound 2.

### Step 2

Magnesium is reacted with compound 2 in a solvent such as various ethers (e.g., ethyl ether, tetrahydrofuran and the like), benzene, toluene, n-hexane, cyclohexane and the like or a mixed solvent thereof. Then, aldehyde such as acetaldehyde, propionaldehyde, benzaldehyde and the like is added to the reaction mixture and the mixture is reacted from under cooling to under heating. This reaction often produces preferable results when lower alkyl halide such as methyl iodide, ethyl iodide, 1,2-dibromoethane, 1,2-diiodoethane and the like or iodine is used with magnesium. The obtained product is reacted with an inorganic acid such as hydrochloric acid, sulfuric acid, hydrobromic acid and the like or an organic acid such as trifluoroacetic acid, formic acid, acetic acid, trifluoromethanesulfonic acid, p-toluenesulfonic acid, methanesulfonic acid, benzenesulfonic acid and the like in water or a mixed solvent of tetrahydrofuran, 1,4-dioxane, ethanol or chloroform, or methylene chloride and water from under cooling to under heating, preferably from room temperature to under heating to give compound 3.

In an alternative method of step 2, compound 2 is reacted with vinyltributyltin in a solvent such as various ethers (e.g., 1,4-dioxane, tetrahydrofuran and the like), benzene, toluene, N,N-dimethylformamide, acetonitrile and the like or a mixed solvent thereof in the presence of a phosphine palladium catalyst such as tetrakis(triphenylphosphine)palladium (O), trans-benzyl(chloro)bis(triphenylphosphine)palladium (II) and the like from under cooling to under heating, preferably from room temperature to under heating; the obtained product is reacted with substituted borans such as diboran, boran-dimethyl sulfide complex, boran-tetrahydrofuran complex, 9-boranbicyclo[3,3,1]nonane, 2,3-dimethyl-2-butylboran, bis(1,2-dimethylpropyl)boran, boran complex and the like in a solvent such as various ethers (e.g., ethyl ether, diglyme, 1,4-dioxane, tetrahydrofuran and the like), benzene, toluene, n-hexane, cyclohexane and the like or a mixed solvent thereof and an aqueous solution of a base such as sodium hydroxide, potassium hydroxide, magnesium hydroxide, calcium hydroxide and the like and aqueous hydrogen peroxide are added to the reaction mixture, and the obtained product is reacted with an acid such as an inorganic acid (e.g., hydrochloric acid, sulfuric acid, hydrobromic acid, and the like) or an organic acid (e.g., trifluoroacetic acid, formic acid, acetic acid, and the like) in water or a mixed solvent of tetrahydrofuran, 1,4-dioxane, ethanol, chloroform or methylene chloride and water from under cooling to under heating, preferably from room temperature to under heating to give compound 3. When Step 2 is used, R₁ of compound 3 is phenyl substituted by hydroxymethyl.

### Step 3

Compound 4 is reacted with a lower alkyl formate halide such as methyl chloroformate, methyl bromoformate, ethyl chloroformate, ethyl bromoformate, propyl chloroformate, propyl bromoformate, isopropyl chloroformate, isopropyl bromoformate and the like or an acid halide such as tosyl chloride, pivaloyl chloride and the like in a solvent such as various ethers (e.g., ethyl ether, 1,4-dioxane, tetrahydrofuran and the like), benzene, toluene, n-hexane, cyclohexane, methylene chloride, chloroform and the like or a mixed solvent thereof in the presence of an organic base such as triethylamine, pyridine, N-methylmorpholine and the like and a solution of lithium borohydride, sodium borohydride, potassium borohydride and the like in water, methanol, ethanol, isopropanol and the like was added to the reaction mixture from under cooling to under heating, preferably from under cooling to room temperature. The obtained product is dissolved in dimethyl sulfoxide or a mixed solvent of dimethyl sulfoxide and methylene chloride, and an organic base such as triethylamine, N-methylmorpholine and the like was added to the reaction mixture in the presence of sulfur trioxide-pyridine complex or oxalyl chloride and the like from under cooling to under heating, preferably from under cooling to room temperature to give compound 3.

### Step 4

Compound 5 is reacted with lithium borohydride, sodium borohydride, potassium borohydride and the like in a solvent such as water, various alcohols (e.g., methanol, ethanol, isopropanol and the like) and the like, or a mixed solvent thereof from under cooling to under heating, preferably from under cooling to room temperature. Then the obtained product is reacted with an inorganic acid such as hydrochloric acid, sulfuric acid, hydrobromic acid and the like or an organic acid such as p-toluenesulfonic acid, benzenesulfonic acid, trifluoroacetic acid, formic acid, acetic acid and the like in water or a mixed solvent of tetrahydrofuran, 1,4-dioxane, methanol, ethanol, chloroform, methylene chloride and the like and water to give compound 3. When Step 4 is used, R₁ of compound 3 is phenyl substituted by hydroxyalkyl.

### Step 5

Compound 6 is reacted with Meldrum's acid in a solvent such as various ethers (e.g., ethyl ether, 1,4-dioxane, 1,2-dimethoxyethane, tetrahydrofuran and the like), benzene, toluene, n-hexane, cyclohexane, methylene chloride, chloroform, N,N-dimethylformamide, dimethyl sulfoxide and the like, or a mixed solvent thereof in the presence of a base such as triethylamine, pyridine, N-methylmorpholine and the like to give compound 7.

### Step 6

Compound 7 is reacted with a lower alcohol such as methanol, ethanol, propanol and the like in a solvent such as benzene, toluene and the like or a mixed solvent thereof or without solvent in the presence of an inorganic acid such as hydrochloric acid, sulfuric acid, hydrobromic acid and the like or an organic acid such as p-toluenesulfonic acid, benzenesulfonic acid, methanesulfonic acid, trifluoromethanesulfonic acid and the like from under cooling to under heating, preferably at refluxing temperature; and the obtained product is reacted in the presence of a alkaline metal halide such as lithium chloride, sodium chloride, potassium chloride and the like in aqueous dimethyl sulfoxide or aqueous N,N-dimethylformamide from under cooling to under heating, preferably at refluxing temperature. The obtained product is reacted with dialkylaluminums such as diisobutylaluminum hydride and the like, lithium trialkylaluminum hydrides such as lithium trimethylaluminum hydride and the like or lithium trialkoxyaluminum hydrides such as lithium tri-tert-butoxyaluminum hydride in a solvent such as various ethers (e.g., ethyl ether, tetrahydrofuran and the like), benzene, toluene, n-hexane, cyclohexane and the like, or a mixed solvent thereof from under cooling to under heating, preferably from under cooling to give compound 3. When Step 6 is used, R₁ of compound 3 is indanyl.

### Step 7

The Compound 3 is reacted with methyl vinyl ketone, ethyl vinyl ketone, propyl vinyl ketone, phenyl vinyl ketone, biphenyl vinyl ketone and the like in a solvent such as various alcohols (e.g., methanol, ethanol and the like), N,N-dimethylformamide, 1,4-dioxane, and the like or a mixed solvent thereof in the presence of quaternary thiazolium salts such as 3-benzyl-5-(2-hydroxyethyl)-4-methyl-1,3-thiazolium chloride, 3-ethyl-5-(2-hydroxyethyl)-4-methyl-1,3-thiazolium bromide, 5-(2-hydroxyethyl)-3,4-dimethylthiazolium iodide and the like and an organic base such as triethylamine, N-methylmorpholine, pyridine and the like or cyanide such as sodium cyanide, potassium cyanide and the like from under cooling to under heating, preferably from room temperature to under heating to give compound [I]-1. 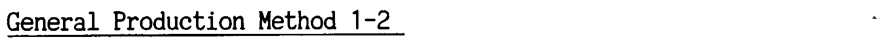 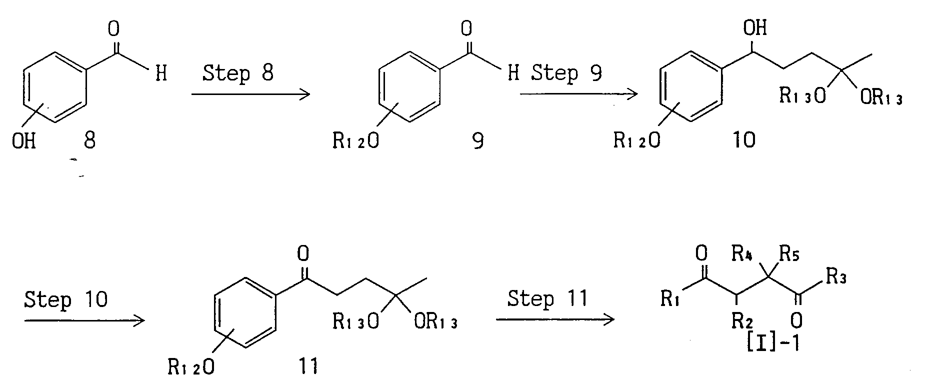 wherein R₁ is as defined above, R₂, R₄ and R₅ are hydrogen atoms, R₃ is methyl, R₁₂ is methoxymethyl, tert-butyldimethylsilyl or tert-butyl, R₁₃ is a lower alkyl such as methyl, ethyl, propyl, butyl and the like, or two R₁₃ combinedly form lower alkylene such as ethylene, trimethylene and the like.

### Step 8

When R₁₂ is methoxymethyl or tert-butyldimethylsilyl: compound 8 is reacted with hydroxy-protecting reagent such as chloromethylmethyl ether, tert-butyldimethylsilyl chloride and the like in a solvent such as various ethers (e.g., ethyl ether, tetrahydrofuran and the like), benzene, toluene, n-hexane, cyclohexane, N,N-dimethylformamide, dimethyl sulfoxide, acetone, acetonitrile and the like, or a mixed solvent thereof in the presence of a base such as sodium hydride, sodium hydroxide, potassium hydroxide, sodium hydrogencarbonate, potassium hydrogencarbonate, triethylamine, N-methylmorpholine, pyridine, imidazole, tert-butyl chloride, 2-methoxyethoxymethyl chloride, potassium carbonate and the like, from under cooling to under heating, preferably from room temperature to under heating to give compound 9. When R₁₂ is tert-butyl: compound 8 is reacted with isobutene in a solvent such as various ethers (e.g., ethyl ether, tetrahydrofuran and the like), benzene, toluene, n-hexane, cyclohexane and the like, or a mixed solvent thereof in the presence of an inorganic acid such as hydrogen chloride, sulfuric acid, hydrogen bromide and the like or an organic acid such as p-toluenesulfonic acid, benzenesulfonic acid, methanesulfonic acid, trifluoromethanesulfonic acid and the like to give compound 9.

### Step 9

Magnesium is reacted with 2-(bromoethyl)-2-methyl-1,3-dioxolane, which reaction often produces preferable results when iodine or 1,2-dibromoethane is used with magnesium, in a solvent such as various ethers (e.g., ethyl ether, 1,2-dimethoxyethane, 1,4-dioxane, tetrahydrofuran and the like), benzene, toluene, n-hexane, cyclohexane and the like, or a mixed solvent thereof, and then compound 9 is added to the reaction mixture from under cooling to under heating, preferably from under cooling to give compound 10.

### Step 10

Compound 10 is added to dimethyl sulfoxide or a mixed solvent of dimethyl sulfoxide and methylene chloride, in the presence of sulfur trioxide-pyridine complex or oxalyl chloride and the like, and further, reacted with an organic base such as triethylamine, N-methylmorpholine and the like from under cooling to under heating, preferably room temperature to under heating to give compound 11.

### Step 11

Compound 11 is reacted with an inorganic acid such as hydrochloric acid, sulfuric acid, hydrobromic acid and the like, or an organic acid such as p-toluenesulfonic acid, benzenesulfonic acid, formic acid, acetic acid, trifluoroacetic acid, methanesulfonic acid, trifluoromethanesulfonic acid and the like in a solvent such as water or various ethers (e.g., 1,4-dioxane, tetrahydrofuran and the like), methylene chloride, chloroform, various alcohols (e.g., methanol, ethanol, propanol, isopropanol and the like) and the like, or a mixed solvent thereof with water from under cooling to under heating, preferably from room temperature to under heating to give compound [I]-1.

When Step 11 is used, R₁ of compound [1]-1 is phenyl substituted by hydroxy. 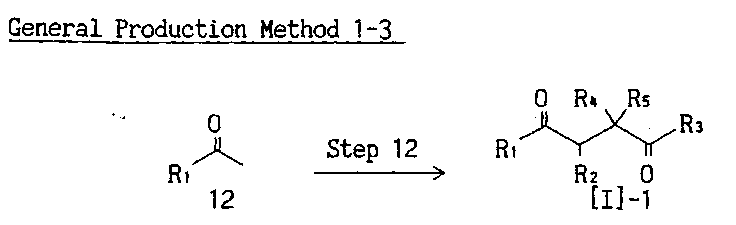 wherein R₁ and R₃ are as defined above, R₂, R₄ and R₅ are each hydrogen atom.

### Step 12

The compound 12 is dissolved in a solvent such as various ethers (e.g., ethyl ether, 1,4-dioxane, tetrahydrofuran and the like), benzene, toluene, n-hexane, cyclohexane, methylene chloride, chloroform, tetrahydrofuran, N,N-dimethylformamide, dimethyl sulfoxide and the like, or a mixed solvent. The compound 12 is reacted with copper halide such as copper chloride, copper bromide, copper iodide and the like, copper acetate, silver oxide and the like in the presence of a base such as lithium diisopropylamide, sodium hexamethyldisilazide and the like from under cooling to under heating, preferably from room temperature to under heating, and from under cooling to give compound [I]-1 wherein R₁ and R₃ are the same group. 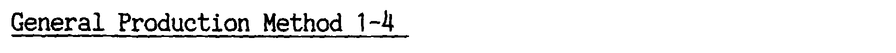 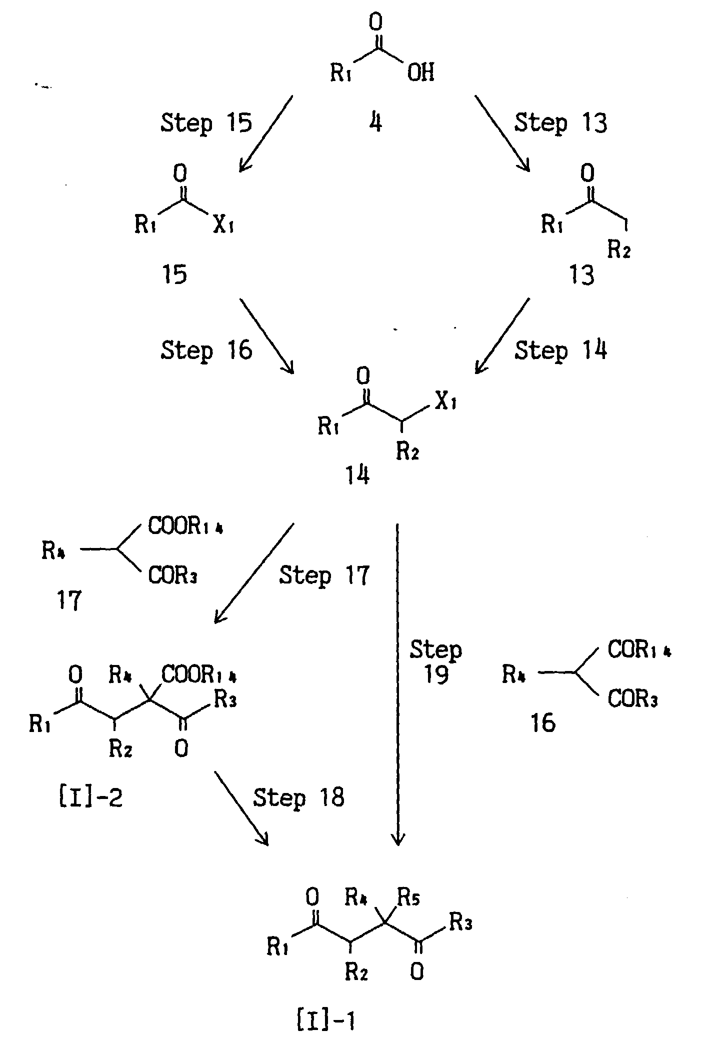 wherein R₁ and R₄ are as defined above, R₂ is lower alkyl such as methyl, ethyl, propyl, butyl and the like, R₃ is lower alkyl such as methyl, ethyl, propyl, isopropyl, butyl, isobutyl, sec-butyl, tert-butyl and the like, phenyl, benzyl or 2-phenylethyl, R₅ is hydrogen atom, acyl, lower alkylcarbonyl or benzylcarbonyl, R₁₄ is a lower alkyl such as methyl, ethyl, propyl, isopropyl, butyl, isobutyl, sec-butyl, tert-butyl and the like, or benzyl, and X₁ is halogen atom such as chlorine atom, bromine atom and iodine atom.

### Step 13

The compound 4 is reacted with lower alkyllithium such as methyllithium, ethyllithium and the like in a solvent such as various ethers (e.g., ethyl ether, 1,2-dimethoxyethane, 1,4-dioxane, tetrahydrofuran and the like), benzene, toluene, n-hexane, cyclohexane, methylene chloride, chloroform and the like, or a mixed solvent thereof from under cooling to under heating, preferably from 0°C to room temperature to give compound 13 wherein R₂ is lower alkyl.

### Step 14

The compound 13 is reacted with halogen such as chlorine and bromine, or 4-(dimethylamino)pyridinium bromide perbomide in a solvent such as various ethers (e.g., ethyl ether, 1,4-dioxane, tetrahydrofuran and the like), benzene, toluene, n-hexane, cyclohexane, methylene chloride, carbon tetrachloride, acetic acid, chloroform, various alcohols (e.g., methanol, ethanol and the like) and the like, or a mixed solvent thereof from under cooling to under heating, preferably from 0°C to room temperature to give compound 14 wherein R₂ is lower alkyl. This reaction often produces preferable results when hydrochloric acid or hydrobromic acid is added.

### Step 15

The compound 4 is reacted with oxalyl chloride, oxalyl bromide, thionyl chloride and the like in a solvent such as benzene, toluene, n-hexane, cyclohexane, methylene chloride, chloroform and the like, or a mixed solvent thereof, or without solvent from under cooling to under heating, preferably from 0°C to under heating to give compound 15. This reaction often produces preferable results when dimethylformamide and the like is added.

### Step 16

The compound 15 is reacted with lower diazoalkyl such as diazomethane, diazoethane and the like in a solvent such as various ethers (e.g., ethyl ether, 1,4-dioxane, tetrahydrofuran and the like), benzene, toluene, n-hexane, cyclohexane, chloroform, dichloromethane and the like, or a mixed solvent thereof from under cooling to under heating, preferably from 0°C to room temperature. The obtained product is reacted with aqueous hydrochloric acid, aqueous hydrobromic acid or hydrogen-bromide in acetic acid solution to give compound 14 wherein R₂ is lower alkyl group.

### Step 17

The compound 14 is reacted with the compound 17 in a solvent such as various ethers (e.g., ethyl ether, 1,4-dioxane, tetrahydrofuran and the like), benzene, toluene, n-hexane, cyclohexane, N,N-dimethylformamide, dimethyl sulfoxide and the like, or a mixed solvent thereof in the presence of a base such as lithium hydride, potassium hydride, sodium hydride, sodium methoxide, sodium ethoxide, lithium diisopropylamide, sodium carbonate, potassium carbonate and the like from under cooling to under heating, preferably from 0°C to room temperature to give compound [I]-2.

### Step 18

When R₁₄ is lower alkyl such as methyl, ethyl, propyl, isopropyl, butyl, isobutyl, sec-butyl and the like:

The compound [I]-2 is deprotected in a solvent of water or a mixed solvent of water and various alcohols (e.g., methanol, ethanol, propanol, isopropanol and the like), or various ethers (e.g., 1,4-dioxane, tetrahydrofuran and the like) and water, in the presence of a base such as lithium hydroxide, sodium hydroxide, potassium hydroxide, calcium hydroxide, barium hydroxide and the like from under cooling to under heating, preferably from 0°C to room temperature to give compound [I]-1.

When R₁₄ is tert-butyl:

The compound [I]-2 is deprotected in a solvent such as benzene, toluene, n-hexane, cyclohexane, methylene chloride, chloroform and the like, or a mixed solvent thereof, or without solvent, in the presence or absence of acid catalyst such as formic acid, acetic acid, trifluoroacetic acid, p-toluenesulfonic acid and the like from under cooling to under heating, preferably from room temperature to under heating to give compound [I]-1. Alternatively, compound [I]-2 is deprotected in a solvent such as dimethyl sulfoxide, N,N-dimethylformamide and the like in the presence of lithium chloride, sodium chloride, potassium chloride, and the like and water from under cooling to under heating, preferably at refluxing temperature to give compound [I]-1.

When R₁₄ is benzyl:

The compound [I]-2 is deprotected in a solvent such as various alcohols (e.g., methanol, ethanol, propanol and the like), various ethers (e.g., 1,4-dioxane, tetrahydrofuran and the like), ethyl acetate and the like, or a mixed solvent thereof, in the presence of palladium catalyst such as palladium-carbon, palladium black and the like under a hydrogen atmosphere from under cooling to under heating, preferably from room temperature to under heating to give compound [I]-1. When Step 18 is used, compound [I]-1 wherein R₂ is lower alkyl, R₃ is lower alkyl, phenyl, benzyl or 2-phenylethyl and R₅ is hydrogen atom can be obtained.

### Step 19

In the same manner as in Step 17, compound [I]-1 can be obtained from compound 16 and compound 14. When Step 19 is used, compound [I]-1 wherein R₂ is lower alkyl, R₃ is lower alkyl, phenyl, benzyl or 2-phenylethyl and R₅ is lower alkylcarbonyl or benzylcarbonyl can be obtained. 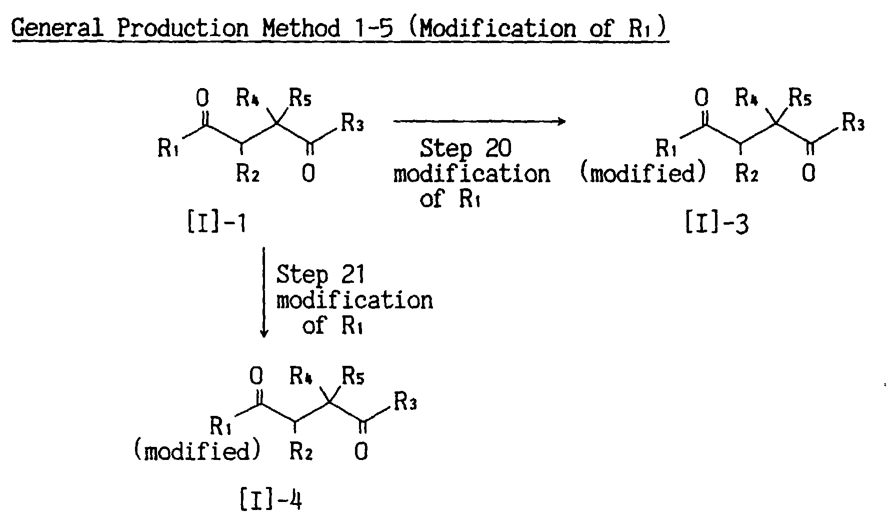 wherein R₁, R₂, R₃, R₄ and R₅ are as defined above.

### Step 20

When R₁ is halogen-substituted aryl, compound [I]-1 is reacted with vinyl tributyltin or isopropenyl tributyltin in a solvent such as various ethers (e.g., 1,4-dioxane, tetrahydrofuran and the like), benzene, toluene, N,N-dimethylformamide, acetonitrile and the like, or a mixed solvent thereof in the presence of phosphine palladium catalyst such as tetrakis(triphenylphosphine)palladium (O), trans-benzyl(chloro)bis(triphenylphosphine)palladium (II) and the like from under cooling to under heating, preferably from room temperature to under heating to give compound [I]-3 wherein R₁ is modified. Alternatively, in a solvent such as various ethers (e.g., ethyl ether, tetrahydrofuran and the like), benzene, toluene, n-hexane, cyclohexane and the like, or a mixed solvent thereof, the Grignard reagent can be obtained from magnesium and halogen-substituted lower alkylaryl such as (o-, m-, p-)chloro-substituted toluene, (o-, m-, p-)bromo-substituted toluene, (o-, m-, p-)chloro-substituted ethylbenzene, (o-, m-, p-)bromo-substituted ethylbenzene, (o-, m-, p-)chloro-substituted propylbenzene, (o-, m-, p-)bromo-substituted propylbenzene and the like or biphenyl halide such as 4-chlorobiphenyl, 4-bromobiphenyl, 3-chlorobiphenyl, 3-bromobiphenyl, 2-chlorobiphenyl, 2-bromobiphenyl and the like. Compound [I]-1 is reacted with the above-mentioned Grignard reagent in the presence of zinc halide such as zinc chloride, zinc bromide and the like, and phosphine palladium catalyst such as tetrakis(triphenylphosphine)palladium (O), trans-benzyl(chloro)bis(triphenylphosphine)palladium (II) and the like to give compound [I]-3 wherein R₁ is modified. The modified R₁ of the compound [I]-3 thus obtained in Step 20 is aryl substituted by lower alkenyl or aryl.

### Step 21

When R₁ is aryl substituted by a hydroxy lower alkyl such as hydroxymethyl, hydroxyethyl, hydroxypropyl, hydroxyisopropyl, hydroxybutyl, hydroxyisobutyl, hydroxy sec-butyl, hydroxy tert-butyl and the like:

Compound [I]-1 is added to a solvent such as dimethyl sulfoxide or a mixed solvent of dimethyl sulfoxide and methylene chloride, in the presence of sulfur trioxide-pyridine complex or oxalyl chloride and the like, and reacted with organic base such as triethylamine, N-methylmorpholine and the like from under cooling to under heating, preferably from under cooling to room temperature to give compound [I]-4 wherein R₁ is substituted by an acyl such as formyl, acetyl, propionyl, butyryl, valeryl and the like. Alternatively, the thus-obtained compound [I]-4 which is substituted by an acyl such as formyl, acetyl, propionyl, butyryl, valeryl and the like is reacted with hydrogen peroxide and oxidizing agent such as sodium chlorite, potassium chlorite and the like in water or aqueous acetonitrile in the presence of disodium hydrogenphosphate from under cooling to under heating, preferably from under cooling to room temperature to give compound [I]-4 wherein R₁ is modified. The modified R₁ of the compound [I]-4 thus obtained in Step 21 is aryl which is substituted by acyl, carboxy, carboxy lower alkyl such as carboxymethyl, carboxyethyl, carboxypropyl and the like. 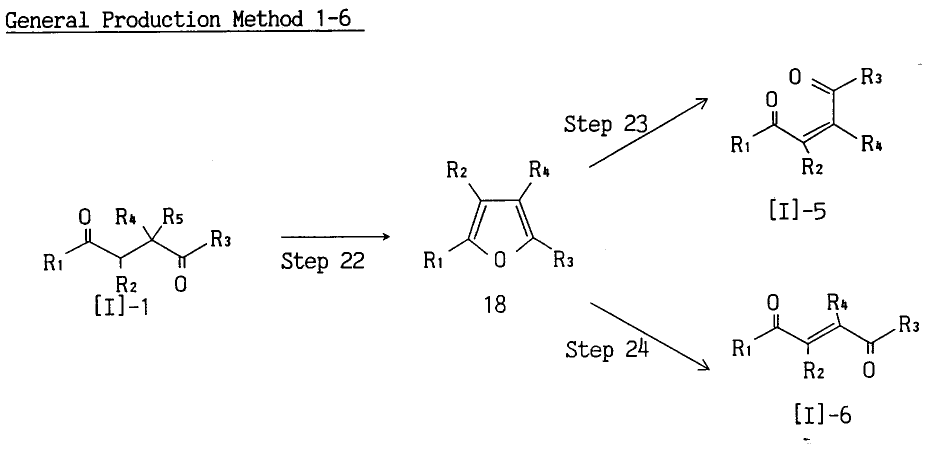 wherein R₁, R₂, R₃ and R₄ are as defined above, and R₅ is hydrogen atom.

### Step 22

The compound [I]-1 is converted to compound 18 in a solvent such as benzene, toluene, n-hexane, cyclohexane, methylene chloride, chloroform, N,N-dimethylformamide, dimethyl sulfoxide, water, various alcohols (e.g., methanol, ethanol and the like), acetic anhydride and the like, or a mixed solvent thereof or without solvent in the presence of inorganic acid such as hydrochloric acid, sulfuric acid, phosphoric acid, polyphosphoric acid, hydrobromic acid and the like, or organic acid such as p-toluenesulfonic acid, benzenesulfonic acid, trifluoroacetic acid, methanesulfonic acid, trifluoromethanesulfonic acid, oxalic acid and the like from under cooling to under heating, preferably from room temperature to under heating.

### Step 23

The compound 18 is reacted with organic peroxide such as m-chloroperbenzoic acid, peracetic acid and the like in a solvent such as benzene, toluene, n-hexane, cyclohexane, methylene chloride, tetracarbon chloride, chloroform and the like, or a mixed solvent thereof from under cooling to under heating, preferably from 0°C to room temperature to give compound [I]-5.

### Step 24

The compound 18 is reacted with halogen such as chlorine, bromine, iodine and the like in an aqueous solvent such as various alcohols (e.g., methanol, ethanol and the like), acetone, acetonitrile and the like, or a mixed solvent thereof from under cooling to under heating, preferably under cooling to give compound [I]-6.

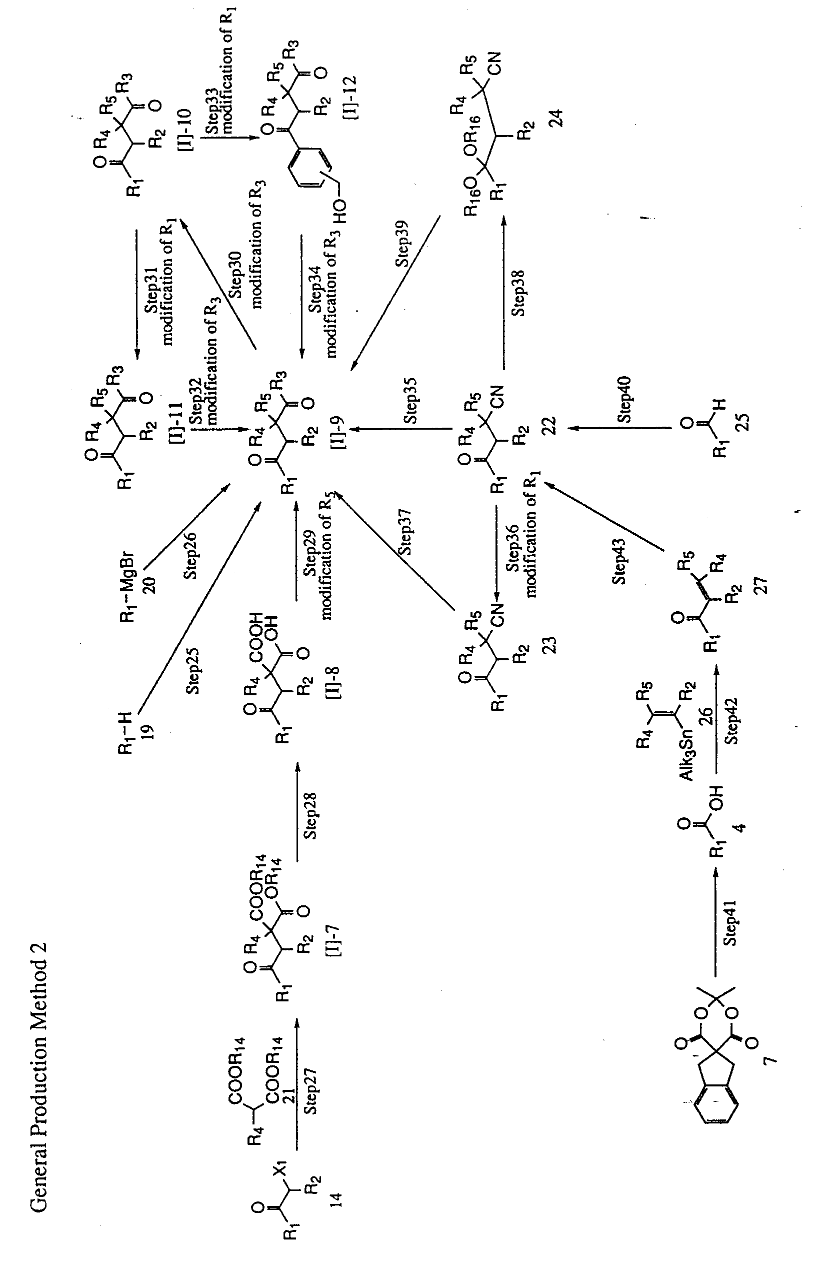

The reaction flow on the previous page schematically show the General Production Method 2, wherein R₁, R₂, R₃, R₄ and R₅ are conveniently used within the general sense of the symbols as used in the compound of the present invention. The same symbol generally shows the same meaning throughout all formulas, though there may occur cases where the range shown by each symbol differs depending on respective reactions from the corresponding symbol.

The General Production Method 2 is described in detail following each step. 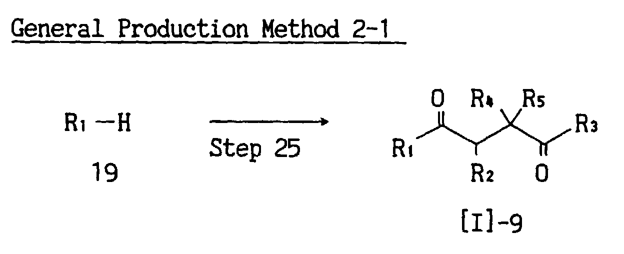 wherein R₁ is as defined above, R₂ and R₅ are each hydrogen atom, R₃ is hydroxy, and R₄ is hydrogen atom or lower alkyl such as methyl, ethyl, propyl and butyl.

### Step 25

The compound 19 is reacted with acid anhydride such as succinic anhydride, maleic anhydride, citraconic anhydride, pyrotartaric anhydride and the like in a solvent such as carbon disulfide, methylene chloride, chloroform, carbon tetrachloride, tetrachloroethane, nitrobenzene and the like, or a mixed solvent thereof, or without solvent in the presence of Lewis acid such as aluminium chloride, zinc chloride, boron trifluoride and the like from under cooling to under heating to give a certain compound [I]-9 wherein R₃ is hydroxy. 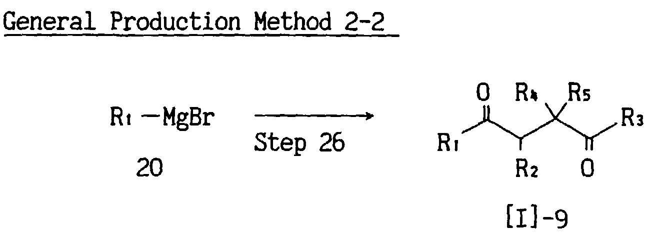 wherein R₁, R₂ and R₅ are as defined above, R₃ is hydroxy and R₄ is hydrogen atom or lower alkyl such as methyl, ethyl, propyl and butyl.

### Step 26

The compound 20 is reacted with succinic anhydride derivative such as succinic anhydride, maleic anhydride, citraconic anhydride, pyrotartaric anhydride, cis-cyclohexanedicarboxylic anhydride, cis-cyclopentanedicarboxylic anhydride and the like in a solvent such as various ethers (e.g., ethyl ether, 1,2-dimethoxyethane, 1,4-dioxane, tetrahydrofuran and the like), benzene, toluene, n-hexane, cyclohexane and the like, or a mixed solvent thereof from under cooling to under heating to give compound [I]-9. 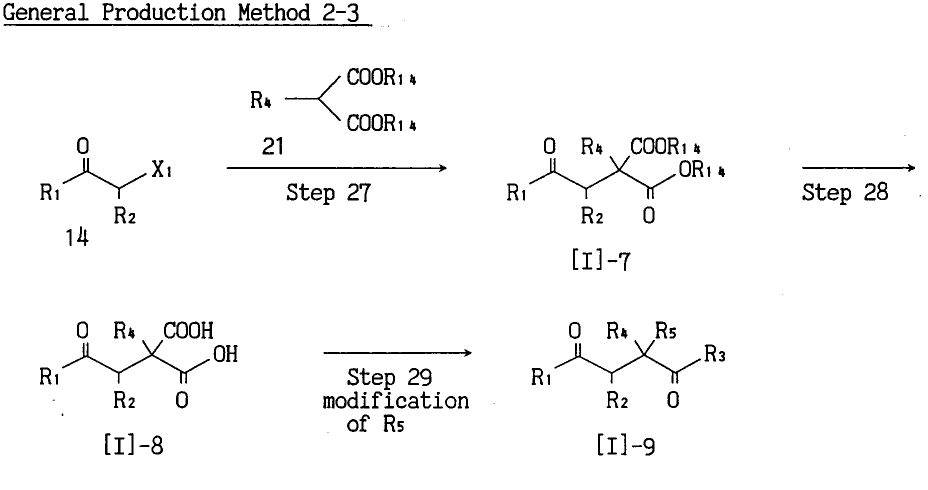 wherein R₁, R₂ and R₄ are as defined above, R₃ is hydroxy, R₅ is hydrogen atom and R₁₄ is lower alkyl such as methyl, ethyl, propyl, isopropyl, butyl, isobutyl, sec-butyl, tert-butyl and the like or benzyl and X₁ is halogen atom such as chlorine atom, bromine atom, iodine atom and the like.

### Step 27

The compound 14 is reacted with compound 21 in a solvent such as various ethers (e.g., ethyl ether, 1,4-dioxane, tetrahydrofuran and the like), benzene, toluene, n-hexane, cyclohexane, N,N-dimethylformamide, dimethyl sulfoxide, water and the like, or a mixed solvent thereof in the presence of a base such as lithium hydride, potassium hydride, sodium hydride, sodium methoxide, sodium ethoxide, lithium diisopropylamide, sodium carbonate, potassium carbonate and the like from under cooling to under heating, preferably from 0°C to room temperature to give compound [I]-7.

### Step 28

The compound [I]-7 is deprotected in water or alcohol solvent such as water and an alcohol such as methanol, ethanol, propanol, isopropanol and the like, or a mixed solvent of water and ethers such as 1,4-dioxane, tetrahydrofuran and the like, in the presence of a base such as lithium hydroxide, sodium hydroxide, potassium hydroxide, calcium hydroxide, barium hydroxide and the like from under cooling to under heating, preferably from 0°C to room temperature to give compound [I]-8.

### Step 29

The compound [I]-9 can be obtained by heating compound [I]-8. 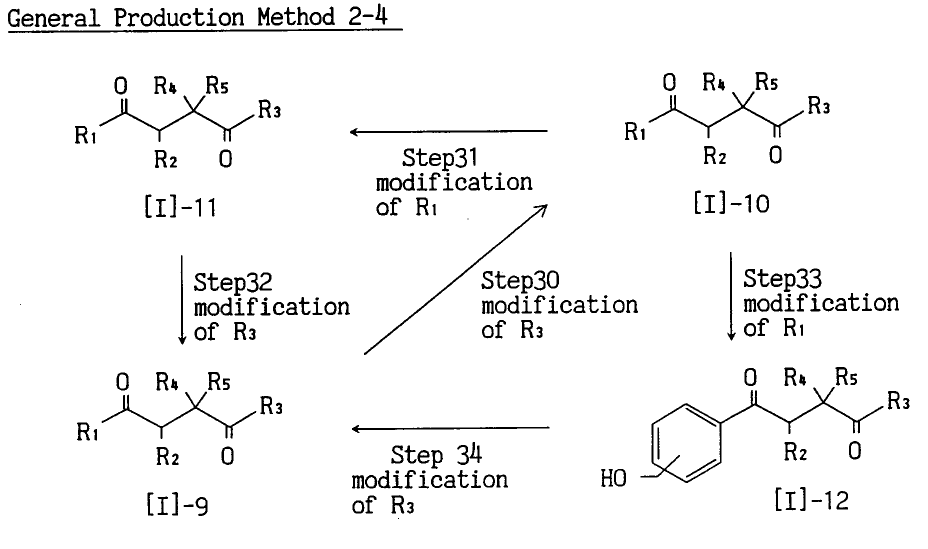 wherein R₁, R₂, R₃, R₄ and R₅ are as defined above, and modified in each step.

### Step 30

When R₃ of compound [I]-9 is hydroxy, compound [I]-9 is converted to compound [I]-10 in a solvent such as benzene, toluene, xylene and the like or a mixed solvent thereof or without solvent in the presence of a lower alcohol such as methanol, ethanol, propanol and the like and inorganic acid such as hydrochloric acid, sulfuric acid, hydrobromic acid and the like, or organic acid such as p-toluenesulfonic acid, benzenesulfonic acid, methanesulfonic acid, trifluoromethanesulfonic acid and the like from under cooling to under heating, preferably at refluxing temperature. In compound [I]-10, R₅ is hydrogen atom and R₃ is lower alkoxy such as methoxy, ethoxy, butoxy and the like.

### Step 31

When R₁ of compound [I]-10 is halogen-substituted aryl, compound [I]-10 is reacted with heterocycle-substituted boric acid such as pyridine-substituted boric acid, thiophene-substituted boric acid, pyperazine-substituted boric acid, benzothiophene-substituted boric acid, benzofuran-substituted boric acid and the like in a solvent such as various ethers (e.g., ethyl ether, tetrahydrofuran and the like), benzene, toluene, n-hexane, cyclohexane, N,N-dimethylformamide, dimethyl sulfoxide, water and the like, or a mixed solvent thereof in the presence of a base such as lithium carbonate, sodium carbonate, potassium carbonate, lithium hydroxide, sodium hydroxide, potassium hydroxide, sodium hydrogencarbonate and the like from under cooling to under heating, preferably from room temperature to refluxing temperature to give compound [I]-11 wherein R₁ is heterocycle-substituted aryl.

### Step 32

When R₃ of compound [I]-11 is lower alkoxy such as methoxy, ethoxy, butoxy and the like, compound [I]-11 is subjected to the same reactions as in Step 28 to give compound [I]-9 wherein R₃ is hydroxy.

### Step 33

When R₁ of compound [I]-10 is phenyl substituted by methyl, compound [I]-10 is reacted with N-halogenated succinimide such as N-chlorosuccinimide, N-bromosuccinimide, N-iodosuccinimide and the like by irradiation of light or addition of radical initiator such as 2,2'-azobis(isobutyronitrile), benzoyl peroxide and the like, from room temperature to refluxing temperature and then the obtained product is converted to compound [I]-12 in the presence of carbonate such as calcium carbonate, magnesium carbonate, barium carbonate and the like under heating in water or a mixed solvent of water and ether such as tetrahydrofuran, 1,4-dioxane and the like. In compound [I]-12, R₁ is phenyl substituted by hydroxymethyl.

### Step 34

When R₃ of compound [I]-12 is lower alkoxy such as methoxy, ethoxy, butoxy and the like, compound [I]-12 is subjected to the same reactions as in Step 28 to give compound [I]-9 wherein R₃ is hydroxy. 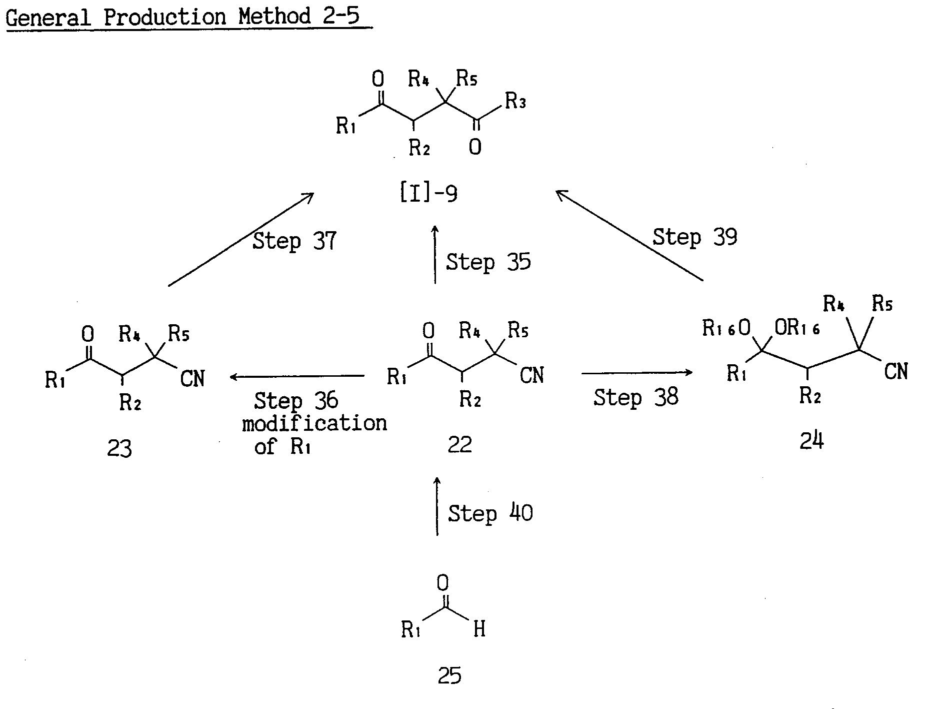 wherein R₁, R₂, R₄ and R₅ are as defined above, R₃ is hydroxy, R₁₆ is lower alkyl such as methyl, ethyl, propyl and the like, or two R₁₆ combinedly show lower alkylene such as ethylene and the like.

### Step 35

Compound 22 is hydrolyzed in a solvent such as water or in a mixed solvent of various alcohols such as methanol, ethanol, propanol and the like or various ethers such as 1,4-dioxane, tetrahydrofuran and the like and water in the presence of a metal hydroxide such as lithium hydroxide, sodium hydroxide, potassium hydroxide, barium hydroxide, calcium hydroxide and the like from under cooling to under heating, preferably from room temperature to under heating to give compound [I]-9. When Step 35 is used, R₃ of compound [I]-9 is hydroxy.

### Step 36

When R₁ of compound 22 is halogen-substituted aryl(phenyl), in a solvent such as various ethers (e.g., ethyl ether, tetrahydrofuran and the like), benzene, toluene, n-hexane, cyclohexane and the like, or a mixed solvent thereof, the Grignard reagent can be obtained from magnesium and halogen-substituted lower alkylaryl such as (o-, m-, p-)chloro-substituted toluene, (o-, m-, p-)bromo-substituted toluene, (o-, m-, p-)chloro-substituted ethylbenzene, (o-, m-, p-)bromo-substituted ethylbenzene, (o-, m-, p-)chloro-substituted propylbenzene, (o-, m-, p-)bromo-substituted propylbenzene and the like, bromo-substituted lower alkoxymethoxybenzene such as bromo-substituted methoxymethoxybenzene, bromo-substituted ethoxymethoxybenzene, bromo-substituted propoxymethoxybenzene, bromo-substituted trimethylsilyloxybenzene, bromo-substituted tert-butyldimethylsilyloxybenzene and the like. The compound 22 is reacted with the above-mentioned Grignard reagent in the presence of zinc halide such as zinc chloride, zinc bromide and the like, and phosphine palladium catalyst such as tetrakis(triphenylphosphine)palladium (O), transbenzyl(chloro)bis(triphenylphosphine)palladium (II) to give compound 23. When Step 36 is used, R₁ of compound 23 is lower alkyl such as methyl, ethyl, propyl and the like or aryl(biphenyl) substituted by lower alkyl-substituted silyloxy.

### Step 37

The compound 23 is hydrolyzed in a solvent such as water or in a mixed solvent of various alcohols such as methanol, ethanol, propanol and the like or various ethers such as 1,4-dioxane, tetrahydrofuran and the like and water in the presence of a metal hydroxide such as lithium hydroxide, sodium hydroxide, potassium hydroxide, barium hydroxide, calcium hydroxide and the like from under cooling to under heating, preferably from room temperature to under heating to give compound [I]-9. When R₁ has a phenolic hydroxyl group having protecting group, the above-mentioned product is deprotected in the presence of an inorganic acid such as hydrochloric acid, sulfuric acid, hydrobromic acid and the like or an organic acid such as trifluoroacetic acid, formic acid, acetic acid, trifluoromethanesulfonic acid, p-toluenesulfonic acid, methanesulfonic acid, benzenesulfonic acid and the like in water or a mixed solvent of tetrahydrofuran, 1,4-dioxane, ethanol, chloroform or methylene chloride with water from under cooling to under heating, preferably from roam temperature to under heating to give compound [I]-9. When Step 37 is used, R₃ of compound [I]-9 is hydroxy.

### Step 38

The compound 22 is converted to compound 24 in a solvent such as benzene, toluene, xylene, methylene chloride, chloroform, 1,2-dichloroethane and the like or a mixed solvent thereof or without solvent in the presence of alcohol such as methanol, ethanol, propanol, ethylene glycol and the like and inorganic acid such as hydrochloric acid, sulfuric acid, hydrobromic acid and the like or organic acid such as p-toluenesulfonic acid, benzenesulfonic acid, methanesulfonic acid, trifluoromethanesulfonic acid and the like from under cooling to under heating, preferably at refluxing temperature.

### Step 39

The compound 24 is hydrolyzed in a solvent such as water and various alcohols such as methanol, ethanol and the like or a mixed solvent thereof in the presence of a metal hydroxide such as lithium hydroxide, sodium hydroxide, potassium hydroxide, barium hydroxide, calcium hydroxide and the like, and reacted from under cooling to under heating, preferably from room temperature to under heating, and then the resulting product is converted to compound [I]-9 in the presence of an inorganic acid such as hydrochloric acid, sulfuric acid, hydrobromic acid and the like or an organic acid such as p-toluenesulfonic acid, benzenesulfonic acid, trifluoroacetic acid, methanesulfonic acid, trifluoromethanesulfonic acid and the like, from under cooling to under heating, preferably from 0°C to room temperature. When Step 39 is used, R₃ of compound [I]-9 is hydroxy.

### Step 40

The compound 25 is reacted with acrylonitrile in a solvent such as N,N-dimethylformamide, 1,4-dioxane, various alcohols (e.g., methanol, ethanol and the like) and the like or a mixed solvent thereof in the presence of quaternary thiazolium salts such as 3-benzyl-5-(2-hydroxyethyl)-4-methyl-1,3-thiazolium chloride, 3-ethyl-5-(2-hydroxyethyl)-4-methyl-1,3-thiazolium bromide, 5-(2-hydroxyethyl)-3,4-dimethylthiazolium iodide and the like and an organic base such as triethylamine, N-methylmorpholine and the like or cyanide such as sodium cyanide, potassium cyanide and the like and reacted from under cooling to under heating, preferably from room temperature to under heating to give compound 22. When Step 40 is used, R₂, R₄ and R₅ of compound 22 are each hydrogen atom. 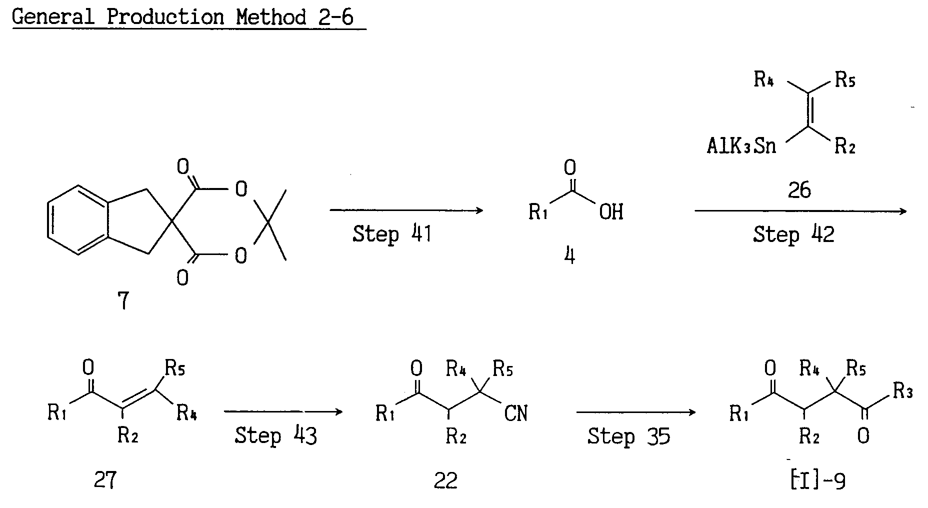 wherein R₁ is indanyl, R₂, R₄ and R₅ are as defined above, R₃ is hydroxy, and Alk is lower alkyl such as methyl, ethyl, propyl, isopropyl, butyl, isobutyl, sec-butyl, tert-butyl and the like.

### Step 41

The compound 7 is converted to dicarboxylic acid in a solvent such as various ethers such as ethyl ether, 1,4-dioxane, tetrahydrofuran and the like or a mixed solvent thereof in the presence of water and an inorganic acid such as hydrochloric acid, sulfuric acid, hydrobromic acid and the like, or an organic acid such as p-toluenesulfonic acid, benzenesulfonic acid, methanesulfonic acid, trifluoromethanesulfonic acid and the like from under cooling to under heating, preferably at refluxing temperature, and successively the above dicarboxylic acid is decarboxylated by heating to give compound 4 wherein R₁ is indanyl.

### Step 42

The compound 4 is reacted with thionyl chloride, oxalyl chloride, oxalyl bromide and the like in a solvent such as benzene, toluene, n-hexane, cyclohexane, methylene chloride, chloroform and the like, or a mixed solvent thereof, or without solvent from under cooling to under heating, preferably from room temperature to under heating (this reaction often produces preferable results when dimethylformamide and the like is added), and then, the above-mentioned resulting product is reacted with compound 26 in a solvent such as various ethers (e.g., ethyl ether, tetrahydrofuran and the like), benzene, toluene, n-hexane, cyclohexane, N,N-dimethylformamide and the like, or a mixed solvent thereof in the presence of phosphine palladium catalyst such as tetrakis(triphenylphosphine)palladium (O), transbenzyl(chloro)bis(triphenylphosphine)palladium (II) and the like from under cooling to under heating, preferably from room temperature to under heating to give compound 27 wherein R₁ is indanyl.

### Step 43

The compound 27 is reacted with acetic acid and cyanide such as lithium cyanide, sodium cyanide, potassium cyanide and the like in a solvent such as N,N-dimethylformamide, dimethyl sulfoxide, water, various alcohols (e.g., methanol, ethanol and the like) and the like or a mixed solvent thereof from under cooling to under heating, preferably from room temperature to under heating to give compound 22 wherein R₁ is indanyl.

By reacting the compound 22 according to Step 35, compound [I]-9 wherein R₁ is indanyl can be obtained.

When Step 43 is used, R₃ of compound [I]-9 is hydroxy. 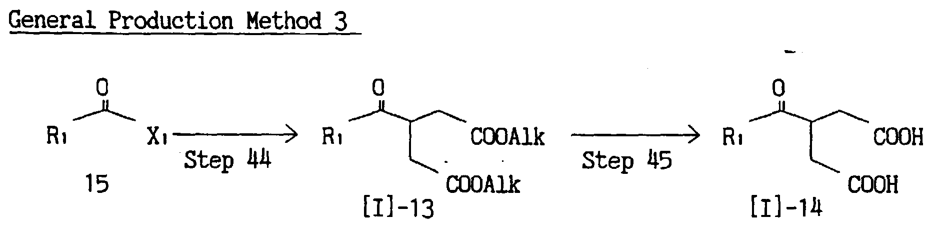 wherein R₁ is as defined above, X₁ is halogen atom such as chlorine atom, bromine atom, iodine atom and the like and Alk is lower alkyl such as methyl, ethyl, propyl, isopropyl, butyl, isobutyl, sec-butyl, tert-butyl and the like.

### Step 44

The compound 15 is reacted with monobenzyl malonate or mono-tert-butyl malonate in a solvent such as various ethers (e.g., ethyl ether, 1,4-dioxane, tetrahydrofuran and the like), benzene, toluene, n-hexane, cyclohexane, N,N-dimethylformamide and the like, or a mixed solvent thereof in the presence of a base such as lithium hydride, sodium hydride, potassium hydride, sodium methoxide, sodium ethoxide, lithium diisopropylamide, butyllithium and the like from under cooling to under heating, preferably under cooling, and then the resulting product is reacted with a halogenated lower alkyl acetate such as methyl chloroacetate, ethyl chloroacetate, propyl chloroacetate, methyl bromoacetate, ethyl bromoacetate, propyl bromoacetate and the like in a solvent such as various ethers (e.g., ethyl ether, 1,4-dioxane, tetrahydrofuran and the like), benzene, toluene, n-hexane, cyclohexane, N,N-dimethylformamide and the like, or a mixed solvent thereof in the presence of a base such as lithium hydride, sodium hydride, potassium hydride, sodium methoxide, sodium ethoxide, lithium diisopropylamide, butyllithium and the like from under cooling to under heating, preferably under cooling. When the obtained product has a benzyl group, the compound is reacted with hydrogen in a solvent such as ethyl acetate, methanol, ethanol, 1,4-dioxane, tetrahydrofuran and the like or a mixed solvent thereof in the presence of palladium catalyst such as palladium-carbon, palladium-black and the like under a hydrogen atmosphere from under cooling to under heating, preferably from room temperature to under heating to give compound [I]-13. When the obtained product has tert-butyl group, the compound is converted to compound [I]-13 in a solvent such as benzene, toluene and the like or a mixed solvent thereof in the presence or absence of an organic acid such as acetic acid, formic acid, p-toluenesulfonic acid and the like from under cooling to under heating, preferably from room temperature to under heating.

### Step 45

The compound [I]-13 is hydrolyzed in a solvent such as water or a mixed solvent of 1,4-dioxane or various alcohols such as methanol, ethanol and the like and water in the presence of a base such as lithium hydroxide, sodium hydroxide, potassium hydroxide, magnesium hydroxide, calcium hydroxide, barium hydroxide and the like from under cooling to under heating, preferably at room temperature to give compound [I]-14. 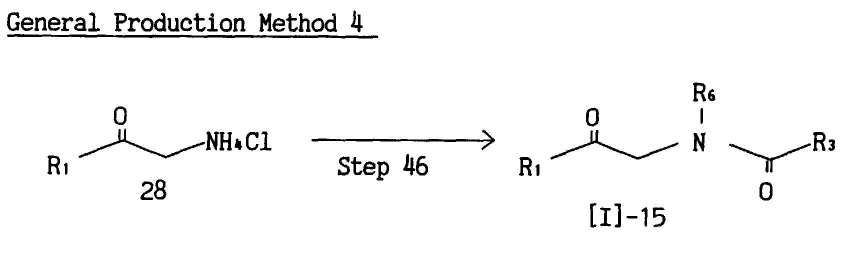 wherein R₁, R₃ and R₆ are as defined above.

### Step 46

The compound 28 is reacted with acyl halide or carboxy anhydride in a solvent such as ethers (e.g., ethyl ether, 1,4-dioxane, tetrahydrofuran and the like), benzene, toluene, n-hexane, cyclohexane, methylene chloride, chloroform, tetracarbon chloride, N,N-dimethylformamide, dimethyl sulfoxide and the like, or a mixed solvent thereof, or without solvent, in the presence of an organic base such as pyridine, triethylamine, N-methylmorpholine and the like from under cooling to under heating, preferably under heating to give compound [I]-15. 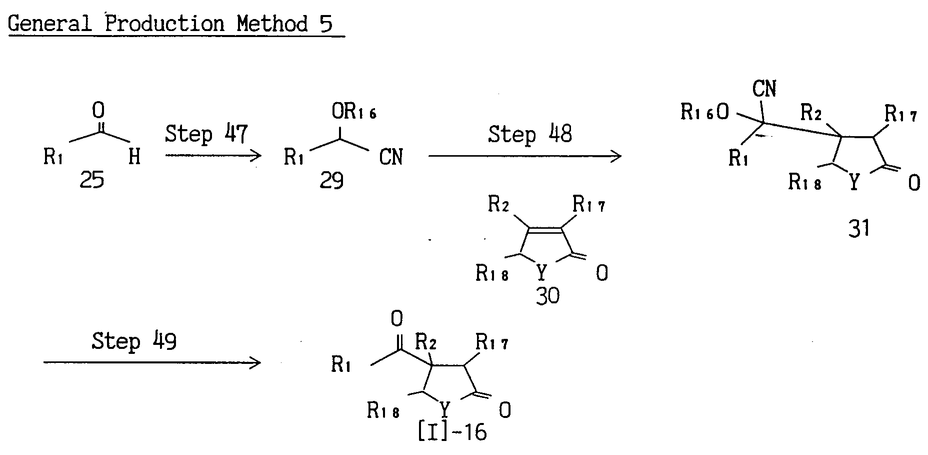 wherein R₁ and R₂ are as defined above, R₁₆ is a protecting group such as substituted silyl such as trimethylsilyl, tert-butyldimethylsilyl and the like, lower alkoxyalkyl such as methoxymethyl, methoxyethyl, ethoxymethyl, ethoxyethyl and the like, R₁₇ and R₁₈ are the same or different and each is hydrogen atom, lower alkyl such as methyl, ethyl and the like, lower alkoxy such as methoxy, ethoxy and the like, lower acyloxy such as acetyloxy, propionyloxy and the like, lower alkoxycarbonyl such as methoxycarbonyl, ethoxycarbonyl, propoxycarbonyl and the like and Y is carbon atom, oxygen atom or nitrogen atom.

### Step 47

When R₁₆ is substituted silyl such as trimethylsilyl, tert-butyldimethylsilyl and the like:

The compound 25 is reacted with substituted silylnitrile having substituted silyl such as trimethylsilyl, tert-butyldimethylsilyl and the like, in a solvent such as various ethers (e.g., ethyl ether, 1,4-dioxane, tetrahydrofuran and the like), benzene, toluene, n-hexane, cyclohexane, methylene chloride, chloroform and the like from under ice-cooling to heating, preferably from under ice-cooling to room temperature to give compound 29. This reaction often produces preferable results when an organic base such as triethylamine, N-methylmorpholine, pyridine, 2,6-ruthidine and the like is added.

When R₁₆ is a lower alkoxyalkyl such as methoxymethyl, ethoxymethyl and the like:

The compound 25 is reacted with cyanide such as sodium cyanide, potassium cyanide, silver cyanide and the like in the presence of chloromethyl methyl ether, bromomethyl methyl ether, chloromethyl ethyl ether and the like, in a solvent such as various alcohols (e.g., methanol, ethanol and the like), N,N-dimethylformamide, dimethyl sulfoxide, 1,4-dioxane, water and the like, or a mixed solvent thereof from under ice-cooling to heating, preferably from under ice-cooling to room temperature to give compound 29.

### Step 48

The compound 29 is reacted with compound 30 in the presence of a base such as lithium diisoproamide, sodium hexamethyldisilazide, sodium methoxide, potassium methoxide, sodium tert-butoxide, potassium tert-butoxide, triethylamine and the like under cooling, preferably from -80°C to under ice-cooling to give compound 31.

### Step 49

The compound 31 is converted to compound [I]-16 in a solvent such as various ethers (e.g., ethyl ether, 1,4-dioxane, tetrahydrofuran and the like), benzene, toluene, n-hexane, cyclohexane, methylene chloride, chloroform, N,N-dimethylformamide, dimethyl sulfoxide, water, various alcohols (e.g., methanol, ethanol and the like), or a mixed solvent thereof from under ice-cooling to heating, preferably from under ice-cooling to room temperature. When R₁₆ is substituted silyl such as trimethylsilyl, tert-butyldimethylsilyl and the like, the addition of fluorine compound such as tetrabutylammonium fluoride, ammonium fluoride, hydrogen fluoride-pyridine, silver fluoride and the like often produces preferable results, and when R₁₆ is a lower alkoxyalkyl such as methoxymethyl, methoxyethyl, ethoxymethyl, ethoxyethyl and the like, addition of inorganic acid such as hydrochloric acid, sulfuric acid, nitric acid and the like to the solvent of water, various alcohols (e.g., methanol, ethanol and the like) and the like often produces preferable results. 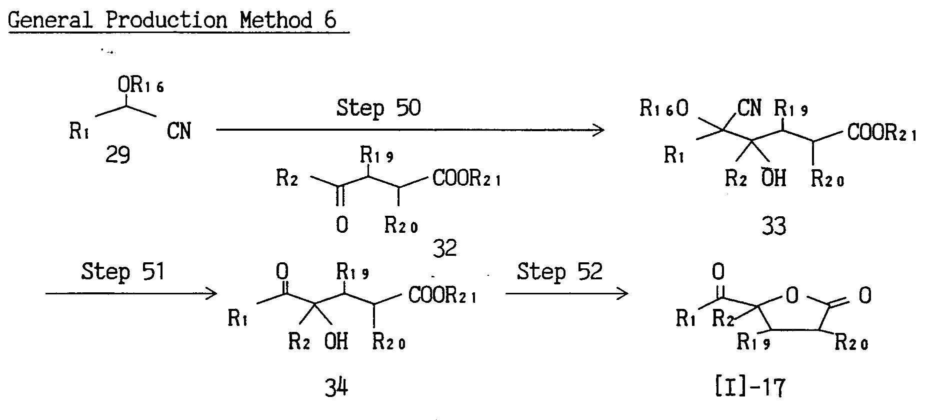 wherein R₁ and R₂ are as defined above, R₁₆ is a protecting group such as substituted silyl such as trimethylsilyl, tert-butyldimethylsilyl and the like, lower alkoxyalkyl such as methoxymethyl, methoxyethyl, ethoxymethyl, ethoxyethyl and the like, R₁₉ and R₂₀ are the same or different and each is hydrogen atom, lower alkyl such as methyl, ethyl and the like, lower alkoxy such as methoxy, ethoxy and the like, lower acyloxy such as acetyloxy, propionyloxy and the like, lower alkoxycarbonyl such as methoxycarbonyl, ethoxycarbonyl, propoxycarbonyl and the like, and R₂₁ is hydrogen atom or lower alkyl such as methyl, ethyl and the like.

### Step 50

The compound 33 can be obtained by subjecting compound 29 and compound 32 to the same reaction as in Step 48.

### Step 51

The compound 34 can be obtained by subjecting compound 33 to the same reaction as in Step 49.

### Step 52

The compound 34 is converted to compound [I]-17 in an inorganic acid such as hydrochloric acid, sulfuric acid, nitric acid, hydrobromic acid and the like or an organic acid such as formic acid, acetic acid, trifluoroacetic acid, propionic acid and the like from 0°C to refluxing temperature. Alternatively, compound 34 is converted to compound [I]-17 in a solvent such as various ethers (e.g., ethyl ether, 1,4-dioxane, tetrahydrofuran and the like), benzene, toluene, n-hexane, cyclohexane, methylene chloride, chloroform, N,N-dimethylformamide, dimethyl sulfoxide, water, various alcohols (e.g., methanol, ethanol and the like) and the like, or a mixed solvent thereof in the presence of an organic acid such as p-toluenesulfonic acid if necessary. 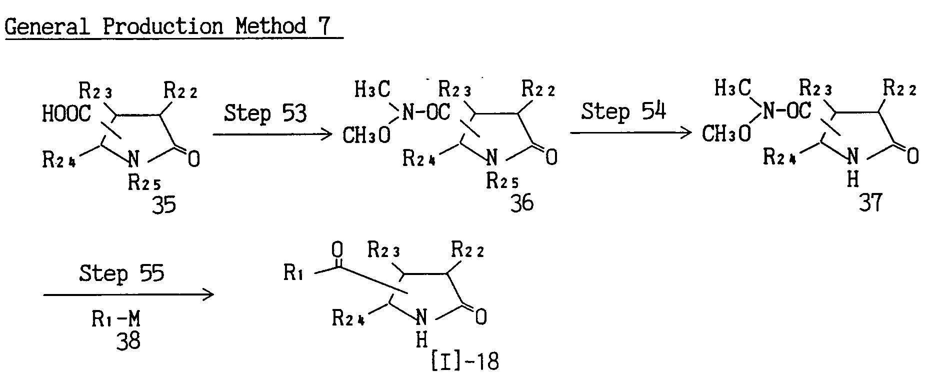 wherein R₁ is as defined above, R₂₂, R₂₃ and R₂₄ are the same or different and each is hydrogen atom, lower alkyl such as methyl, ethyl and the like, or lower alkoxy such as methoxy, ethoxy and the like, R₂₅ is amino-protecting group such as carbobenzoxy, carbo-tert-butoxy and the like, and M is lithium or magnesium halide.

### Step 53

The compound 35 is reacted with N,O-dimethylhydroxyamine in a solvent such as various ethers (e.g., ethyl ether, 1,4-dioxane, tetrahydrofuran and the like), benzene, toluene, n-hexane, cyclohexane, methylene chloride, chloroform, N,N-dimethylformamide, dimethyl sulfoxide and the like, or a mixed solvent thereof in the presence of a dicyclohexylcarbodiimide, 1-(3-dimethylaminopropylyl)-3-ethylcarbodiimide or a salt thereof from under ice-cooling to under heating, preferably from under ice-cooling to room temperature to give compound 36. The addition of 1-hydroxybenzotriazole, N,N-dimethylaminopyridine and the like often produces preferable results.

### Step 54

The compound 36 is reacted with hydrogen in a solvent such as various ethers (e.g., 1,4-dioxane, tetrahydrofuran and the like), water, various alcohols (e.g., methanol, ethanol and the like) and the like, or a mixed solvent thereof in the presence of a palladium catalyst such as palladium-carbon, palladium black and the like under a hydrogen atmosphere at room temperature to give compound 37.

### Step 55

The compound 37 is reacted with compound 38 in a solvent such as various ethers (e.g., ethyl ether, 1,4-dioxane, tetrahydrofuran and the like), benzene, toluene, n-hexane and the like, or a mixed solvent thereof from under cooling to under heating, preferably from -80°C to under ice-cooling to give compound [I]-18. 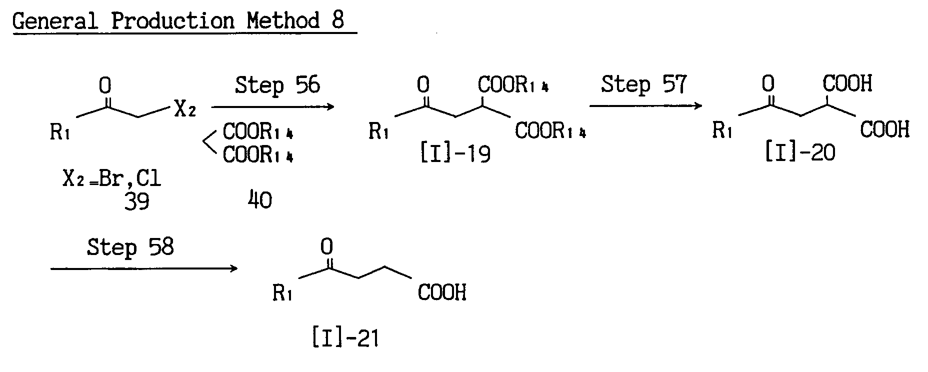 wherein R₁ is as defined above, R₁₄ is a lower alkyl such as methyl, ethyl, propyl, isopropyl, butyl, isobutyl, sec-butyl, tert-butyl and the like or benzyl, and X₂ is Br or Cl.

### Step 56

The compound 39 is reacted with compound 40 in a solvent such as various ethers (e.g., ethyl ether, 1,4-dioxane, tetrahydrofuran and the like), benzene, toluene, n-hexane, cyclohexane, N,N-dimethylformamide, dimethyl sulfoxide and the like, or a mixed solvent thereof in the presence of a base such as lithium hydride, sodium hydride, sodium methoxide, sodium ethoxide, lithium diisopropylamide, sodium carbonate, potassium carbonate and the like from under cooling to under heating to give compound [I]-19.

### Step 57

The compound [I]-19 is deprotected in water or a mixed solvent of water and alcohols (e.g., methanol, ethanol and the like) or cyclic ethers (e.g., 1,4-dioxane, tetrahydrofuran and the like) in the presence of alkali such as lithium hydroxide, sodium hydroxide, sodium hydride and the like from under cooling to under heating to give compound [I]-20.

### Step 58

The compound [I]-21 can be obtained by heating compound [I]-20 for decarboxylation. 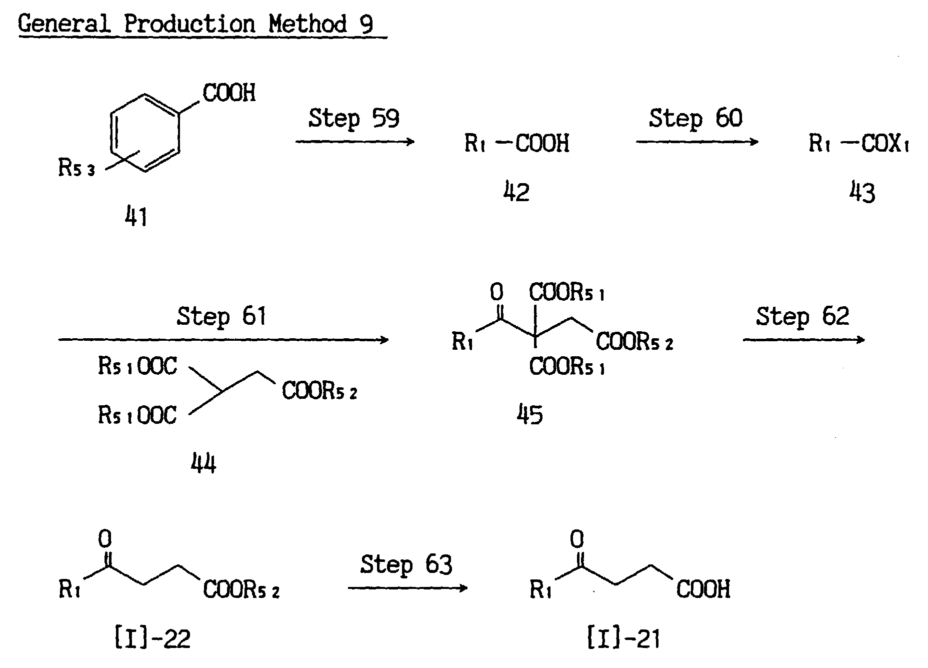 wherein R₁ is cyclohexyl substituted by a lower alkyl such as methyl, ethyl, propyl, butyl and the like, R₅₁ is tert-butyl, R₅₂ is a linear chain lower alkyl such as methyl, ethyl, propyl and the like, R₅₃ is lower alkyl, and X₁ is halogen atom such as chlorine atom, bromine atom, iodine atom and the like.

### Step 59

The compound 41 is reacted with hydrogen in a solvent such as alcohols (e.g., methanol, ethanol and the like), ethyl acetate, acetic acid, 1,4-dioxane and the like using a platinum catalyst such as platinum-carbon, platinum-alumina, platinum black, platinum oxide and the like under 1-5 atm under a hydrogen atmosphere from room temperature to under heating to give compound 42.

### Step 60

The compound 42 is reacted with oxalyl chloride, oxalyl bromide, thionyl chloride and the like in a solvent such as benzene, toluene, n-hexane, cyclohexane, methylene chloride, chloroform and the like, or a mixed solvent thereof, or without solvent from under cooling to under heating, preferably from 0°C to under heating to give compound 43. This reaction often produces preferable results when dimethylformamide and the like is added.

### Step 61

The compound 43 is reacted with compound 44 in a solvent such as ethers (e.g., ethyl ether, 1,4-dioxane, tetrahydrofuran and the like), benzene, toluene, n-hexane, cyclohexane, N,N-dimethylformamide, dimethyl sulfoxide and the like, or a mixed solvent thereof, in the presence of a base such as lithium hydride, sodium hydride, sodium methoxide, sodium ethoxide, lithium diisopropylamide, sodium carbonate, potassium carbonate and the like from under cooling to under heating to give compound 45.

### Step 62

The compound 45 is deprotected and decarboxylated in a solvent such as benzene, toluene, n-hexane, cyclohexane, methylene chloride, chloroform and the like, or a mixed solvent thereof, or without solvent in the presence of acid catalyst such as formic acid, trifluoroacetic acid, p-toluenesulfonic acid and the like from under cooling to under heating, preferably from room temperature to under heating to give compound [I]-22.

### Step 63

The compound [I]-22 is hydrolyzed in water or a mixed solvent of water and alcohols (e.g., methanol, ethanol and the like) or cyclic ethers (e.g., 1,4-dioxane, tetrahydrofuran and the like) in the presence of alkali such as lithium hydroxide, potassium hydroxide, sodium hydride and the like from under cooling to under heating to give compound [I]-21. 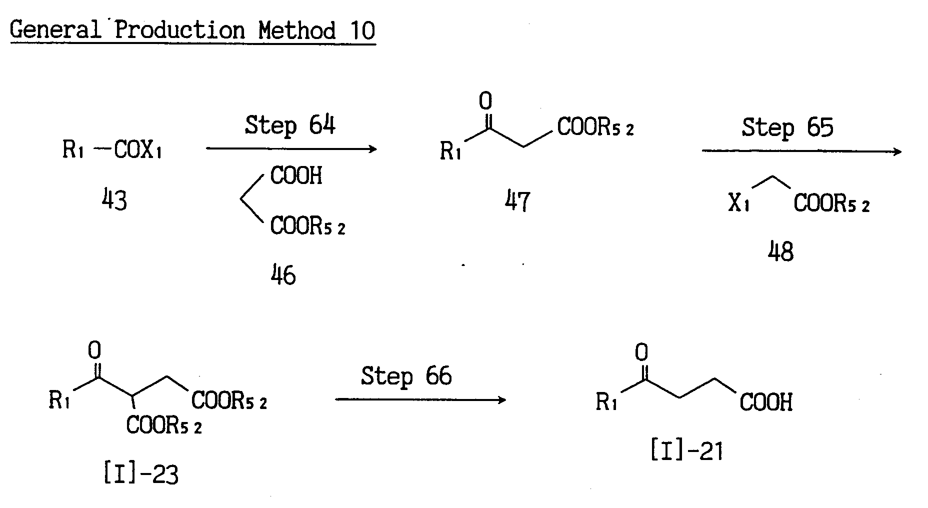 wherein R₁ is as defined above, R₅₂ is a lower alkyl such as methyl, ethyl, propyl and the like, and X₁ is halogen atom such as chlorine atom, bromine atom, iodine atom and the like.

### Step 64

The compound 43 is reacted with compound 46 in a solvent such as ethers (e.g., ethyl ether, 1,4-dioxane, tetrahydrofuran and the like), benzene, toluene, n-hexane, cyclohexane and the like, or a mixed solvent thereof, in the presence of a base such as n-butyllithium, lithium diisopropylamide and the like from under cooling to room temperature to give compound 47.

### Step 65

The compound 47 is reacted with compound 48 in the presence of sodium alkoxide such as sodium methoxide, sodium ethoxide and the like (the alkyl group of R₅₂, alcohol and alkoxide are preferably the same) in a solvent such as alcohols (e.g., methanol, ethanol and the like), benzene, toluene, n-hexane, cyclohexane and the like, or a mixed solvent thereof, from under cooling to under heating, preferably with refluxing under heating to give compound [I]-23.

### Step 66

The compound [I]-23 is hydrolyzed in water or a mixed solvent of water and alcohols (e.g., methanol, ethanol and the like) or cyclic ethers (e.g., 1,4-dioxane, tetrahydrofuran and the like) in the presence of alkali such as lithium hydroxide, potassium hydroxide, sodium hydride and the like from under cooling to under heating, and then was decarboxylated in a solvent such as benzene, toluene, xylene and the like, or a mixed solvent thereof, or without solvent, in the presence or absence of acid catalyst such as formic acid, trifluoroacetic acid, p-toluenesulfonic acid and the like from under cooling to under heating, preferably from room temperature to under heating to give compound [I]-21. 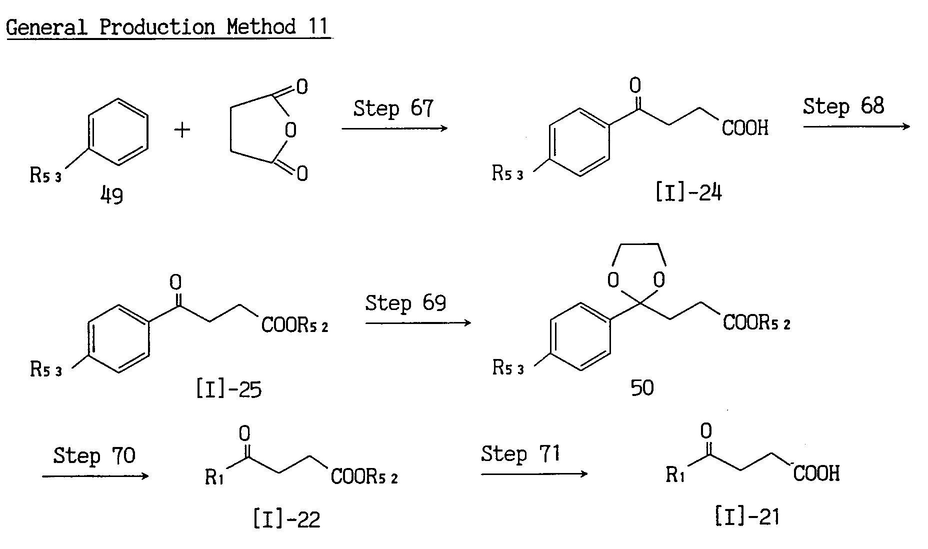 wherein R₁ is a cycloalkyl substituted by lower alkyl such as methyl, ethyl, propyl, butyl and the like, R₅₂ is determined according to the alcohol to be used, and R₅₃ is hydrogen atom or lower alkyl such as methyl, ethyl, propyl, isopropyl and the like.

### Step 67

The compound 49 is reacted with succinic anhydride in a solvent such as carbon disulfide, methylene chloride, chloroform, carbon tetrachloride, tetrachloroethane, nitrobenzene and the like, or a mixed solvent thereof, or without solvent in the presence of Lewis acid such as aluminium chloride, zinc chloride, boron trifluoride and the like from under cooling to under heating to give compound [I]-24.

### Step 68

The compound [I]-24 is esterified in a solvent of alcohols (e.g., methanol, ethanol, propanol and the like) in the presence of inorganic acid such as hydrogen chloride, sulfuric acid and the like or acid catalyst such as p-toluenesulfonic acid, benzenesulfonic acid, methanesulfonic acid and the like from under cooling to under heating, preferably from room temperature to refluxing temperature to give compound [I]-25.

### Step 69

The compound [I]-25 is reacted with ethylene glycol in the presence of pyridinium p-toluenesulfonate and orthoformic triester such as trimethyl orthoformate and triethyl orthoformate from room temperature to under heating, preferably under heating while removing the generated alcohol to give compound 50.

### Step 70

The compound 50 is reacted with a hydrogen in a solvent such as alcohols (e.g., methanol, ethanol, propanol and the like), ethyl acetate, acetic acid and the like, or a mixed solvent thereof, in the presence of platinum catalyst such as platinum-carbon, platinum-alumina, platinum black, platinum oxide and the like or rhodium catalyst such as rhodium-carbon, rhodium-alumina and the like under 1-5 atm under a hydrogen atmosphere from room temperature to under heating, and then converted to compound [I]-22 in acetic acid from room temperature to under heating, preferably under heating.

### Step 71

The compound [I]-22 is converted to compound [I]-21 in water or a mixed solvent of water and alcohols (e.g., methanol, ethanol, propanol and the like) or cyclic ethers (e.g., 1,4-dioxane, tetrahydrofuran and the like) in the presence of alkali such as lithium hydroxide, potassium hydroxide, sodium hydride and the like from under cooling to under heating. 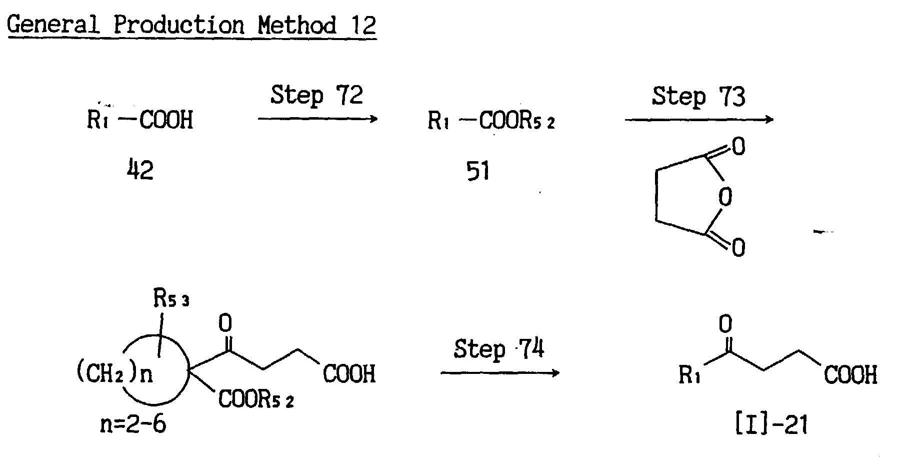 wherein R₁ is a cycloalkyl substituted by lower alkyl such as methyl, ethyl, propyl, butyl and the like, R₅₂ is determined according to the alcohol to be used, R₅₃ is lower alkyl and n is an integer of 2 to 6.

### Step 72

The compound 42 is converted to compound 51 in a solvent of alcohols (e.g., methanol, ethanol, propanol and the like) in the presence of inorganic acid such as hydrogen chloride, sulfuric acid and the like or acid catalyst such as p-toluenesulfonic acid, benzenesulfonic acid, methanesulfonic acid and the like from under cooling to under heating, preferably from room temperature to refluxing temperature.

### Step 73

The compound 51 is reacted with succinic anhydride in a solvent such as ethers (e.g., ethyl ether, 1,4-dioxane, tetrahydrofuran and the like), benzene, toluene, n-hexane, cyclohexane and the like, or a mixed solvent thereof, in the presence of a base such as n-butyllithium, lithium diisopropylamide, sodium hydroxide and the like from under cooling to under heating to give compound [I]-26.

### Step 74

The compound [I]-26 is converted to compound [I]-21 in water or a mixed solvent of water and alcohols (e.g., methanol, ethanol, propanol and the like) in the presence of alkali such as lithium hydroxide, potassium hydroxide, sodium hydride and the like from room temperature to under heating. 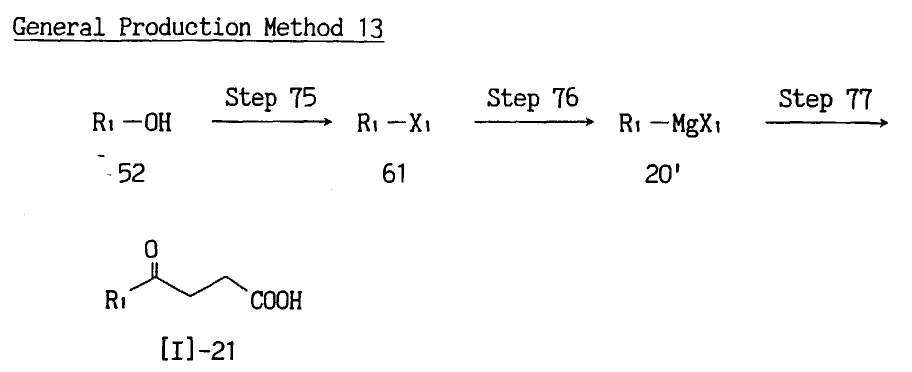 wherein R₁ is as defined above, and X₁ is halogen atom such as chlorine atom, bromine atom, iodine atom and the like.

### Step 75

The compound 52 is reacted with thionyl chloride, phosphorus trichloride, phosphorus pentachloride, hydrogen chloride gas, phosphorus oxychloride and the like in a solvent such as hexane, cyclohexane, methylene chloride, chloroform, carbon tetrachloride and the like, or without solvent from under cooling to under heating, preferably under cooling to give compound 61.

### Step 76

The compound 61 is reacted with magnesium in a solvent such as ethers (e.g., ethyl ether, tetrahydrofuran and the like) in a nitrogen stream from room temperature to under heating to give compound 20'. The addition of iodine at the beginning of the reaction may increase the yield.

### Step 77

The compound 20' is reacted with succinic anhydride in a solvent such as ethers (e.g., ethyl ether, tetrahydrofuran and the like) from under cooling to under heating, preferably under cooling, to give compound [I]-21. The addition of iron (III)-acetylacetonate as a catalyst may increase the yield. 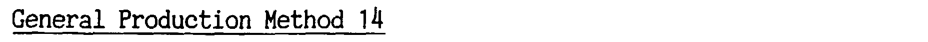 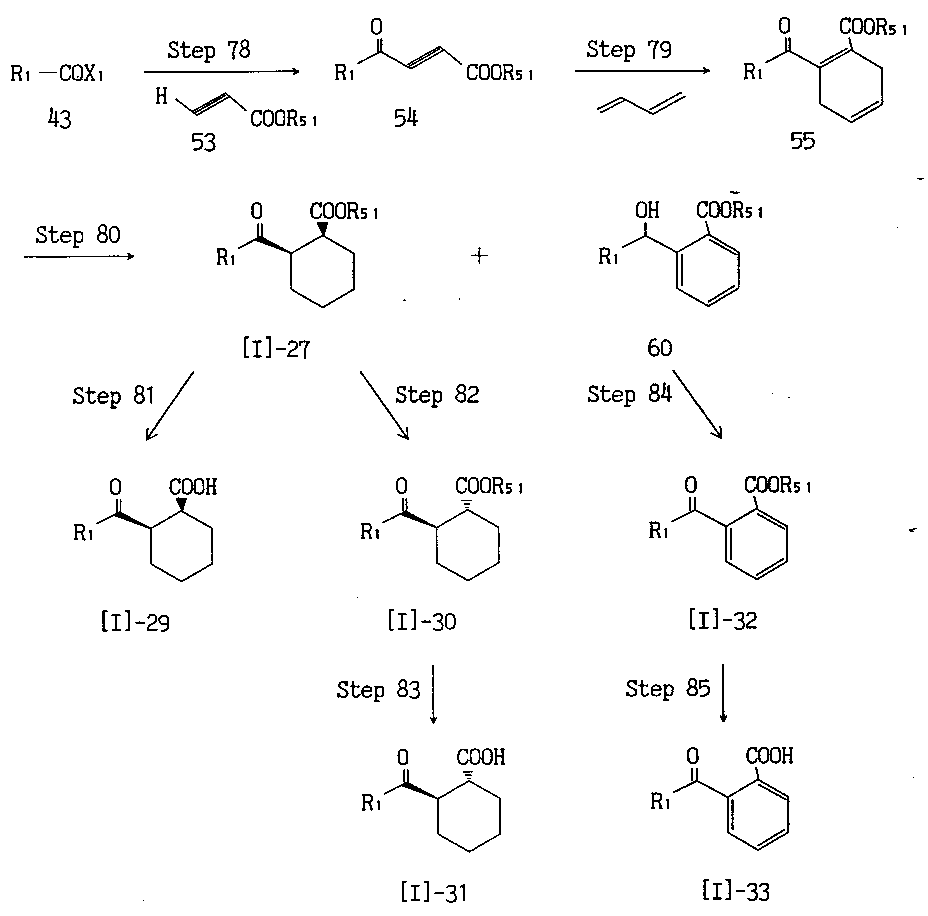 wherein R₁ is as defined above, R₅₁ is tert-butyl, and X₁ is halogen atom such as chlorine atom, bromine atom, iodine atom and the like.

### Step 78

The compound 43 is reacted with compound 53 in a solvent such as various ethers (e.g., ethyl ether, tetrahydrofuran and the like), benzene, toluene, n-hexane, cyclohexane and the like, or a mixed solvent thereof, in the presence of copper iodide, phosphine palladium catalyst such as bis(triphenylphosphine)palladium (II) chloride and the like, and an organic base such as triethylamine, N-methylmorpholine and the like from under cooling to under heating, preferably at room temperature to give compound 54.

### Step 79

The compound 54 is reacted with butadiene preferably under high pressure and under heating in a solvent such as chloroform, dichloromethane, ethyl ether and the like or without solvent to give compound 55.

### Step 80

The compound 55 is reacted with hydrogen in a solvent such as various alcohols (e.g., methanol, ethanol, propanol and the like) using palladium catalyst such as palladium-carbon, palladium black, palladium hydroxide and the like under 1-5 atm under a hydrogen atmosphere at room temperature to give compounds [I]-27 and 60.

### Step 81

The compound [I]-27 is converted to compound [I]-29 in an organic solvent such as formic acid and trifluoroacetic acid from under cooling to under heating, preferably at room temperature.

### Step 82

The compound [I]-27 is converted to compound [I]-30 in the presence of a base such as lithium hydroxide, potassium hydroxide, sodium hydride and the like in a solvent such as various alcohols (e.g., methanol, ethanol, propanol, tert-butanol and the like) from under cooling to under heating.

### Step 83

The compound [I]-31 can be obtained by subjecting compound [I]-30 to the same reaction as in Step 81.

### Step 84

The compound 60 is converted to compound [I]-32 in dimethyl sulfoxide or a mixed solvent of dimethyl sulfoxide and methylene chloride in the presence of sulfur trioxide-pyridine complex or oxalyl chloride and the like, and an organic base such as triethylamine, N-methylmorpholine from under cooling to under heating, preferably at room temperature.

### Step 85

The compound [I]-33 can be obtained by subjecting compound [I]-32 to the same reaction as in Step 81. 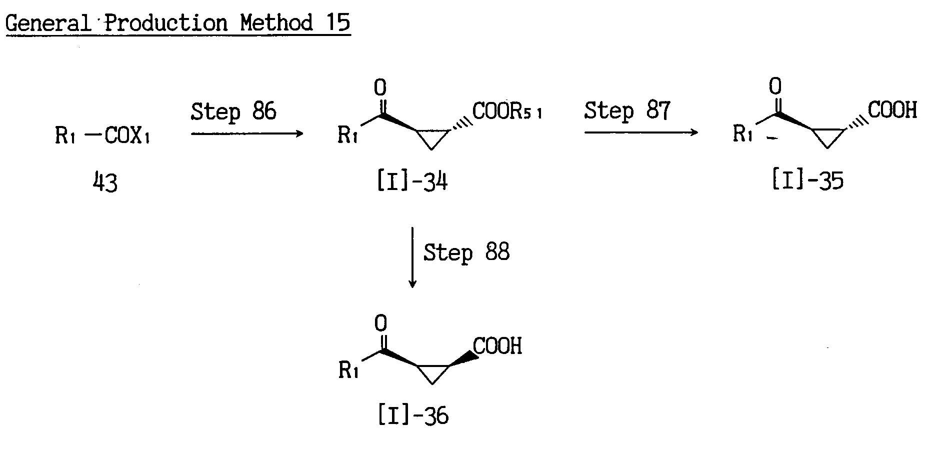 wherein R₁ is as defined above, X₁ is halogen atom such as chlorine atom, bromine atom, iodine atom and the like, and R₅₁ is tert-butyl.

### Step 86

The compound 43 is reacted with diazomethane in a solvent such as ethers (e.g., ethyl ether, tetrahydrofuran and the like), chloroform, methylene chloride and the like, from under cooling to room temperature, and successively the resulting product is reacted with tert-butyl acrylate in a solvent such as ethyl ether, tetrahydrofuran, hexane, cyclohexane, benzene, toluene, chloroform, methylene chloride and the like or without solvent under heating to give compound [I]-34.

### Step 87

The compound [I]-34 is converted to compound [I]-35 in an acidic solvent such as formic acid, trifluoroacetic acid, hydrogen bromideacetic acid solution, hydrogen chloride-dioxane solution and the like from under cooling to under heating.

### Step 88

The compound [I]-34 is reacted with a base such as lithium hydroxide, potassium hydroxide, sodium hydride, sodium methoxide, sodium ethoxide, potassium tert-butoxide and the like in a solvent such as various alcohols (e.g., methanol, ethanol, propanol, tert-butanol and the like) from under cooling to under heating, and successively the resulting product is reacted with hydrochloric acid in water or a mixed solvent of water and any one of methanol, ethanol, tetrahydrofuran, 1,4-dioxane and the like from room temperature to under heating to give compound [I]-36. 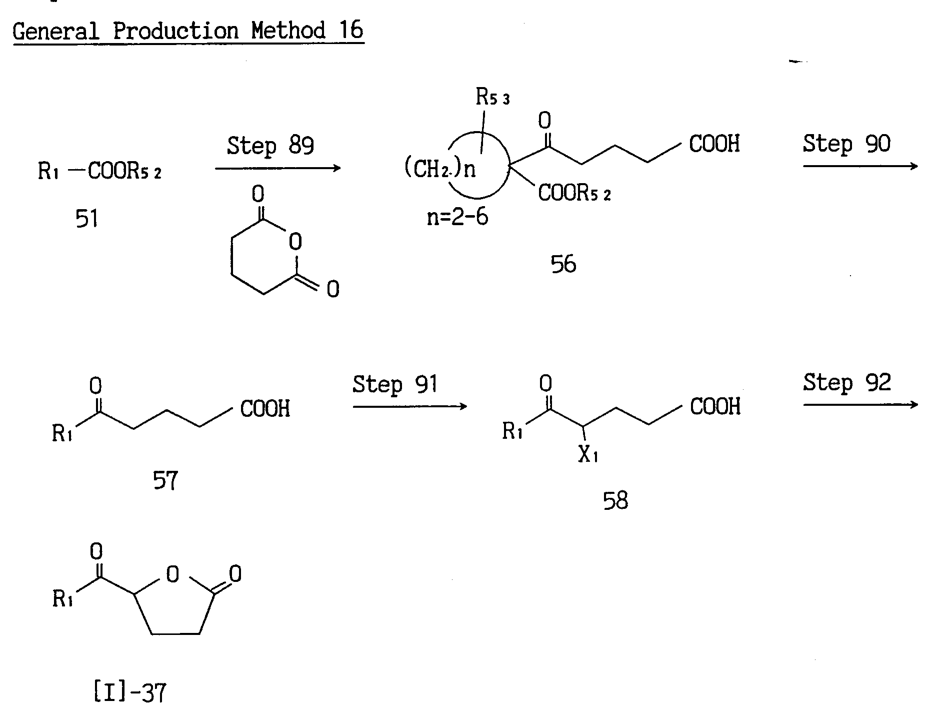 wherein R₁ is a cycloalkyl substituted by lower alkyl such as methyl, ethyl, propyl, butyl and the like, R₅₂ is determined according to the alcohol to be used, R₅₃ is lower alcohol, n is an integer of 2 to 6, and X₁ is halogen atom such as chlorine atom, bromine atom, iodine atom and the like.

### Step 89

The compound 56 can be obtained by subjecting compound 51 and glutaric anhydride to the same reaction as in Step 73.

### Step 90

The compound 57 can be obtained by subjecting compound 56 to the same reaction as in Step 74.

### Step 91

The compound 57 is reacted with bromine in a solvent such as ethyl ether, 1,4-dioxane, tetrahydrofuran, methanol, ethanol, benzene, toluene, n-hexane, cyclohexane, methylene chloride, chloroform and acetic acid, from under cooling to under heating, preferably from 0°C to room temperature to give compound 58.

### Step 92

The compound 58 is converted to compound [I]-37 in a solvent such as tetrahydrofuran, dimethylformamide, acetonitrile and the like in the presence of inorganic base such as sodium hydrogencarbonate, potassium carbonate, sodium carbonate and the like or organic base such as triethylamine, N-methylmorpholine and the like from room temperature to under heating.

In the above reactions, when R₁ is a cyclohexyl substituted by lower alkyl, compound [I]-21 shows a thermodynamically stable isomer from the cis-trans isomers. The other isomer which is not thermodynamically stable can be obtained by reacting the mixture of cis-trans isomers with isobutene in a solvent such as ethyl ether, chloroform, methylene chloride and the like in the presence of acid catalyst such as sulfuric acid from under cooling to room temperature to give a tert-butyl ester compound, separating the cis-trans isomers by HPLC and deesterifying the tert-butyl ester compound of the desired isomer in formic acid or trifluoroacetic acid.

### Example 1 1-phenyl-1,4-pentanedione

### a) methyl 2-acetyl-4-oxo-4-phenylbutanoate

To a suspension of sodium hydride (60% in oil dispersion, 1.05 g) in tetrahydrofuran (10 ml) was dropwise added a solution of methyl acetoacetate (2.70 ml) in tetrahydrofuran (10 ml) at 0°C, and the mixture was stirred for 1 hour. Then, a solution of 2-bromoacetophenone (4.99 g) in tetrahydrofuran (8 ml) was dropwise added, and the mixture was stirred at room temperature for 2 hr. A saturated aqueous ammonium chloride solution was added to the reaction mixture and the mixture was extracted with ethyl acetate. The extract was dried over magnesium sulfate, concentrated and purified by silica gel column chromatography to give the title compound (4.89 g).

### b) 1-phenyl-1,4-pentanedione

To a solution of methyl 2-acetyl-4-oxo-4-phenylbutanoate (2.35 g) obtained in Example 1 a) in tetrahydrofuran (20 ml) was added 2N aqueous sodium hydroxide solution (10.5 ml) at room temperature, and the mixture was stirred for 12 hr. Then, 10% aqueous citric acid solution was added to the reaction mixture. The mixture was stirred, extracted with ethyl acetate. The extract was dried over magnesium sulfate, concentrated and purified by silica gel column chromatography to give the title compound (0.96 g).

### Example 2 1-(4-biphenylyl)-1,4-pentanedione

In the same manner as in Example 1, the title compound was obtained from 2-bromo-4'-phenylacetophenone.

### Example 3 1-(2-naphthyl)-1,4-pentanedione

In the same manner as in Example 1, the title compound was obtained from 2-bromo-2'-acetonaphthone.

### Example 4 1-(1-naphthyl)-1,4-pentanedione

### a) 2-bromo-1'-acetonaphthone

To a solution of 1'-acetonaphthone (15.2 ml) in acetic acid (200 ml) was added concentrated hydrochloric acid (0.2 ml). Then, a solution of bromine (5.2 ml) in acetic acid (20 ml) was dropwise added at room temperature, which was followed by stirring for 1 hr. The reaction mixture was concentrated, and the obtained residue was dissolved in ethyl acetate, washed successively with distilled water, saturated aqueous sodium hydrogencarbonate solution, distilled water and saturated brine, dried over magnesium sulfate and concentrated to give the title compound (25.86 g).

### b) 1-(1-naphthyl)-1,4-pentanedione

In the same manner as in Example 1, the title compound was obtained from 2-bromo-1'-acetonaphthone obtained in Example 4 a).

### Example 5 1-cyclohexyl-1,4-pentanedione

### a) 1-cyclohexyl-1-ethanone

To a solution of cyclohexanecarboxylic acid (12.9 g) in diethyl ether (700 ml) was dropwise added a 1.09M solution (190 ml) of methyllithium in diethyl ether at 0 °C. The mixture was stirred at room temperature for 4 hr. The reaction mixture was poured into a mixture of 1N hydrochloric acid (250 ml) and ice (50 g), and the mixture was stirred. The mixture was extracted with diethyl ether, and the organic layer was washed with saturated aqueous sodium hydrogencarbonate solution and saturated brine, dried over magnesium sulfate and concentrated to give the title compound (9.47 ml).

### b) 2-bromo-1-cyclohexyl-1-ethanone

Bromine (0.55 ml) was dropwise added to a solution of 1-cyclohexyl-1-ethanone (1.75 g) obtained in Example 5 a) in methanol (6.0 ml) at 0°C. The mixture was stirred for 30 min. Distilled water was added at room temperature, followed by stirring. The mixture was extracted with diethyl ether, and the organic layer was washed with saturated aqueous sodium hydrogencarbonate solution and distilled water, dried over magnesium sulfate and concentrated to give the title compound (2.40 g).

### c) 1-cyclohexyl-1,4-pentanedione

In the same manner as in Example 1, the title compound was obtained from 2-bromo-1-cyclohexyl-1-ethanone obtained in Example 5 b).

### Example 6 1-(1-methyl-1-cyclohexyl)-1,4-pentanedione

In the same manner as in Example 5, the title compound was obtained from 1-methyl-1-cyclohexanecarboxylic acid.

### Example 7 1-cyclopentyl-1,4-pentanedione

In the same manner as in Example 5, the title compound was obtained from cyclopentanecarboxylic acid.

### Example 8 1-cycloheptyl-1,4-pentanedione

In the same manner as in Example 5, the title compound was obtained from cycloheptanecarbaxylic acid.

### Example 9 1-cyclohexyl-2,5-hexanedione

### a) cyclohexylacetyl chloride

To a solution of cyclohexylacetic acid (8.0 g) in dichloromethane (50 ml) was dropwise added oxalyl chloride (5.89 ml) at 0°C. Then, dimethylformamide (0.1 ml) was added. The mixture was warmed to room temperature and stirred for 1 hr. The reaction mixture was concentrated to give the title compound (8.43 g).

### b) 1-bromo-3-cyclohexyl-2-propanone

To a solution of cyclohexylacetyl chloride (8.43 g) obtained in Example 9 a) in diethyl ether (100 ml) was added a solution (0.7 M, 350 ml) of diazomethane in diethyl ether, and the mixture was stirred at room temperature for 12 hr. The reaction mixture was concentrated and a solution (16.99 ml) of 25% hydrogen bromide in acetic acid was gradually added dropwise to the obtained residue at 0°C. The reaction mixture was concentrated, and the residue was dissolved in diethyl ether and washed with saturated brine. The organic layer was dried over sodium sulfate and concentrated to give the title compound (10.19 g).

### c) 1-cyclohexyl-2,5-hexanedione

In the same manner as in Example 1, the title compound was obtained from 1-bromo-3-cyclohexyl-2-propanone obtained in Example 9 b).

### Example 10 1-(2-methylphenyl)-1,4-pentanedione

### a) 2-bromo-2'-methylacetophenone

To a solution of 2'-methylacetophenone (5.0 g) in acetic acid (93 ml) was added 4-(dimethylamino)pyridinium bromide perbromide (14.87 g) at room temperature, and the mixture was stirred for 3 hr. Distilled water was added to the reaction mixture, and the mixture was extracted with dichloromethane. The organic layer was washed successively with saturated aqueous sodium hydrogencarbonate solution and saturated brine, dried over sodium sulfate and concentrated to give the title compound (7.94 g).

### b) tert-butyl 2-acetyl-4-(2-methylphenyl)-4-oxobutanoate

In the same manner as in Example 1 a), the title compound was obtained from 2-bromo-2'-methylacetophenone obtained in Example 10 a) and tert-butyl acetoacetate.

### c) 1-(2-methylphenyl)-1,4-pentanedione

tert-Butyl 2-acetyl-4-(2-methylphenyl)-4-oxobutanoate (3.45 g) obtained in Example 10 b) was heated at 185°C for 2.5 hr. The mixture was purified by silica gel column chromatography to give the title compound (0.912 g).

### Example 11 1-(3-methylphenyl)-1,4-pentanedione

### a) tert-butyl 2-acetyl-4-(3-methylphenyl)-4-oxobutanoate

In the same manner as in Example 10 a) and b), the title compound was obtained from 3'-methylacetophenone.

### b) 1-(3-methylphenyl)-1,4-pentanedione

To a solution of tert-butyl 2-acetyl-4-(3-methylphenyl)-4-oxobutanoate (4.0 g) obtained in Example 11 a) in dimethyl sulfoxide (20 ml) were added distilled water (0.27 ml) and lithium chloride (0.64 g), and the mixture was stirred at 185°C for 1 hr. Distilled water was added to the reaction mixture, and the mixture was stirred and extracted with chloroform. The organic layer was washed with saturated brine, dried over sodium sulfate, concentrated and purified by silica gel column chromatography to give the title compound (0.50 g).

### Example 12 1-(4-tert-butylphenyl)-1,4-pentanedione

### a) 2-bromo-4'-tert-butylacetophenone

In the same manner as in Example 4 a), the title compound was obtained from 4'-tert-butylacetophenone.

### b) tert-butyl 2-acetyl-4-(4-tert-butylphenyl)-4-oxobutanoate

In the same manner as in Example 10 b), the title compound was obtained from 2-bromo-4'-tert-butylacetophenone obtained in Example 12 a).

### c) 1-(4-tert-butylphenyl)-1,4-pentanedione

tert-Butyl 2-acetyl-4-(4-tert-butylphenyl)-4-oxobutanoate (50.1 mg) obtained in Example 12 b) was dissolved in formic acid (1.0 ml), and the mixture was stirred for 4 hr at room temperature, and then at 150°C for 10 min. The mixture was concentrated and purified by silica gel column chromatography to give the title compound (26.6 mg).

### Example 13 1-(4-cyclohexylphenyl)-1,4-pentanedione

### a) tert-butyl 2-acetyl-4-(4-cyclohexylphenyl)-4-oxobutanoate

In the same manner as in Example 10 a) and b), the title compound was obtained from 4'-cyclohexylacetophenone.

### b) 1-(4-cyclohexylphenyl)-1,4-pentanedione

In the same manner as in Example 11 b), the title compound was obtained from tert-butyl 2-acetyl-4-(4-cyclohexylphenyl)-4-oxobutanoate obtained in Example 13 a).

### Example 14 2-methyl-4,7-octanedione

### a) 1-bromo-4-methyl-2-pentanone

In the same manner as in Example 9 b), the title compound was obtained from isovaleryl chloride.

### b) 2-methyl-4,7-octanedione

In the same manner as in Example 10 b) and c), the title compound was obtained from 1-bromo-4-methyl-2-pentanone obtained in Example 14 a).

### Example 15 trans-1-(4-methylcyclohexyl)-1,4-pentanedione

### a) trans-2-bromo-1-(4-methylcyclohexyl)-1-ethanone

In the same manner as in Example 9 a) and b), the title compound was obtained from 4-methyl-1-cyclohexanecarboxylic acid.
CDCl₃ 300MHz
   0.90(3H,d,J=6.0Hz), 0.97(2H,dq,J=3.0Hz,12.0Hz), 1.41(2H,dq,J=3.0Hz, 12.0Hz), 1.26-1.43(1H,m), 1.79(2H,dd,J=3.0Hz,12.0Hz), 1.90(2H,dd, J=3.0Hz,12.0Hz), 2.64(1H,tt,J=3.0Hz,12.0Hz), 3.97(2H,s).

### b) trans-1-(4-methylcyclohexyl)-1,4-pentanedione

To a solution of benzyl acetoacetate (3.16 g) in tetrahydrofuran (60 ml) was added sodium hydride (60% in oil dispersion, 0.69 g) at 0°C, and the mixture was stirred for 30 min. Then, a solution of trans-2-bromo-1-(4-methylcyclohexyl)-1-ethanone (4.0 g) obtained in Example 15 a) in tetrahydrofuran (20 ml) was added dropwise. The mixture was stirred at room temperature for 2 hr. Saturated aqueous ammonium chloride solution was added to the reaction mixture, and the mixture was stirred and extracted with ethyl acetate. The organic layer washed with saturated brine, dried over sodium sulfate, concentrated and purified by silica gel column chromatography to give benzyl trans-2-acetyl-4-(4-metylcyclohexyl)-4-oxobutanoate (3.96 g). The obtained benzyl trans-2-acetyl-4-(4-metylcyclohexyl)-4-oxobutanoate (3.96 g) was dissolved in ethanol (59 ml), and 10% palladium-carbon (0.791 g) was added. The mixture was stirred at room temperature for 1 hr under a hydrogen atmosphere. 10% Palladium-carbon was filtered off, concentrated and purified by silica gel column chromatography to give the title compound (2.00 g).

### Example 16 1-(1-adamantyl)-1,4-pentanedione

### a) 1-(1-adamantyl)-2-bromo-1-ethanone

In the same manner as in Example 5 a) and b), the title compound was obtained from 1-adamantanecarboxylic acid.

### b) 1-(1-adamantyl)-1,4-pentanedione

In the same manner as in Example 15 b), the title compound was obtained from 1-(1-adamantyl)-2-bromo-1-ethanone obtained in Example 16 a).

### Example 17 trans-1-(4-phenylcyclohexyl)-1,4-pentanedione

### a) trans-4-phenylcyclohexanecarboxylic acid

4-Phenylbenzoic acid (4.0 g) was added to isoamyl alcohol (400 ml) and the mixture was heated to 130°C, which was followed by portionwise addition of sodium (28.0 g). The mixture was heated to 150°C and stirred for 15 hr. The reaction mixture was poured into saturated aqueous ammonium chloride solution under ice-cooling. The mixture was extracted with ethyl acetate. The organic layer was washed with saturated brine, dried over sodium sulfate and concentrated to give the title compound (4.12 g).
CD₃OD 300MHz
   1.47-1.70(4H,m), 1.92-1.96(2H,m), 2.09-2.12(2H,m), 2.28(1H,tt,J=3.6 Hz,11.7Hz), 2.55(1H,tt,J=3.0Hz,11.7Hz), 7.14-7.31(5H,m).

### b) trans-1-(4-phenylcyclohexyl)-1,4-pentanedione

In the same manner as in Example 15, the title compound was obtained from trans-4-phenylcyclohexanecarboxylic acid obtained in Example 17 a).

### Example 18 1-(2-pyridyl)-1,4-pentanedione

To a solution of picolinaldehyde (1.5 g) in ethanol (30 ml) were added methyl vinyl ketone (1.17 ml), 3-benzyl-5- (2-hydroxyethyl)-4-methylthiazolium chloride (378 mg) and triethylamine (0.782 ml) at room temperature, and the mixture was stirred for 12 hr. The reaction mixture was poured into 5% aqueous potassium hydrogensulfate solution, and the aqueous layer was washed with chloroform. The aqueous layer was adjusted to pH 8 with sodium hydrogencarbonate and extracted with chloroform. The organic layer was washed with saturated brine, dried over sodium sulfate, concentrated and purified by silica gel column chromatography to give the title compound (329 mg).

### Example 19 1-(3-pyridyl)-1,4-pentanedione

In the same manner as in Example 18, the title compound was obtained from nicotinaldehyde.

### Example 20 1-(4-hydroxymethylphenyl)-1,4-pentanedione

### a) 4-hydroxymethylbenzaldehyde

To a solution of sodium borohydride (7.26 g) in methanol (400 ml) was added dropwise a solution of terephthalaldehyde monodiethyl acetal (26.1 ml) in methanol (20 ml), and the mixture was stirred at room temperature for 2 hr. The reaction mixture was concentrated, and brine was added to the obtained residue, which was followed by extraction with diethyl ether. The organic layer was washed with saturated brine and dried over magnesium sulfate. The residue obtained by concentration of the organic layer was dissolved in chloroform (80 ml). A 50% aqueous trifluoroacetic acid solution (40 ml) was added at 0°C and the mixture was stirred for 30 min. The reaction mixture was neutralized with sodium hydrogencarbonate, and chloroform was evaporated. The residue was dissolved in ethyl acetate, washed with distilled water, dried over magnesium sulfate and concentrated to give the title compound (15.09 g).

### b) 1-(4-hydroxymethylphenyl)-1,4-pentanedione

To a solution of 4-hydroxymethylbenzaldehyde (1.94 g) obtained in Example 20 a) in ethanol (20 ml) were added methyl vinyl ketone (2.5 ml), triethylamine (0.85 ml) and 3-benzyl-5-(2-hydroxyethyl)-4-methylthiazolium chloride (0.410 g), and the mixture was refluxed for 8 hr. Distilled water was added to the reaction mixture, and the mixture was extracted with diethyl ether. The organic layer was dried over magnesium sulfate, concentrated and purified by silica gel column chromatography to give the title compound (1.32 g).

### Example 21 1-[4-(1-hydroxyethyl)phenyl]-1,4-pentanedione

### a) 4-bromobenzaldehyde ethylene acetal

To a solution of 4-bromobenzaldehyde (25.27 g) in benzene (150 ml) were added ethylene glycol (11 ml) and p-toluenesulfonic acid monohydrate (0.264 g), and the mixture was refluxed in a Dean-Stark trap for 24 hr. The reaction mixture was neutralized with potassium carbonate, and the organic layer was washed with distilled water and saturated brine, dried over magnesium sulfate and concentrated to give the title compound (30.49 g).

### b) 4-(1-hydoroxyethyl)benzaldehyde

To a mixture of magnesium (turnings 2.434 g) and tetrahydrofuran (10 ml) was added, under an argon atmosphere, 10 ml of a solution of 4-bromobenzaldehyde ethylene acetal (21.83 g) obtained in Example 21 a) in tetrahydrofuran (70 ml), and 1,2-dibromoethane (0.2 ml) was added with heating. After the reaction was started, the remaining solution was added dropwise at a rate to maintain gentle reflux. Then, the mixture was refluxed for 30 min, and cooled to room temperature. A solution of acetaldehyde (5.3 ml) in tetrahydrofuran (40 ml) was added dropwise, and the mixture was stirred for 1 hr. After the reaction was completed, saturated aqueous ammonium chloride solution was added, and the mixture was stirred, extracted with ethyl acetate, dried over potassium carbonate, concentrated and purified by silica gel column chromatography to give 4-(1-hydoroxyethyl)benzaldehyde ethylene acetal (7.58 g). To a solution of 4- (1-hydoroxyethyl)benzaldehyde ethylene acetal (2.92 g) in tetrahydrofuran (10 ml) was added 1N hydrochloric acid (2 ml) at room temperature, and the mixture was stirred for 2 hr. The reaction mixture was diluted with distilled water and extracted with ethyl acetate. The organic layer was washed with distilled water, saturated aqueous sodium hydrogencarbonate solution and saturated brine, dried over magnesium sulfate and concentrated to give the title compound (2.14 g).

### c) 1-[4-(1-hydroxyethyl)phenyl]-1,4-pentanedione

In the same manner as in Example 20 b), the title compound was obtained from 4-(1-hydoroxyethyl)benzaldehyde obtained in Example 21 b).

### Example 22 1-[4-(2-hydroxyethyl)phenyl]-1,4-pentanedione

### a) 4-vinylbenzaldehyde ethylene acetal

To a solution of 4-bromobenzaldehyde ethylene acetal (5.455 g) obtained in Example 21 a) in toluene (10 ml) were added, under an argon atmosphere, vinyltributyltin (8.35 g) and tetrakis(triphenylphosphine)palladium(O) (0.555 g), and the mixture was refluxed for 2 hr. Saturated aqueous sodium fluoride solution (20 ml) was added to the reaction mixture, and the mixture was stirred for 12 hr at room temperature, after which the mixture was filtered through a Celite pad. The filtrate was extracted with ethyl acetate, washed with saturated brine, dried over magnesium sulfate, concentrated and purified by silica gel column chromatography to give the title compound (3.188 g).

### b) 4-(2-hydroxyethyl)benzaldehyde

To a solution of 4-vinylbenzaldehyde ethylene acetal (1.417 g) obtained in Example 22 a) in tetrahydrofuran (10 ml) was added dropwise under an argon atmosphere at 0°C borane-tetrahydrofuran complex (1.0 M, 10 ml), and the mixture was stirred for 1 hr. 4M Aqueous sodium hydroxide solution (6.0 ml) was added to the reaction mixture and then 30% aqueous hydrogen peroxide (10 ml) was added to the reaction mixture and stirred. Sodium chloride was added to the reaction mixture and extracted with diethyl ether. The organic layer was washed with saturated brine, dried over magnesium sulfate, and concentrated. The obtained residue was dissolved in tetrahydrofuran (20 ml) and 1N hydrochloric acid (5 ml) was added. The mixture was stirred for 12 hr, concentrated and purified by silica gel column chromatography to give the title compound (0.261 g).

### c) 1-[4-(2-hydroxyethyl)phenyl]-1,4-pentanedione

In the same manner as in Example 20 b), the title compound was obtained from 4-(2-hydroxyethyl)benzaldehyde obtained in Example 22 b).

### Example 23 1-(benzo[b]thiophen-2-yl)-1,4-pentanedione

### a) (benzo[b]thiophen-2-yl)methanol

To a solution of benzo[b]thiophen-2-carboxylic acid (8.925 g) in tetrahydrofuran (50 ml) was added triethylamine (7.7 ml) at 0°C. Then, a solution of isopropyl chloroformate (6.816 g) in tetrahydrofuran (40 ml) was dropwise added, and the mixture was stirred at 0°C for 1 hr. The reaction mixture was filtered through a Celite pad, and the filtrate was poured into a solution of sodium borohydride (3.801 g) in water (50 ml) at 0°C. The mixture was stirred at room temperature for 1.5 hr. 1N Hydrochloric acid was added to the reaction mixture at 0°C to acidify the mixture, and the mixture was extracted with ethyl acetate. The organic layer was washed successively with saturated aqueous sodium hydrogencarbonate solution and saturated brine, dried over magnesium sulfate and concentrated to give the title compound (7.89 g).

### b) benzo[b]thiophene-2-carbaldehyde

To a solution of (benzo[b]thiophen-2-yl)methanol (4.94 g) obtained in Example 23 a) in dimethyl sulfoxide (35 ml) was added triethylamine (29 ml), and a solution of sulfur trioxide-pyridine complex (14.78 g) in dimethyl sulfoxide (40 ml) was added dropwise. The mixture was stirred for 1 hr. Ice water was added to the reaction mixture, and the mixture was extracted with ethyl acetate. The organic layer was washed successively with distilled water, 1N hydrochloric acid, aqueous sodium hypochlorite solution, saturated aqueous ammonium chloride solution and saturated brine, dried over magnesium sulfate, concentrated and purified by silica gel column chromatography to give the title compound (4.41 g).

### c) 1-(benzo[b]thiophen-2-yl)-1,4-pentanedione

In the same manner as in Example 20 b), the title compound was obtained from benzo[b]thiophene-2-carbaldehyde obtained in Example 23 b).

### Example 24 1-(indan-2-yl)-1,4-pentanedione

### a) 2,2-dimethyl-(spiro[1,3]dioxane-5,2'-indan)-4,6-dione

To a solution of Meldrum's acid (5.78 g) in dimethyl sulfoxide (150 ml) was added triethylamine (11.2 ml) at room temperature, and then a solution of α,α'-dibromo-O-xylene (10.56 g) in dimethyl sulfoxide (50 ml) was added. The mixture was stirred for 20 hr. Distilled water was added to the reaction mixture, and the mixture was extracted with ethyl acetate and concentrated to give the title compound (6.56 g).

### b) ethyl indan-2-carboxylate

To a solution of 2,2-dimethyl-(spiro[1,3]dioxane-5,2'-indan)-4,6-dione (6.56 g) obtained in Example 24 a) in ethanol (50ml) was added p-toluenesulfonic acid monohydrate (0.232 g), and the mixture was refluxed for 24 hr. The reaction mixture was concentrated and the residue was dissolved in dimethyl sulfoxide (140 ml). Distilled water (0.89 ml) and lithium chloride (1.05 g) were added, and the mixture was stirred for 1 hr at 150 °C. Distilled water was added to the reaction mixture, and after stirring, the mixture was extracted with ethyl acetate. The organic layer was washed with saturated brine, dried over magnesium sulfate, concentrated and purified by silica gel column chromatography to give the title compound (2.183 g).

### c) 2-indancarbaldehyde

To a solution of ethyl indan-2-carboxylate (1.98 g) obtained in Example 24 b) in toluene (10 ml) was dropwise added under an argon atmosphere at -72°C a solution (1.01 M, 12.5 ml) of diisobutylaluminium hydride in toluene. The mixture was stirred for 1 hr. 1N Aqueous citric acid solution was added to the reaction mixture, and the mixture was stirred and extracted with ethyl acetate. The organic layer was washed with distilled water, dried over magnesium sulfate, concentrated and purified by silica gel column chromatography to give the title compound (0.951 g).

### d) 1-(indan-2-yl)-1,4-pentanedione

In the same manner as in Example 20 b), the title compound was obtained from 2-indancarbaldehyde obtained in Example 24 c).

### Example 25 1-(4-vinylphenyl)-1,4-pentanedione

### a) 1-(4-bromophenyl)-1,4-pentanedione

In the same manner as in Example 20 b), the title compound was obtained from 4-bromobenzaldehyde.

### b) 1-(4-vinylphenyl)-1,4-pentanedione

A solution of 1-(4-bromophenyl)-1,4-pentanedione (3.83 g) obtained in Example 25 a), vinyltributyltin (8.80 ml) and tetrakis(triphenylphosphine)palladium(O) (0.346 g) in toluene (20 ml) was refluxed for 1 hr under an argon atmosphere. An aqueous sodium fluoride solution (10 ml) was added to the reaction mixture, and the mixture was stirred at room temperature for 3 hr and filtered through a Celite pad. Ethyl acetate was added to the filtrate, and the mixture was washed with distilled water and saturated brine, dried over magnesium sulfate, concentrated and purified by silica gel column chromatography to give the title compound (2.26 g).

### Example 26 1-(4-isopropenylphenyl)-1,4-pentanedione

### a) isopropenylmagnesium bromide

To a mixture of magnesium (turnings, 6.33 g) and tetrahydrofuran (20 ml) was added dropwise 10 ml of a solution of 2-bromopropene (20 ml) in tetrahydrofuran (230 ml) under an argon atmosphere with heating. After the reaction was started, the remaining solution was dropwise added at a rate that maintained refluxing. The mixture was refluxed for 1 hr, and cooled to room temperature to give a solution (0.82 M) of the title compound in tetrahydrofuran.

### b) isopropenyltributyltin

To a solution (0.82 M, 170 ml) of isopropenylmagnesium bromide obtained in Example 26 a) in tetrahydrofuran was dropwise added under an argon atmosphere a solution of tributyltin chloride (27.0 ml) in tetrahydrofuran (100 ml), and the mixture was refluxed for 12 hr. Tetrahydrofuran was evaporated from the reaction mixture, and the residue was distilled (b.p._{1.4} Torr 104-108°C) to give the title compound (32.56 g).

### c) 1-(4-isopropenylphenyl)-1,4-pentanedione

In the same manner as in Example 25 b), the title compound was obtained from 1-(4-bromophenyl)-1,4-pentanedione obtained in Example 25 a) and isopropenyltributyltin obtained in Example 26 b).

### Example 27 1-(p-terphenyl-4-yl)-1,4-pentanedione

### a) 4-biphenylylmagnesium bromide

In the same manner as in Example 26 a), the title compound was obtained from 4-bromobiphenyl.

### b) 1-(p-terphenyl-4-yl)-1,4-pentanedione

To a suspension of zinc bromide (1.09 g) in tetrahydrofuran (10 ml) was added dropwise under an argon atmosphere at 0°C, a solution (0.73 M, 6.50 ml) of 4-biphenylylmagnesium bromide obtained in Example 27 a) in tetrahydrofuran. Then, bis(triphenylphosphine)palladium(II) chloride (56.0 mg) was added, and the mixture was stirred at room temperature for 16 hr. To the reaction mixture was added 1N aqueous citric acid solution, and the mixture was stirred and extracted with chloroform. The organic layer was washed with distilled water, saturated aqueous sodium hydrogencarbonate solution and saturated brine, dried over magnesium sulfate, concentrated and purified by silica gel column chromatography to give the title compound (434 mg).

### Example 28 1-(4-formylphenyl)-1,4-pentanedione

To a solution of 1-(4-hydroxymethylphenyl)-1,4-pentanedione (1.05 g) obtained in Example 20 b) in dimethyl sulfoxide (12 ml) was added dropwise, under an argon atmosphere, triethylamine (4.50 ml) and a solution of sulfur trioxide-pyridine complex (2.39 g) in dimethyl sulfoxide (12 ml), and the mixture was stirred at room temperature for 30 min. To the reaction mixture was added 1N aqueous citric acid solution (50 ml), and the mixture was stirred, extracted with ethyl acetate, and washed with distilled water and saturated brine. After drying over magnesium sulfate, the residue was concentrated and purified by silica gel column chromatography to give the title compound (0.85 g).

### Example 29 4-(1,4-dioxopentyl)benzoic acid

To a solution of 1-(4-formylphenyl)-1,4-pentanedione (0.824 g) obtained in Example 28 in acetonitrile (8 ml) was added a solution of disodium hydrogenphosphate (94.8 mg) in water (1 ml) at 0°C, and then 30% hydrogen peroxide (0.43 ml) was added. A solution of sodium chlorite (0.645 g) in water (6 ml) was added dropwise, and the mixture was stirred for 3 hr. 10% Sodium thiosulfate (2 ml) was added to the reaction mixture, and 1N hydrochloric acid (6 ml) was added to acidify the reaction mixture. Distilled water was added and the mixture was stirred, which was followed by extraction with ethyl acetate. The organic layer was washed with distilled water and saturated brine, dried over magnesium sulfate and concentrated to give the title compound (0.815 g).

### Example 30 1-(4-hydroxyphenyl)-1,4-pentanedione

### a) 4-(methoxymethoxy)benzaldehyde

Potassium carbonate (13.81 g) was added to a solution of 4-hydroxybenzaldehyde (6.823 g) in acetone (150 ml) at 0°C, and chloromethyl methyl ether (5.70 ml) was added dropwise. The mixture was stirred at room temperature for 10 hr. The reaction mixture was filtered through a Celite pad, and the filtrate was concentrated to give the title compound (9.19 g).

### b) 1-[4-(methoxymethoxy)phenyl]-4,4-ethylenedioxy-1-pentanol

To a mixture of magnesium (turnings 1.686 g) and tetrahydrofuran (4 ml) was added 1,2-dibromoethane (30 µl) under an argon atmosphere. A solution of 2-(bromoethyl)-2-methyl-1,3-dioxolane (4.503 g) in tetrahydrofuran (15 ml) was added dropwise at a rate to keep the reaction mixture at 25°C. After the completion of the dropwise addition, the mixture was stirred at room temperature for 1 hr. To a solution of 4-(methoxymethoxy)benzaldehyde (1.664 g) obtained in Example 30 a) in tetrahydrofuran (10 ml) was added dropwise the above-mentioned mixture (10.5 ml) at 0°C under an argon atmosphere. The mixture was stirred for 4 hr. To the reaction mixture was added saturated aqueous ammonium chloride solution, and the mixture was stirred, extracted with ethyl acetate, dried over magnesium sulfate, concentrated and purified by silica gel column chromatography to give the title compound (2.068 g).

### c) 1-[4-(methoxymethoxy)phenyl]-4,4-ethylenedioxy-1-pentanone

To a solution of 1-[4-(methoxymethoxy)phenyl]-4,4-ethylenedioxy-1-pentanol (2.068 g) obtained in Example 30 b) in dimethyl sulfoxide (10 ml) was added triethylamine (6.6 ml), and then a solution of sulfur trioxide-pyridine complex (3.495 g) in dimethyl sulfoxide (10 ml) was added. The mixture was stirred for 1 hr. The reaction mixture was poured into ice water and extracted with ethyl acetate. The organic layer was washed successively with aqueous sodium hypochlorite solution, 1N aqueous citric acid solution and saturated brine. The mixture was dried over magnesium sulfate, concentrated and purified by silica gel column chromatography to give the title compound (1.493 g).

### d) 1-(4-hydroxyphenyl)-1,4-pentanedione

1-[4-(Methoxymethoxy)phenyl]-4,4-ethylenedioxy-1-pentanone (1.446 g) obtained in Example 30 c) was dissolved in tetrahydrofuran (4.0 ml). Isopropanol (4.0 ml) and concentrated hydrochloric acid (2.0 ml) were added to the mixture. The mixture was stirred at room temperature for 12 hr. Distilled water was added to the reaction mixture and the mixture was extracted with ethyl acetate. The organic layer was washed with saturated aqueous ammonium chloride solution and saturated brine. The mixture was dried over magnesium sulfate, concentrated and purified by silica gel column chromatography to give the title compound (0.901 g).

### Example 31 1-(4-biphenylyl)-4-phenyl-1,4-butanedione

### a) ethyl 2-benzoyl-4-(4-biphenylyl)-4-oxobutanoate

In the same manner as in Example 1 a), the title compound was obtained from 2-bromo-4'-phenylacetophenone and ethyl benzoylacetate.

### b) 1-(4-biphenylyl)-4-phenyl-1,4-butanedione

In the same manner as in Example 1 b), the title compound was obtained from ethyl 2-benzoyl-4-(4-biphenylyl)-4-oxobutanoate obtained in Example 31 a).

### Example 32 1-(4-biphenylyl)-4-cyclohexyl-1,4-butanedione

### a) 3-dimethylamino-4'-phenylpropiophenone hydrochloride

To a solution of 4-acetylbiphenyl (10 g) in isoamyl alcohol (40 ml) were added paraformaldehyde (2.76 g), dimethylamine hydrochloride (5.61 g) and concentrated hydrochloric acid (0.1 ml), and the mixture was refluxed for 2 hr. The reaction mixture was filtered while it was hot, and acetone (100 ml) was added to the filtrate. The resulting precipitate was collected by filtration to give the title compound (6.51 g).

### b) 3-(4-biphenylyl)-3-oxopropyltrimethylammonium iodide

3-Dimethylamino-4'-phenylpropiophenone hydrochloride (6.51 g) obtained in Example 32 a) was dissolved in saturated aqueous potassium carbonate solution and the mixture was extracted with diethyl ether, dried over sodium sulfate and concentrated, and the residue was dissolved in ethanol (30 ml). Iodomethane (2.24 ml) was added and the mixture was refluxed for 2 hr. The mixture was cooled to room temperature and the resulting precipitate was collected by filtration to give the title compound (8.01 g).

### c) 4-biphenylyl vinyl ketone

3-(4-Biphenylyl)-3-oxopropyltrimethylammonium iodide (8.01 g) obtained in Example 32 b) was dissolved in 16% aqueous sodium hydrogencarbonate solution (500 ml) and the solution was extracted with diethyl ether. The organic layer was washed with 0.1N hydrochloric acid and then saturated brine, dried over sodium sulfate and concentrated to give the title compound (1.62 g).

### d) 1-(4-biphenylyl)-4-cyclohexyl-1,4-butanedione

In the same manner as in Example 20 b), the title compound was obtained from 4-biphenylyl vinyl ketone obtained in Example 32 c) and cyclohexanecarbaldehyde.

### Example 33 5-(4-biphenylyl)-1-phenyl-2,5-pentanedione

In the same manner as in Example 20 b), the title compound was obtained from phenylacetaldehyde and 4-biphenylyl vinyl ketone obtained in Example 32 c).

### Example 34 1-(4-biphenylyl)-1,4-heptanedione

In the same manner as in Example 20 b), the title compound was obtained from 1-butanal and 4-biphenylyl vinyl ketone obtained in Example 32 c).

### Example 35 1-methoxy-5-phenyl-2,5-pentanedione

### a) methyl 2-(methoxyacetyl)-4-oxo-4-phenylbutanoate

In the same manner as in Example 1 a), the title compound was obtained from 2-bromoacetophenone and methyl 4-methoxyacetoacetate.

### b) 1-methoxy-5-phenyl-2,5-pentanedione

In the same manner as in Example 1 b), the title compound was obtained from methyl 2-(methoxyacetyl)-4-oxo-4-phenylbutanoate obtained in Example 35 a).

### Example 36 1,4-dicyclohexyl-1,4-pentanedione

To a solution of lithium diisopropylamide (1.35 g) in tetrahydrofuran (26.4 ml) was added dropwise a solution of cyclohexyl methyl ketone (1.51 g) in tetrahydrofuran (5 ml) under an argon atmosphere at -78°C, and the mixture was stirred for 15 min. Then, a 0.66 M solution of copper(II) chloride in tetrahydrofuran (19 ml) was dropwise added, and the mixture was stirred for 40 min. 10% Aqueous citric acid solution was added to the reaction mixture and the mixture was extracted with diethyl ether. The organic layer was washed successively with 10% aqueous citric acid solution and saturated brine, dried over magnesium sulfate, concentrated and purified by silica gel column chromatography to give the title compound (0.72 g).

### Example 37 1,4-bis(1-methylcyclohexyl)-1,4-pentanedione

### a) methyl 1-methylcyclohexyl ketone

In the same manner as in Example 5 a), the title compound was obtained from 1-methyl-1-cyclohexanecarboxylic acid.

### b) 1,4-bis(1-methylcyclohexyl)-1,4-pentanedione

In the same manner as in Example 36, the title compound was obtained from methyl 1-methylcyclohexyl ketone obtained in Example 37 a).

### Example 38 (Z)-1-(4-biphenylyl)-2-pentene-1,4-dione

### a) 2-(4-biphenylyl)-5-methylfuran

To a solution of 1-(4-biphenylyl)-1,4-pentanedione (7.53 g) obtained in Example 2 in benzene (100 ml) was added p-toluenesulfonic acid monohydrate (0.383 g), and the mixture was subjected to azeotropic dehydration in a Dean-Stark trap for 2 hr. The reaction mixture was concentrated, and the obtained residue was dissolved in ethyl acetate, washed with saturated aqueous sodium hydrogencarbonate solution and distilled water, dried over magnesium sulfate, concentrated and purified by silica gel column chromatography to give the title compound (3.19 g).

### b) (Z)-1-(4-biphenylyl)-2-pentene-1,4-dione

m-Chloroperbenzoic acid (1.01 g) was added to a solution of 2- (4-biphenylyl)-5-methylfuran (1.00 g) obtained in Example 38 a) in dichloromethane (10 ml) at 0°C, and the mixture was stirred for 1 hr. Saturated aqueous sodium hydrogen carbonate solution and 10% aqueous sodium thiosulfate solution were added to the reaction mixture to adjust to pH 8 and the mixture was extracted with ethyl acetate. The organic layer was washed with saturated brine, dried over magnesium sulfate and concentrated to give the title compound (1.07 g).

### Example 39 (Z)-1-cyclohexyl-2-pentene-1,4-dione

In the same manner as in Example 38, the title compound was obtained from 1-cyclohexyl-1,4-pentanedione obtained in Example 5.

### Example 40 (Z)-1-(1-methyl-1-cyclohexyl)-2-pentene-1,4-dione

In the same manner as in Example 38, the title compound was obtained from 1-(1-methyl-1-cyclohexyl)-1,4-pentanedione obtained in Example 6.

### Example 41 (E)-1-(4-biphenylyl)-2-pentene-1,4-dione

2-(4-Biphenylyl)-5-methylfuran (1.0 g) obtained in Example 38 a) was dissolved in aqueous acetone (acetone:distilled water=85:15, 100 ml), and an aqueous acetone solution (0.65 M, 6.6 ml) of bromine was added at -25°C, which was followed by stirring at room temperature for 1 hr. Sodium hydrogencarbonate (4.0 g) was added to the reaction mixture and the mixture was concentrated. The residue was dissolved in distilled water. 10% Aqueous sodium thiosulfate solution was added, and after stirring, the mixture was extracted with ethyl acetate. The organic layer was washed successively with saturated aqueous sodium hydrogencarbonate solution, distilled water and saturated brine, dried over magnesium sulfate and concentrated to give the title compound (1.12 g).

### Example 42 3-acetyl-1-(4-biphenylyl)-1,4-pentanedione

In the same manner as in Example 1 a), the title compound was obtained from 2-bromo-4'-phenylacetophenone and 2,4-pentanedione.

### Example 43 3-acetyl-1-(4-biphenylyl)-3-methyl-1,4-pentanedione

In the same manner as in Example 1 a), the title compound was obtained from 2-bromo-4'-phenylacetophenone and 3-methyl-2,4-pentanedione.

### Example 44 3-acetyl-1-(1-methyl-1-cyclohexyl)-1,4-pentanedione

### a) 2-bromo-1-(1-methyl-1-cyclohexyl)-1-ethanone

In the same manner as in Example 5 b), the title compound was obtained from methyl 1-methylcyclohexyl ketone obtained in Example 37 a).

### b) 3-acetyl-1-(1-methyl-1-cyclohexyl)-1,4-pentanedione

In the same manner as in Example 1 a), the title compound was obtained from 2-bromo-1-(1-methyl-1-cyclohexyl)-1-ethanone obtained in Example 44 a) and 2,4-pentanedione.

### Example 45 1-acetyl-1-(4-biphenylyl)-2-pentene-1,4-dione

### a) 3-acetyl-5-(4-biphenylyl)-2-methylfuran

In the same manner as in Example 38 a), the title compound was obtained from 3-acetyl-1-(4-biphenylyl)-1,4-pentanedione obtained in Example 42.

### b) 1-acetyl-1-(4-biphenylyl)-2-pentene-1,4-dione

In the same manner as in Example 41, the title compound was obtained from 3-acetyl-5-(4-biphenylyl)-2-methylfuran obtained in Example 45 a).

### Example 46 4-oxo-4-phenylbutanoic acid

To a solution of succinic anhydride (2.00 g) in tetrahydrofuran (25 ml) was added dropwise, under an argon atmosphere, a solution (1M, 20 ml) of phenylmagnesium bromide in tetrahydrofuran and the mixture was refluxed for 3 hr. 1N Aqueous citric acid solution was added to the reaction mixture, and the mixture was stirred and extracted with ethyl acetate. The obtained organic layer was extracted with 1N aqueous sodium hydroxide solution, and 1N aqueous citric acid solution was added until the aqueous layer was acidified. The aqueous layer was extracted with ethyl acetate and washed with brine. The organic layer was dried over magnesium sulfate and concentrated to give the title compound (3.20 g).

### Example 47 4-(4-biphenylyl)-4-oxobutanoic acid

To a solution of biphenyl (5.20 g) and succinic anhydride (3.0 g) in 1,1,2,2,-tetrachloroethane (50 ml) was added aluminum chloride (9.47 g) at 0°C, which was followed by stirring at room temperature for 6 hr. The reaction mixture was poured into ice water and acidified with concentrated hydrochloric acid. The mixture was extracted with ethyl acetate. 1N Aqueous sodium hydroxide solution was added to the organic layer for extraction into the aqueous layer. The obtained aqueous layer was acidified with concentrated hydrochloric acid and extracted again with ethyl acetate, washed with saturated brine, dried over sodium sulfate and concentrated to give the title compound (6.40 g).

### Example 48 4-(2-fluorenyl)-4-oxobutanoic acid

In the same manner as in Example 47, the title compound was obtained from fluorene.

### Example 49 4-[4-(hydroxymethyl)phenyl]-4-oxobutanoic acid

### a) 4-(4-methylphenyl)-4-oxobutanoic acid

To a solution of toluene (5.52 g) and succinic anhydride (4.0 g) in 1,1,2,2,-tetrachloroethane (40 ml) was added aluminum chloride (12.78 g) at 0°C, which was followed by stirring at room temperature for 2 hr. Ice water was poured onto the reaction mixture and acidified with concentrated hydrochloric acid. The mixture was extracted with ethyl acetate. 1N Aqueous sodium hydroxide solution was added to the organic layer for extraction into the aqueous layer. The obtained aqueous layer was acidified with concentrated hydrochloric acid, extracted with ethyl acetate, washed with saturated brine, dried over sodium sulfate and concentrated to give the title compound (7.50 g).

### b) methyl 4-(4-methylphenyl)-4-oxobutanoate

To a solution of 4-(4-methylphenyl)-4-oxobutanoic acid (7.05 g) obtained in Example 49 a) in methanol (140 ml) was added p-toluenesulfonic acid monohydrate (0.698 g) and the mixture was refluxed for 3 hr. The reaction mixture was concentrated, dissolved in ethyl acetate, washed successively with saturated aqueous sodium hydrogencarbonate solution and saturated brine, dried over sodium sulfate and concentrated to give the title compound (6.43 g).

### c) methyl 4-[4-(hydroxymethyl)]phenyl-4-oxobutanoate

To a solution of methyl 4-(4-methylphenyl)-4-oxobutanoate (6.43 g) obtained in Example 49 b) in carbon tetracholoride (193 ml) was added N-bromosuccinimide (5.30 g). The mixture was refluxed for 30 min under irradiation of a 100W lamp. The insoluble matter was filtered off from the reaction mixture, and the filtrate was concentrated. The obtained residue was dissolved in 1,4-dioxane (75 ml). Distilled water (75 ml) and calcium carbonate (13.4 g) were added and the mixture was refluxed for 12 hr. The mixture was poured into 1N hydrochloric acid and the mixture was extracted with chloroform. The organic layer was washed successively with saturated aqueous sodium hydrogencarbonate solution and saturated brine, dried over sodium sulfate, concentrated and purified by silica gel column chromatography to give the title compound (3.55 g).

### d) 4-[4-(hydroxymethyl)phenyl]-4-oxobutanoic acid

To a solution of methyl 4-[4-(hydroxymethyl)phenyl]-4-oxobutanoate (3.55 g) obtained in Example 49 c) in tetrahydrofuran (35.5 ml) was added 1N aqueous sodium hydroxide solution (33.0 ml) at 0°C, and the mixture was stirred for 1 hr at room temperature. The reaction mixture was neutralized with 1N hydrochloric acid, and concentrated to remove tetrahydrofuran. The mixture was acidified with 1N hydrochloric acid, and was extracted with ethyl acetate. The organic layer was washed with saturated brine, dried over sodium sulfate and concentrated to give the title compound (2.89 g).

### Example 50 (E)-4-oxo-4-phenyl-2-butenoic acid

To a solution of maleic anhydride (3.43 g) in benzene (20 ml) was added aluminum chloride (10.5 g) at room temperature, and the mixture was refluxed for 1 hr. Distilled water was added to the reaction mixture at 0°C and concentrated hydrochloric acid was added. Benzene was evaporated and the residue was filtered. The filtrate was extracted with ethyl acetate. The organic layer was washed with distilled water. The organic layer was extracted with 1N aqueous sodium hydroxide solution and the aqueous layer was made acidic with concentrated hydrochloric acid, extracted with ethyl acetate, dried over magnesium sulfate and concentrated to give the title compound (5.32 g).

### Example 51 4-(4'-methylbiphenyl-4-yl)-4-oxobutanoic acid

### a) 4-(4-bromophenyl)-4-oxobutanenitrile

To a solution of sodium cyanide (3.36 g) in dimethylformamide (50 ml) was added dropwise at 45°C a solution of 4-bromobenzaldehyde (25.57 g) in dimethylformamide (100 ml), and then a solution of acrylonitrile (8.90 ml) in dimethylformamide (100 ml) was added dropwise, which was followed by stirring at 40°C for 2 hr. Ice water was added to the reaction mixture, and the mixture was extracted with ethyl acetate. The organic layer was washed with saturated brine, dried over magnesium sulfate and concentrated to give the title compound (19.29 g).

### b) 4-(4'-methylbiphenyl-4-yl)-4-oxobutanenitrile

To a mixture of magnesium (turnings, 2.68 g) and tetrahydrofuran (10 ml) was added dropwise, with heating under an argon atmosphere, 10 ml of a solution of 4-bromotoluene (17.1 g) in tetrahydrofuran (75.2 ml). After the reaction was started, the remaining solution was added dropwise at a rate to maintain gentle reflux. After the completion of the dropwise addition, the mixture was refluxed for 3 hr. Then, to a mixture of zinc bromide (1.694 g) and tetrahydrofuran (20 ml) was added dropwise the above-mentioned mixture (7.5 ml) under an argon atmosphere at 0°C, and the mixture was stirred at room temperature for 1 hr. 4-(4-Bromophenyl)-4-oxobutanenitrile (1.19 g) obtained in Example 51 a) and tetrakis(triphenylphosphine)palladium(O) (0.119 g) were added at 0°C, and the mixture was stirred. The mixture was refluxed for 3 hr. After the reaction was completed, distilled water and saturated aqueous ammonium chloride solution were added, and the mixture was extracted with ethyl acetate, dried over magnesium sulfate, concentrated and purified by silica gel column chromatography to give the title compound (0.740 g).

### c) 4-(4'-methylbiphenyl-4-yl)-4-oxobutanoic acid

To a solution of 4-(4'-methylbiphenyl-4-yl)-4-oxobutanenitrile (0.537 g) obtained in Example 51 b) in ethanol (2.0 ml) was added 2N aqueous sodium hydroxide solution (3.0 ml) and the mixture was refluxed for 12 hr. 1N Hydrochloric acid was added to the reaction mixture and the mixture was extracted with ethyl acetate. The organic layer was washed with saturated brine, dried over magnesium sulfate and concentrated to give the title compound (0.502 g).

### Example 52 4-(3'-methylbiphenyl-4-yl)-4-oxobutanoic acid

In the same manner as in Example 51 b) and c), the title compound was obtained from 3-bromotoluene and 4-(4-bromophenyl)-4-oxobutanenitrile obtained in Example 51 a).

### Example 53 4-(2'-methylbiphenyl-4-yl)-4-oxobutanoic acid

In the same manner as in Example 51 b) and c), the title compound was obtained from 2-bromotoluene and 4-(4-bromophenyl)-4-oxobutanenitrile obtained in Example 51 a).

### Example 54 4-(2'-hydroxybiphenyl-4-yl)-4-oxobutanoic acid

### a) 1-bromo-2-(methoxymethoxy)benzene

To a solution of 2-bromophenol (17.27 g) in acetone (20 ml) was added at 0°C potassium carbonate (27.8 g), and chloromethyl methyl ether (12.0 ml) was added dropwise. The mixture was stirred at room temperature for 1 hr and filtered through a Celite pad. The filtrate was concentrated to give the title compound (20.42 g).

### b) 4-[2'-(methoxymethoxy)biphenyl-4-yl]-4-oxobutanenitrile

To a mixture of magnesium (turnings, 1.337 g) and tetrahydrofuran (5 ml) was added dropwise, with heating under an argon atmosphere, 5 ml of a solution of 1-bromo-2-(methoxymethoxy)benzene (10.86 g) obtained in Example 54 a) in tetrahydrofuran (37 ml). After the reaction was started, the remaining solution was added dropwise at a rate to maintain gentle reflux. After the completion of the dropwise addition, the mixture was refluxed for 3 hr. Then, to a mixture of zinc bromide (2.266 g) and tetrahydrofuran (10 ml) was added dropwise the above-mentioned mixture (9.0 ml) under an argon atmosphere at 0 °C, and the mixture was stirred at room temperature for 1 hr. 4-(4-Bromophenyl)-4-oxobutanenitrile (1.19 g) obtained in Example 51 a) and tetrakis(triphenylphosphine)palladium(O) (0.119 g) were added at 0°C, and the mixture was stirred. The mixture was refluxed for 3 hr. Distilled water and saturated aqueous ammonium chloride solution were added to the reaction mixture, and the mixture was extracted with ethyl acetate, dried over magnesium sulfate, concentrated and purified by silica gel column chromatography to give the title compound (0.761 g).

### c) 4-(2'-hydroxybiphenyl-4-yl)-4-oxobutanoic acid

To a solution of 4-[2'-(methoxymethoxy)biphenyl-4-yl]-4-oxobutanenitrile (0.761 g) obtained in Example 54 b) in ethanol (3 ml) was added 2N aqueous sodium hydroxide solution (4.0 ml), and the mixture was refluxed for 4 hr. Distilled water was added to the reaction mixture, and the mixture was washed with ethyl acetate. The aqueous layer was acidified with 1N hydrochloric acid, extracted with ethyl acetate, washed with saturated brine, dried over magnesium sulfate and concentrated. The obtained residue was dissolved in tetrahydrofuran (4 ml), to which concentrated hydrochloric acid (1 ml) was added, and the mixture was stirred at room temperature for 12 hr. Tetrahydrofuran was evaporated and the residue was extracted with ethyl acetate, washed with saturated brine, dried over magnesium sulfate and concentrated to give the title compound (0.405 g).

### Example 55 4-(3'-hydroxybiphenyl-4-yl)-4-oxobutanoic acid

In the same manner as in Example 54, the title compound was obtained from 3-bromophenol.

### Example 56 4-(4'-hydroxybiphenyl-4-yl)-4-oxobutanoic acid

In the same manner as in Example 54, the title compound was obtained from 4-bromophenol.

### Example 57 4-oxo-4-[4-(3-thienyl)phenyl]butanoic acid

### a) 4-(4-bromophenyl)-4-oxobutanoic acid

In the same manner as in Example 51 c), the title compound was obtained from 4-(4-bromophenyl)-4-oxobutanenitrile obtained in Example 51 a).

### b) methyl 4-(4-bromophenyl)-4-oxobutanoate

In the same manner as in Example 49 b), the title compound was obtained from 4-(4-bromophenyl)-4-oxobutanoic acid obtained in Example 57 a).

### c) methyl 4-oxo-4-[4-(3-thienyl)phenyl]butanoate

To a solution of methyl 4-(4-bromophenyl)-4-oxobutanoate (0.542 g) obtained in Example 57 b) in benzene (4 ml) were added, under an argon atmosphere, a solution of thiophene-3-boric acid (0.445 g) in ethanol (0.5 ml) and 2M aqueous sodium carbonate solution (2 ml), and the mixture was refluxed for 1 hr. Diethyl ether was added to the reaction mixture, and the mixture was washed with saturated brine. The organic layer was dried over sodium sulfate and concentrated. The obtained crude product was purified by silica gel column chromatography to give the title compound (0.586 g).

### d) 4-oxo-4-[4-(3-thienyl)phenyl]butanoic acid

In the same manner as in Example 49 d), the title compound was obtained from methyl 4-oxo-4-[4-(3-thienyl)phenyl]butanoate obtained in Example 57 c).

### Example 58 4-cyclohexyl-4-oxobutanoic acid

### a) dimethyl 2-(2-cyclohexyl-2-oxoethyl)propanedioate

To a solution of dimethyl malonate (2.17 g) in tetrahydrofuran (60 ml) was added sodium hydride (60% in oil dispersion, 0.69 g) at 0°C. After stirring for 30 min, a solution of 2-bromo-1-cyclohexyl-1-ethanone (3.75 g) obtained in Example 5 b) in tetrahydrofuran (20 ml) was added dropwise. After the completion of the dropwise addition, the mixture was stirred at room temperature for 1 hr. Saturated aqueous ammonium chloride solution was added to the reaction mixture, and after stirring, the mixture was extracted with ethyl acetate. The organic layer was washed with saturated brine, dried over sodium sulfate, concentrated and purified by silica gel column chromatography to give the title compound (3.70 g).

### b) 2-(2-cyclohexyl-2-oxoethyl)propanedioic acid

To a solution of dimethyl 2-(2-cyclohexyl-2-oxoethyl)propanedioate (2.97 g) obtained in Example 58 a) in tetrahydrofuran (30 ml) was added 1N aqueous sodium hydroxide solution (24.3 ml) at 0°C, and the mixture was stirred for 4 hr. Tetrahydrofuran was evaporated from the reaction mixture and distilled water was added. The mixture was washed with diethyl ether, and the aqueous layer was acidified with 5% aqueous potassium hydrogensulfate solution, extracted with ethyl acetate. The extract was dried over sodium sulfate and concentrated to give the title compound (1.94 g).

### c) 4-cyclohexyl-4-oxobutanoic acid

2-(2-Cyclohexyl-2-oxoethyl)propanedioic acid (1.94 g) obtained in Example 58 b) was heated and decarboxylated to give the title compound (1.50 g).

### Example 59 methyl 4-cyclohexyl-4-oxobutanoate

To a solution of 4-cyclohexyl-4-oxobutanoic acid (1.22 g) obtained in Example 58 in methylene chloride (20 ml) was added methanol (12 ml) at room temperature. Then, a solution (10%, 14 ml) of (trimethylsilyl) diazomethane in hexane was added, and the mixture was stirred for 1 hr. The reaction mixture was concentrated and purified by silica gel column chromatography to give the title compound (1.19 g).

### Example 60 dimethyl 2-[2-(1-methylcyclohexyl)-2-oxoethyl]propenedioate

In the same manner as in Example 58 a), the title compound was obtained from 2-bromo-1-(1-methyl-1-cyclohexyl)-1-ethanone obtained in Example 44 a).

### Example 61 4-(1-methylcyclohexyl)-4-oxobutanoic acid

In the same manner as in Example 58, the title compound was obtained from 2-bromo-1-(1-methyl-1-cyclohexyl)-1-ethanone obtained in Example 44 a).

### Example 62 methyl 4-(1-methylcyclohexyl)-4-oxobutanoate

In the same manner as in Example 59, the title compound was obtained from 4-(1-methylcyclohexyl)-4-oxobutanoic acid obtained in Example 61.

### Example 63 methyl 2-acetyl-4-(1-methylcyclohexyl)-4-oxobutanoate

In the same manner as in Example 1 a), the title compound was obtained from 2-bromo-1-(1-methyl-1-cyclohexyl)-1-ethanone obtained in Example 44 a).

### Example 64 4-cycloheptyl-4-oxobutanoic acid

### a) 2-bromo-1-cycloheptyl-1-ethanone

In the same manner as in Example 5 a) and b), the title compound was obtained from cycloheptanecarboxylic acid.

### b) 4-cycloheptyl-4-oxobutanoic acid

In the same manner as in Example 58, the title compound was obtained from 2-bromo-1-cycloheptyl-1-ethanone obtained in Example 64 a).

### Example 65 trans-4-(4-methylcyclohexyl)-4-oxobutanoic acid

### Production Method 1

### a) dimethyl 2-[2-(trans-4-methylcyclohexyl)-2-oxoethyl]propanedioate

To a solution of dimethyl malonate (2.17 g) in tetrahydrofuran (60 ml) was added sodium hydride (60% in oil dispersion, 0.694 g) at 0°C. After stirring for 30 min, a solution of trans-2-bromo-1-(4-methylcyclohexyl)-1-ethanone (4.00 g) obtained in Example 15 a) in tetrahydrofuran (20 ml) was added dropwise. After the completion of the dropwise, the mixture was stirred at room temperature for 1 hr. Saturated aqueous ammonium chloride solution was added to the reaction mixture, and the mixture was stirred and extracted with ethyl acetate. The organic layer was washed with saturated brine, dried over sodium sulfate, concentrated and purified by silica gel column chromatography to give the title compound (3.901 g).

### b) 2-[2-(trans-4-methylcyclohexyl)-2-oxoethyl]propanedioic acid

To a solution of dimethyl 2-[2-(trans-4-methylcyclohexyl)-2-oxoethyl]propanedioate (3.901 g) obtained in Example 65, Production Method 1 a) in methanol (39.0 ml) was added 1N aqueous sodium hydroxide solution (30.30 ml) at 0°C, and the mixture was stirred for 2 hr. The reaction mixture was acidified with 5% aqueous potassium hydrogensulfate solution, extracted with ethyl acetate, washed with saturated brine, dried over sodium sulfate and concentrated to give the title compound (3.50 g).

### c) trans-4-(4-methylcyclohexyl)-4-oxobutanoic acid

2-[2-(trans-4-Methylcyclohexyl)-2-oxoethyl]propanedioic acid (3.50 g) obtained in Example 65, Production Method 1 b) was heated for 2 hr and purified by recrystallization from ethyl acetate and hexane to give the title compound (1.42 g).

### Production Method 2

### a) 4-methylcyclohexanecarbonyl chloride

To a solution of 4-methyl-1-cyclohexanecarboxylic acid (36.05 g) in methylene chloride (500 ml) were added dropwise dimethylformamide (0.1 ml) and oxalyl chloride (24.33 ml) at 0°C. Then, the mixture was warmed to room temperature, stirred for 1 hr and concentrated to give the title compound (36.30 g).

### b) ethyl 4-(4-methylcyclohexyl)-4-oxo-3,3-bis(tert-butoxycarbonyl)butanoate

To a suspension of sodium hydride (60% in oil dispersion, 17.62 g) in tetrahydrofuran (500 ml) was added dropwise, at room temperature, a solution of di-tert-butyl malonate (98.88 ml) in tetrahydrofuran (500 ml), and the mixture was stirred for 2 hr. A solution of ethyl bromoacetate (37.57 ml) in tetrahydrofuran (250 ml) was dropwise added at 0°C and the mixture was stirred at room temperature for 4.5 hr. Saturated ammonium chloride solution was added to the reaction mixture, and tetrahydrofuran was evaporated under reduced pressure. The resulting mixture was extracted with ethyl acetate, washed with saturated brine, dried over sodium sulfate, concentrated, and distilled under reduced pressure to give di-tert-butyl 2-(ethoxycarbonylmethyl)propanedioate (68.32 g). Then, to a suspension of sodium hydride (60% in oil dispersion, 9.49 g) in tetrahydrofuran (100 ml) was added dropwise di-tert-butyl 2-(ethoxycarbonylmethyl)propanedioate (68.32 g) in tetrahydrofuran (642 ml) at 0°C, and the mixture was stirred at room temperature for 1 hr, to which a solution of 4-methylcyclohexanecarbonyl chloride (36.30 g) obtained in Example 65, Production Method 2 a) in tetrahydrofuran (180 ml) was added dropwise at 0°C. Then, the mixture was stirred at room temperature for 2 hr. Saturated aqueous ammonium chloride solution was added to the reaction mixture, and the mixture was extracted with ethyl acetate, washed successively with saturated aqueous sodium hydrogencarbonate solution and saturated brine, dried over sodium sulfate and concentrated to give the title compound (91.47 g).

### c) ethyl trans-4-(4-methylcyclohexyl)-4-oxobutanoate

p-Toluenesulfonic acid monohydrate (4.08 g) was added to a solution of ethyl 4-(4-methylcyclohexyl)-4-oxo-3,3-bis(tert-butoxycarbonyl)butanoate (91.47 g) obtained in Example 65, Production Method 2 b) in toluene (914 ml), and the mixture was refluxed for 12 hr. Ethyl acetate was added to dilute the reaction mixture, and the mixture was washed successively with saturated aqueous sodium hydrogencarbonate solution and saturated brine, dried over sodium sulfate and concentrated to give the title compound (46.64 g).

### d) trans-4-(4-methylcyclohexyl)-4-oxobutanoic acid

To a solution of ethyl trans-4-(4-methylcyclohexyl)-4-oxobutanoate (46.64 g) obtained in Example 65, Production Method 2 c) in ethanol (500 ml) was added 1N aqueous lithium hydroxide solution (226.7 ml), and the mixture was stirred at room temperature for 12 hr. The reaction mixture was neutralized with saturated aqueous ammonium chloride solution and ethanol was evaporated under reduced pressure. The obtained residue was dissolved in saturated aqueous sodium hydrogencarbonate solution. The obtained aqueous solution was washed with diethyl ether. The aqueous layer was acidified with potassium hydrogensulfate, extracted with ethyl acetate, washed with saturated brine, dried over sodium sulfate, concentrated and purified by recrystallization from ethyl acetate and hexane to give the title compound (27.0 g) and a mixture (8.16 g) of cis-4-(4-methylcyclohexyl)-4-oxobutanoic acid and trans-4-(4-methylcyclohexyl)-4-oxobutanoic acid.

### Production Method 3

### a) ethyl hydrogen malonate

To a solution of potassium ethyl malonate (50 g) in water (30 ml) was added dropwise concentrated hydrochloric acid (25 ml) at a temperature of 5 to 10°C. The precipitated potassium chloride was filtered off and the filtrate was extracted with diethyl ether, dried over magnesium sulfate and concentrated to give the title compound (38.8 g).

### b) ethyl (4-methylcyclohexylcarbonyl)acetate

To a solution of ethyl hydrogen malonate (3.50 g) obtained in Example 65, Production Method 3 a) in tetrahydrofuran (80 ml) was added dropwise a solution (1.68 M, 31.5 ml) of n-butyllithium in hexane at a temperature of from -78°C to -30°C. The mixture was stirred at -10°C for 30 min. Then, a solution of 4-methylcyclohexanecarbonyl chloride (2.0 g) obtained in Example 65, Production Method 2 a) in tetrahydrofuran (20 ml) was dropwise added at -78°C. After the completion of the dropwise addition, the mixture was warmed to room temperature and stirred for 1 hr. A 5% aqueous potassium hydrogensulfate solution was added to the reaction mixture, and the mixture was extracted with diethyl ether, washed successively with saturated aqueous sodium hydrogencarbonate solution and saturated brine, dried over sodium sulfate and concentrated to give the title compound (2.38 g).

### c) diethyl (4-methylcyclohexylcarbonyl)butanedioate

To a solution of sodium ethoxide (0.321 g) in ethanol (40 ml) was added dropwise a solution of ethyl (4-methylcyclohexylcarbonyl)acetate (1.0 g) obtained in Example 65, Production Method 3 b) in ethanol (4.0 ml) at room temperature. Then, the mixture was refluxed for 30 min, and ethyl bromoacetate (0.522 ml) was added dropwise. The mixture was refluxed for 30 min. The reaction mixture was neutralized with saturated aqueous ammonium chloride solution and ethanol was evaporated under reduced pressure. The residue was dissolved in ethyl acetate, washed with saturated brine, dried over sodium sulfate and concentrated to give the title compound (1.40 g).

### d) trans-4-(4-methylcyclohexyl)-4-oxobutanoic acid

To a solution of diethyl (4-methylcyclohexylcarbonyl)butanedioate (1.40 g) obtained in Example 65, Production Method 3 c) in ethanol (20 ml) was added 1M aqueous lithium hydroxide solution (20 ml), and the mixture was stirred at room temperature for 1 hr. The reaction mixture was neutralized witn saturated aqueous ammonium chloride solution and ethanol was evaporated under reduced pressure. The obtained residue was dissolved in diethyl ether and extracted with saturated aqueous sodium hydrogencarbonate solution. The aqueous layer was acidified with potassium hydrogensulfate, extracted with ethyl acetate, washed with saturated brine, dried over sodium sulfate and concentrated. The residue was dissolved in toluene (30 ml), to which p-toluenesulfonic acid monohydrate (80 mg) was added and the mixture was refluxed for 4 hr. The reaction mixture was diluted with ethyl acetate, washed with saturated brine, dried over sodium sulfate, concentrated and purified by recrystallization from ethyl acetate and hexane to give the title compound (0.612 g).

### Production Method 4

### a) 4-(4-methylphenyl)-4-oxobutanoic acid

To a solution of aluminum chloride (100 g) in methylene chloride (450 ml) was added toluene (44.0 ml), and succinic anhydride (34.02 g) was added at room temperature. Then, the mixture was stirred for 12 hr. The reaction mixture was poured on ice (650 g), and the mixture was acidified with concentrated hydrochloric acid and extracted with ethyl acetate. The organic layer was washed with water and saturated brine, dried over sodium sulfate and concentrated to give the title compound (62.1 g).

### b) methyl 4-(4-methylphenyl)-4-oxobutanoate

To a solution of 4-(4-methylphenyl)-4-oxobutanoic acid (91.2 g) obtained in Example 65, Production Method 4 a) in methanol (760 ml) was added concentrated sulfuric acid (1.33 ml), and the mixture was refluxed for 2 hr. The reaction mixture was cooled to room temperature and methanol was evaporated under reduced pressure. The obtained residue was dissolved in ethyl acetate, washed successively with water, saturated aqueous sodium hydrogencarbonate solution and saturated brine, dried over sodium sulfate and concentrated to give the title compound (99.4 g).

### c) methyl 4,4-ethylenedioxy-4-(4-methylphenyl)butanoate

To a solution of methyl 4-(4-methylphenyl)-4-oxobutanoate (20.0 g) obtained in Example 65, Production Method 4 b) in ethylene glycol (100 ml) were added pyridinium p-toluenesulfonate (2.44 g) and trimethyl orthoformate (64 ml), and the mixture was heated at 95°C for 30 hr while removing the generated methanol. The reaction mixture was cooled to room temperature and distilled water was added. The mixture was extracted with toluene, and the organic layer was washed successively with 5% aqueous citric acid solution, water, saturated aqueous sodium hydrogencarbonate solution, water and saturated brine, and concentrated to give the title compound (23.5 g).

### d) methyl 4-(4-methylcyclohexyl)-4-oxobutanoate

To a solution of methyl 4,4-ethylenedioxy-4-(4-methylphenyl)butanoate (1.0 g) obtained in Example 65, Production Method 4 c) in ethyl acetate (10.0 ml) was added 5% rhodium-carbon (0.2 g), and the mixture was stirred for 5 hr at room temperature under a hydrogen atmosphere at 3 atm. 5% Rhodium-carbon was filtered off, and the filtrate was concentrated. The obtained residue was dissolved in acetic acid (8.0 ml) and water (2.0 ml) was added. The mixture was refluxed for 1 hr. The reaction mixture was cooled to room temperature, and acetic acid was evaporated under reduced pressure. Water was added to the obtained residue, and the mixture was extracted with toluene. The organic layer was washed successively with water, saturated aqueous sodium hydrogencarbonate solution, water and saturated brine, dried over sodium sulfate and concentrated to give the title compound (0.787 g).

### e) trans-4-(4-methylcyclohexyl)-4-oxobutanoic acid

To a solution of methyl 4-(4-methylcyclohexyl)-4-oxobutanoate (21.22 g) obtained in Example 65, Production Method 4 d) in methanol (15 ml) was added 1N aqueous sodium hydroxide solution (150 ml), and the mixture was refluxed for 2 hr. The reaction mixture was ice-cooled, and was acidified with concentrated hydrochloric acid. The mixture was extracted with ethyl acetate, washed with saturated brine, dried over sodium sulfate, concentrated and purified by recrystallization from ethyl acetate and hexane and then was recrystallized from ethanol and water, to give the title compound (10.7 g).

### Production Method 5

### a) methyl 4-methylcyclohexanecarboxylate

To a solution of 4-methyl-1-cyclohexanecarboxylic acid (25 g) in methanol (250 ml) was added concentrated sulfuric acid (0.94 ml), and the mixture was stirred at room temperature for 12 hr. The reaction mixture was concentrated, diluted with ethyl acetate, washed with saturated aqueous sodium hydrogencarbonate solution and saturated brine, dried over sodium sulfate and concentrated to give the title compound (27.0 g).

### b) 4-(1-methoxycarbonyl-4-metylcyclohexyl)-4-oxobutanoic acid

To a solution of methyl 4-methylcyclohexanecarboxylate (11.87 g) obtained in Example 65, Production Method 5 a) in tetrahydrofuran (15 ml) was added dropwise a solution (1.5 M, 53.1 ml) of lithium diisopropylamide in cyclohexane at -5°C, and the mixture was stirred for 2 hr. The obtained solution was added dropwise to a solution of succinic anhydride (7.61 g) in tetrahydrofuran (91 ml) at room temperature, and the mixture was stirred for 1 hr. Saturated aqueous ammonium chloride solution was added to the reaction mixture, and tetrahydrofuran was evaporated under reduced pressure. The reaction mixture was acidified with concentrated hydrochloric acid and the mixture was extracted with ethyl acetate, washed with water and saturated brine, dried over magnesium sulfate and concentrated to give the title compound (17.55 g).

### c) trans-4-(4-methylcyclohexyl)-4-oxobutanoic acid

4-(1-Methoxycarbonyl-4-methylcyclohexyl)-4-oxobutanoic acid (17.55 g) obtained in Example 65, Production Method 5 b) was dissolved in a 2N aqueous sodium hydroxide solution (120 ml), and the mixture was refluxed for 2 hr. The reaction mixture was acidified with concentrated hydrochloric acid under ice-cooling, and the mixture was extracted with ethyl acetate. The organic layer was washed with water and saturated brine, dried over sodium sulfate and concentrated. The residue was purified by recrystallization from ethyl acetate and hexane to give the title compound (6.50 g).

### Production Method 6

### a) 1-chloro-4-methylcyclohexane

Phosphorus pentachloride (497.07 g) was suspended in hexane (2.43 L), and 4-methylcyclohexanol (248.7 g) was added dropwise at 5°C under a nitrogen stream. The reaction mixture was neutralized with 5N aqueous sodium hydroxide solution. The organic layer was washed with distilled water, dried over sodium sulfate, concentrated and purified by distillation (b.p.₄₀, 70-71 °C) to give the title compound (110.65 g).

### b) trans-4-(4-methylcyclohexyl)-4-oxobutanoic acid

To a mixture of magnesium (9.27 g) and tetrahydrofuran (5.0 ml) was added, under a nitrogen stream, 1-chloro-4-methylcyclohexane (1.00 g) obtained in Example 65, Production Method 6 a) and then was added iodine (50 mg), and the mixture was stirred for 5 min. Tetrahydrofuran (150 ml) was added, and 1-chloro-4-methylcyclohexane (49.00 g) obtained in Example 65, Production Method 6 a) was added dropwise over 30 min while refluxing. The mixture was refluxed for 1 hr and cooled to room temperature. Then, succinic anhydride (34.21 g) and iron(III) acetylacetonate (3.07 g) were dissolved in tetrahydrofuran (330 ml), to which the mixture (210.86 ml) prepared above was dropwise added at 5°C, and the mixture was stirred for 30 min. Concentrated hydrochloric acid (50 ml) was dropwise added to the reaction mixture at 10°C, and the precipitated salt was filtered off and tetrahydrofuran was evaporated. The residue was dissolved in toluene, washed with water. Toluene was evaporated and the residue was dissolved in a mixed solution of 5N aqueous sodium hydroxide solution (100 ml) and ethanol (50 ml). The mixture was stirred at 3°C for 12 hr. The mixture was neutralized with concentrated hydrochloric acid at 0°C, and the precipitated crystals were collected and purified by recrystallization from ethanol and water to give the title compound (36.68 g).

### Example 66 1,2,3,4-tetrahydro-1-oxo-2-naphthaleneacetic acid

### a) 2-bromo-3,4-dihydro-1(2H)-naphthalenone

To a solution of 3,4-dihydro-1(2H)-naphthalenone (1.99 ml) in acetic acid (30 ml) was added dropwise bromine (0.85 ml) at room temperature, and the mixture was stirred for 1 hr. A 5% aqueous solution of sodium hypochlorite was added to the reaction mixture under ice-cooling, and the mixture was stirred. Then, the mixture was extracted with diethyl ether. The organic layer was washed with saturated aqueous sodium hydrogencarbonate solution and saturated brine, dried over sodium sulfate and concentrated to give the title compound (3.24 g).

### b) 1,2,3,4-tetrahydro-1-oxo-2-naphthaleneacetic acid

In the same manner as in Example 58, the title compound was obtained from 2-bromo-3,4-dihydro-1(2H)-naphthalenone obtained in Example 66 a).

### Example 67 dimethyl 2-(3-cyclohexyl-2-oxopropyl)propanedioate

In the same manner as in Example 58 a), the title compound was obtained from 1-bromo-3-cyclohexyl-2-propanone obtained in Example 9 b).

### Example 68 2-(2-oxo-2-phenylethyl)propanedioic acid

In the same manner as in Example 58 a) and b), the title compound was obtained from 2-bromoacetophenone.

### Example 69 2-[2-(4-biphenylyl)-2-oxoethyl]propanedioic acid

In the same manner as in Example 58 a) and b), the title compound was obtained from 2-bromo-4'-phenylacetophenone.

### Example 70 2-[2-(4-biphenylyl)-2-oxoethyl]-2-methylpropanedioic acid

In the same manner as in Example 58 a) and b), the title compound was obtained from 2-bromo-4'-phenylacetophenone and diethyl methylmalonate.

### Example 71 3-methyl-4-oxo-4-phenylbutanoic acid

### a) 2-bromopropiophenone

In the same manner as in Example 5 b), the title compound was obtained from propiophenone.

### b) dimethyl 2-(2-oxo-2-phenyl-1-methylethyl)propanedioate

In the same manner as in Example 58 a), the title compound was obtained from 2-bromopropiophenone obtained in Example 71 a).

### c) methyl 3-methyl-4-oxo-4-phenylbutanoate

To a solution of dimethyl 2-(2-oxo-2-phenyl-1-methylethyl)propanedioate (2.64 g) obtained in Example 71 b) in dimethyl sulfoxide (60 ml) were added distilled water (0.36 ml) and lithium chloride (0.424 g) at room temperature, and the mixture was stirred at 170°C for 2.5 hr.

Distilled water was added to the reaction mixture, and the mixture was stirred and extracted with ethyl acetate. The organic layer was washed with saturated brine, dried over magnesium sulfate, concentrated and purified by silica gel column chromatography to give the title compound (1.33 g).

### d) 3-methyl-4-oxo-4-phenylbutanoic acid

In the same manner as in Example 49 d), the title compound was obtained from methyl 3-methyl-4-oxo-4-phenylbutanoate obtained in Example 71 c).

### Example 72 2-methyl-4-oxo-4-phenylbutanoic acid

### a) 2-methyl-2-(2-oxo-2-phenylethyl)propanedioic acid

In the same manner as in Example 70, the title compound was obtained from 2-bromoacetophenone.

### b) 2-methyl-4-oxo-4-phenylbutanoic acid

In the same manner as in Example 58 c), the title compound was obtained from 2-methyl-2-(2-oxo-2-phenylethyl)propanedioic acid obtained in Example 72 a).

### Example 73 4-oxo-2,4-diphenylbutanoic acid

### a) 4-oxo-2,4-diphenylbutanenitrile

To a solution of trans-chalcone (20.8 g) in ethanol (350 ml) was added acetic acid (5.7 ml) at 35°C, and a solution of potassium cyanide (13.0 g) in water (30 ml) was dropwise added, and the mixture was stirred at 40°C for 4 hr. Then the mixture was left standing at 0°C for 12 hr and the precipitated crystals were collected by filtration to give the title compound (21.0 g).

### b) 4-oxo-2,4-diphenylbutanoic acid

In the same manner as in Example 51 c), the title compound was obtained from 4-oxo-2,4-diphenylbutanenitrile obtained in Example 73 a).

### Example 74 4-(2-naphthyl)-4-oxobutanoic acid

### a) 4-(2-naphthyl)-4-oxobutanenitrile

In the same manner as in Example 51 a), the title compound was obtained from 2-naphthaldehyde.

### b) 4-(2-naphthyl)-4-oxobutanoic acid

In the same manner as in Example 51 c), the title compound was obtained from 4-(2-naphthyl)-4-oxobutanenitrile obtained in Example 74 a).

### Example 75 4-(indan-2-yl)-4-oxobutanoic acid

### a) indan-2-carboxylic acid

To a solution of 2,2-dimethyl-(spiro[1,3]dioxane-5,2'-indan)-4,6-dione (14.1 g) in tetrahydrofuran (100 ml) was added 1N hydrochloric acid (30 ml), and the mixture was refluxed for 4 hr. 1N Aqueous sodium hydroxide solution was added to the reaction mixture. The mixture was washed with ethyl acetate and the aqueous layer was acidified with concentrated hydrochloric acid. The mixture was extracted with ethyl acetate, dried over magnesium sulfate and concentrated. The obtained residue was heated at 170 °C for 1 hr to give the title compound (2.53 g).

### b) 1-(indan-2-yl)-2-propen-1-one

To a solution of indan-2-carboxylic acid (1.624 g) obtained in Example 75 a) in dichloromethane (20 ml) was added oxalyl chloride (2.0 ml) at 0°C. Dimethylformamide (0.05 ml) was added and the the mixture was stirred for 2 hr. The reaction mixture was concentrated and the obtained residue was dissolved in chloroform (10 ml), to which were added vinyltributyltin (3.2 ml) and trans-benzyl(chloro)bis(triphenylphosphine)palladium(II) (0.070 g), and the mixture was refluxed for 1 hr. Saturated aqueous sodium fluoride solution (10 ml) was added to the reaction mixture and the mixture was stirred at room temperature for 12 hr. The mixture was filtered through a Celite pad and the filtrate was extracted with ethyl acetate, dried over magnesium sulfate, concentrated and purified by silica gel column chromatography to give the title compound (1.186 g).

### c) 4-(indan-2-yl)-4-oxobutanenitrile

To a solution of 1-(indan-2-yl)-2-propen-1-one (1.186 g) obtained in Example 75 b) in ethanol (24 ml) was added acetic acid (0.40 ml), and a solution of sodium cyanide (0.667 g) in water (2.6 ml) was dropwise added at 40°C, which was followed by stirring for 30 min. Ethanol was evaporated from the reaction mixture, and distilled water was added. After stirring, the reaction mixture was extracted with ethyl acetate. The organic layer was washed with distilled water and saturated brine, dried over magnesium sulfate, concentrated and purified by silica gel column chromatography to give the title compound (1.04 g).

### d) 4,4-ethylenedioxy-4-(indan-2-yl)butanenitrile

To a solution of 4-(indan-2-yl)-4-oxobutanenitrile (1.00 g) obtained in Example 75 c) in benzene (5.0 ml) were added ethylene glycol (0.6 ml) and p-toluenesulfonic acid monohydrate (0.101 g), and the mixture was refluxed in a Dean-Stark trap for 12 hr. Benzene was added to the reaction mixture and the mixture was washed with saturated sodium hydrogencarbonate solution and saturated brine, dried over magnesium sulfate, concentrated and purified by silica gel column chromatography to give the title compound (1.185 g).

### e) 4-(indan-2-yl)-4-oxobutanoic acid

To a solution of 4,4-ethylenedioxy-4-(indan-2-yl)butanenitrile (0.735 g) obtained in Example 75 d) in ethanol (3.0 ml) was added 2N aqueous sodium hydroxide solution (4.5 ml) and the mixture was refluxed for 3 hr. The reaction mixture was acidified with 1N hydrochloric acid and extracted with ethyl acetate, washed with saturated brine, dried over magnesium sulfate and concentrated. The obtained residue was dissolved in tetrahydrofuran (4 ml), to which was added concentrated hydrochloric acid (1 ml) and the mixture was stirred at room temperature for 12 hr. Tetrahydrofuran was evaporated, and the residue was extracted with ethyl acetate, washed with saturated brine, dried over magnesium sulfate and concentrated to give the title compound (0.597 g).

### Example 76 (2R*,3S*)-2,3-dimethyl-4-oxo-4-phenylbutanoic acid

### a) 2-phenyl-2-(trimethylsilyloxy)ethanenitrile

To a solution of benzaldehyde (10.2 ml) in dichloromethane (200 ml) was added trimethylsilyl cyanide (13.4 ml), and triethylamine (1.40 ml) was added dropwise at 0°C. The reaction mixture was concentrated and the obtained residue was distilled under reduced pressure (b.p._{1.1}Torr 88-97°C) to give the title compound (19.193 g).

### b) methyl 2,3-dimethyl-4-cyano-4-phenyl-4-(trimethylsilyloxy)butanoate

A solution (1.5M, 7.5 ml) of lithium diisopropylamide in cyclohexane was diluted with tetrahydrofuran (10 ml) at -78°C under an argon atmosphere. A solution of 2-phenyl-2-(trimethylsilyloxy)ethanenitrile (2.059 g) obtained in Example 76 a) in tetrahydrofuran (6.0 ml) was added dropwise, and the mixture was stirred for 30 min. Methyl tiglate was added, and the mixture was stirred for 30 min. Distilled water was added to the reaction mixture, and the mixture was warmed to room temperature. 1N Hydrochloric acid was added and the mixture was extracted with diethyl ether. The organic layer was washed with saturated brine, dried over magnesium sulfate and concentrated to give the title compound (3.148 g).

### c) methyl (2R*,3S*)-2,3-dimethyl-4-oxo-4-phenylbutanoate

To a solution of methyl 2,3-dimethyl-4-cyano-4-phenyl-4-(trimethylsilyloxy)butanoate (3.08 g) obtained in Example 76 b) in tetrahydrofuran (10 ml) was added a solution (1M, 10 ml) of tetrabutylammonium fluoride in tetrahydrofuran, and the mixture was stirred for 30 min. Distilled water was added to the reaction mixture, and the mixture was extracted with diethyl ether, washed with saturated brine, dried over magnesium sulfate and concentrated. The obtained residue was dissolved in methanol (10 ml), to which sodium methoxide (0.227 g) was added, and the mixture was stirred for 1 hr at room temperature. Saturated aqueous ammonium chloride solution was added to the reaction mixture, and the mixture was extracted with diethyl ether. The organic layer was washed with distilled water and saturated brine, dried over magnesium sulfate, concentrated and purified by silica gel column chromatography to give the title compound (0.412 g).

### d) (2R*,3S*)-2,3-dimethyl-4-oxo-4-phenylbutanoic acid

To a solution of methyl (2R*,3S*)-2,3-dimethyl-4-oxo-4-phenylbutanoate (0.218 g) obtained in Example 76 c) in methanol (2.0 ml) was added 2N aqueous sodium hydroxide solution at 0°C, and the mixture was stirred for 1 hr. 1N Aqueous citric acid solution was added to the reaction mixture, and the mixture was extracted with ethyl acetate. The organic layer was washed with distilled water and saturated brine, dried over magnesium sulfate and concentrated to give the title compound (0.197 g).

### Example 77 (1R*,2S*)-2-benzoylcyclohexanecarboxylic acid

To a solution of cis-cyclohexanedicarboxylic anhydride (1.543 g) in diethyl ether (30 ml) was added dropwise, under an argon atmosphere at 0°C, a solution (1.0M, 12.0 ml) of phenylmagnesium bromide in tetrahydrofuran, and the mixture was stirred for 1.5 hr. 1N Hydrochloric acid was added to the reaction mixture, and then distilled water was added. The mixture was extracted with ethyl acetate. The organic layer was washed with distilled water and saturated brine, dried over magnesium sulfate and concentrated to give the title compound (2.368 g).

### Example 78 (1R*,2S*)-2-benzoylcyclopentanecarboxylic acid

### a) 1-bromocyclopentyl phenyl ketone

In the same manner as in Example 4 a), the title compound was obtained from cyclopentyl phenyl ketone.

### b) 1-cyclopentenyl phenyl ketone

A solution of 1-bromocyclopentyl phenyl ketone (12.66 g) obtained in Example 78 a) in pyridine (20 ml) was refluxed for 12 hr. The reaction mixture was poured into 1N hydrochloric acid under ice-cooling, and the mixture was extracted with diethyl ether. The organic layer was washed successively with distilled water, saturated aqueous sodium hydrogencarbonate solution and saturated brine, dried over magnesium sulfate, concentrated and purified by silica gel column chromatography to give the title compound (4.70 g).

### c) (1R*,2R*)-2-benzoylcyclopentanecarboxylic acid

In the same manner as in Example 73, the title compound was obtained from 1-cyclopentenyl phenyl ketone obtained in Example 78 b).

### d) tert-butyl (1R*,2R*)-2-benzoylcyclopentanecarboxylate

To a solution of (1R*,2R*)-2-benzoylcyclopentanecarboxylic acid (0.654 g) obtained in Example 78 c) in dichloromethane (10 ml) was added concentrated hydrochloric acid (0.1 ml) and then isobutene (3.0 ml) was added at -20°C, and the mixture was warmed to room temperature and stirred for 12 hr. Saturated aqueous sodium hydrogencarbonate solution was added to the reaction mixture, and the mixture was extracted with diethyl ether, dried over magnesium sulfate, concentrated and purified by silica gel column chromatography to give the title compound (0.425 g).

### e) tert-butyl (1R*,2S*)-2-benzoylcyclopentanecarboxylate

A solution (1.5M, 1.40 ml) of lithium diisopropylamide in tetrahydrofuran was diluted with tetrahydrofuran (3.0 ml) under an argon atmosphere at -78°C, and a solution of tert-butyl (1R*,2R*)-2-benzoylcyclopentanecarboxylate (0.277 g) obtained in Example 78 d) in tetrahydrofuran (3.0 ml) was added dropwise. The mixture was stirred for 2 hr. Saturated aqueous ammonium chloride solution was added to the reaction mixture and the mixture was warmed to room temperature, and 1N aqueous citric acid solution was added. The mixture was extracted with diethyl ether, and the organic layer was washed with saturated brine, dried over magnesium sulfate, concentrated and purified by silica gel column chromatography to give the title compound (0.122 g).

### f) (1R*,2S*)-2-benzoylcyclopentanecarboxylic acid

tert-Butyl (1R*,2S*)-2-benzoylcyclopentanecarboxylate (94 mg) obtained in Example 78 e) was dissolved in formic acid (2 ml), and the mixture was stirred at room temperature for 1.5 hr. Formic acid was evaporated and distilled water was added. The mixture was stirred and extracted with ethyl acetate. The extract was dried over magnesium sulfate and concentrated to give the title compound (74 mg).

### Example 79 3-(4-phenylbenzoyl)pentanedioic acid

### a) benzyl hydrogen malonate

To a solution of dibenzyl malonate (5.0 ml) in benzyl alcohol (10 ml) was added dropwise a solution of potassium hydroxide (1.164 g) in benzyl alcohol (10 ml) at room temperature, and the mixture was stirred for 3 hr. Diethyl ether was added to the reaction mixture. The aqueous layer was washed with diethyl ether. The aqueous layer was acidified with concentrated hydrochloric acid, and the mixture was extracted with ethyl acetate. The organic layer was washed with saturated brine, dried over magnesium sulfate and concentrated to give the title compound (3.296 g).

### b) benzyl (4-phenylbenzoyl)acetate

To a solution of 4-phenylbenzoic acid (17.98 g) in dichloromethane (200 ml) was added oxalyl chloride (12.69 g) at 0°C. Then, dimethylformamide (0.1 ml) was added, and the mixture was stirred at room temperature for 1 hr and then stirred at 50°C for 4 hr. The reaction mixture was concentrated to give 4-phenylbenzoyl chloride (20.11 g). Then, to a solution of benzyl hydrogen malonate (1.98 g) obtained in Example 79 a) in tetrahydrofuran (25 ml) was dropwise added a solution (1.69M, 12 ml) of n-butyllithium in hexane under an argon atmosphere at -78°C. The mixture was stirred for 30 min. Then, a solution of 4-phenylbenzoyl chloride (2.178 g) in tetrahydrofuran (25 ml) was dropwise added and the mixture was stirred for 30 min. 1N Hydrochloric acid was added to the reaction mixture at room temperature, and the mixture was extracted with ethyl acetate. The organic layer was washed with saturated brine, dried over magnesium sulfate, concentrated and purified by silica gel column chromatography to give the title compound (1.285 g).

### c) diethyl 3-benzyloxycarbonyl-3-(4-phenylbenzoyl)pentanedioate

To a solution of benzyl (4-phenylbenzoyl)acetate (0.229 g) obtained in Example 79 b) in dimethylformamide (3.0 ml) was added sodium hydride (60% in oil dispersion, 30 mg) at room temperature and then ethyl bromoacetate (80 µl) was added, and the mixture was stirred for 30 min. Sodium hydride (60% in oil dispersion, 30 mg) was further added to the reaction mixture. Ethyl bromoacetate (150 µl) was added, and the mixture was stirred for 12 hr. Distilled water was added to the reaction mixture, and the mixture was stirred and extracted with ethyl acetate. The organic layer was washed with distilled water and saturated brine, concentrated and purified by silica gel column chromatography to give the title compound (0.263 g).

### d) diethyl 3-(4-phenylbenzoyl)pentanedioate

To a solution of diethyl 3-benzyloxycarbonyl-3-(4-phenylbenzoyl)pentanedioate (0.263 g) obtained in Example 79 c) in ethyl acetate (6.0 ml) was added 10% palladium-carbon (0.301 g), and the mixture was stirred at room temperature under a hydrogen atmosphere for 2 hr. Palladium-carbon was filtered off through a Celite pad, and the filtrate was concentrated and purified by silica gel column chromatography to give the title compound (0.093 g).

### e) 3-(4-phenylbenzoyl)pentanedioic acid

To a solution of diethyl 3-(4-phenylbenzoyl)pentanedioate (93 mg) obtained in Example 79 d) in methanol (2.5 ml) was added 1M aqueous lithium hydroxide solution (0.75 ml) at room temperature, and the mixture was stirred for 12 hr. The reaction mixture was diluted with distilled water and 1N hydrochloric acid was added. The mixture was extracted with ethyl acetate. The organic layer was washed with distilled water and saturated brine, dried over magnesium sulfate and concentrated to give the title compound (70 mg).

### Example 80 2-acetaminoacetophenone

To a solution of 2-aminoacetophenone hydrochloride (2.0 g) in pyridine (20 ml) was added dropwise acetyl chloride (0.829 ml), and the mixture was refluxed for 20 min. The reaction mixture was poured into ice water, and the mixture was extracted with ethyl acetate. The organic layer was washed successively with 50% aqueous potassium hydrogensulfate solution, saturated aqueous sodium hydrogencarbonate solution and saturated brine, dried over sodium sulfate and purified by silica gel column chromatography to give the title compound (1.180 g).

### Example 81 (±)-4-benzoyldihydro-2(3H)-furanone

### a) (±)-2-trimethylsilyloxyphenylacetanitrile

To a solution of benzaldehyde (10.2 ml) and trimethylsilylnitrile (13.4 ml) in methylene chloride (200 ml) was added dropwise triethylamine (140 ml), and the mixture was stirred at room temperature for 3 hr. The solvent was evaporated and the residue was distilled (b.p._{1.1} Torr 88-97°C) to give the title compound (19.2 g).

### b) (±)-4-benzoyldihydro-2(3H)-furanone

To a solution of lithium diisopropylamide (35 ml, 1.5M in cyclohexane solution) in tetrahydrofuran (10 ml) was added dropwise, under an argon atmosphere, a solution of (±)-2-trimethylsilyloxyphenylacetonitrile (1.02 g) obtained in Example 81 a) in tetrahydrofuran (5 ml), and the mixture was stirred at -72°C for 30 min. Then, 2(5H)-furanone (0.36 ml) was dropwise added, and the mixture was stirred for 1 hr. The reaction was quenched with saturated aqueous ammonium chloride solution and the reaction mixture was warmed to room temperature. The mixture was extracted with ethyl acetate, and the organic layer was washed with distilled water and saturated brine and dried over magnesium sulfate. The solvent was evaporated, and to a solution of the residue and acetic acid (0.50 ml) in tetrahydrofuran (5 ml) was added dropwise a 1.0M tetrabutylammonium fluoride/tetrahydrofuran solution (5.5 ml). The mixture was stirred at room temperature for 30 min. Distilled water was added to the reaction mixture, and the mixture was extracted with ethyl acetate. The organic layer was washed with distilled water and saturated brine, and dried over magnesium sulfate. The solvent was evaporated and the residue was purified by silica gel column chromatography (hexane:ethyl acetate=62:38) to give the title compound (0.68 g).

### Example 82 (±)-dihydro-4-(4'-phenylbenzoyl)-2(3H)-furanone

### a) (±)-2-trimethylsilyloxy-4'-biphenylacetonitrile

In the same manner as in Example 81 a), the title compound was obtained from 4-phenylbenzaldehyde.

### b) (±)-dihydro-4-(4'-phenylbenzoyl)-2(3H)-furanone

In the same manner as in Example 81 b), the title compound was obtained from (±)-2-trimethylsilyloxy-4'-biphenylacetonitrile obtained in Example 82 a).

### Example 83 (±)-dihydro-4-methyl-4-(4'-phenylbenzoyl)-2(3H)-furanone

In the same manner as in Example 81 b), the title compound was obtained from (±)-2-trimethylsilyloxy-4'-biphenylacetonitrile obtained in Example 82 a) and 4-methyl-2(5H)-furanone.

### Example 84 (±)-3-benzoyl-1-cyclopentanone

In the same manner as in Example 81 b), the title compound was obtained from (±)-2-trimethylsilyloxyphenylacetonitrile obtained in Example 81 a) and 2-cyclopenten-1-one.

### Example 85 (±)-3-benzoyl-3-methyl-1-cyclopentanone

In the same manner as in Example 81 b), the title compound was obtained from (±)-2-trimethylsilyloxyphenylacetonitrile obtained in Example 81 a) and 3-methyl-2-cyclopenten-1-one.

### Example 86 (±)-3-methyl-3-(4'-phenylbenzoyl)-1-cyclopentanone

In the same manner as in Example 81 b), the title compound was obtained from (±)-2-trimethylsilyloxy-4'-biphenylacetonitrile obtained in Example 82 a) and 3-methyl-2-cyclopenten-1-one.

### Example 87 (±)-dihydro-4-(4'-hydroxymethylbenzoyl)-2(3H)-furanone

### a) 4-hydroxymethylbenzaldehyde

To a solution of sodium tetrahydroborate (12.6 g) in methanol (500 ml) was dropwise added a solution of terephthalaldehyde monodiethyl acetal (60.5 ml) in methanol (100 ml), and the mixture was stirred at room temperature for 4 hr. Methanol was evaporated, to which saturated brine was added, and the mixture was extracted with ether. The organic layer was washed with water and saturated brine, and dried over magnesium sulfate. The solvent was evaporated, and the residue was dissolved in chloroform (200 ml) and 25% aqueous trifluoroacetic acid solution (100 ml) was added. The mixture was stirred at room temperature for 2 hr. The organic layer was washed with water and saturated aqueous sodium hydrogencarbonate solution and dried over potassium carbonate. The solvent was evaporated to give the title compound.

### b) (±)-4'-tert-butyldimethylsilyloxymethyl-2-trimethylsilyloxyphenylacetonitrile

To a solution of 4-hydroxymethylbenzaldehyde (32.8 g) obtained in Example 87 a) and imidazole (40.9 g) in dimethylformamide (200 ml) was added tert-butylchlorodimethylsilane (43.6 g) and the mixture was stirred at room temperature overnight. The reaction mixture was poured into ice water, and the mixture was extracted with ether. The organic layer was washed with water and saturated brine and dried over potassium carbonate. The solvent was evaporated, and the residue was treated in the same manner as in Example 81 a) to give the title compound (b.p._{0.29}Torr 145-152°C).

### c) (±)-dihydro-4-(4'-hydroxymethylbenzoyl)-2(3H)-furanone

In the same manner as in Example 81 b), the title compound was obtained from (±)-4'-tert-butyldimethylsilyloxymethyl-2-trimethylsilyloxyphenylacetonitrile obtained in Example 87 b).

### Example 88 (±)-3-(4'-hydroxymethylbenzoyl)-3-methyl-1-cyclopentanone

In the same manner as in Example 81 b), the title compound was obtained from (±)-4'-tert-butyldimethylsilyloxymethyl-2-trimethylsilyloxyphenylacetonitrile obtained in Example 87 b) and 3-methyl-2-cyclopenten-1-one.

### Example 89 (±)-5-benzoyldihydro-2(3H)-furanone

### a) ethyl (±)-4-benzoyl-4-hydroxybutyrate

In the same manner as in Example 81 b), the title compound was obtained from (±)-2-trimethylsilyloxyphenylacetonitrile obtained in Example 81 a) and ethyl semialdehydesuccinate.

### b) (±)-5-benzoyldihydro-2(3H)-furanone

(±)-4-Benzoyl-4-hydroxybutyric acid (176 mg) was refluxed in acetic acid (2 ml) for 2 hr and the solvent was evaporated to give the title compound.

### Example 90 (±)-dihydro-5-(4'-phenylbenzoyl)-2(3H)-furanone

In the same manner as in Example 89, the title compound was obtained from (±)-2-trimethylsilyloxy-4'-biphenylacetonitrile obtained in Example 82 a).

### Example 91 (±)-dihydro-5-(4'-hydroxymethylbenzoyl)-2(3H)-furanone

### a) ethyl (±)-4-hydroxy-4-(4'-hydroxymethylbenzoyl)butyrate

In the same manner as in Example 89 a), the title compound was obtained from (±)-4'-tert-butyldimethylsilyloxymethyl-2-trimethylsilyloxyphenylacetonitrile obtained in Example 87 b).

### b) (±)-dihydro-5-(4'-hydroxymethylbenzoyl)-2(3H)-furanone

A solution of ethyl (±)-4-hydroxy-4-(4'-hydroxymethylbenzoyl)butyrate (0.248 g) obtained in Example 91 a) and p-toluenesulfonic acid hydrate (19 mg) in benzene (5 ml) was refluxed for 3 hr. The reaction mixture was washed with water and saturated brine and dried over magnesium sulfate. The solvent was evaporated to give the title compound.

### Example 92 (±)-dihydro-5-(4'-hydroxymethylbenzoyl)-5-methyl-2(3H)-furanone

In the same manner as in Example 91, the title compound was obtained from (±)-4'-tert-butyldimethylsilyloxymethyl-2-trimethylsilyloxyphenylacetonitrile obtained in Example 87 b) and ethyl levulinate.

### Example 93 (-)-dihydro-5-methyl-5-(4'-phenylbenzoyl)-2(3H)-furanone

### a) (±)-4-hydroxy-4-(4'-phenylbenzoyl)valeric acid

Ethyl (±)-4-hydroxy-4-(4'-phenylbenzoyl)valerate obtained in the same manner as in Example 81 b) from (±)-2-trimethylsilyloxy-4'-biphenylacetonitrile obtained in Example 82 a) and ethyl levulinate was dissolved in methanol. 1M Aqueous lithium hydroxide solution was added, and the mixture was stirred at room temperature overnight. Methanol was evaporated and the mixture was acidified with 10% aqueous citric acid solution. The mixture was extracted with ethyl acetate and dried over magnesium sulfate. The solvent was evaporated to give the title compound.

### b) (-)-4-hydroxy-4-(4'-phenylbenzoyl)valeric acid

A mixture of (±)-4-hydroxy-4-(4'-phenylbenzoyl)valeric acid (0.597 g) obtained in Example 93 a) and (S)-(-)-1-phenylethylamine (0.260 ml) was recrystallized from a mixed solvent of methanol (10 ml) and diisopropyl ether (10 ml). The obtained crystals were dissolved in 10% aqueous citric acid solution and the mixture was extracted with ethyl acetate. The organic layer was washed with water and dried over magnesium sulfate. The solvent was evaporated to give the title compound.

### c) (-)-dihydro-5-methyl-5-(4'-phenylbenzoyl)-2(3H)-furanone

(-)-4-Hydroxy-4-(4'-phenylbenzoyl)valeric acid (77 mg) obtained in Example 93 b) was refluxed in acetic acid (1 ml) for 1.5 hr. The solvent was evaporated to give the title compound.

### Example 94 (+)-dihydro-5-methyl-5-(4'-phenylbenzoyl)-2(3H)-furanone

In the same manner as in Example 93 b), the title compound was obtained from (±)-4-hydroxy-4-(4'-phenylbenzoyl)valeric acid obtained in Example 93 a) and (R)-(+)-1-phenylethylamine.

### Example 95 (R)-(+)-5-benzoyl-2-pyrrolidinone

### a) (R)-Nα-carbobenzoxy-N-methoxy-N-methylpyroglutamide

To a solution of (R)-(+)-N-carbobenzoxypyroglutamic acid (10.0 g) and N,O-dimethylhydroxyamine hydrochloride (3.71 g) in methylene chloride (50 ml) were added triethylamine (4.23 g) and 1-hydroxybenzotriazole hydrate (5.65 g) under ice-cooling. Then, 1-(3-dimethylaminopropyl)-3-ethylcarbodiimide hydrochloride (7.65 g) was added. The reaction mixture was warmed to room temperature and stirred for 2.5 hr. The reaction mixture was diluted with methylene chloride (100 ml), washed with saturated aqueous sodium hydrogencarbonate solution, 10% aqueous citric acid solution and saturated brine, dried over sodium sulfate and the solvent was evaporated to give the title compound (10.0 g).

### b) (R)-N-methoxy-N-methylpyroglutamide

(R)-Nα-Carbobenzoxy-N-methoxy-N-methylpyroglutamide (5.44 g) obtained in Example 95 a) was dissolved in methanol (100 ml) and 5% palladium-carbon (0.52 g) was added. The mixture was stirred under a hydrogen atmosphere at room temperature for 1 hr. The reaction mixture was filtered through Celite, and the solvent was evaporated to give the title compound (3.61 g).

### c) (R)-(+)-5-benzoyl-2-pyrrolidinone

(R)-N-Methoxy-N-methylpyroglutamide (0.850 g) obtained in Example 95 b) was dissolved in dry tetrahydrofuran (50 ml), and 1.8M phenyllithium/cyclohexane ether solution (9.30 ml) was added dropwise at -78°C under an argon atmosphere, the mixture was stirred for 1 hr while gradually warming to -30°C. The reaction was quenched with aqueous ammonium chloride solution and aqueous citric acid solution. The mixture was extracted with ethyl acetate. The organic layer was washed with saturated brine and dried over sodium sulfate. The solvent was evaporated and the residue was purified by silica gel column chromatography (chloroform:methanol =97:3) to give the title compound.

### Example 96 (S)-(-)-5-benzoyl-2-pyrrolidinone

In the same manner as in Example 95, the title compound was obtained using (S)-(-)-N-carbobenzoxypyroglutamic acid.

### Example 97 (±)-5-benzoyl-2-pyrrolidinone

In the same manner as in Example 95, the title compound was obtained using (±)-N-carbobenzoxypyroglutamic acid.

### Example 98 (S)-(-)-5-(4'-phenylbenzoyl)-2-pyrrolidinone

To a solution of 4-bromobiphenyl (2.55 g) in dry tetrahydrofuran (30 ml) was added dropwise, at -78°C under an argon atmosphere, 1.6M n-butyllithium/hexane solution (6.50 ml). The mixture was stirred for 10 min and the reaction mixture was added dropwise to a solution of (S)-N-methoxy-N-methylpyroglutamide (0.857 g) in dry tetrahydrofuran (30 ml) at -78°C under an argon atmosphere. The mixture was warmed to -40°C and stirred for 1 hr, which was followed by addition of ammonium chloride and aqueous citric acid solution. The mixture was extracted with ethyl acetate. The organic layer was washed with saturated brine and dried over sodium sulfate. The solvent was evaporated and the residue was recrystallized from ethanol and ethyl acetate to give the title compound (0.465 g).

### Example 99 (S)-(-)-5-(4'-hydroxymethylbenzoyl)-2-pyrrolidinone

### a) 1-bromo-4-tert-butyldimethylsilyloxymethylbenzene

To a solution of 4-bromobenzyl alcohol (65.6 g) and imidazole (55.0 g) in N,N-dimethylformamide (300 ml) was added tert-butyldimethylsilyl chloride (58.1 g) and the mixture was stirred at room temperature overnight. The reaction mixture was poured into ice water, and the mixture was extracted with ether. The organic layer was washed with water and saturated brine and dried over potassium carbonate. The solvent was evaporated, and the residue was distilled (b.p._{1.5}Torr 129-133°C) to give the title compound (101 g).

### b) (S)-Nα-carbobenzoxy-N-methoxy-N-methylpyroglutamide

To a solution of (S)-(-)-N-carbobenzoxypyroglutamic acid (100 g), N,O-dimethylhydroxylamine hydrochloride (37.1 g) in methylene chloride (500 ml) were added triethylamine (42.3 g) and 1-hydroxybenzotriazole hydrate (56.5 g) under ice-cooling, and 1-(3-dimethylaminopropyl)-3-ethylcarbodiimide hydrochloride (76.5 g) was added. The mixture was warmed to room temperature and stirred overnight. The reaction mixture was washed with saturated aqueous sodium hydrogencarbonate solution, 5% aqueous potassium hydrogensulfate solution and saturated brine and dried over sodium sulfate. The solvent was evaporated to give the title compound (108 g).

### c) (S)-N-methoxy-N-methylpyroglutamide

(S)-Nα-Carbobenzoxy-N-methoxy-N-methylpyroglutamide (108 g) obtained in Example 99 b) was dissolved in methanol (800 ml) and 5% palladium-carbon (10.0 g) was added. The mixture was stirred under a hydrogen atmosphere at room temperature for 6.5 hr. The reaction mixture was filtered through a Celite pad, and the solvent was evaporated to give the title compound (58.9 g).

### d) (S)-5-[4'-(tert-butyldimethylsilyloxymethyl)benzoyl]-2-pyrrolidinone

To a solution of 1-bromo-4-tert-butyldimethylsilyloxymethylbenzene (73.5 g) obtained in Example 99 a) in dry tetrahydrofuran (610 ml) was dropwise added 1.6M n-butyllithium/hexane solution (141 ml) at -78°C under an argon atmosphere. The mixture was stirred for 1 hr, and the reaction mixture was added dropwise to a solution of (S)-N-methoxy-N-methylpyroglutamide (20.0 g) in dry tetrahydrofuran (580 ml) at -78°C under an argon atmosphere. The mixture was warmed to -40°C and stirred for 1 hr, which was followed by addition of an aqueous solution of ammonium chloride. The mixture was extracted with ethyl acetate. The organic layer was washed with 10% aqueous citric acid solution and saturated brine, and dried over sodium sulfate. The solvent was evaporated and the residue was purified by silica gel column chromatography (chloroform:methanol=99:1) to give the title compound.

### e) (S)-5-(4'-hydroxymethylbenzoyl)-2-pyrrolidinone

A mixed solution of (S)-5-[4'-(tert-butyldimethylsilyloxymethyl)benzoyl]-2-pyrrolidinone (0.406 g) obtained in Example 99 d) in tetrahydrofuran (1 ml), distilled water (1 ml) and acetic acid (3 ml) was stirred at room temperature overnight. The solvent was evaporated and the residue was washed with a mixed solvent of hexane/ether (4 ml/1 ml) to give the title compound (0.240 g).

### Example 100 (±)-4-benzoyl-2-pyrrolidinone

### a) methyl (±)-2-oxo-4-pyrrolidinecarboxylate

Dimethyl itaconate (25.0 g) was stirred in 6.2% ammonia/methanol solution (85 ml) at room temperature overnight. The solvent was evaporated and the residue was purified by silica gel column chromatography (chloroform:methanol=9:1) to give the title compound (11.7 g).

### b) methyl (±)-N-carbobenzoxy-2-oxo-4-pyrrolidinecarboxylate

A solution of methyl (±)-2-oxo-4-pyrrolidinecarboxylate (21.9 g) obtained in Example 100 a) in dry tetrahydrofuran (150 ml) was dropwise added to a suspension of sodium hydride (3.6 g) in dry tetrahydrofuran (150 ml) at 0°C. A solution of benzyl chloroformate (23.8 ml) in dry tetrahydrofuran (170 ml) was added dropwise, and the mixture was warmed to room temperature. The mixture was stirred for 3.5 hr. The reaction mixture was poured into ice water, and the mixture was extracted with ethyl acetate. The organic layer was washed with distilled water and saturated brine and dried over sodium sulfate. The solvent was evaporated and the residue was purified by silica gel column chromatography (hexane:ethyl acetate=1:1) to give the title compound (11.5 g).

### c) (±)-N-carbobenzoxy-2-oxo-4-pyrrolidinecarboxylic acid

To a solution of methyl (±)-N-carbobenzoxy-2-oxo-4-pyrrolidinecarboxylate (10.3 g) obtained in Example 100 b) in methanol (350 ml) was added 0.1N aqueous potassium carbonate solution (556 ml), and the mixture was stirred at room temperature for 3 hr. The reaction mixture was washed with ether, acidified with 1N hydrochloric acid and extracted with ethyl acetate. The organic layer was washed with saturated brine and dried over sodium sulfate, The solvent was evaporated to give the title compound (6.48 g).

### d) (±)-N-methoxy-N-methyl-2-oxo-4-pyrrolidinecarboxamide

In the same manner as in Example 95 a) and b), the title compound was obtained from (±)-N-carbobenzoxy-2-oxo-4-pyrrolidinecarboxylic acid obtained in Example 100 c).

### e) (±)-4-benzoyl-2-pyrrolidinone

In the same manner as in Example 95 c), the title compound was obtained from (±)-N-methoxy-N-methyl-2-oxo-4-pyrrolidinecarboxamide obtained in Example 100 d).

### Example 101 (±)-4-(4'-hydroxymethylbenzoyl)-2-pyrrolidinone

In the same manner as in Example 99 d) and e), the title compound was obtained from (±)-N-methoxy-N-methyl-2-oxo-4-pyrrolidinecarboxamide obtained in Example 100 d).

### Example 102 trans-4-(4-ethylcyclohexyl)-4-oxobutanoic acid

### a) 4-ethylcyclohexanecarboxylic acid

To a solution of 4-ethylbenzoic acid (10 g) in acetic acid (150 ml) was added platinum(IV) oxide (1.0 g), and the mixture was stirred at room temperature under a hydrogen atmosphere at 3 atm for 3 hr. The platinum catalyst was filtered off through a Celite pad, and the filtrate was concentrated to give the title compound (10.0 g).

### b) trans-4-(4-ethylcyclohexyl)-4-oxobutanoic acid

In the same manner as in Example 65, Production Method 2, the title compound was obtained from 4-ethylcyclohexanecarboxylic acid obtained in Example 102 a).

### Example 103 trans-4-(4-isopropylcyclohexyl)-4-oxobutanoic acid

In the same manner as in Example 102, the title compound was obtained from cumic acid.

### Example 104 trans-4-(4-tert-butylcyclohexyl)-4-oxobutanoic acid

In the same manner as in Example 65, Production Method 2, the title compound was obtained from 4-tert-butylcyclohexanecarboxylic acid.

### Example 105 trans-4-(4-phenylcyclohexyl)-4-oxobutanoic acid

In the same manner as in Example 65, Production Method 2, the title compound was obtained from trans-4-phenylcyclohexanecarboxylic acid obtained in Example 17 a).

### Example 106 cis-4-(4-methylcyclohexyl)-4-oxobutanoic acid

### a) tert-butyl cis-4-(4-methylcyclohexyl)-4-oxobutanoate

To a solution of the mixture (2.027 g) obtained in Example 65, Production Method 2 d) of trans-4-(4-methylcyclohexyl)-4-oxobutanoic acid and cis-4-(4-methylcyclohexyl)-4-oxobutanoic acid in methylene chloride (20 ml) was added concentrated sulfuric acid (0.2 ml), and isobutene (5.0 ml) was added at -20°C. The mixture was warmed to room temperature and stirred for 12 hr. To the reaction mixture was added saturated aqueous sodium hydrogencarbonate solution, and the mixture was extracted with ethyl acetate, dried over magnesium sulfate, concentrated and purified by HPLC to give the title compound (0.500 g).

### b) cis-4-(4-methylcyclohexyl)-4-oxobutanoic acid

A solution of tert-butyl cis-4-(4-methylcyclohexyl)-4-oxobutanoate (0.500 g) obtained in Example 106 a) in formic acid (5.0 ml) was stirred at room temperature for 2 hr, and formic acid was evaporated under reduced pressure. Distilled water was added, and the mixture was extracted with ethyl acetate, dried over magnesium sulfate and concentrated to give the title compound (0.355 g).

### Example 107 4-(3-methylcyclohexyl)-4-oxobutanoic acid

In the same manner as in Example 65, Production Method 2, the title compound was obtained from 3-methyl-1-cyclohexanecarboxylic acid.

### Example 108 cis-2- (trans-4-methylcyclohexylcarbonyl)cyclohexanecarboxylic acid

### a) trans-4-methylcyclohexanecarbonyl chloride

To a solution of trans-4-methyl-1-cyclohexanecarboxylic acid (7.12 g) in methylene chloride (50 ml) were added dropwise oxalyl chloride (5.5 ml) and dimethylformamide (0.05 ml) at room temperature, and the mixture was stirred for 1 hr. The reaction mixture was concentrated and distilled under reduced pressure to give the title compound (7.24 g).

### b) tert-butyl trans-4-(4-methylcyclohexyl)-4-oxo-2-butynoate

To a suspension of copper(I) iodide (38 mg) and bis(triphenylphosphine)palladium(II) chloride (38 mg) in benzene (20 ml) were added tert-butyl propiolate (3.30 ml) and triethylamine (3.10 ml). Then, a solution of trans-4-methylcyclohexanecarbonyl chloride (3.21 g) obtained in Example 108 a) in benzene (20 ml) was dropwise added at room temperature, and the mixture was stirred for 1 hr. The reaction mixture was ice-cooled and distilled water was added. The mixture was extracted with hexane, washed with distilled water and saturated brine, dried over magnesium sulfate, concentrated and purified by silica gel column chromatography to give the title compound (3.87 g).

### c) tert-butyl 2-(trans-4-methylcyclohexylcarbonyl)-1,4-cyclohexadiene-1-carboxylate

To a solution of tert-butyl trans-4-(4-methylcyclohexyl)-4-oxo-2-butynoate (3.86 g) obtained in Example 108 b) in toluene (20 ml) was added 2,6-di-tert-butyl-4-methylphenol (0.05 g), and 1,3-butadiene (1.0 g) was bubbled into the reaction mixture. The mixture was stirred at 60°C for 12 hr with heating. 1,3-Butadiene (1.0 g) was bubbled again and the mixture was stirred at 80 °C for 24 hr with heating. The reaction mixture was concentrated and purified by silica gel column chromatography to give the title compound (4.34 g).

### d) tert-butyl cis-2-(trans-4-methylcyclohexylcarbonyl)cyclohexanecarboxylate

To a solution of tert-butyl 2-(trans-4-methylcyclohexylcarbonyl)-1,4-cyclohexadiene-1-carboxylate (4.34 g) obtained in Example 108 c) in ethanol (14 ml) was added 5% palladium-carbon (0.434 g), and the mixture was stirred under a hydrogen atmosphere at 1 atm for 8 hr. Palladium-carbon was filtered off through a Celite pad, and the filtrate was concentrated and purified by silica gel column chromatography to give tert-butyl 2-[1-hydroxy-1-(trans-4-methylcyclohexyl)methyl]benzoate (1.26 g) and the title compound (2.42 g).

### e) cis-2-(trans-4-methylcyclohexylcarbonyl)cyclohexanecarboxylic acid

A solution of tert-butyl cis-2-(trans-4-methylcyclohexylcarbonyl)cyclohexanecarboxylate (0.623 g) obtained in Example 108 d) in formic acid (5.0 ml) was stirred at room temperature for 4 hr. The reaction mixture was concentrated and the obtained residue was dissolved in diethyl ether. The mixture was washed with distilled water, dried over magnesium sulfate, concentrated, and purified by recrystallization from diethyl ether and hexane to give the title compound (0.224 g).

### Example 109 trans-2-(trans-4-methylcyclohexylcarbonyl)cyclohexanecarboxylic acid

### a) tert-butyl trans-2- (trans-4-methylcyclohexylcarbonyl)cyclohexanecarboxylate

To a solution of tert-butyl cis-2- (trans-4-methylcyclohexylcarbonyl)cyclohexanecarboxylate (0.626 g) obtained in Example 108 d) in tert-butanol (10 ml) was added potassium tert-butoxide (35 mg) at room temperature, and the mixture was stirred for 12 hr. A 10% aqueous citric acid solution was added to the reaction mixture, and tert-butanol was evaporated under reduced pressure. The residue was extracted with diethyl ether, washed with water, saturated aqueous sodium hydrogencarbonate solution, dried over magnesium sulfate, concentrated and purified by silica gel column chromatography to give the title compound (0.526 g).

### b) trans-2-(trans-4-methylcyclohexylcarbonyl)cyclohexanecarboxylic acid

A solution of tert-butyl trans-2- (trans-4-methylcyclohexylcarbonyl)cyclohexanecarboxylate (0.526 g) obtained in Example 109 a) in formic acid (5.0 ml) was stirred at room temperature for 2 hr. The reaction mixture was concentrated and purified by recrystallization from ethyl acetate and hexane to give the title compound (0.181 g).

### Example 110 2-(trans-4-methylcyclohexylcarbonyl)benzoic acid

### a) tert-butyl 2-(trans-4-methylcyclohexylcarbonyl)benzoate

To a solution of tert-butyl 2-[1-hydroxy-1-(trans-4-methylcyclohexyl)methyl]benzoate (1.26 g) obtained in Example 108 d) in dimethyl sulfoxide (5.0 ml) was added triethylamine (2.60 ml), and a solution of sulfur trioxide-pyridine complex (1.30 g) in dimethyl sulfoxide (5.0 ml) was added, which was followed by stirring for 1 hr. The reaction mixture was poured into water and extracted with ethyl ether. The organic layer was washed successively with 5% aqueous citric acid solution, water, aqueous sodium hypochlorite solution and water, dried over magnesium sulfate, concentrated and purified by silica gel column chromatography to give the title compound (0.735 g).

### b) 2-(trans-4-methylcyclohexylcarbonyl)benzoic acid

A solution of tert-butyl 2-(trans-4-methylcyclohexylcarbonyl)benzoate (0.753 g) obtained in Example 110 a) in formic acid (5.0 ml) was stirred at room temperature for 2 hr. The reaction mixture was concentrated, and washed with hexane to give the title compound (0.480 g).

### Example 111 trans-2- (trans-4-methylcyclohexylcarbonyl)cyclopropanecarboxylic acid

### a) tert-butyl trans-2-(trans-4-methylcyclohexylcarbonyl)cyclopropanecarboxylate

To a solution (1M, 80 ml) of diazomethane in diethyl ether was added dropwise a solution of trans-4-methylcyclohexanecarbonyl chloride (3.218 g) obtained in Example 108 a) in diethyl ether (20 ml), and the mixture was stirred at room temperature for 12 hr. The reaction mixture was concentrated, and the obtained residue was dissolved in tert-butyl acrylate (30 ml). The solution was stirred at 120°C for 1 hr and the reaction mixture was purified by silica gel column chromatography to give the title compound (0.850 g).

### b) trans-2-(trans-4-methylcyclohexylcarbonyl)cyclopropanecarboxylic acid

A solution of tert-butyl trans-2-(trans-4-methylcyclohexylcarbonyl)cyclopropanecarborylate (0.538 g) obtained in Example 111 a) in formic acid (10 ml) was stirred at room temperature for 1 hr. The reaction mixture was concentrated and purified by recrystallization from diethyl ether and hexane to give the title compound (0.360 g).

### Example 112 cis-2-(trans-4-methylcyclohexylcarbonyl)cyclopropanecarboxylic acid

To a solution of tert-butyl trans-2-(trans-4-methylcyclohexylcarbonyl)cyclopropanecarboxylate (0.238 g) obtained in Example 111 a) in methanol (10 ml) was added sodium methoxide (0.103 g), and the mixture was refluxed for 4 hr and methanol was evaporated under reduced pressure. The obtained residue was dissolved in tetrahydrofuran (10 ml), to which was added 1N hydrochloric acid (5 ml) and the mixture was refluxed for 2 hr. The reaction mixture was concentrated and the residue was dissolved in saturated aqueous sodium hydrogencarbonate solution. The aqueous layer was washed with diethyl ether, and acidified with hydrochloric acid. The mixture was extracted with diethyl ether, concentrated and purified by recrystallization from methanol and water to give the title compound (0.107 g).

### Example 113 trans-5-(4-methylcyclohexylcarbonyl)-dihydro-2(3H)-furanone

### a) 5-(1-methoxycarbonyl-4-methylcyclohexyl)-5-oxopentanoic acid

A solution (1.5M, 100 ml) of lithium diisopropylamide in cyclohexane was added dropwise to a solution of methyl 4-methylcyclohexanecarboxylate (22.34 g) obtained in Example 65, Production Method 5 a) in tetrahydrofuran (40 ml) at -5°C, and the mixture was stirred at 0°C for 30 min. Then, the mixture prepared above was added dropwise to a solution of glutaric anhydride (16.32 g) in tetrahydrofuran (160 ml) at 5°C, and the mixture was stirred at room temperature for 2 hr. A 5% aqueous potassium hydrogensulfate solution was added to the reaction mixture, and the mixture was extracted with ethyl acetate. The organic layer was washed with saturated brine, dried over sodium sulfate and concentrated to give the title compound (28.75 g).

### b) trans-5-(4-methylcyclohexyl)-5-oxopentanoic acid

5-(1-Methoxycarbonyl-4-methylcyclohexyl)-5-oxopentanoic acid (28.75 g) obtained in Example 113 a) was dissolved in a solution of sodium hydroxide (14.83 g) in water (230 ml), which was refluxed for 2 hr. Concentrated hydrochloric acid was added under ice-cooling to acidify the reaction mixture and extracted with ethyl acetate. The organic layer was washed with water and saturated brine, dried over sodium sulfate and concentrated. The residue was purified by recrystallization from ethyl acetate and hexane to give the title compound (8.88 g).

### c) 4-bromo-5-(trans-4-methylcyclohexyl)-5-oxopentanoic acid

To a solution of trans-5-(4-methylcyclohexyl)-5-oxopentanoic acid (2.0 g) obtained in Example 113 b) in acetic acid (30 ml) was added bromine (0.485 ml), and the mixture was stirred at room temperature for 1 hr. The reaction mixture was poured into water and extracted with chloroform. The organic layer was washed with saturated brine, dried over sodium sulfate and concentrated to give the title compound (2.70 g).

### d) trans-5-(4-methylcyclohexylcarbonyl)-dihydro-2(3H)-furanone

To a solution of 4-bromo-5-(trans-4-methylcyclohexyl)-5-oxopentanoic acid (2.70 g) obtained in Example 113 c) in dimethyl formamide (6.0 ml) was added sodium hydrogencarbonate (1.85 g), and the mixture was stirred at room temperature for 2 hr. Water was added to the reaction mixture and the mixture was extracted with ethyl acetate. The extract was washed with saturated brine, dried over sodium sulfate and concentrated. The residue was purified by recrystallization from ethyl acetate and hexane to give the title compound (1.28 g).

The properties of the compounds of the Examples are shown in the following.

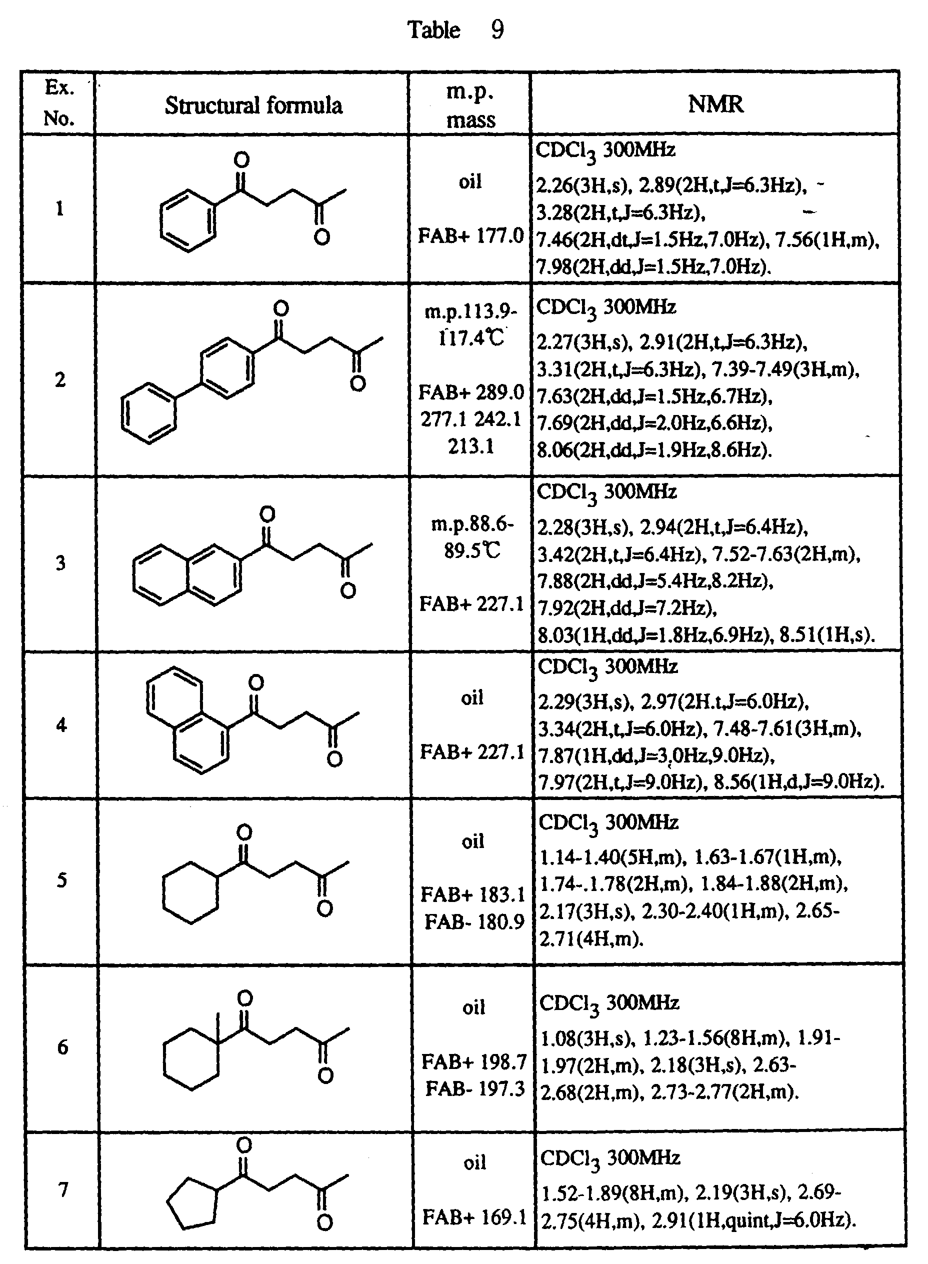

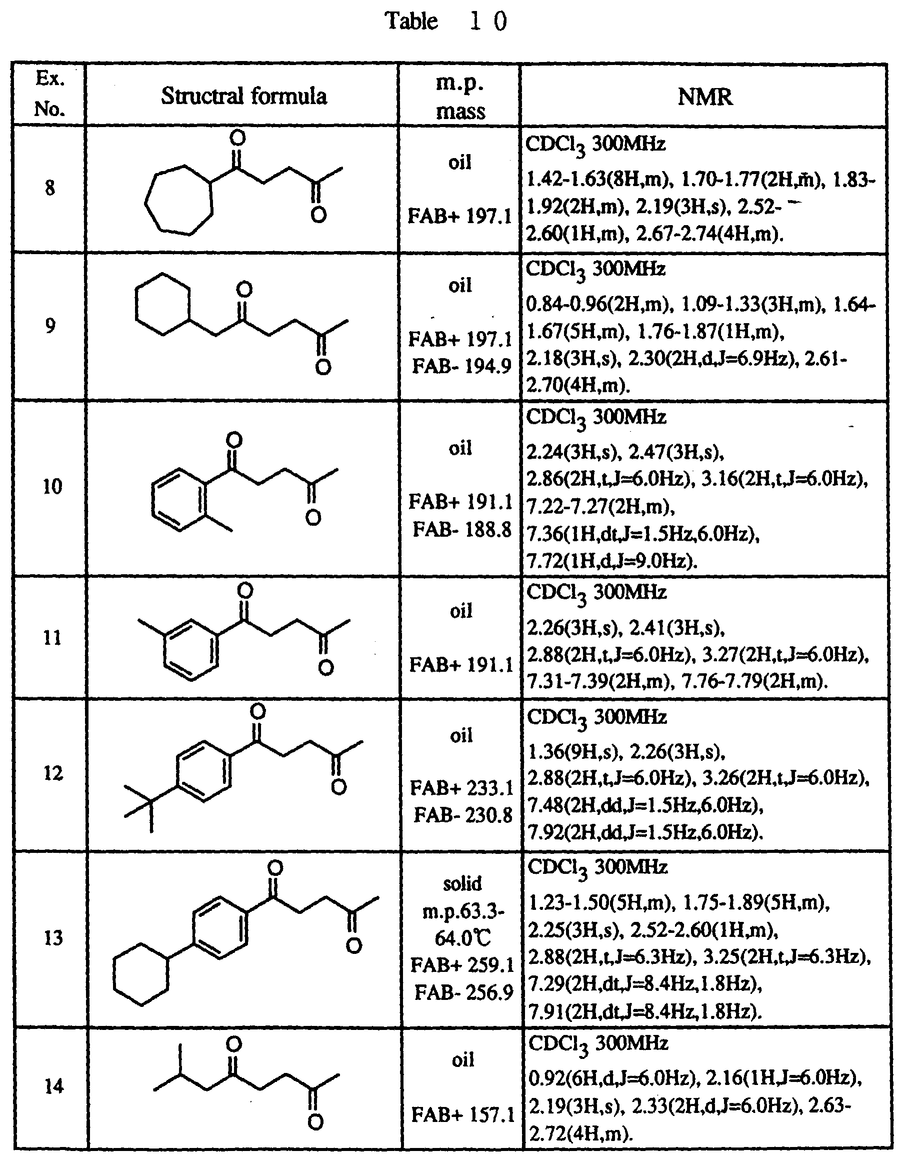

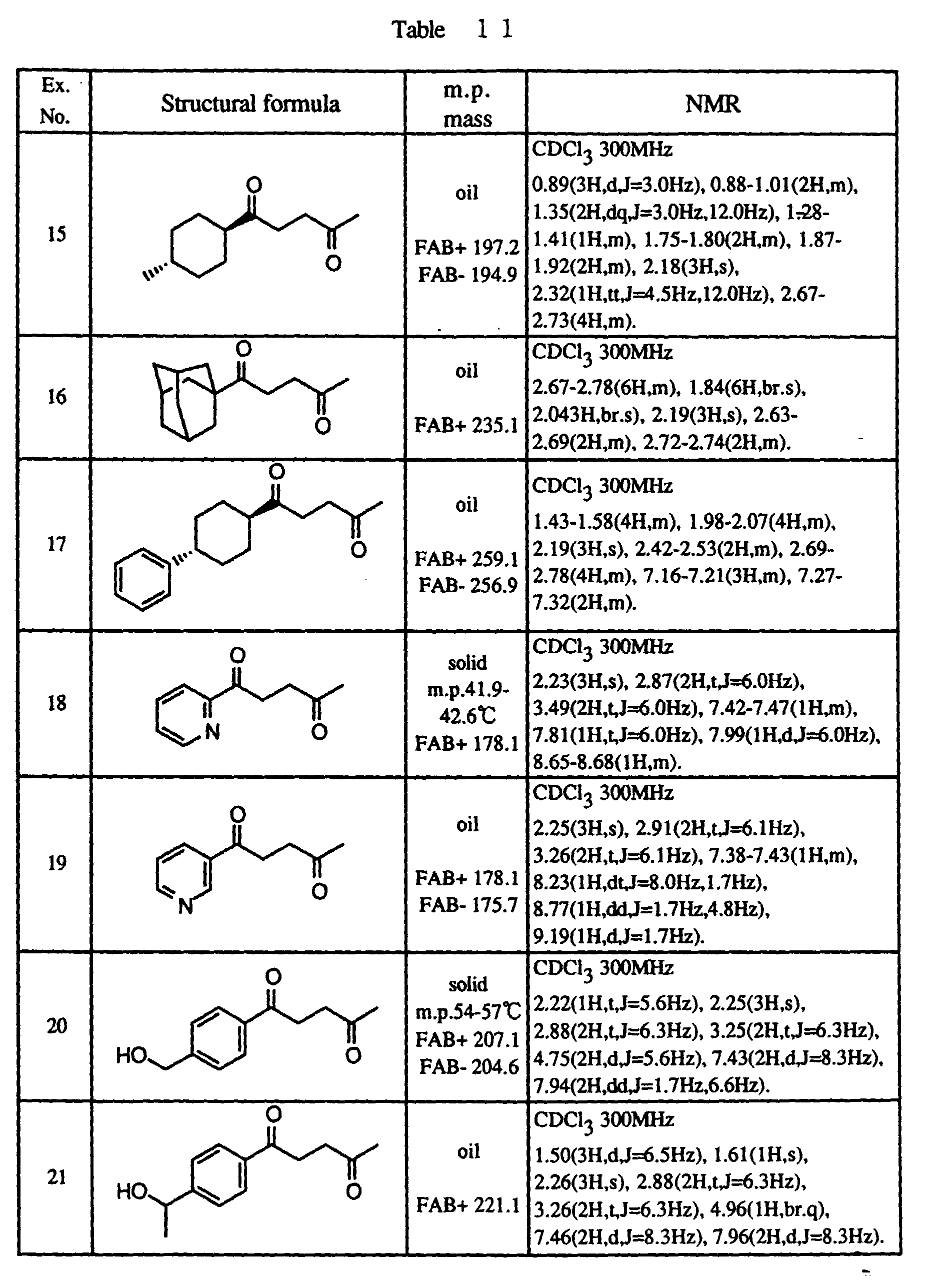

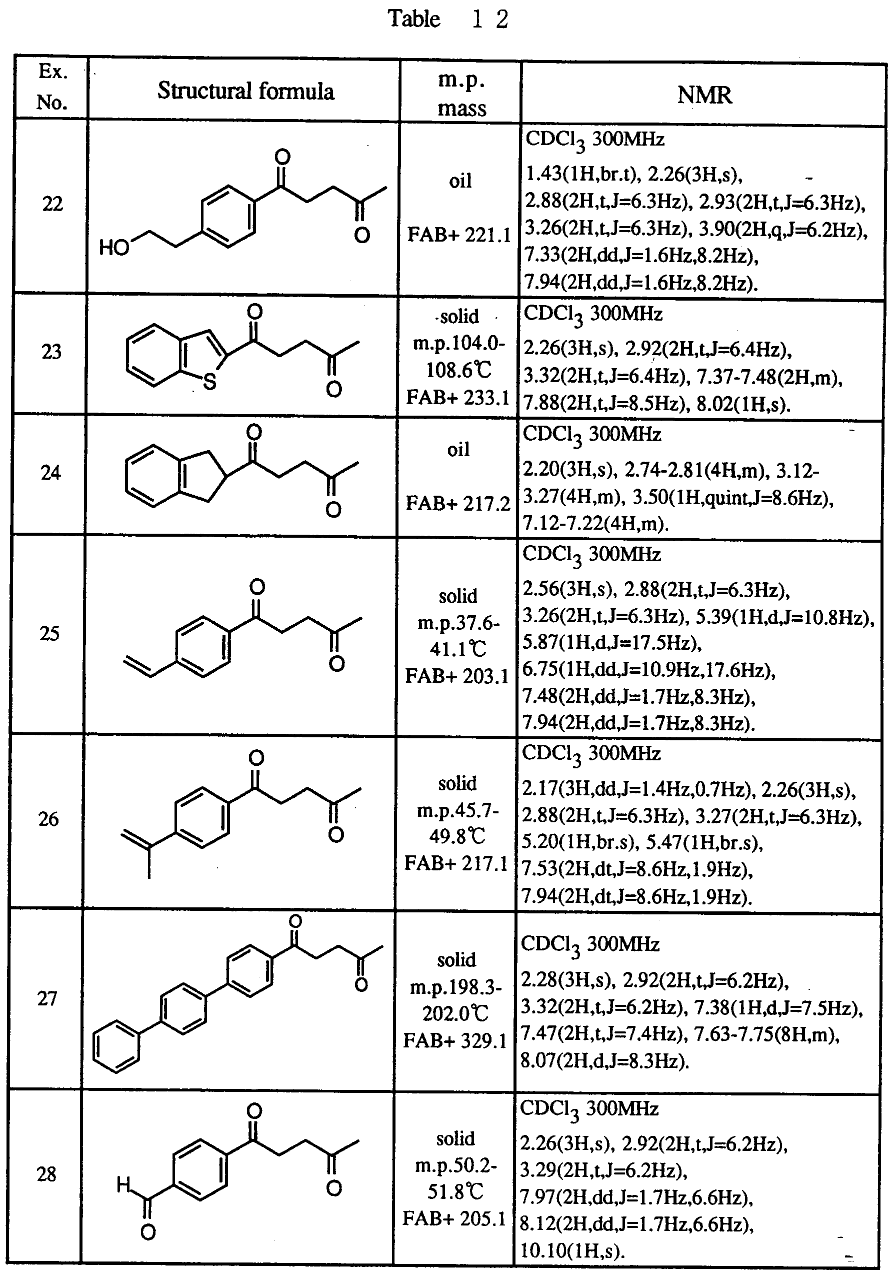

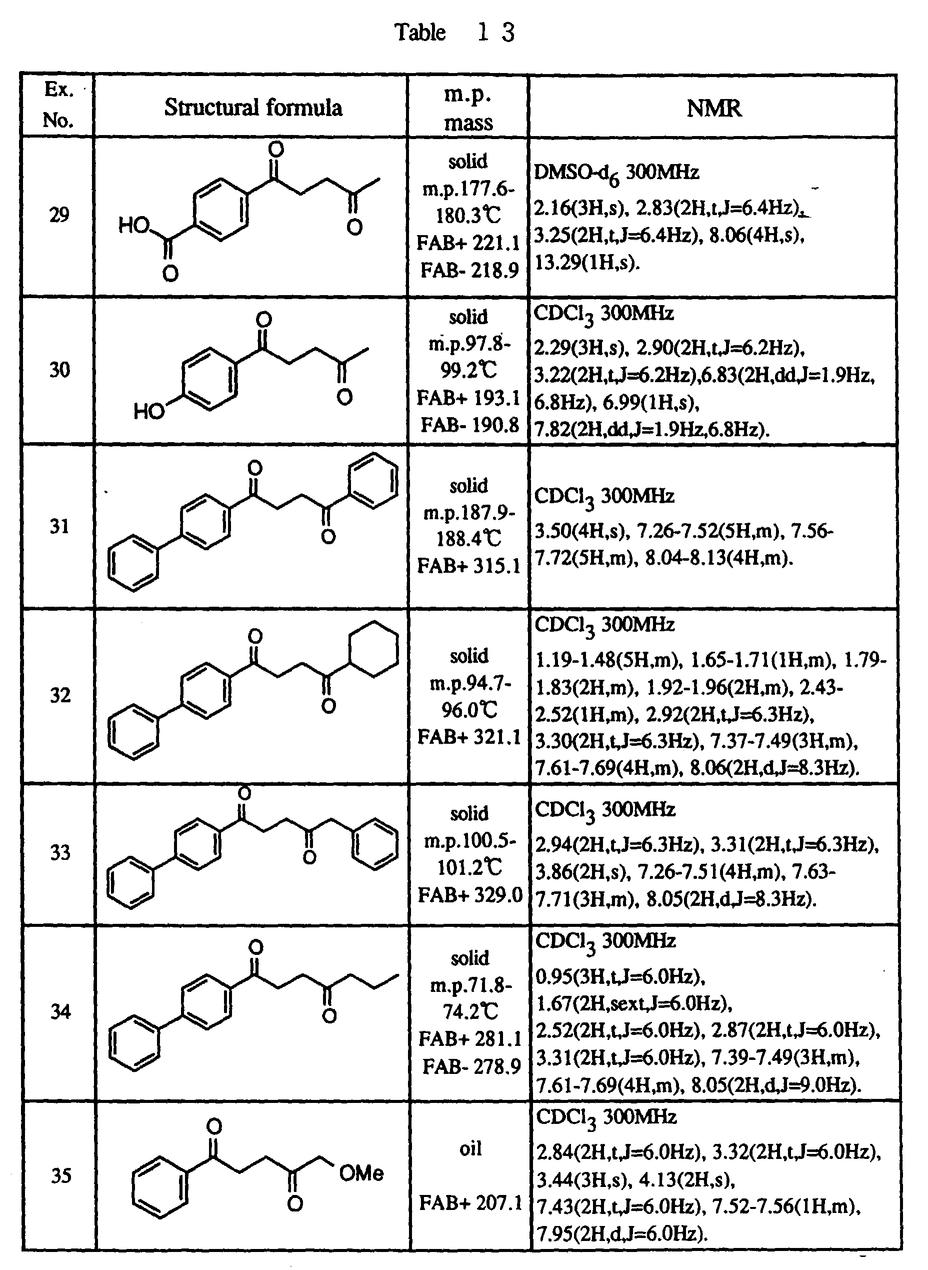

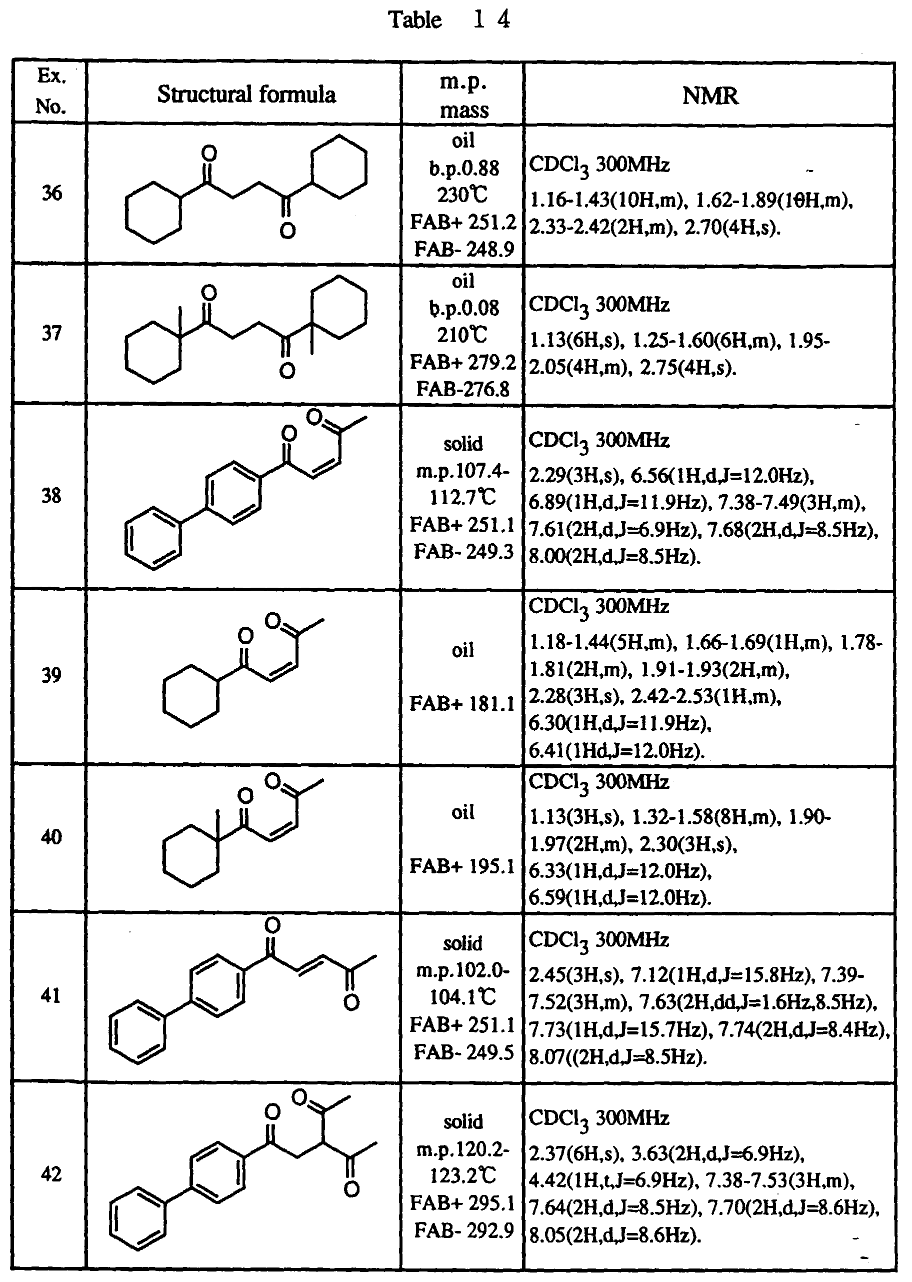

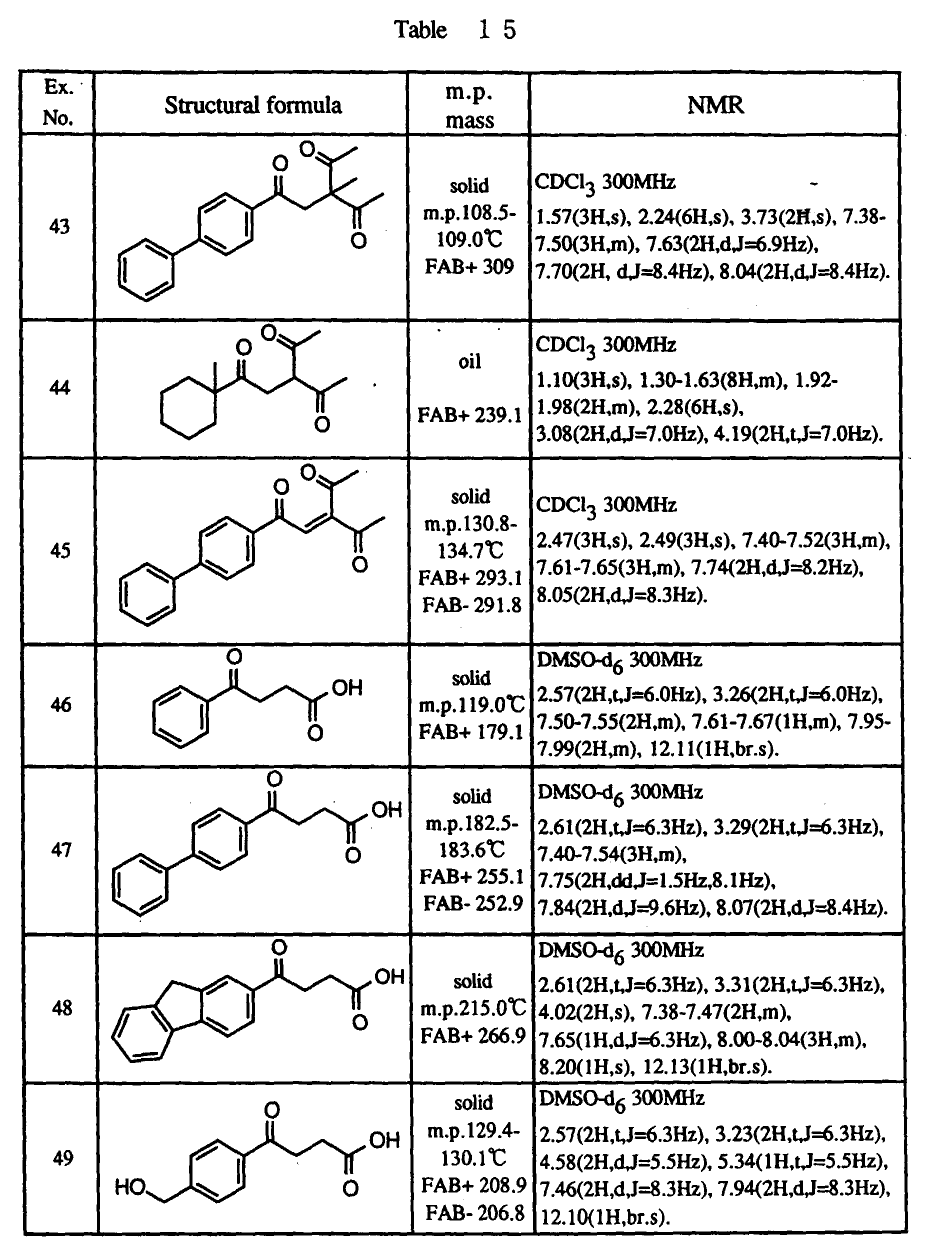

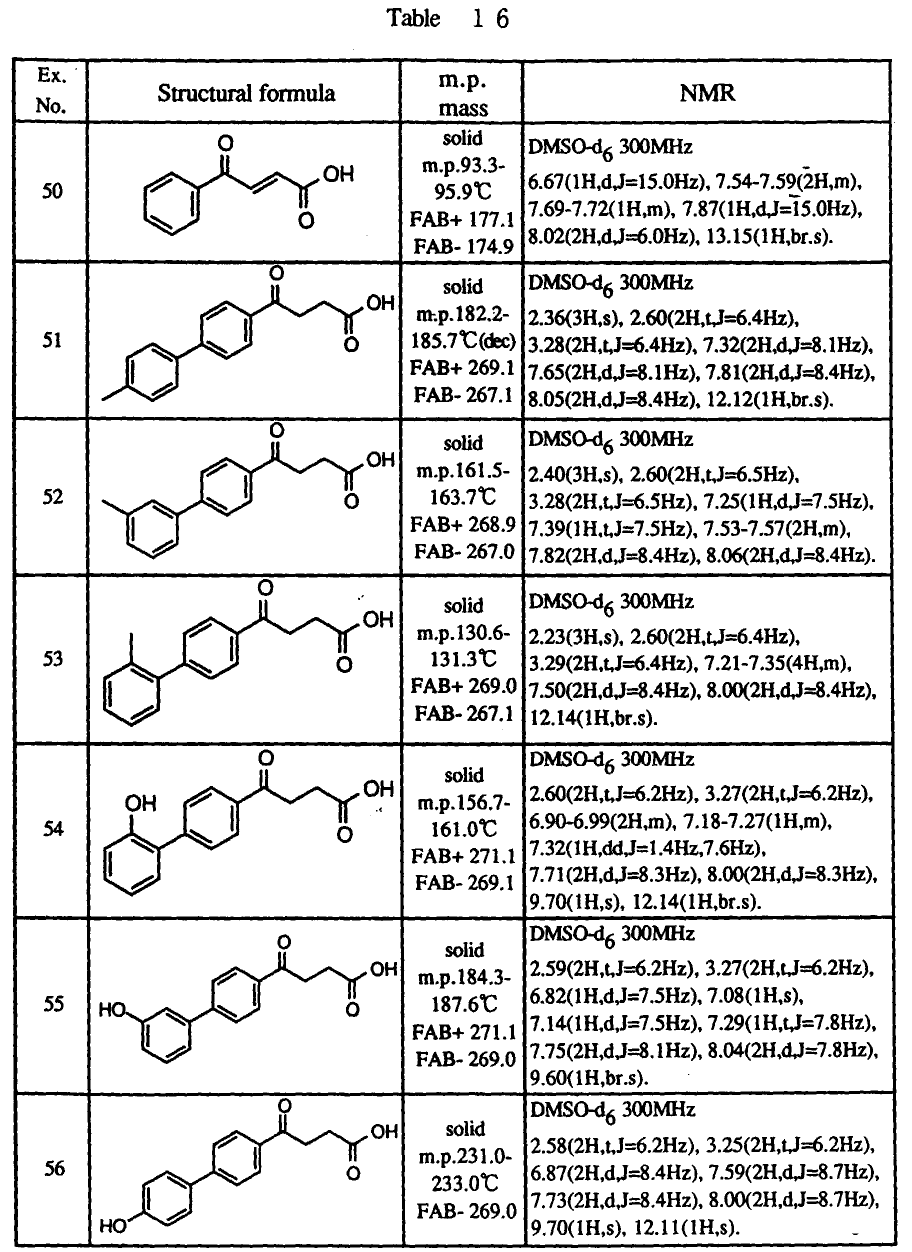

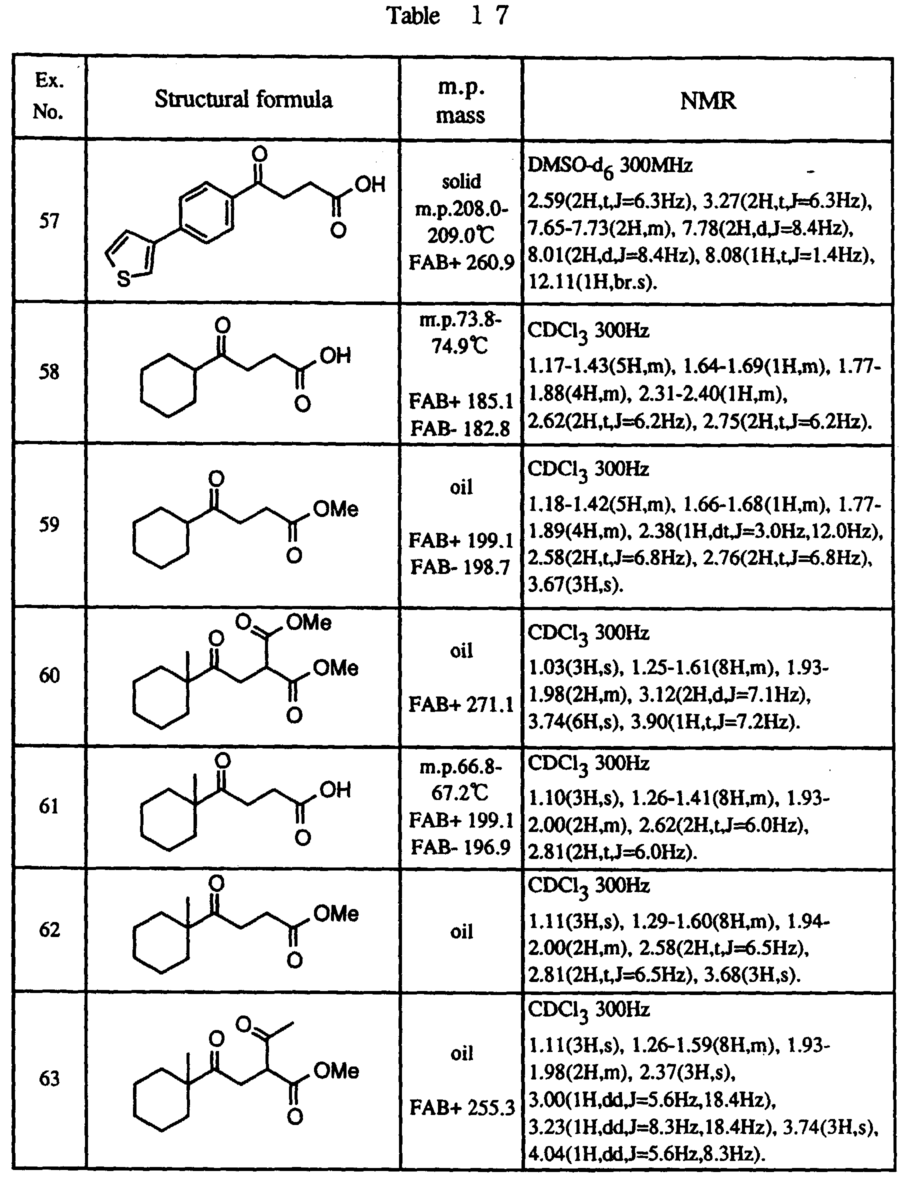

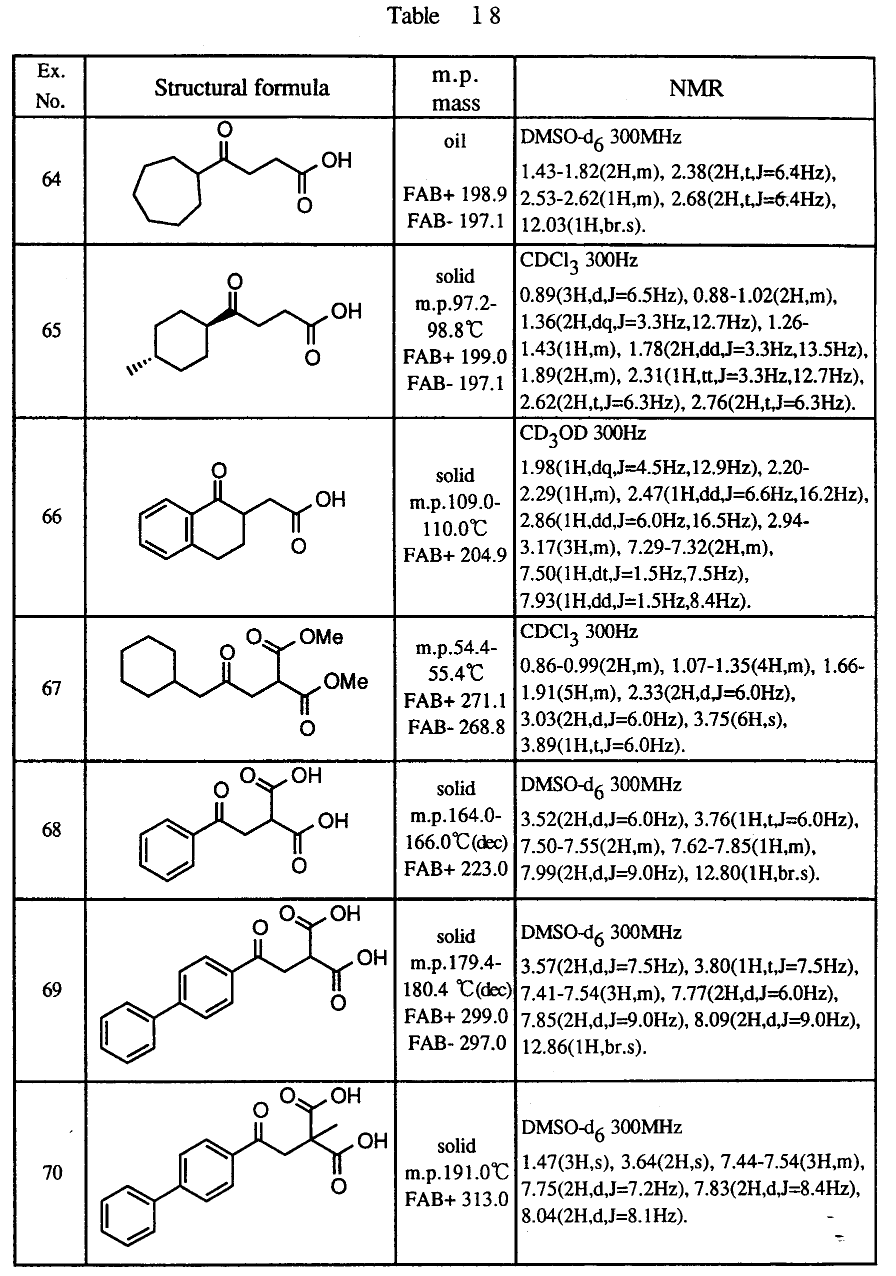

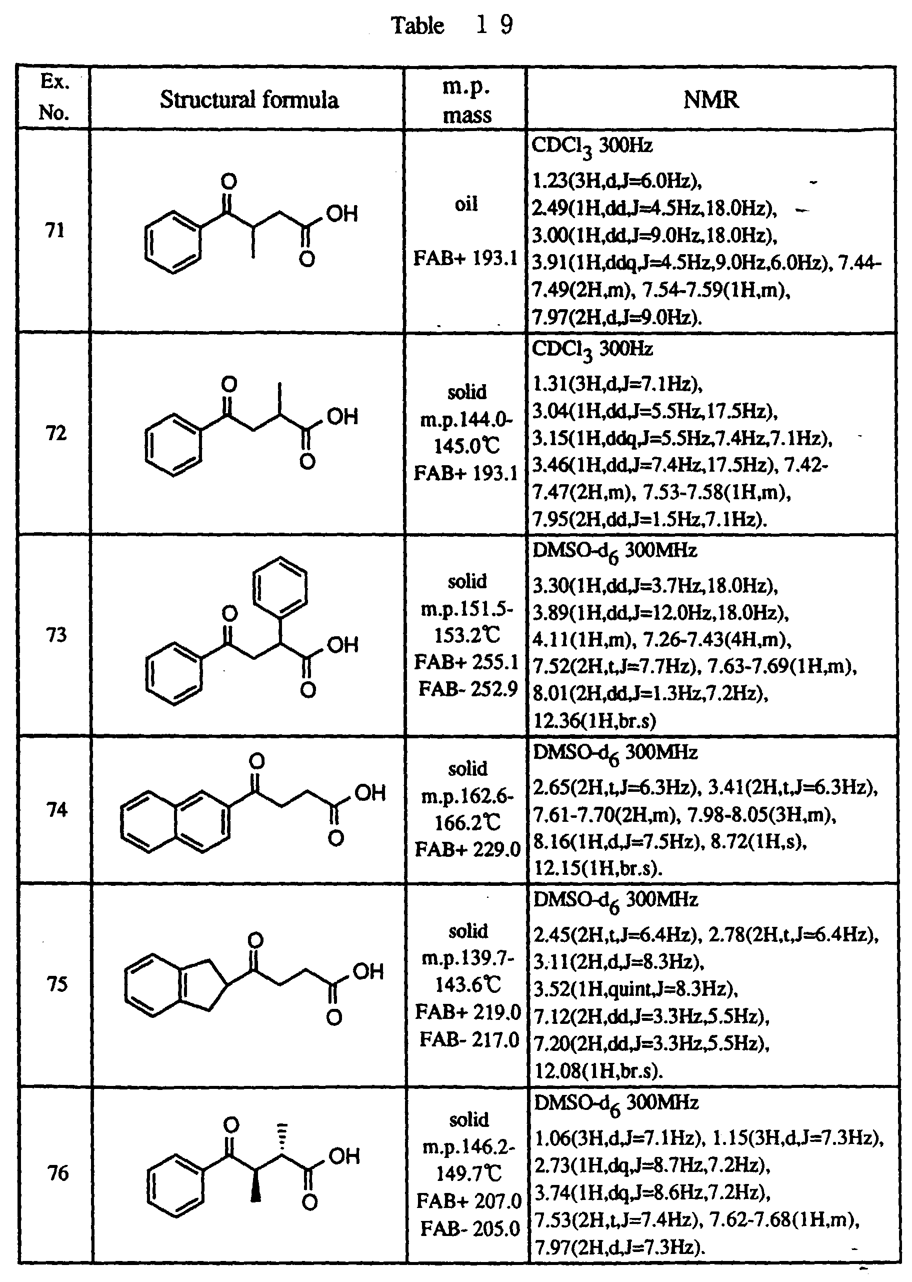

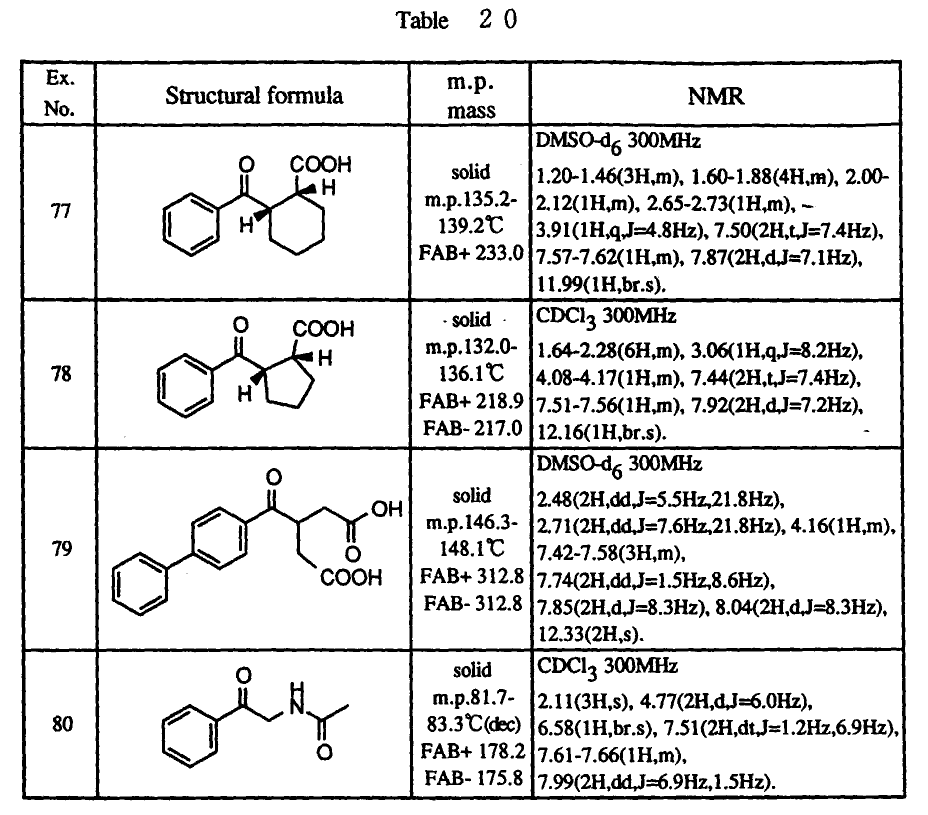

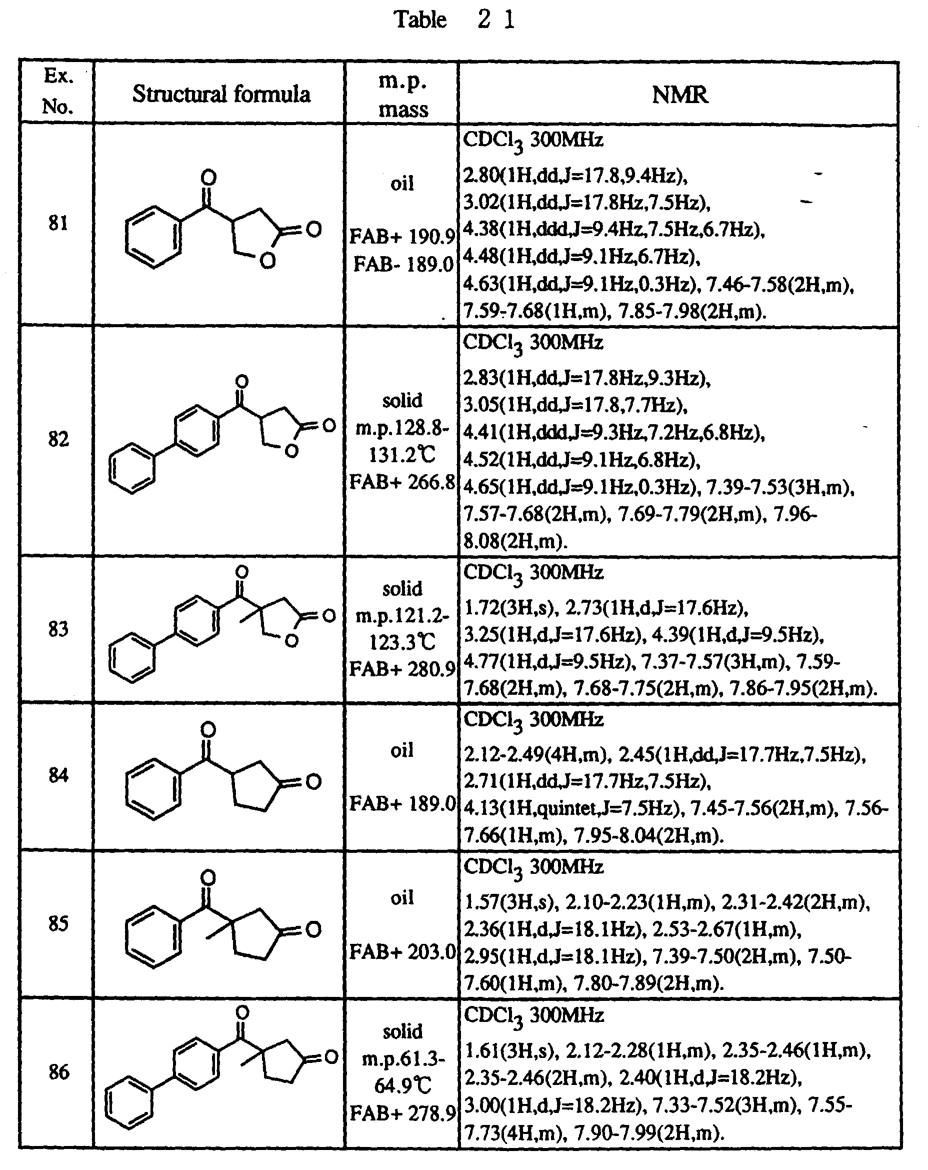

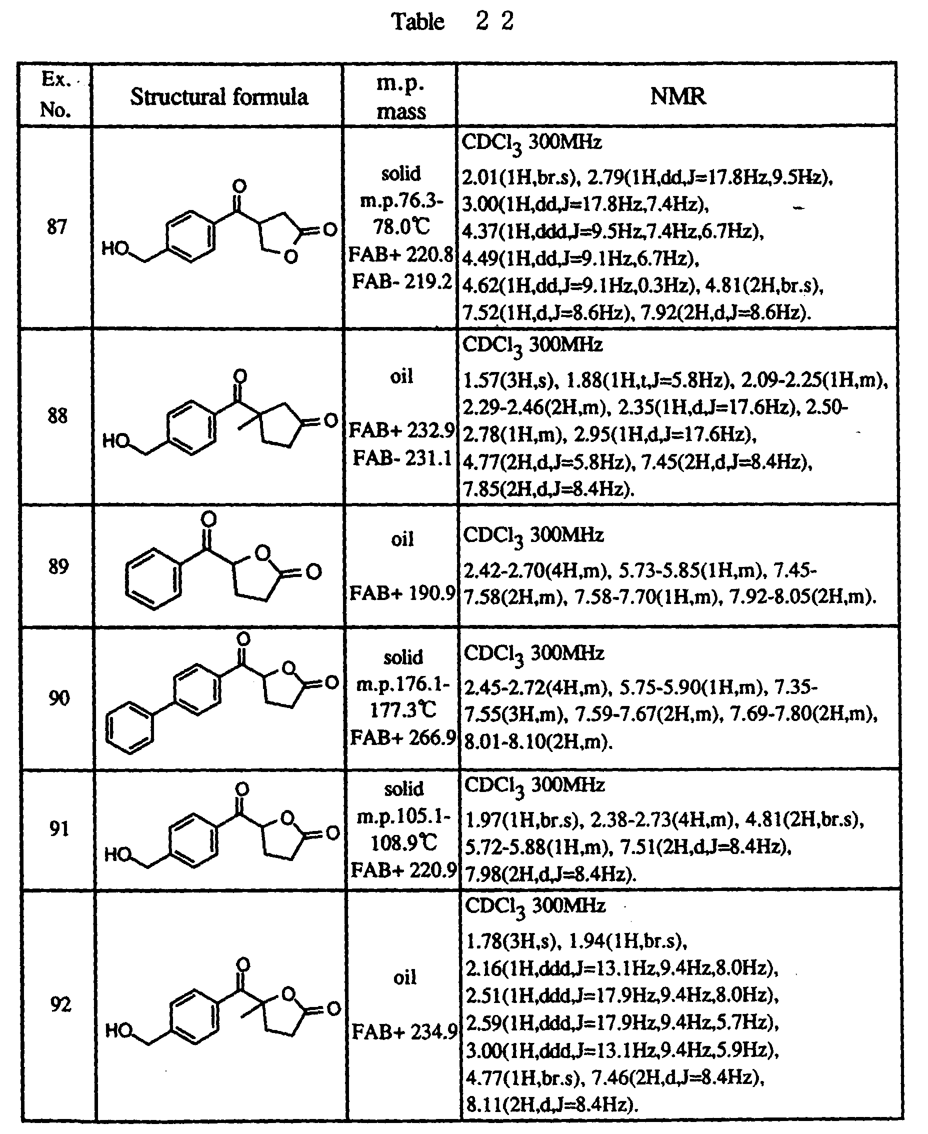

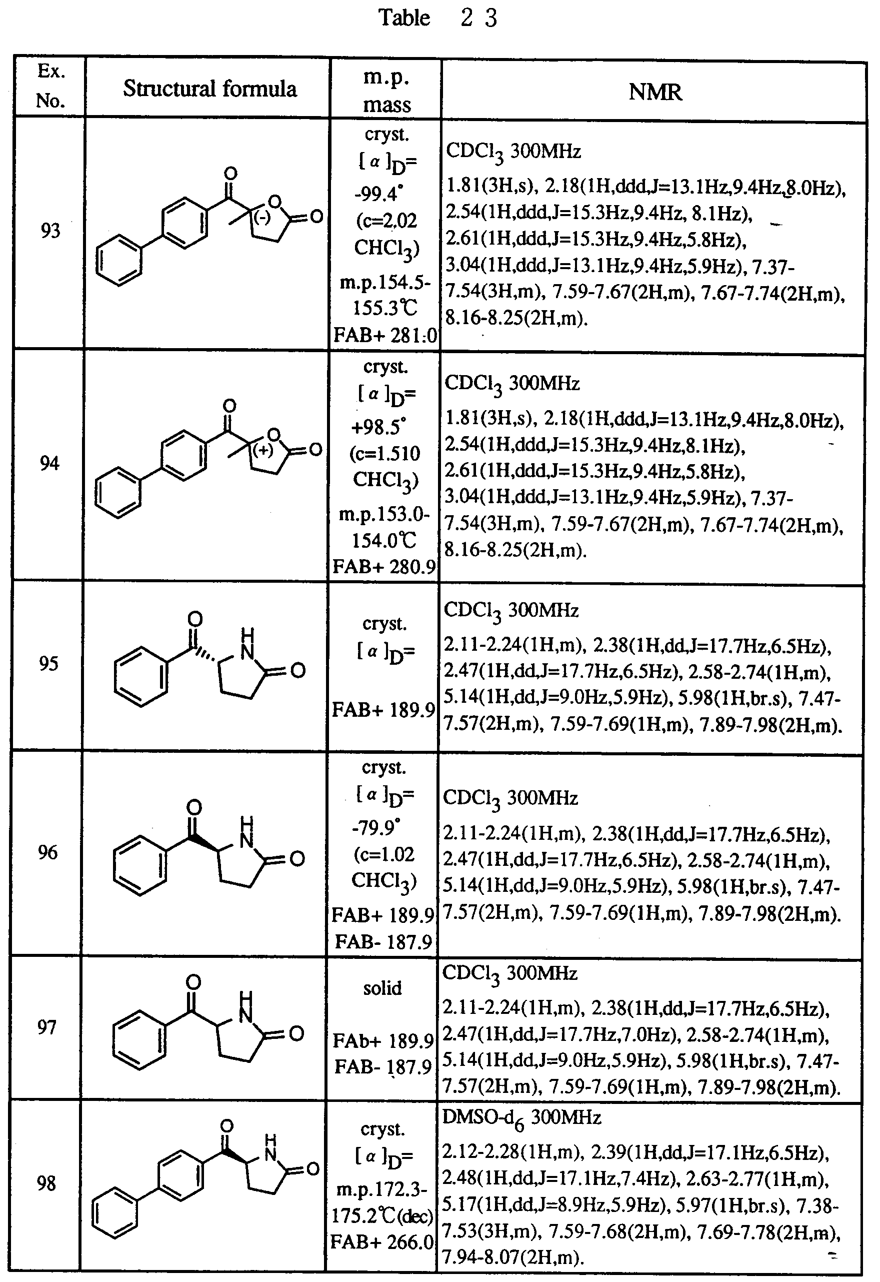

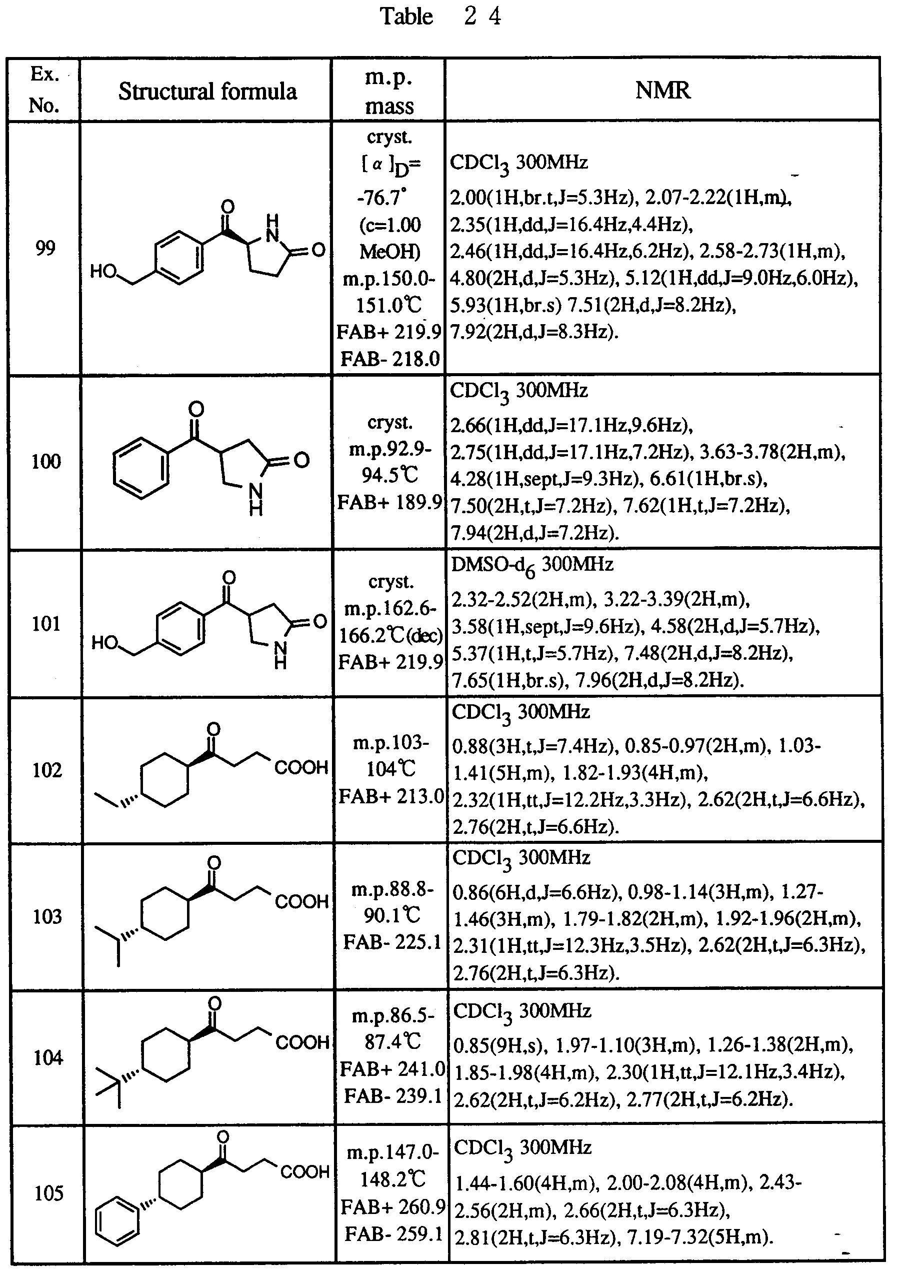

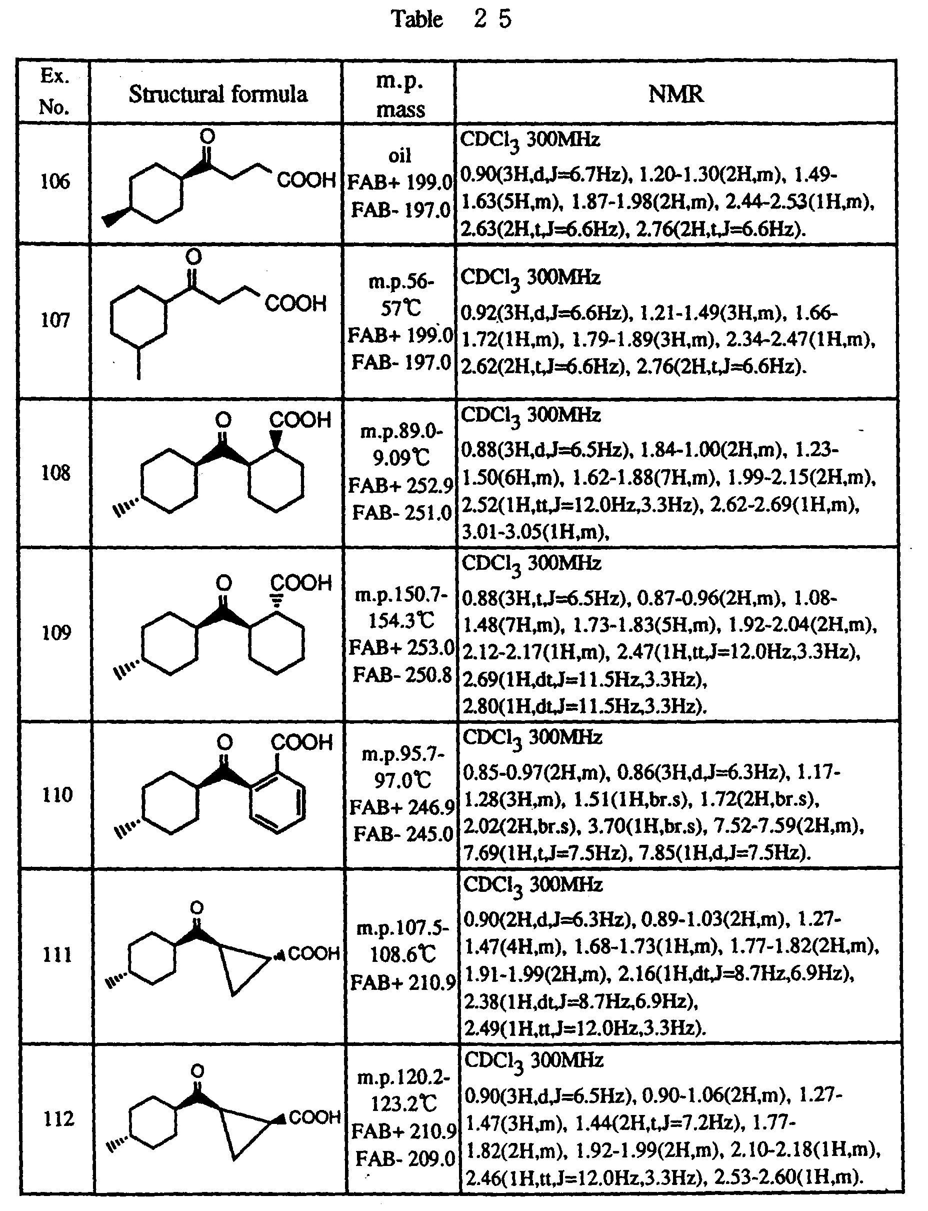

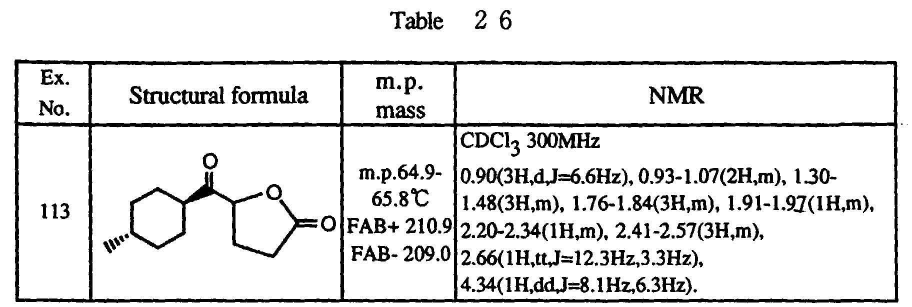

### Formulation Example

The present invention is specifically described in the following by way of formulation example of the preparation.

### Preparation Formulation Example

| | |
|---|---|
| Compound of formula [I] | 3.0 mg |
| Crystalline cellulose | 67.0 mg |
| Corn starch | 25.0 mg |
| Talc | 4.0 mg |
| Magnesium stearate | 1.0 mg |

The above ingredients were thoroughly mixed, granulated, dried and tableted by tableting machine to give tablets weighing 100 mg per tablet.

### Experimental Example

The present invention is specifically described in the following by way of experimental example.

### Experimental Example 1

### Glucose tolerance test using fasted rats

Male Wistar rats (Japan Charles River) weighing about 250 g were fasted for 16 hr from the previous day and subjected to the test. Glucose (1 g/kg) was intraperitoneally administered and blood (0.2 ml) was taken at 30, 60 and 120 minutes later from the tail vein. After obtaining serum, blood glucose was determined by the hexokinase method. The test drug was suspended in 0.5% carboxymethylcellulose or corn oil, and orally administered 30 min before glucose loading. As a control, used was carboxymethylcellulose. The suppression of increase in blood glucose was expressed by inhibition ratio (%) in the group administered with test drug, relative to the increase in blood glucose in the control group at 30 min after glucose loading, which was taken as 100%. The blood glucose after 120 min was also shown by the ratio (%) of changes relative to the control.

The results are shown in the following Tables.

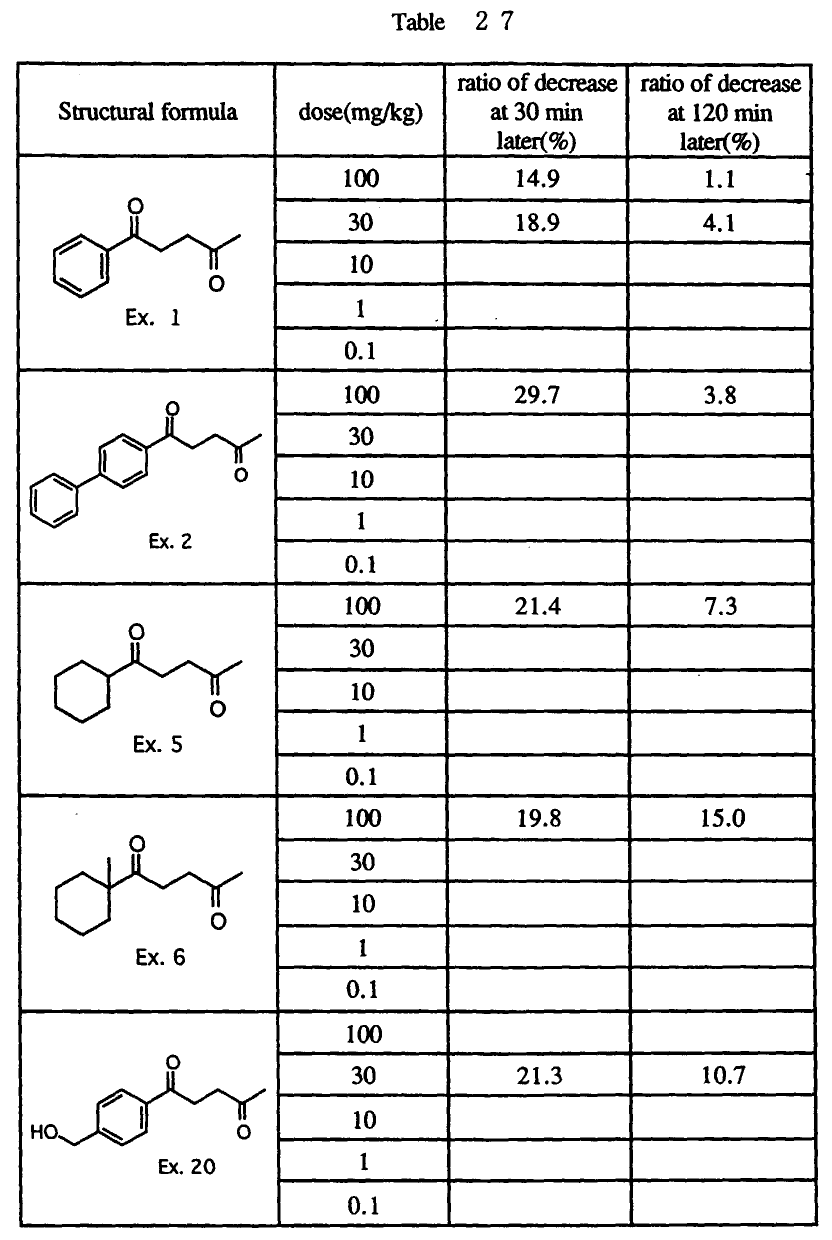

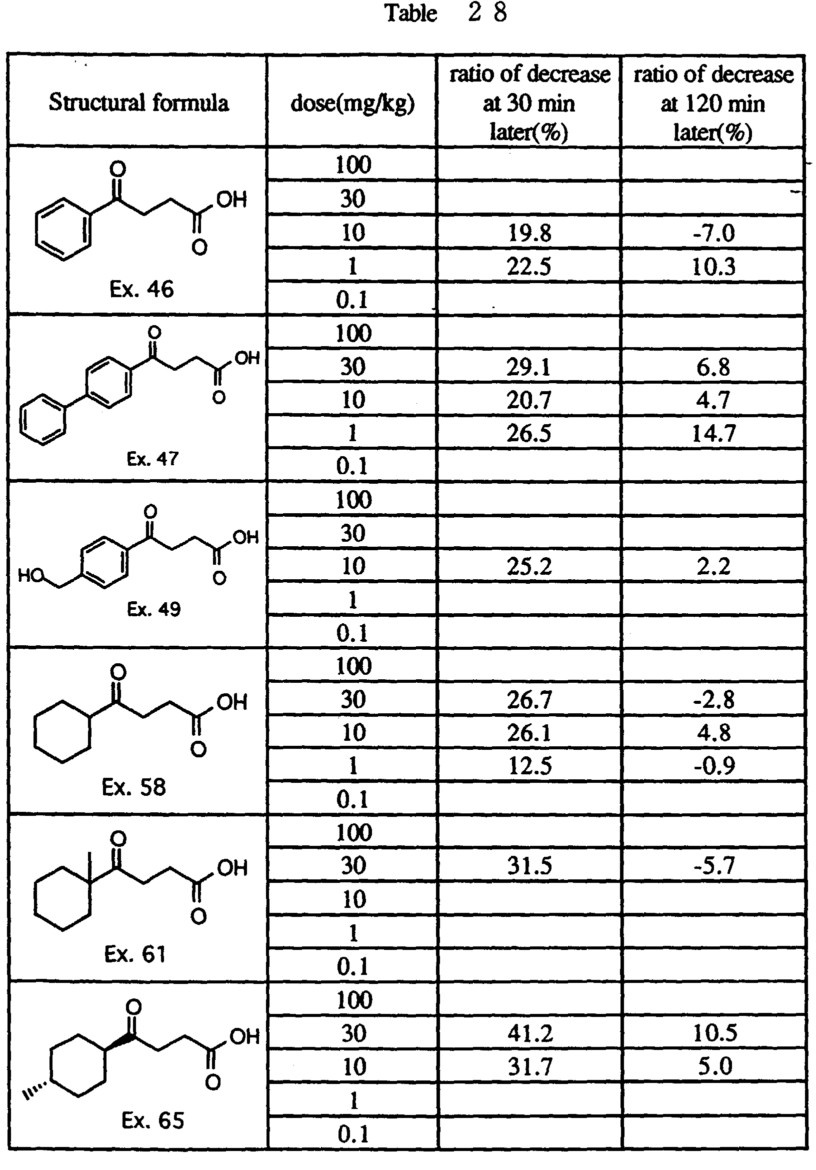

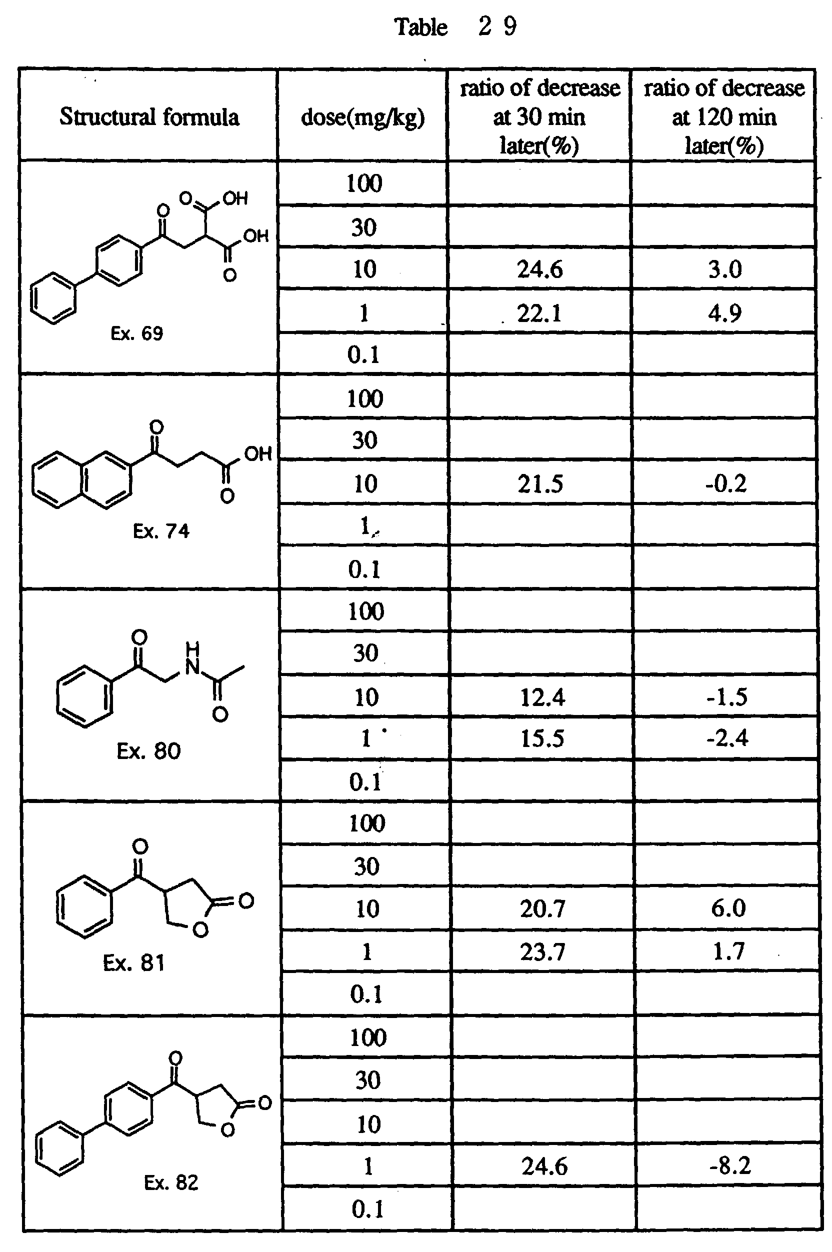

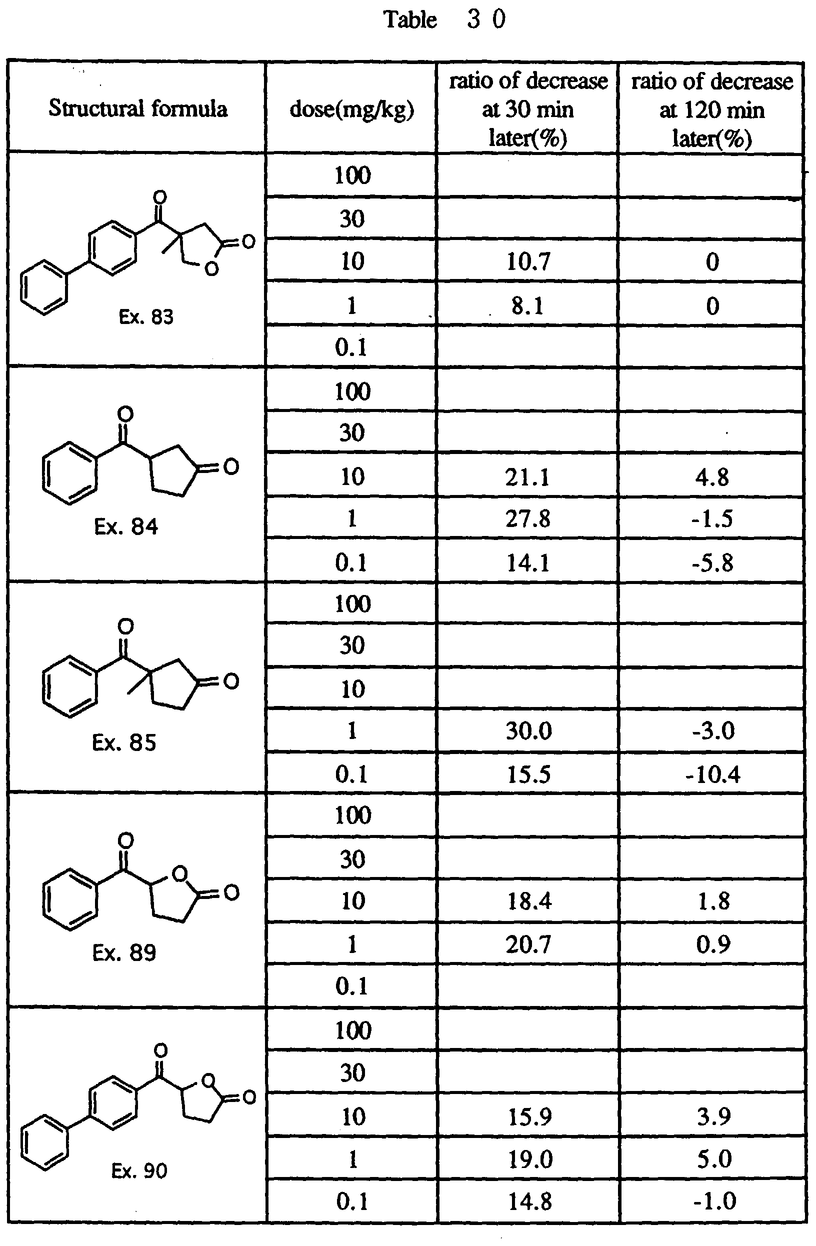

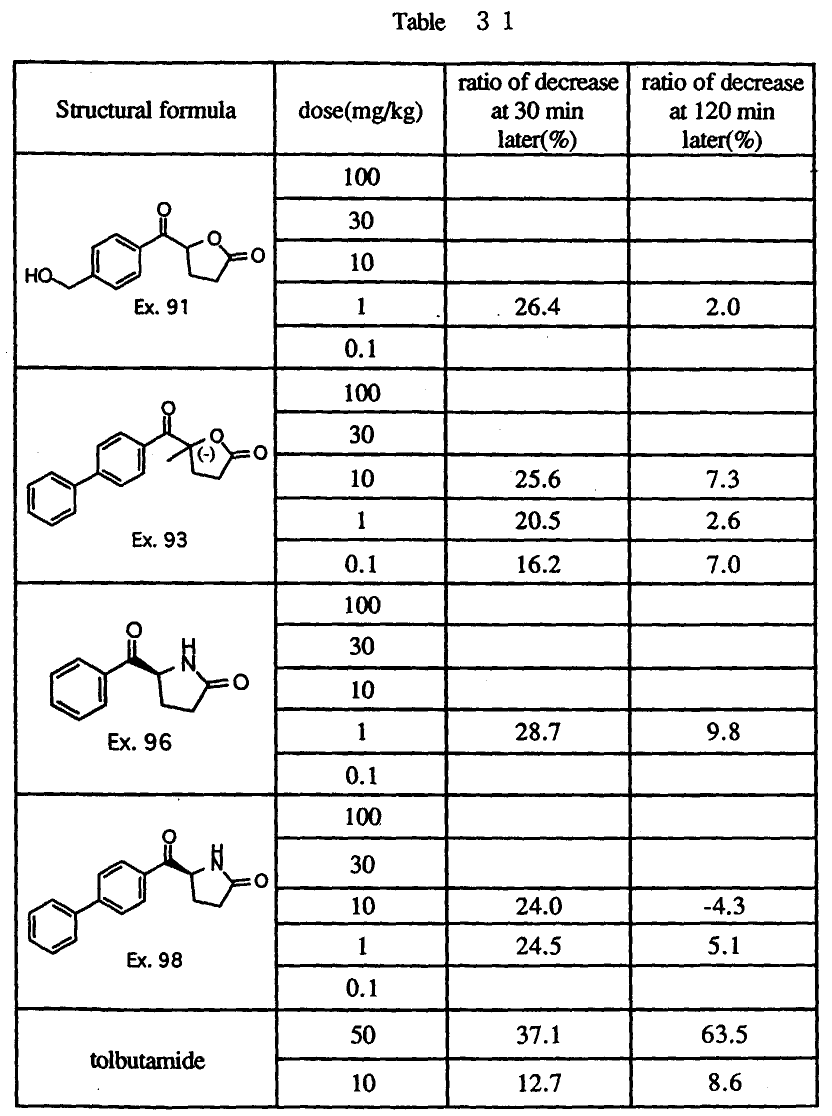

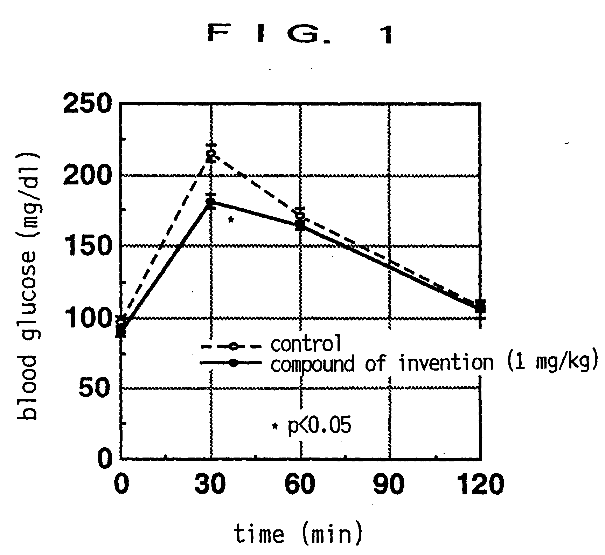

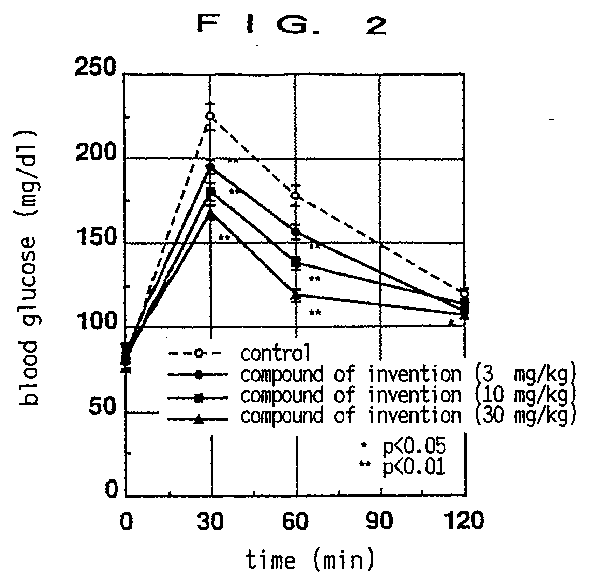

As one example, Fig. 1 shows time-course changes in blood glucose level when the compounds of the present invention (Example 93) and control were orally administered, and Fig. 2 shows time-course changes in blood glucose level when the compounds of the present invention (Example 65) and control were orally administered. In Fig. 2, the dose of the compound of the present invention was varied and the blood glucose was determined. According to Fig. 1, the compound of the present invention significantly decreased the blood glucose in hyperglycemia at 30 min after glucose loading, whereas it did not unnecessarily lower the blood glucose level 120 min later when hyperglycemia was not observed. According to Fig. 2, moreover, the compound of the present invention tends to show significant decrease in the blood glucose level in hyperglycemia at 30 min after glucose loading, irrespective of the dose thereof, whereas it does not unnecessarily lower the blood glucose level 120 min later when hyperglycemia is not observed. The compounds of other Examples showed the same tendency.

### Experimental Example 2

### Glucose tolerance by tolbutamide in fasted rats

Male Wistar rats (Japan Charles River) weighing about 250 g were fasted for 16 hr from the previous day and subjected to the test. Glucose (1 g/kg) was intraperitoneally administered and blood (0.2 ml) was taken at 30, 60 and 120 minutes later from the tail vein. After obtaining serum, blood glucose was determined by the hexokinase method. Tolbutamide was suspended in 0.5% carboxymethylcellulose or corn oil, and orally administered 30 min before glucose loading. As a control, used was carboxymethylcellulose.

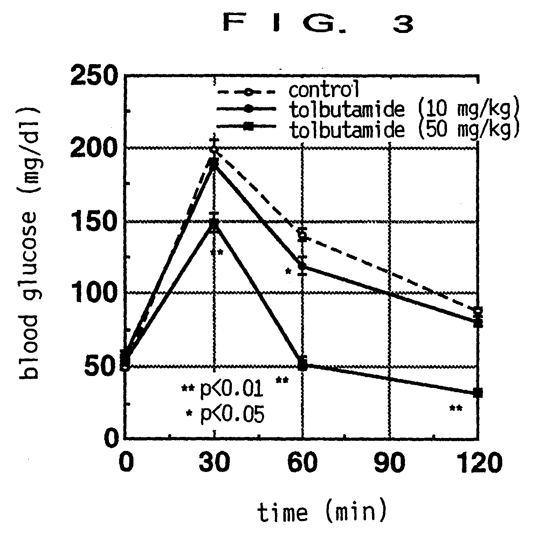

The time-course changes in blood glucose level after oral administration of tolbutamide and control are shown in Fig. 3. According to Fig. 3, tolbutamide did not show effective results as a therapeutic agent for diabetes for treating hyperglycemia in the dose that does not cause hypoglycemia, and when used in the dose effective for treating hyperglycemia, it induced hypoglycemia 120 min later. In other words, when administered in a dose effective as a therapeutic agent for diabetes, tolbutamide always decreases blood glucose whether hyperglycemic condition or normal blood glucose level, to result in hypoglycemic condition 120 min later.

### Experimental Example 3

### Effects on blood glucose level of fasted rats

Male Wistar rats (Japan Charles River) weighing about 250 g were fasted for 16 hr from the previous day and subjected to the test. The test drug was suspended in 0.5% carboxymethylcellulose or corn oil, and blood (0.2 ml) was taken at 30, 60 and 120 minutes later from the tail vein. After obtaining serum, blood glucose was determined by the hexokinase method. As a control, used was carboxymethylcellulose, and blood glucose after administration of tolbutamide and the compounds of the present invention was determined.

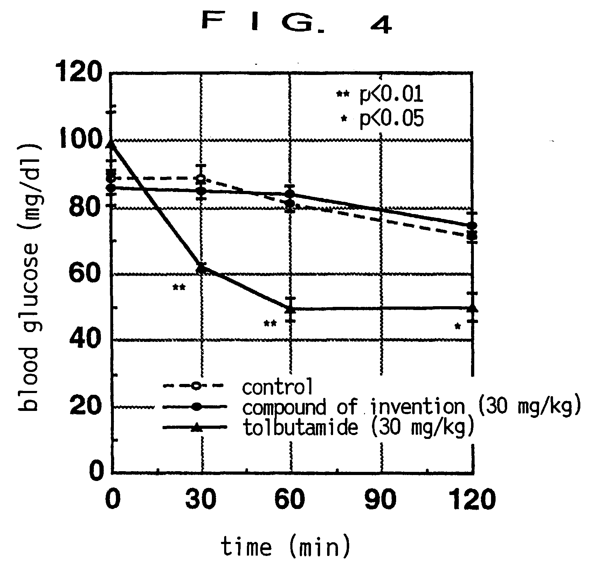

As one example, Fig. 4 shows time-course changes in blood glucose level when the compound of the present invention (Example 93), tolbutamide and control were orally administered. According to Fig. 4, tolbutamide lowered the fasting blood glucose, whereas the compound of the present invention did not lower the fasting blood glucose. The compounds of other Examples showed the same action.

### Industrial Applicability

The compound of the present invention shows superior blood glucose decreasing action in hyperglycemia but does not cause serious side effects such as hypoglycemia. Therefore, the compound of the present invention is useful as a therapeutic agent for diabetes and also useful as a preventive of the chronic complications of diabetes.

This application is based on application No. 56883/1996 filed in Japan, the content of which is incorporated hereinto by reference.

## Claims

1. A therapeutic agent for diabetes, which comprises a compound of the formula [I] 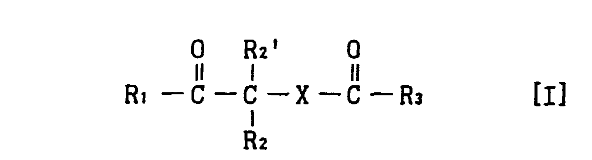 wherein
X is a group of the formula 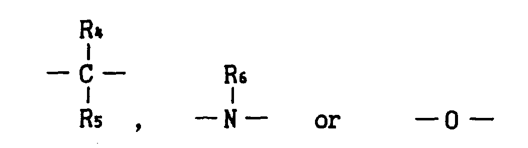 wherein
R₄ and R₅ are the same or different and each is a hydrogen atom, an optionally substituted alkyl having 1 to 5 carbon atoms, an optionally substituted alkenyl having 2 to 6 carbon atoms, an optionally substituted aryl, an optionally substituted acyl having 2 to 5 carbon atoms, a carboxy or an optionally substituted alkoxycarbonyl having 2 to 5 carbon atoms, and R₆ is a hydrogen atom or an amino-protecting group;
R₁ is an optionally substituted alkyl having 1 to 5 carbon atoms, an optionally substituted alkenyl having 2 to 6 carbon atoms, an optionally substituted aryl, an optionally substituted heterocyclic group having at least one nitrogen atom, oxygen atom or sulfur atom, an optionally substituted cycloalkyl having 3 to 7 carbon atoms, a cycloalkenyl having 5 to 7 carbon atoms having at least one double bond in the ring, an optionally substituted adamantyl, an optionally substituted indanyl, an optionally substituted fluorenyl, or a group of the formula 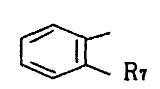
R₂ is a hydrogen atom, an optionally substituted alkyl having 1 to 5 carbon atoms, an optionally substituted alkenyl having 2 to 6 carbon atoms, an optionally substituted aryl, an optionally substituted acyl having 2 to 5 carbon atoms, a carboxy or an optionally substituted alkoxycarbonyl having 2 to 5 carbon atoms;
R₂ 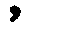 is a hydrogen atom;
R₃ is an optionally substituted alkyl having 1 to 5 carbon atoms, an optionally substituted alkoxy having 1 to 4 carbon atoms, a hydroxy, an optionally substituted aryl, an optionally substituted cycloalkyl having 3 to 7 carbon atoms or an amino;
R₂ and R₇ combinedly form a group of the formula
―CH₂― or ―(CH₂)₂― ;
R₂ and R₅ combinedly form a bond, or a group of the formula
―CH₂― , ―(CH₂)₂―, ―(CH₂)₃― or ―(CH₂)₄― ;
R₂, R₂', R₄ and R₅ combinedly form a group of the formula
=CH―CH=CH―CH=;
R₃ and R₅ combinedly form a group of the formula 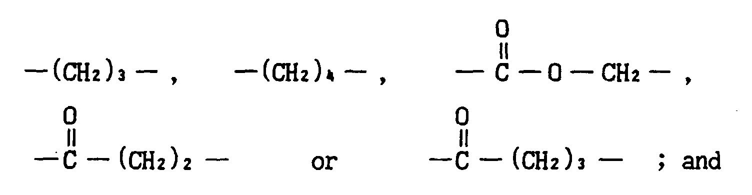
R₂' and R₃ combinedly form a group of the formula 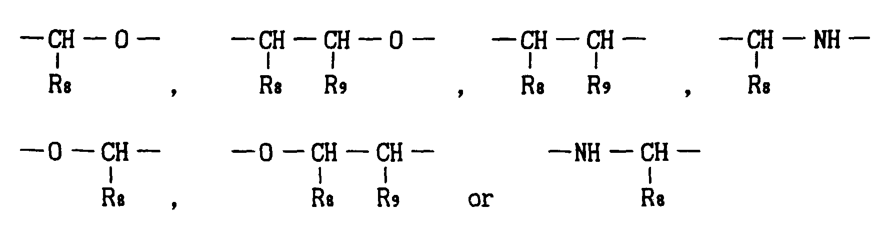 wherein
R₈ and R₉ are the same or different and each is a hydrogen atom, an optionally substituted alkyl having 1 to 5 carbon atoms, an optionally substituted alkoxy having 1 to 4 carbon atoms, an optionally substituted alkoxycarbonyl having 2 to 5 carbon atoms or an optionally substituted acyloxy having 2 to 5 carbon atoms;
a prodrug thereof, a pharmaceutically acceptable salt thereof, a hydrate thereof or a solvate thereof.

2. The therapeutic agent for diabetes of claim 1, wherein, in the formula [I],
X is a group of the formula 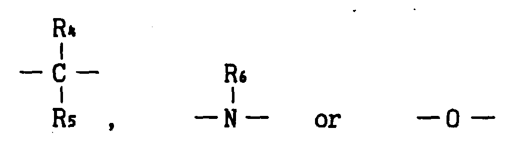 wherein
R₄ is a hydrogen atom, an alkyl having 1 to 5 carbon atoms, an aryl, an acyl having 2 to 5 carbon atoms which is optionally substituted by alkoxy having 1 to 4 carbon atoms, a carboxy or an alkoxycarbonyl having 2 to 5 carbon atoms, R₅ is a hydrogen atom or an alkyl having 1 to 5 carbon atoms, and R₆ is a hydrogen atom or an acyl having 2 to 5 carbon atoms;
R₁ is an alkyl having 1 to 5 carbon atoms which is optionally substituted by cycloalkyl having 3 to 7 carbon atoms or aryl, an alkenyl having 2 to 6 carbon atoms which is optionally substituted by cycloalkyl having 3 to 7 carbon atoms or aryl, an aryl selected from the group consisting of phenyl, biphenyl, naphthyl and terphenyl (these aryl groups being optionally substituted by one or more substituents selected from the group consisting of alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, alkoxycarbonyl having 2 to 5 carbon atoms, hydroxy, cycloalkyl having 3 to 7 carbon atoms, alkoxy having 1 to 4 carbon atoms, amino, alkyl-substituted amino having 1 to 5 carbon atoms, C₁-C₅ alkyl-disubstituted amino, acyl-substituted amino having 2 to 5 carbon atoms, C₂-C₅ acyl-disubstituted amino, heterocyclic group and halogen atom), a heterocyclic group selected from the group consisting of furyl, thienyl, pyridyl, benzothienyl and benzofuryl (these heterocyclic groups being optionally substituted by alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, alkoxycarbonyl having 2 to 5 carbon atoms, hydroxy, cycloalkyl having 3 to 7 carbon atoms, alkoxy having 1 to 4 carbon atoms, amino, alkyl-substituted amino having 1 to 5 carbon atoms, C₁-C₅ alkyl-disubstituted amino, acyl-substituted amino having 2 to 5 carbon atoms, C₂-C₅ acyl-disubstituted amino and halogen atom), a cycloalkyl having 3 to 7 carbon atoms which is optionally substituted by alkyl having 1 to 5 carbon atoms or aryl, a cycloalkenyl having 5 to 7 carbon atoms having at least one double bond in the ring, an adamantyl, an indanyl optionally substituted by alkyl having 1 to 5 carbon atoms or aryl, a fluorenyl, or a group of the formula 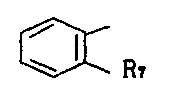
R₂ is a hydrogen atom or an alkyl having 1 to 5 carbon atoms which is optionally substituted by aryl or carboxy;
R₂' is a hydrogen atom;
R₃ is an alkyl having 1 to 5 carbon atoms which is optionally substituted by alkoxy having 1 to 4 carbon atoms or aryl, an alkoxy having 1 to 4 carbon atoms, a hydroxy, an aryl selected from the group consisting of phenyl, biphenyl, naphthyl and terphenyl, a cycloalkyl having 3 to 7 carbon atoms which is optionally substituted by alkyl having 1 to 5 carbon atoms or an amino;
R₂ and R₇ combinedly form a group of the formula
―CH₂― or ―(CH₂)₂― ;
R₂ and R₅ combinedly form a bond or a group of the formula
―CH₂― , ―(CH₂)₂― , ―(CH₂)₃― or ―(CH₂)₄― ;
R₂, R₂', R₄ and R₅ combinedly form a group of the formula
=CH―CH=CH―CH ;
R₃ and R₅ combinedly form a group of the formula 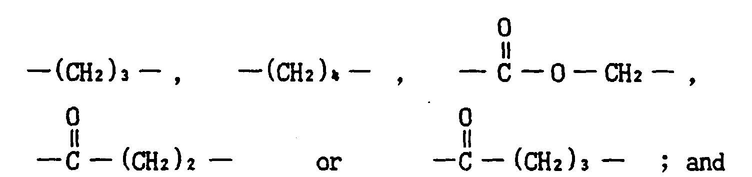
R₂' and R₃ combinedly form a group of 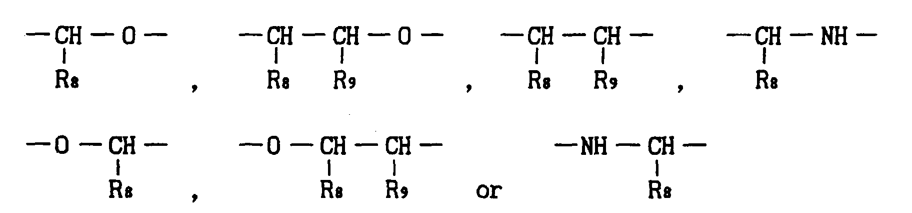 the formula wherein
R₈ and R₉ are the same or different and each is a hydrogen atom, an alkyl having 1 to 5 carbon atoms, an alkoxy having 1 to 4 carbon atoms, an alkoxycarbonyl having 2 to 5 carbon atoms or an acyloxy having 2 to 5 carbon atoms.

3. The therapeutic agent for diabetes of claim 1, wherein, in the formula [I],
X is a group of the formula 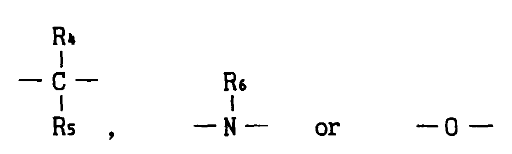 wherein R₄ is a hydrogen atom, an alkyl having 1 to 5 carbon atoms, a phenyl, an acyl having 2 to 5 carbon atoms, a carboxy or an alkoxycarbonyl having 2 to 5 carbon atoms, R₅ is a hydrogen atom or an alkyl having 1 to 5 carbon atoms, and R₆ is a hydrogen atom or an acyl having 2 to 5 carbon atoms;
R₁ is an alkyl having 1 to 5 carbon atoms which is optionally substituted by cycloalkyl having 3 to 7 carbon atoms or aryl, an aryl selected from the group consisting of phenyl, biphenyl, naphthyl and terphenyl (these aryl groups being optionally substituted by one or more substituents selected from the group consisting of alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group and hydroxy), a heterocyclic group selected from the group consisting of furyl, thienyl, benzothienyl and pyridyl (these heterocyclic groups being optionally substituted by alkyl having 1 to 5 carbon atoms, alkyl having 1 to 5 carbon atoms which is substituted by hydroxy, alkenyl having 2 to 6 carbon atoms, acyl having 1 to 5 carbon atoms, carboxy, cycloalkyl having 3 to 7 carbon atoms or hydroxy), a cycloalkyl having 3 to 7 carbon atoms which is optionally substituted by alkyl having 1 to 5 carbon atoms or aryl, an adamantyl, an indanyl, a fluorenyl or a group of the formula 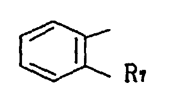
R₂ is a hydrogen atom or an alkyl having 1 to 5 carbon atoms;
R₂' is a hydrogen atom;
R₃ is an alkyl having 1 to 5 carbon atoms which is optionally substituted by alkoxy having 1 to 4 carbon atoms or aryl, alkoxy having 1 to 4 carbon atoms, a hydroxy, an aryl selected from the group consisting of phenyl, biphenyl, naphthyl and terphenyl or a cycloalkyl having 3 to 7 carbon atoms which is optionally substituted by alkyl having 1 to 5 carbon atoms;
R₂ and R₇ combinedly form a group of the formula
―CH₂― or ―(CH₂)₂― ;
R₂ and R₅ combinedly form a bond or a group of the formula
―CH₂― , ―(CH₂)₂― , ―(CH₂)₃― or ―(CH₂)₄― ;
R₂, R₂', R₄ and R₅ combinedly form a group of the formula
=CH―CH=CH―CH= ;
R₃ and R₅ combinedly form a group of the formula 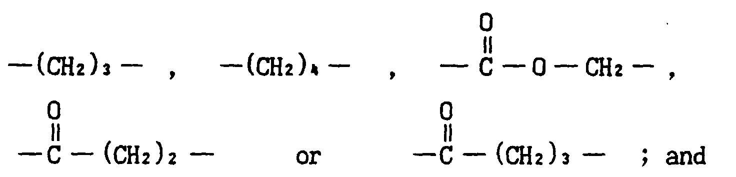
R₂' and R₃ combinedly form a group of the formula 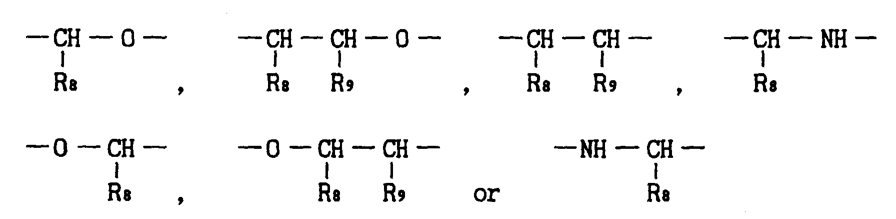 wherein
R₈ and R₉ are the same or different and each is a hydrogen atom, an alkyl having 1 to 5 carbon atoms, an alkoxy having 1 to 4 carbon atoms, an alkoxycarbonyl having 2 to 5 carbon atoms or an acyloxy having 2 to 5 carbon atoms.

4. The therapeutic agent for diabetes of claim 1, wherein, in the formula [I],
X is a group of the formula 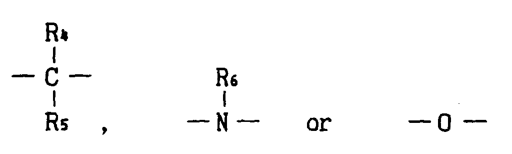 wherein
R₄ is a hydrogen atom, an alkyl having 1 to 5 carbon atoms, an acyl having 2 to 5 carbon atoms, a carboxy or an alkoxycarbonyl having 2 to 5 carbon atoms, R₅ is a hydrogen atom or an alkyl having 1 to 5 carbon atoms, and R₆ is a hydrogen atom;
R₁ is an alkyl having 1 to 5 carbon atoms which is optionally substituted by cycloalkyl having 3 to 7 carbon atoms or aryl, an aryl selected from the group consisting of phenyl, biphenyl, naphthyl and terphenyl (these aryl groups being optionally substituted by one or more substituents selected from the group consisting of alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group and hydroxy), a heterocyclic group selected from furyl, thienyl, benzothienyl and pyridyl, a cycloalkyl having 3 to 7 carbon atoms which is optionally substituted by alkyl having 1 to 5 carbon atoms or aryl, an adamantyl, an indanyl, a fluorenyl, or a group of the formula 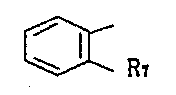
R₂ is a hydrogen atom or an alkyl having 1 to 5 carbon atoms;
R₂' is a hydrogen atom;
R₃ is an alkyl having 1 to 5 carbon atoms which is optionally substituted by alkoxy having 1 to 4 carbon atoms or aryl, an alkoxy having 1 to 4 carbon atoms, a hydroxy or an aryl selected from the group consisting of phenyl, biphenyl, naphthyl and terphenyl;
R₂ and R₇ combinedly form a group of the formula
―(CH₂)₂― ;
R₂ and R₅ combinedly form a bond or a group of the formula
―CH₂― , ―(CH₂)₂― , ―(CH₂)₃― or ―(CH₂)₄― ;
R₂, R₂', R₄ and R₅ combinedly form a group of the formula
=CH―CH=CH―CH= ; and
R₂' and R₃ combinedly form a group of the formula 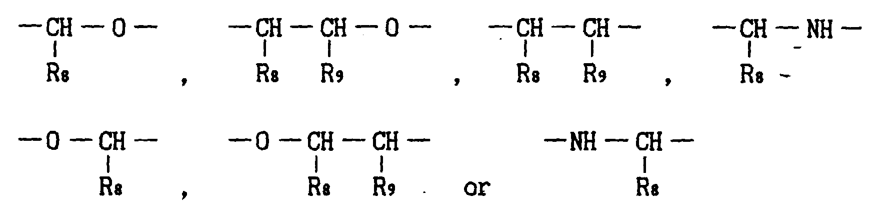 wherein R₈ and R₉ are each a hydrogen atom.

5. The therapeutic agent for diabetes of claim 1, wherein, in the formula [I],
X is a group of the formula 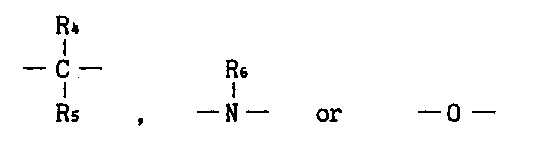 wherein
R₄ is a hydrogen atom, an alkyl having 1 to 5 carbon atoms, an acyl having 2 to 5 carbon atoms, carboxy or an alkoxycarbonyl having 2 to 5 carbon atoms, R₅ is a hydrogen atom or an alkyl having 1 to 5 carbon atoms, and R₆ is a hydrogen atom;
R₁ is an alkyl having 1 to 5 carbon atoms optionally substituted by cycloalkyl having 3 to 7 carbon atoms or aryl, an aryl selected from the group consisting of phenyl, biphenyl, naphthyl and terphenyl (these aryl groups being optionally substituted by alkyl having 1 to 5 carbon atoms, alkyl having 1 to 5 carbon atoms which is substituted by hydroxy, alkyl having 1 to 5 carbon atoms substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group or hydroxy), a heterocyclic group selected from the group consisting of furyl, thienyl, benzothienyl and pyridyl, a cycloalkyl having 3 to 7 carbon atoms which is optionally substituted by alkyl having 1 to 5 carbon atoms or aryl, an adamantyl, an indanyl or a fluorenyl;
R₂ is a hydrogen atom, or an alkyl having 1 to 5 carbon atoms;
R₂' is a hydrogen atom;
R₃ is an alkyl having 1 to 5 carbon atoms, an alkoxy having 1 to 4 carbon atoms, a hydroxy or an aryl selected from the group consisting of phenyl, biphenyl, naphthyl and terphenyl;
R₂ and R₅ together form a bond or a group of the formula
―(CH₂)₃― or ―(CH₂)₄― ; and
R₂' and R₃ combinedly form a group of t 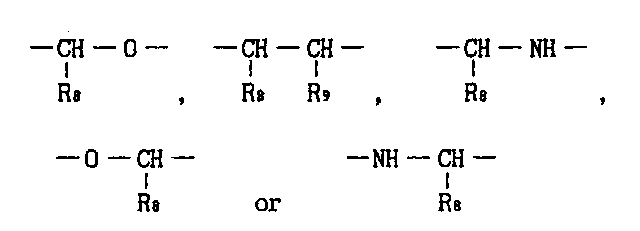 he formula wherein R₈ and R₉ are each hydrogen atom.

6. The therapeutic agent for diabetes of claim 1, wherein, in the formula [I],
X is a group of the formula 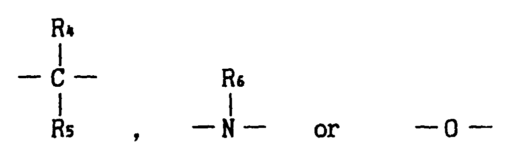 wherein
R₄ is a hydrogen atom, an alkyl having 1 to 5 carbon atoms, an acyl having 2 to 5 carbon atoms, a carboxy or an alkoxycarbonyl having 2 to 5 carbon atoms, R₅ is a hydrogen atom or an alkyl having 1 to 5 carbon atoms and R₆ is a hydrogen atom;
R₁ is an alkyl having 1 to 5 carbon atoms optionally substituted by cycloalkyl having 3 to 7 carbon atoms or aryl, an aryl selected from phenyl, biphenyl, naphthyl and terphenyl (these aryl groups being optionally substituted by alkyl having 1 to 5 carbon atoms, alkyl having 1 to 5 carbon atoms substituted by hydroxy, alkyl having 1 to 5 carbon atoms substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group or hydroxy), a cycloalkyl having 3 to 7 carbon atoms which is optionally substituted by alkyl having 1 to 5 carbon atoms or aryl, an adamantyl, an indanyl or a fluorenyl;
R₂ is a hydrogen atom;
R₂ 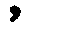 is a hydrogen atom;
R₃ is an alkyl having 1 to 5 carbon atoms, alkoxy having 1 to 4 carbon atoms or hydroxy;
R₂ and R₅ combinedly show a bond; and
R₂ 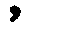 and R₃ combinedly form a group of the formula 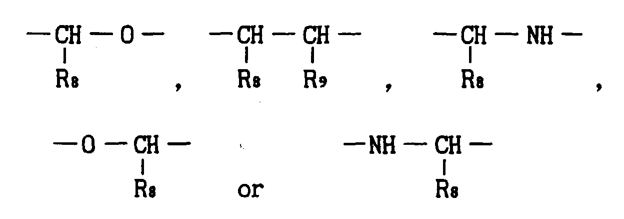 wherein R₈ and R₉ are each hydrogen atom.

7. The therapeutic agent for diabetes of claim 1, wherein, in the formula [I],
X is a group of the formula 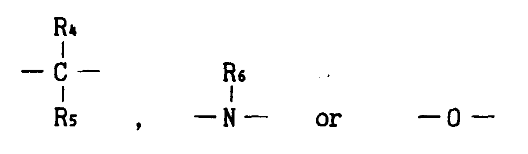 wherein
R₄ is a hydrogen atom, an alkyl having 1 to 5 carbon atoms, an acyl having 2 to 5 carbon atoms, a carboxy or an alkoxycarbonyl having 2 to 5 carbon atoms, R₅ is a hydrogen atom or an alkyl having 1 to 5 carbon atoms, and R₆ is a hydrogen atom;
R₁ is an alkyl having 1 to 5 carbon atoms optionally substituted by cycloalkyl having 3 to 7 carbon atoms or aryl, an aryl selected from phenyl, biphenyl, naphthyl and terphenyl (these aryl groups being optionally substituted by alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group or hydroxy), or cycloalkyl having 3 to 7 carbon atoms which is optionally substituted by alkyl having 1 to 5 carbon atoms or aryl;
R₂ is a hydrogen atom;
R₂ 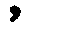 is a hydrogen atom;
R₃ is an alkyl having 1 to 5 carbon atoms, an alkoxy having 1 to 4 carbon atoms or a hydroxy; and
R₂ 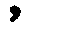 and R₃ combinedly form a group of the formula 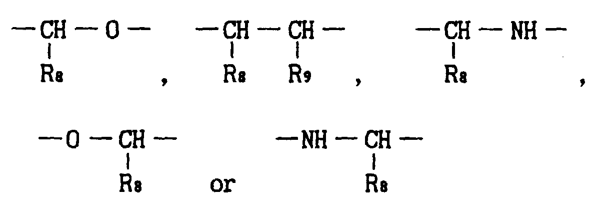 wherein R₈ and R₉ are each a hydrogen atom.

8. The therapeutic agent for diabetes of claim 1, wherein, in the formula [I],
X is a group of the formula 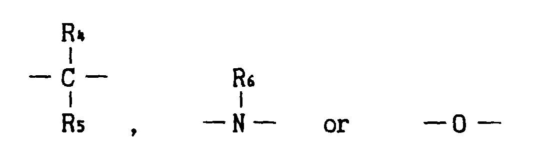 wherein
R₄ is a hydrogen atom, an alkyl having 1 to 5 carbon atoms, an acyl having 2 to 5 carbon atoms, a carboxy or an alkoxycarbonyl having 2 to 5 carbon atoms, R₅ is a hydrogen atom or an alkyl having 1 to 5 carbon atoms, and R₆ is a hydrogen atom;
R₁ is an aryl selected from the group consisting of phenyl, biphenyl, naphthyl and terphenyl (these aryl groups being optionally substituted by alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group or hydroxy), or a cycloalkyl having 3 to 7 carbon atoms which is optionally substituted by alkyl having 1 to 5 carbon atoms or aryl;
R₂ is a hydrogen atom;
R₂ 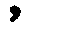 is a hydrogen atom; and
R₃ is an alkyl having 1 to 5 carbon atoms, an alkoxy having 1 to 4 carbon atoms or a hydroxy.

9. The therapeutic agent for diabetes of claim 1, wherein, in the formula [I],
X is a group of the formula 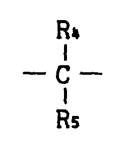 wherein
R₄ is a hydrogen atom, an alkyl having 1 to 5 carbon atoms, an acyl having 2 to 5 carbon atoms, a carboxy or an alkoxycarbonyl having 2 to 5 carbon atoms, and R₅ is a hydrogen atom or an alkyl having 1 to 5 carbon atoms;
R₁ is an aryl selected from phenyl, biphenyl, naphthyl and terphenyl (these aryl groups being optionally substituted by alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group or hydroxy), or a cycloalkyl having 3 to 7 carbon atoms which is optionally substituted by alkyl having 1 to 5 carbon atoms or aryl;
R₂ is a hydrogen atom;
R₂ 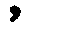 is a hydrogen atom; and
R₃ is an alkyl having 1 to 5 carbon atoms, an alkoxy having 1 to 4 carbon atoms or a hydroxy.

10. The therapeutic agent for diabetes of claim 1, wherein, in the formula [I],
X is a group of the formula 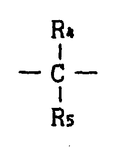 wherein
R₄ and R₅ are each a hydrogen atom;
R₁ is an aryl selected from the group consisting of phenyl, biphenyl, naphthyl and terphenyl (these aryl groups being optionally substituted by alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group or hydroxy), or cycloalkyl having 3 to 7 carbon atoms which is optionally substituted by alkyl having 1 to 5 carbon atoms or aryl;
R₂ is a hydrogen atom;
R₂ 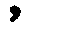 is a hydrogen atom; and
R₃ is an alkyl having 1 to 5 carbon atoms, an alkoxy having 1 to 4 carbon atoms or a hydroxy.

11. The therapeutic agent for diabetes of claim 1, wherein, in the formula [I],
X is a group of the formula 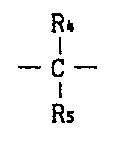 wherein
R₄ and R₅ are each a hydrogen atom;
R₁ is an aryl selected from the group consisting of phenyl, biphenyl, naphthyl and terphenyl (these aryl groups being optionally substituted by alkyl having 1 to 5 carbon atoms, alkyl having 1 to 5 carbon atoms which is substituted by hydroxy, alkyl having 1 to 5 carbon atoms which is substituted by alkoxy having 1 to 4 carbon atoms, alkenyl having 2 to 6 carbon atoms, acyl having 1 to 5 carbon atoms, carboxy, cycloalkyl having 3 to 7 carbon atoms, heterocyclic group or hydroxy), or a cycloalkyl having 3 to 7 carbon atoms which is optionally substituted by alkyl having 1 to 5 carbon atoms or aryl;
R₂ is a hydrogen atom;
R₂ 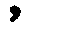 is a hydrogen atom; and
R₃ is a hydroxy.

12. A compound of the formula 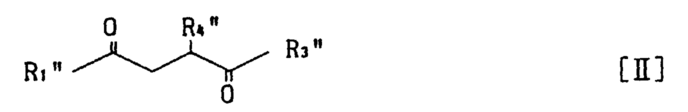 wherein,
when R₃'' is a hydroxy,
R₄'' is a hydrogen atom, an acyl having 2 to 5 carbon atoms, a carboxy or an alkoxycarbonyl having 2 to 5 carbon atoms, and
R₁'' is a cycloalkyl having 3 to 7 carbon atoms which is mono-substituted by a substituent selected from the group consisting of alkyl having 1 to 4 carbon atoms, hydroxy, alkoxy having 1 to 4 carbon atoms, aryl, acyl having 2 to 5 carbon atoms, amino, carboxy and alkoxycarbonyl having 2 to 5 carbon atoms; and
when R₃'' is an alkyl having 1 to 4 carbon atoms or an alkoxy having 1 to 4 carbon atoms,
R₄'' is an acyl having 2 to 5 carbon atoms, a carboxy or an alkoxycarbonyl having 2 to 5 carbon atoms, and
R₁'' is a cycloalkyl having 3 to 7 carbon atoms which is mono-substituted by a substituent selected from the group consisting of alkyl having 1 to 4 carbon atoms, hydroxy, alkoxy having 1 to 4 carbon atoms, aryl, acyl having 2 to 5 carbon atoms, amino, carboxy and alkoxycarbonyl having 2 to 5 carbon atoms;
a prodrug thereof, a pharmaceutically acceptable salt thereof, a hydrate thereof or a solvate thereof.

13. The compound of claim 12, wherein R₃'' is a hydroxy; a prodrug thereof, a pharmaceutically acceptable salt thereof, a hydrate thereof or a solvate thereof.

14. The compound of claim 12 or 13, wherein R₄'' is a hydrogen atom; a prodrug thereof, a pharmaceutically acceptable salt thereof, a hydrate thereof or a solvate thereof.

15. The compound of any one of claims 12 to 14, wherein R₁'' is a cycloalkyl having 3 to 7 carbon atoms which is mono-substituted by alkyl having 1 to 4 carbon atoms; a prodrug thereof, a pharmaceutically acceptable salt thereof, a hydrate thereof or a solvate thereof.

16. The compound of any one of claims 12 to 15, wherein R₁'' is a cycloalkyl having 3 to 7 carbon atoms which is mono-substituted by methyl; a prodrug thereof, a pharmaceutically acceptable salt thereof, a hydrate thereof or a solvate thereof.

17. The compound of any one of claims 12 to 16, which is selected from the group consisting of
1) 4-(1-methylcyclohexyl)-4-oxobutanoic acid,
2) trans-4-(4-methylcyclohexyl)-4-oxobutanoic acid,
3) trans-4-(4-ethylcyclohexyl)-4-oxobutanoic acid,
4) trans-4-(4-isopropylcyclohexyl)-4-oxobutanoic acid,
5) trans-4-(4-tert-butylcyclohexyl)-4-oxobutanoic acid,
6) trans-4-(4-phenylcyclohexyl)-4-oxobutanoic acid,
7) cis-4-(4-methylcyclohexyl)-4-oxobutanoic acid,
8) 4-(3-methylcyclohexyl)-4-oxobutanoic acid,
9) dimethyl 2-[2-(1-methylcyclohexyl)-2-oxoethyl]propenedioate and
10) methyl 2-acetyl-4-(1-methylcyclohexyl)-4-oxobutanoate;
a prodrug thereof, a pharmaceutically acceptable salt thereof, a hydrate thereof or a solvate thereof.

18. The compound of any one of claims 12 to 17, which is selected from the group consisting of
1) 4-(1-methylcyclohexyl)-4-oxobutanoic acid,
2) trans-4-(4-methylcyclohexyl)-4-oxobutanoic acid,
3) trans-4-(4-ethylcyclohexyl)-4-oxobutanoic acid,
4) trans-4-(4-isopropylcyclohexyl)-4-oxobutanoic acid,
5) trans-4-(4-tert-butylcyclohexyl)-4-oxobutanoic acid,
6) trans-4-(4-phenylcyclohexyl)-4-oxobutanoic acid,
7) cis-4-(4-methylcyclohexyl)-4-oxobutanoic acid and
8) 4-(3-methylcyclohexyl)-4-oxobutanoic acid;
a prodrug thereof, a pharmaceutically acceptable salt thereof, a hydrate thereof or a solvate thereof.

19. A compound of the formula 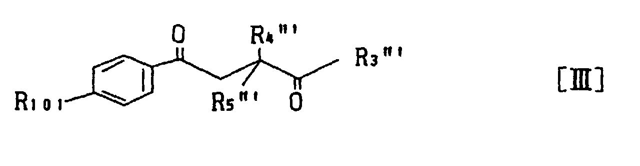 wherein,
when R₃''' is a hydroxy,
R₄''' is a hydrogen atom, an alkyl having 1 to 4 carbon atoms, an acyl having 2 to 5 carbon atoms, a carboxy or an alkoxycarbonyl having 2 to 5 carbon atoms,
R₅''' is a hydrogen atom, an alkyl having 1 to 4 carbon atoms, an acyl having 2 to 5 carbon atoms, a carboxy or an alkoxycarbonyl having 2 to 5 carbon atoms, and
R₁₀₁ is an alkyl having 1 to 4 carbon atoms which is substituted by hydroxy;
when R₃''' is an alkyl having 1 to 4 carbon atoms,
R₄''' is a hydrogen atom, an alkyl having 1 to 4 carbon atoms, an acyl having 2 to 5 carbon atoms, a carbon or an alkoxycarbonyl having 2 to 5 carbon atoms,
R₅''' is a hydrogen atom, an alkyl having 1 to 4 carbon atoms, an acyl having 2 to 5 carbon atoms, a carboxy or an alkoxycarbonyl having 2 to 5 carbon atoms, and
R₁₀₁ is an alkyl having 1 to 4 carbon atoms which is substituted by hydroxy or an aryl substituted by a substituent selected from the group consisting of carboxy, alkoxycarbonyl having 2 to 5 carbon atoms, hydroxy, alkyl having 1 to 4 carbon atoms, alkoxy having 1 to 4 carbon atoms, aryl, acyl having 2 to 5 carbon atoms and amino; and
when R₃''' is an alkoxy having 1 to 4 carbon atoms,
R₄''' is a hydrogen atom, an alkyl having 1 to 4 carbon atoms, an acyl having 2 to 5 carbon atoms, a carboxy or an alkoxycarbonyl having 2 to 5 carbon atoms,
R₅''' is a hydrogen atom, an alkyl having 1 to 4 carbon atoms, an acyl having 2 to 5 carbon atoms, a carboxy or an alkoxycarbonyl having 2 to 5 carbon atoms, and
R₁₀₁ is an alkyl having 1 to 4 carbon atoms which is substituted by hydroxy, or an aryl substituted by a substituent selected from the group consisting of carboxy, alkoxycarbonyl having 2 to 5 carbon atoms, hydroxy, aryl, acyl having 2 to 5 carbon atoms and amino;
a prodrug thereof, a pharmaceutically acceptable salt thereof, a hydrate thereof or a solvate thereof.

20. The compound of claim 19, wherein R₃''' is a hydroxy; a prodrug thereof, a pharmaceutically acceptable salt thereof, a hydrate thereof or a solvate thereof.

21. The compound of claim 19 or 20, wherein R₄''' or R₅''' is a hydrogen atom; a prodrug thereof, a pharmaceutically acceptable salt thereof, a hydrate thereof or a solvate thereof.

22. The compound of any one of claims 19 to 21, wherein R₄''' and R₅''' are each a hydrogen atom; a prodrug thereof, a pharmaceutically acceptable salt thereof, a hydrate thereof or a solvate thereof.

23. The compound of any one of claims 19 to 22, wherein R₁₀₁ is an alkyl having 1 to 4 carbon atoms which is substituted by hydroxy; a prodrug thereof, a pharmaceutically acceptable salt thereof, a hydrate thereof or a solvate thereof.

24. The compound of any one of claims 19 to 23, which is selected from the group consisting of
1) 4-[4-(hydroxymethyl)phenyl]-4-oxobutanoic acid,
2) 1-(4-hydroxymethylphenyl)-1,4-pentanedione,
3) 1-[4-(1-hydroxyethyl)phenyl]-1,4-pentanedione and
4) 1-[4-(2-hydroxyethyl)phenyl]-1,4-pentanedione;
a prodrug thereof, a pharmaceutically acceptable salt thereof, a hydrate thereof or a solvate thereof.

25. A compound of the formula 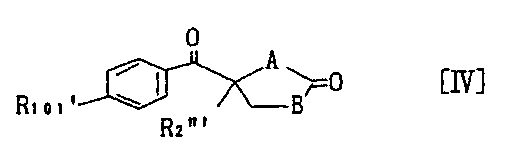 wherein A and B are the same or different and each is C, NH or O; and
when R₂''' is a hydrogen atom,
R₁₀₁' is an alkyl having 1 to 4 carbon atoms which is substituted by hydroxy or an optionally substituted aryl; and
when R₂''' is an alkyl having 1 to 4 carbon atoms,
R₁₀₁' is a hydrogen atom, an alkyl having 1 to 4 carbon atoms which is substituted by hydroxy or an optionally substituted aryl;
a prodrug thereof, a pharmaceutically acceptable salt thereof, a hydrate thereof or a solvate thereof.

26. The compound of claim 25, wherein R₂''' is an alkyl having 1 to 4 carbon atoms; a prodrug thereof, a pharmaceutically acceptable salt thereof, a hydrate thereof or a solvate thereof.

27. The compound of claim 25 or 26, wherein R₂''' is a methyl; a prodrug thereof, a pharmaceutically acceptable salt thereof, a hydrate thereof or a solvate thereof.

28. The compound of any one of claims 25 to 27, which is selected from the group consisting of
1) (±)-3-benzoyl-3-methyl-1-cyclopentanone
2) (±)-dihydro-4-(4'-phenylbenzoyl)-2(3H)-furanone
3) (±)-dihydro-4-methyl-4-(4'-phenylbenzoyl)-2(3H)-furanone
4) (±)-dihydro-4-(4'-hydroxymethylbenzoyl)-2(3H)-furanone
5) (±)-dihydro-5-(4'-phenylbenzoyl)-2(3H)-furanone
6) (±)-dihydro-5-(4'-hydroxymethylbenzoyl)-2(3H)-furanone
7) (±)-dihydro-5-(4'-hydroxymethylbenzoyl)-5-methyl-2(3H)-furanone
8) (-)-dihydro-5-methyl-5-(4'-phenylbenzoyl)-2(3H)-furanone
9) (+)-dihydro-5-methyl-5-(4'-phenylbenzoyl)-2(3H)-furanone
10) (S)-(-)-5-(4'-phenylbenzoyl)-2-pyrrolidinone and
11) (±)-4-(4'-hydroxymethylbenzoyl)-2-pyrrolidinone;
a prodrug thereof, a pharmaceutically acceptable salt thereof, a hydrate thereof or a solvate thereof.

29. A pharmaceutical agent comprising the compound of any one of claims 12-16, 19-23 and 25-27, a prodrug thereof, a pharmaceutically acceptable salt thereof, a hydrate thereof or a solvate thereof.

30. A pharmaceutical composition comprising the compound of any one of claims 12-16, 19-23 and 25-27, a prodrug thereof, a pharmaceutically acceptable salt thereof, a hydrate thereof or a solvate thereof.

31. A pharmaceutical agent comprising the compound of any one of claims 17, 18, 24 and 28, a prodrug thereof, a pharmaceutically acceptable salt thereof, a hydrate thereof or a solvate thereof.

32. A pharmaceutical composition comprising the compound of any one of claims 17, 18, 24 and 28, a prodrug thereof, a pharmaceutically acceptable salt thereof, a hydrate thereof or a solvate thereof.

33. An agent for treating diabetes, comprising the compound of any one of claims 17, 18, 24 and 28, a prodrug thereof, a pharmaceutically acceptable salt thereof, a hydrate thereof or a solvate thereof.
